# EUROPEAN PATENT SPECIFICATION

(11) **EP 1 576 154 B1**
(45) Date of publication and mention of the grant of the patent: **23.02.2011**
(21) Application number: 03779576.2
(22) Date of filing: 18.12.2003
(51) Int. Cl.: C12N 15/10, C12N 15/63

(54) **IN VIVO AFFINITY MATURATION SCHEME**
IN-VIVO AFFINITÄTSREIFUNGSSCHEMA
SCHEMA DE MATURATION PAR AFFINITE IN VIVO

(30) Priority: 18.12.2002 AU 2002953381
(43) Date of publication of application: 21.09.2005
(73) Proprietor: Quintain Consulting Pty Ltd, Oakleigh, VIC (AU)
(72) Inventor: IRVING, Robert, Alexander, Wheelers Hill, Victoria 3150 (AU); HUDSON, Peter, John, Blackburn, Victoria 3130 (AU); MUSTAFA, Huseyin, Glenroy, Victoria 3046 (AU); WARK, Kim, Elwood, Victoria 3184 (AU); ABREGU, Matias, Ezequiel, St. Albans, Victoria 3021 (AU)
(74) Representative: Brasnett, Adrian Hugh
(86) International application number: PCT/AU2003/001697
(87) International publication number: WO 2004/055182

(56) References cited:
- WO-A-00/22111
- US-A- 5 885 827
- US-A- 5 885 827
- YELAMOS J ET AL: "Targeting of non-lg sequences in place of the V segment by somatic hypermutation" NATURE, NATURE PUBLISHING GROUP, LONDON, GB, vol. 376, no. 6537, 1995, pages 225-229, XP002211125 ISSN: 0028-0836
- MARTIN ET AL: "Activation-induced cytidine deaminase turns on somatic hypermutation in hybridomas" NATURE, NATURE PUBLISHING GROUP, LONDON, GB, vol. 415, 14 February 2002 (2002-02-14), pages 802-806, XP002253537 ISSN: 0028-0836
- YELAMOS J. ET AL.: 'Targeting of non-Ig sequences in place of the V segment by somatic hypermutation' NATURE vol. 376, 1995, pages 225 - 229, XP002211125
- MARTIN A, SCHARFF M. D.: 'Somatic hypermutation of the AID transgene in B and non-B cells' PROCEEDINGS OF THE NATIONAL ACADEMY OF SCIENCES USA vol. 99, no. 19, 2002, pages 12304 - 12308, XP002964738
- IRVING R. A. ET AL.: 'Affinity maturation of recombinant antibodies using E.coli mutator cells' IMMUNOTECHNOLOGY vol. 2, 1996, pages 127 - 143, XP000612236

### SEQUENCE LISTING

<110> Diatech Pty Ltd
<120> In vivo affinity maturation scheme
<130> 501974
<150> AU 2002953381
<151> 2002-12-18
<160> 120
<170> PatentIn version 3.1
<210> 1
   <211> 8699
   <212> DNA
   <213> Homo sapiens
<220>
   <221> misc_feature
   <222> (2697)..(2697)
   <223> n = unknown
<220>
   <221> misc_feature
   <222> (4014)..(4014)
   <223> n = unknown
<400> 1
   gcttcattgt tccacgcatt acgtctcacc agtttagtca atatggatta aatatgagag     60
   tgtggcaatt cgcaaactct atctgaggag gaaaatcgga taaaaaatgt tatgaaaaat    120
   aaagcaattt gaagcctctg acttcagcaa cttcaccact aatgaaatga tgtaaccctc    180
   attggcctca aatttagttt tcacggggca tctgcagggt tccaaagtga gaccaggtga    240
   attcaatgtg catgcacttc ccaagtgtcc acttgtattc tgtttcttta cttctgttta    300
   cagaaagtag acacatattc agtcttagta ccagtgtagg gagcgctttc catgagatgg    360
   ataccagaaa aaaatggcaa acatgggatc cgttaatata aaaattagcc acgatgtata    420
   tatatatatg tgtgtgtgtg tgtgtgtgtg tgtgtacaca cgcgcgcgca tgtgtgagtt    480
   gaatagcaga gttggagtgg gtttctatcc acatgtacct gcacctgcag gtattctcag    540
   gtgccataat caactgtagg accctaaagg aaataagagt ctcccctcaa cccctgaaga    600
   gtgtttgggt tcaccgtgtg tccaatgatt ctgtgcctct tgagctccag gaaagggctc    660
   cctggtgatg catgagatct tttcttggag tctctctgca gagttcactg ggtttcctaa    720
   aggcaattca ctatttcaaa agatggtgtg aggagcatgt ggtgtcccta aaggagaatt    780
   ctgagccagg gcacaaccac tttatactga gctggataca ctggtaggaa tatactctgt    840
   cagctcagat agaaacctcc ctgcatggtt ggggctgggc tgcagggggc gctccggata 900
   cacccagcac aggctcccgc cccagagcag gtgcacagga ggctggggag aggttcctcc 960
   cagggcctgg gacttccttt aaaaatatct aaaataagta tttcacaaag actgctgaag 1020
   tttgtataaa tatctattca attgtgagca tttatcaaac tggatgttgt aatgagaacc 1080
   acttttacaa tggggatttc aaactctgct ggaggtcagg aagagatcct ttcttataaa 1140
   taaatgcaat ttttggataa acacagtcat tccctaaata acgcattcac atattatggt 1200
   ctagaaatga tgcaagttga ccctgagaca gtcaaatgtg gtttcaaagt gaggtgctgt 1260
   ccttgaggag cttgttctcc agtgggggaa gctctgtcaa cacagagttc agggatgtgt 1320
   aggggacaca tggcctctaa caggattacg gcttgaaccc tcagcttcta cagttgtgtc 1380
   acccatgtgt ctgtttctca tactgggtca ggaattgggc tattaaatag catccttcat 1440
   gaatatgcaa ttaactgagg tgactatagt atctctgtac cctgaaagca tcacccaaca 1500
   acaacatccc tccttgggag aatcccctag agcacagctc ctcaccatgg actggacctg 1560
   gagcatcctc ttcttggtgg cagcagcaac aggtaagggg ctccccagtc tcggggttga 1620
   ggcagaaacc aggccactca agtgaggctt tacccacccc tgtgtcctct ccacaggtac 1680
   ctactcccag gtgcagctgg tgcagtctgg ccatgaggtg aagcagcctg gggcctcagt 1740
   gaaggtctcc tgcaagtctt ctggttacac cttcaccatc tatggtatga attgggtatg 1800
   atagacccct ggacagggct ttgagtggat gtgatggatc atcacctaca ctgggaaccc 1860
   aacgtatacc cacggcttca caggatggtt tgtcttctcc atggacacgt ctgtcagcac 1920
   ggcgtgtctt cagatcagca gcctaaaggc tgaggacacg gccgagtatt actgtgcgaa 1980
   gtacacagtg tggaaaccca catcccgaga gtttcagaaa gcctgaggaa ggaggcagct 2040
   gtgctgagct gaggcagtgg tacagcagtt ttctgaactt ccatagtatc tcattttgca 2100
   ttgagttccg ctttaatatt agccaagaat atgggataga cgggtgctcc taagagatcc 2160
   ttaacttgcc cattttgatg ggttttccca aagacgtgag aagccacttt tttcgcaaag 2220
   catcccaaag ccatgccctg ctccagaaac acgtgtatcc atttcctggt ctttgattaa 2280
   ctgacaaact ctcatcagcg cacctgggct aatttcacat caggtagaaa tatgtgcttt 2340
   aaagcaaggc taacgttgta atagcaattc ctgcttaata accttcagca ttgttgttgt 2400
   gtgctccatc aactaattac gttagttcaa ggttctcaat gggagtttct aataaatata 2460
   agggatgtat agaagttccc ctaattaaaa caattgtgaa gacaacctca gtgttcaacc 2520
   atatttcaac ccttcaccac aaaggaactt tcatctctcc tggaagttgg gttcattttc 2580
   aaattagttt ttttatttta atatctcaag attattgtat gtgactattt tagcagaaag 2640
   tgaattatgg gaacttgaac taaccaactg aaaatacatt cagaactaat taaacangat 2700
   gccagaatgt gattggctcc aggcatttta aattcaacag gttatgtaac caggctttaa 2760
   atttgcacat cttcgtgtta ccttcatgac acagtcaact cccattatgt aagaaatggt 2820
   gagtgcattc ccaagggtct tgcacagtta taaaaataga cttgatgagg tgaggagttg 2880
   tttaaattcc cctctgaaga agcagcatca acccaacaaa ccactctctt ccctctgtga 2940
   ctagagctct gtcacaggcc acatggacct aaatccttga tggagattac aggactacgt 3000
   aaattggact gatcgttttt atgctgttaa attaataggt gagtctgcac tccagcctgg 3060
   gcaacagaat aatcttgtct gtaaaataca aaagaaagat aaattaatag atactgactt 3120
   tgacatttcg gataataata ttttcataaa ccgaatttaa ttatacccac attgttacct 3180
   acaccttcac tgaaaagttc ctagttatgt tgagttccat caacactcca catgttcaaa 3240
   tctggacatc caagagagtc tagagaataa aacgcaatga gggcagtgaa acttgcgtat 3300
   attcagcacc tcttaactca ggaggactca atacaccctg gaacactctg cttttctgaa 3360
   tggctcacaa tgactccagc tcactctcca acctccgcaa acatctggcc tctgtttgcc 3420
   ctaagttcac gctctgctct tagtctatgt tctgaagtct ttgtagaggt gaaaatgagc 3480
   tgtcagatgg atcttccttc tcactgcaac atggaatttg ctatttcact taatgaccac 3540
   tctttccaca atggttgatt tcttttggcc tgttcattac tggtgatttt caagggaatc 3600
   tcagttgaat ctttactgtt ttgcattttg tctccatgac aatgttggga agtttttctt 3660
   ctagcagcat aacatgatct agtgacctga cacatttgca gcaaacaata cctacaaatt 3720
   cagaagctct ttggttttct ttccacgaaa tataattctt gctcttctgt gtatgagcac 3780
   atcctagcat ccctgtacac acccaggtag atgtctacac gccgatgaaa tattccctgt 3840
   aaataaaaaa agtatctcag tttctctcaa tgttcataat tctcctgagg gtgaggaagg 3900
   tacttctggg tctgctcaaa caaatggccc agagaccacc tggtaggtag gtaaggagct 3960
   cacctcgctc tggatattga gtctgtctct ttccctctgt cgtctcatag aagnccagcc 4020
   cacttgttca gctcctaaga agagagccca ggtttatcca gattatacaa cacaaccagc 4080
   ttctgatgac tctcctgtta caacatccat ggagatattt tgtgtattat ataattcacc 4140
   aaactaatgt gaaatgccca agttgcaata ctgcacaccc tagggtatgt tcttgcaatt 4200
   cagcggagga gaaattcttt cagagacaga tggatctgaa ttggtaaata tgtgggtacg 4260
   aattctgggc ttgagtgtca ttgtccagcc atgtttcaca ggtgtgacct gtcagggaag 4320
   aaccagagtt ccttgttctc tcagagggta gagctcacag aggtcctctc tggttcccag 4380
   gaaaggtaat ttcactaatc ttggtgatga gactatcctc cagtgctgat gtactataga 4440
   gttttcatct gaagctgtca ctgctatccc caatgtacat cttttcacac agaaatgttt 4500
   agaggtcagg ccatattctc agggttacac attgagaagg atggagatat attctactac 4560
   cttctcctga gatctcacac acaatctcaa atttcaaaag gtctcagaag ggcagctctc 4620
   aggtactatt taaaaataac ccacttcctg ggacaggtag catccttcta accatgatgg 4680
   atgttctgaa ctacagtaca cattgcatgg atccaggttt gtctcaattc actgtgatta 4740
   ttacactcag cagctgtttc aatatgtctg aaggggtaaa tgacaattta ggtgacctgg 4800
   gtgtatggtt ggtgttatat gaatctttaa atgtagaaca gtattaactg tattccaaaa 4860
   tctgtctttg atccatgatc acacttgtct cccagaccag ctccttcagc acatttccta 4920
   cctggaagaa gaggactctg ggtttggtga ggggaggcca caggaagaga actgagttct 4980
   cagagggcac agccagcata cacctcccag ggtgagccca aaagactggg gcctccctca 5040
   tcccttttta cctatccata caaaggcacc acccacatgc aaatcctcac ttaggcaccc 5100
   acaggaaatg actacacatt tccttaaatt cagggtccag ctcacatggg aagtgctttc 5160
   tgagagtcat ggacctcctg cacaagaaca tgaaacacct gtggttcttc ctcctcctgg 5220
   tggcagctcc cagatgtgag tgtctcagga atgcggatat gaagatatga gatgctgcct 5280
   ctgatcccag ggctcactgt gggtttttct gttcacaggg gtcctgtccc aggtgcagct 5340
   acagcagtgg ggcgcaggac tgttgaagcc ttcggagacc ctgtccctca cctgcggtgt 5400
   ttatggtggg tccttcagtg gttactactg gagctggatc cgccagcccc cagggaaggg 5460
   gctggagtgg attggggaaa tcaatcatag tggaagcacc aactacaacc cgtccctcaa 5520
   gagtcgagtc accatatcag tagacacgtc caagaagcag ctctccctga agttgagctc 5580
   tgtgaacgcc gcggacacgg ctgtgtatta ctgtgcgaga gttattacta gggcgagtcc 5640
   tggcacagac gggaggtacg gtatggacgt ctggggccaa gggaccacgg tcaccgtctc 5700
   ctcaggtgag aatggccact ctagggcctc tgttctctgc tactgcctgt ggggtttcct 5760
   gagcattgca ggttggtcct cggggcatgt tccgagggga cctgggcgga ctggccagga 5820
   ggggacgggc actggggtgc cttgaggatc tgggagcctc tgtggatttt ccgatgcctt 5880
   tggaaaatgg gactcaggtt gggtgcgtct gatggagtaa ctgagcctgg gggcttgggg 5940
   agccacattt ggacgagatg cctgaacaaa ccaggggtct tagtgatggc tgaggaatgt 6000
   gtctcaggag cggtgtctgt aggactgcaa gatcgctgca cagcagcgaa tcgtgaaata 6060
   ttttctttag aattatgagg tgcgctgtgt gtcaacctgc atcttaaatt ctttattggc 6120
   tggaaagaga actgtcggag tgggtgaatc cagccaggag ggacgcgtag ccccggtctt 6180
   gatgagagca gggttggggg caggggtagc ccagaaacgg tggctgccgt cctgacaggg 6240
   gcttagggag gctccaggac ctcagtgcct tgaagctggt ttccatgaga aaaggattgt 6300
   ttatcttagg aggcatgctt actgttaaaa gacaggatat gtttgaagtg gcttctgaga 6360
   aaaatggtta agaaaattat gacttaaaaa tgtgagagat tttcaagtat attaattttt 6420
   ttaactgtcc aagtatttga aattcttatc atttgattaa cacccatgag tgatatgtgt 6480
   ctggaattga ggccaaagca agctcagcta agaaatacta gcacagtgct gtcggccccg 6540
   atgcgggact gcgttttgac catcataaat caagtttatt tttttaatta attgagcgaa 6600
   gctggaagca gatgatgaat tagagtcaag atggctgcat gggggtctcc ggcacccaca 6660
   gcaggtggca ggaagcaggt caccgcgaga gtctatttta ggaagcaaaa aaacacaatt 6720
   ggtaaattta tcacttctgg ttgtgaagag gtggttttgc ccaggcccag atctgaaagt 6780
   gctctactga gcaaaacaac acctggacaa tttgcgtttc taaaataagg cgaggctgac 6840
   cgaaactgaa aaggcttttt ttaactatct gaatttcatt tccaatctta gcttatcaac 6900
   tgctagtttg tgcaaacagc atatcaactt ctaaactgca ttcattttta aagtaagatg 6960
   tttaagaaat taaacagtct tagggagagt ttatgactgt attcaaaaag ttttttaaat 7020
   tagcttgtta tcccttcatg tgataattaa tctcaaatac tttttcgata cctcagagca 7080
   ttattttcat aatgactgtg ttcacaatct ttttaggtta actcgttttc tctttgtgat 7140
   taaggagaaa cactttgata ttctgataga gtggccttca ttttagtatt tttcaagacc 7200
   acttttcaac tactcacttt aggataagtt ttaggaaaaa tgtgcatcat tatcctgaat 7260
   tatttcagtt aagcatgtta gttggtggca taagagaaaa ctcaatcaga tagtgctgaa 7320
   gacaggactg tggagacacc ttagaaggac agattctgtt ccgaatcacc gatgcggcgt 7380
   cagcaggact ggcctagcgg aggctctggg agggtggctg ccaggcccgg cctgggctct 7440
   gggtctcccc ggactaccca gagctgggat gcgtggcttc tgctgccggg ccgactggct 7500
   gctcaggccc cagcccttgt taatggactt ggaggaatga ttccatgcca aagctttgca 7560
   aggctcgcag tgaccaggcg cccgacatgg taagagacag gcagccgccg ctgctgcatt 7620
   tgcttctctt aaaactttgt atttgacgtc ttatttccac tagaagggga actggtctta 7680
   attgcttgat gaagagcagg agactcattt atgtgagtct tttgagtgac cattgtctgg 7740
   gtcactccca tttaactttc cctaaagccc atttgaagga gaggtcgcac gagctgctcc 7800
   acaacctctg aatggggatg gcatgggtaa tgatgcttga gaacatacca agtcccactg 7860
   gcatcgccct tgtctaagtc attgactgta ggtcatcatc gcacccttga aagtagccca 7920
   tgccttccaa agcgatttat ggtaaatggc agaattttaa gtggcaaatt cagataaaat 7980
   gcatttcttg gttgtttcca atgatgactg ttatctagag ggaatttaaa ggcaggggtt 8040
   tactgcagac tcagaaggga ggggatgctc cgggaaggtg gaggctctga gcatctcaat 8100
   accctcctct tggtgcagaa gatatgctgc cacttctaga gcaaggggac ctgctcattt 8160
   ttatcacagc acaggctcct aaattcttgg tctcattctc aagatgtttt aatgacttta 8220
   aagcagcaaa gaaatattcc acccaggtag tggagggtgg taatgattgg taatgctttg 8280
   gaaccaaaac ccaggtggcg ctggggcagg actgcaggga actggggtat caagtagagg 8340
   gagacaaaag atggaagcca gcctggctgt gcaggaaccc ggcaatgaga tggctttagc 8400
   tgagacaagc aggtctggtg ggctgaccat ttctggccat gacaactcca tccagttttc 8460
   agaaatggac tcagatgggc aaaactgacc taagttgacc tagactaaac aaggccgaac 8520
   tgggctgagc tgagctgaac tgggctgagt tgaactgggc tgagctgagc tgagctgagc 8580
   tgggctaagt tgcaccaggt gagctgagtt gagctgggct tggctgcatt aaggtgggct 8640
   gagctgggca gggctgggct gaattgagct gggctgggct gagctaggct gggctgggc 8699
<210> 2
   <211> 338
   <212> DNA
   <213> Home sapiens
<220>
   <221> misc_feature
   <222> (54)..(54)
   <223> n = unknown
<220>
   <221> misc_feature
   <222> (86)..(86)
   <223> n = unknown
<400> 2
   taagtgaatc ctggtgtgtc tgaactcaag tgattgttac attaagctgc tgtnccaatc 60
   tgtttcctca cctgggaaaa gagganccag gacatagtga gttgaggccc caggaagata 120
   actgaattct cagagggcac agccagcatc ctcctcccag ggagagtcta aaagactggg 180
   gcctccctca tcccttttca cctgtccata cagaggcacc acccacatgc aaatctcact 240
   taggcaccca cagaaaacca ccacacattt ccttaaattc agggtcctgc tcacatggga 300
   aatactttct gagagtcctg gacctcctgt gcaagaac 338
<210> 3
   <211> 339
   <212> DNA
   <213> Homo sapiens
<400> 3
   taagtgaatc ctggtgtgtc tgaactcaca tgattgttac attaagctgc tgttgcaatc      60
   tgtttcctca cctgggaaaa gaggagccag gacatagtga gttgaggccc caggaagata     120
   actgaattct cagagggcac aaccagcatc ctccttgcca gggagagcct aaaagactgg     180
   ggcctccctc atcccttttc acctctccat acagaggcac cacccacatg caaatctcac     240
   ttaggcaccc aagggaaacc atcacacatt tccttaaatt cagggtcctg ctcacatggg     300
   aaatactttc tgagagctct ggacctcctg tgcaagaac                            339
<210> 4
   <211> 338
   <212> DNA
   <213> Homo sapiens
<220>
   <221> misc_feature
   <222> (106)..(106)
   <223> n = unknown
<400> 4
   attccaaaat ctgtctttga tccatgatca cacttgtctc ccagaccagc tccttcagca      60
   catttcctac ctggaagaag aggactctgg gtttggtgag gggagnccac aggaagagaa     120
   ctgagttctc agagggcaca gccagcatac acctcccagg gtgagcccaa aagactgggg     180
   cctccctcat ccctttttac ctatccatac aaaggcacca cccacatgca aatcctcact      240
   taggcaccca caggaaatga ctacacattt ccttaaattc agggtccagc tcacatggga     300
   agtgctttct gagagtcatg gacctcctgc acaagaac                             338
<210> 5
   <211> 339
   <212> DNA
   <213> Homo sapiens
<400> 5
   attccaaaat ctgtccctga tccaagatca cactgatctc ccagagcagc atcttcagca 60
   catttcccta cctggaagaa gaggactatg ggcttggtaa ggggaggcca caggaagaga 120
   actgagttct cagagggcac agccagcttc ctactcccag ggcaagccca aaagactggg 180
   gcctccctcc tcccttttca cctgtccata caaagtcacc gcccacatgc aaatcctcac 240
   ttaggcacct acaggaaacc agcacacatt tccttaaatt tgggatccag ctcacatggg 300
   aaatactttc tgagactcat gggcctcctg cacaagaac 339
<210> 6
   <211> 338
   <212> DNA
   <213> Homo sapiens
<220>
   <221> misc_feature
   <222> (16)..(16)
   <223> n = unknown
<400> 6
   atcccaaaat ctgtcnttga tccaggatca cactcatctc tcagaccagc tccttcagca 60
   catctcttta cctggaagaa gaggactctg ggcttggaga ggggagcccc caagaagaga 120
   actgagttct caaagggcac agccagcatt ctcctcccag ggtgagctca aaagactggc 180
   gcctctctca tcccttttca ctgctccgta caaacgcacc acccccatgc aaatcctcac 240
   ttaggcgccc acaggaagcc accacacatt tccttaaatt caggtccaac tcataaggga 300
   aatgctttct gagagtcatg gatctcatgt gcaagaaa 338
<210> 7
   <211> 334
   <212> DNA
   <213> Artificial Sequence
<220>
<223> Consensus sequence for human immunoglobulin heavy chain promoter for VH4 alleles
<400> 7
   atccaaaatc tgtctctgat ccaagatcac acttatctcc agagcagctc ttcagcacat 60
   ttccttacct ggaagaagag gactctgggc ttggtgaggg gaggccccag gaagagaact 120
   gagttctcag agggcacagc cagcatcctc ctcccagggg agcccaaaag actggggcct 180
   ccctcatccc ttttcacctt ccatacaaag gcaccaccca catgcaaatc ctcacttagg 240
   cacccacagg aaaccaccac acatttcctt aaattcaggg tccagctcac atgggaaata 300
   ctttctgaga gtcatggacc tcctgtgcaa gaac 334
<210> 8
   <211> 3693
   <212> DNA
   <213> Artificial Sequence
<220>
<223> Plasmid pME18Sasfp499
<400> 8
   aagcttggct gtggaatgtg tgtcagttag ggtgtggaaa gtccccaggc tccccagcag 60
   gcagaagtat gcaaagcatg catctcaatt agtcagcaac catagtcccg cccctaactc 120
   cgcccatccc gcccctaact ccgcccagtt ccgcccattc tccgccccat ggctgactaa 180
   ttttttttat ttatgcagag gccgaggccg cctcggcctc tgagctattc cagaagtagt 240
   gaggaggctt ttttggaggc ctaggctttt gcaaaaagct cctcgatcga ggggctcgca 300
   tctctccttc acgcgcccgc cgccctacct gaggccgcca tccacgccgg ttgagtcgcg 360
   ttctgccgcc tcccgcctgt ggtgcctcct gaactgcgtc cgccgtctag gtaagtttaa 420
   agctcaggtc gagaccgggc ctttgtccgg cgctcccttg gagcctacct agactcagcc 480
   ggctctccac gctttgcctg accctgcttg ctcaactcta cgtctttgtt tcgttttctg 540
   ttctgcgccg ttacagatcc aagctctgaa aaaccagaaa gttaactggt aagtttagtc 600
   tttttgtctt ttatttcagg tcccggatcc ggtggtggtg caaatcaaag aactgctcct 660
   cagtggatgt tgcctttact tctaggcctg tacggaagtg ttacttctgc tctaaaagct 720
   gcgctcgaga tgtatccttc catcaaggaa accatgcgcg ttcagctttc tatggagggt 780
   agtgttaact accacgcctt caagtgcact ggaaaaggag agggaaaacc atacgaaggc 840
   acccaaagcc tgaatattac aataactgaa ggaggtcctc tgccatttgc ttttgacatt 900
   ctgtcacacg cctttcagta tggcatcaag gtcttcgcca agtaccccaa agaaattcct 960
   gacttcttta agcagtctct acctggtggt ttttcttggg aaagagtaag cacctatgaa 1020
   gatggaggag tgctttcagc tacccaagaa acaagtttgc agggtgattg catcatctgc 1080
   aaagttaaag tccttggcac caattttccc gcaaacggtc cagtgatgca aaagaagacc 1140
   tgtggatggg agccatcaac tgaaacagtc atcccacgag atggtggact tctgcttcgc 1200
   gatacccccg cacttatgct ggctgacgga ggtcatcttt cttgcttcat ggaaacaact 1260
   tacaagtcga agaaagaggt aaagcttcca gaacttcact ttcatcattt gcgtatggaa 1320
   aagctgaaca taagtgacga ttggaagacc gttgagcagc acgagtctgt ggtggctagc 1380
   tactcccaag tgccttcgaa attaggacat aacgcggccg ctgattataa agatgatgat 1440
   gataaagatt ataaagatga tgatgataaa gattataaag atgatgatga taaagattat 1500
   aaagatgatg atgataaata ataaactagt ctagagaaaa aacctcccac acctccccct 1560
   gaacctgaaa cataaaatga atgcaattgt tgttgttaac ttgtttattg cagcttataa 1620
   tggttacaaa taaagcaata gcatcacaaa tttcacaaat aaagcatttt tttcactgca 1680
   ttctagttgt ggtttgtcca aactcatcaa tgtatcttat catgtctgga tccccgggta 1740
   ccgagctcga attaattcct cttccgcttc ctcgctcact gactcgctgc gctcggtcgt 1800
   tcggctgcgg cgagcggtat cagctcactc aaaggcggta atacggttat ccacagaatc 1860
   aggggataac gcaggaaaga acatgtgagc aaaaggccag caaaaggcca ggaaccgtaa 1920
   aaaggccgcg ttgctggcgt ttttccatag gctccgcccc cctgacgagc atcacaaaaa 1980
   tcgacgctca agtcagaggt ggcgaaaccc gacaggacta taaagatacc aggcgtttcc 2040
   ccctggaagc tccctcgtgc gctctcctgt tccgaccctg ccgcttaccg gatacctgtc   2100
   cgcctttctc ccttcgggaa gcgtggcgct ttctcatagc tcacgctgta ggtatctcag   2160
   ttcggtgtag gtcgttcgct ccaagctggg ctgtgtgcac gaaccccccg ttcagcccga   2220
   ccgctgcgcc ttatccggta actatcgtct tgagtccaac ccggtaagac acgacttatc   2280
   gccactggca gcagccactg gtaacaggat tagcagagcg aggtatgtag gcggtgctac   2340
   agagttcttg aagtggtggc ctaactacgg ctacactaga agaacagtat ttggtatctg   2400
   cgctctgctg aagccagtta ccttcggaaa aagagttggt agctcttgat ccggcaaaca   2460
   aaccaccgct ggtagcggtg gtttttttgt ttgcaagcag cagattacgc gcagaaaaaa   2520
   aggatctcaa gaagatcctt tgatcttttc tacggggtct gacgctcagt ggaacgaaaa   2580
   ctcacgttaa gggattttgg tcatgagatt atcaaaaagg atcttcacct agatcctttt   2640
   aaattaaaaa tgaagtttta aatcaatcta aagtatatat gagtaaactt ggtctgacag   2700
   ttaccaatgc ttaatcagtg aggcacctat ctcagcgatc tgtctatttc gttcatccat   2760
   agttgcctga ctccccgtcg tgtagataac tacgatacgg gagggcttac catctggccc   2820
   cagtgctgca atgataccgc gagacccacg ctcaccggct ccagatttat cagcaataaa   2880
   ccagccagcc ggaagggccg agcgcagaag tggtcctgca actttatccg cctccatcca   2940
   gtctattaat tgttgccggg aagctagagt aagtagttcg ccagttaata gtttgcgcaa   3000
   cgttgttgcc attgctacag gcatcgtggt gtcacgctcg tcgtttggta tggcttcatt   3060
   cagctccggt tcccaacgat caaggcgagt tacatgatcc cccatgttgt gcaaaaaagc   3120
   ggttagctcc ttcggtcctc cgatcgttgt cagaagtaag ttggccgcag tgttatcact   3180
   catggttatg gcagcactgc ataattctct tactgtcatg ccatccgtaa gatgcttttc   3240
   tgtgactggt gagtactcaa ccaagtcatt ctgagaatag tgtatgcggc gaccgagttg   3300
   ctcttgcccg gcgtcaatac gggataatac cgcgccacat agcagaactt taaaagtgct   3360
   catcattgga aaacgttctt cggggcgaaa actctcaagg atcttaccgc tgttgagatc   3420
   cagttcgatg taacccactc gtgcacccaa ctgatcttca gcatctttta ctttcaccag   3480
   cgtttctggg tgagcaaaaa caggaaggca aaatgccgca aaaaagggaa taagggcgac   3540
   acggaaatgt tgaatactca tactcttcct ttttcaatat tattgaagca tttatcaggg   3600
   ttattgtctc atgagcggat acatatttga atgtatttag aaaaataaac aaataggggt   3660
   tccgcgcaca tttccccgaa aagtgccacc tgc                                 3693
<210> 9
   <211> 581
   <212> DNA
<213> Homo sapiens
<400> 9
   cggccccgat gcgggactgc gttttgacca tcataaatca agtttatttt tttaattaat     60
   tgagcgaagc tggaagcaga tgatgaatta gagtcaagat ggctgcatgg gggtctccgg    120
   cacccacagc aggtggcagg aagcaggtca ccgcgagagt ctattttagg aagcaaaaaa    180
   acacaattgg taaatttatc acttctggtt gtgaagaggt ggttttgccc aggcccagat    240
   ctgaaagtgc tctactgagc aaaacaacac ctggacaatt tgcgtttcta aaataaggcg    300
   aggctgaccg aaactgaaaa ggcttttttt aactatctga atttcatttc caatcttagc    360
   ttatcaactg ctagtttgtg caaacagcat atcaacttct aaactgcatt catttttaaa    420
   gtaagatgtt taagaaatta aacagtctta gggagagttt atgactgtat tcaaaaagtt    480
   ttttaaatta gcttgttatc ccttcatgtg taattaatct caaatacttt ttcgatacct    540
   cagagcatta ttttcataat gactgtgttc acaatctttt t                        581
<210> 10
   <211> 582
   <212> DNA
   <213> Homo sapiens
<400> 10
   cggccccgat gcgggactgc gttttgacca tcataaatca agtttatttt tttaattaat     60
   tgagcgaagc tggaagcaga tgatgaatta gagtcaagat ggctgcatgg gggtctccgg    120
   cacccacagc aggtggcagg aagcaggtca ccgcgagagt ctattttagg aagcaaaaaa    180
   acacaattgg taaatttatc acttctggtt gtgaagaggt ggttttgccc aggcccagat    240
   ctgaaagtgc tctactgagc aaaacaacac ctggacaatt tgcgtttcta aaataaggcg    300
   aggctgaccg aaactgaaaa ggcttttttt aactatctga atttcatttc caatcttagc    360
   ttatcaactg ctagtttgtg caaacagcat atcaacttct aaactgcatt catttttaaa    420
   gtaagatgtt taagaaatta aacagtctta gggagagttt atgactgtat tcaaaaagtt    480
   ttttaaatta gcttgttatc ccttcatgtg ataattaatc tcaaatactt tttcgatacc    540
   tcagagcatt attttcataa tgactgtgtt cacaatcttt tt                       582
<210> 11
   <211> 666
   <212> DNA
   <213> Ovis aries
<400> 11
   ctgcgaatac cgagacgggg cctctcaaag ccacccctga tagtctggaa aattgaaact     60
   ttaaaaagag agatgtttaa agtattttaa atttttatca tttaattaac aactgcgaat    120
   catggctttg gagagttgag taagagtttg gctgaaaagt actaactagg ttccatcggc    180
   cctcggcccc aattcagggc tgttttgaga ataataaatt cagcttattt ttttaatgta    240
   attggtggtg ccgagttagt caagatggcc acgggccaga ctgaccacct gcagcaggtg    300
   gcaggaagca tgtccacttg agagtctgtt tttggaagca agaaaaaaca gttggtaaat    360
   ttatcgcttc tggtttccaa aaggtggttt gcggctggtt ttgcccagcc ccacagaacc    420
   gaaagtgttc cactgagcac aacagcacct ggctaatttg catttctaaa ataaggcgca    480
   gatgctgacc gaaactggaa ggttcctctt ctaactattt gagttaactt cagctttagc    540
   ttatcaactg ctcacttatc ttcattttca aagtcgatgt ttaagaaagc cacctgtctc    600
   gggtgcactg tctcggtgca ttgctgcact ctctgatgag ccgtccttca aggtggttga    660
   gctgag                                                               666
<210> 12
   <211> 1067
   <212> DNA
   <213> Mus musculus
<400> 12
   ctagatactg agttctggtt ctaataactg gctcctgtac tgatggatgg gtcctgacta      60
   gtcattgggc cctgatcctc aacattgact tcaaaacctg aactctagcc ccatgcctca     120
   ttcacattag gatgatccct acaggggatt cctgcagaag attccagaat ccccacaaca     180
   ctgttcacac actgggctgc aactgggaca gtgacccttt tgactcatag gacttgccag     240
   gcacagaggc acagaatgga gacaaagcaa gcccaggacc ctggagatgg agcctctggt     300
   ggggtctaca gatgtggggt cagcatcgta gggaggtttg cagggcaggt gtggggcagg     360
   gcagaggtag tcatgcttat agatactatt tttctctcct ctggagcctc ctttgtctat     420
   cacctgctgt cctgggatct ctatctgggg tcaacaatgt ttgcagtaca ggtgtggggg     480
   tagggcaggg atgctcacat tagcaacttg tttttctctc ttctgaagtc tctgttgtct     540
   atcacctgct gaaacattca aagcagctct cagctgaggg cagctgagtc atcctgagcc     600
   tgtctcagca caggtgcccc aaaccagagc tactgttctg agaatcacat cacactggac     660
   caggccaggt gggcctggga catggatgag gggtgggagc caggggagcc tgccaggggc      720
   tgaggaggcc ccaaccccca ctacccaagg ccatccacac ctgtgcctta gtgaggccat     780
   gttctgtccc aatgagaaca agtccaatta agattaagta tggtcttccc aggactatcc     840
   agagctaagg ggtgtcagcc agggacaacc cagaccagcc tgaggtcagc cagcatcacc     900
   caaggccaca cagctattct ggctagagga ctagatagct agctcatcga ggccctggag     960
   atgcagaatg gaagagttta tccctgccag acagggctca tcagaaaggc aggtatctca    1020
   ctacacatga cctccctgaa tatttcccag agtccagttg gttctag                  1067
<210> 13
   <211> 221
   <212> DNA
   <213> Mus musculus
<400> 13
   agtcaagatg gccgatcaga accagaacac ctgcagcagc tggcaggaag caggtcatgt      60
   ggcaaggcta tttggggaag ggaaaataaa accactaggt aaacttgtag ctgtggtttg     120
   aagaagtggt tttgaaacac tctgtccagc cccaccaaac cgaaagtcca ggctgagcaa     180
   aacaccacct gggtaatttg catttctaaa ataagttgag g                         221
<210> 14
   <211> 808
   <212> DNA
   <213> Mus musculus
<400> 14
   agctcaaacc agcttaggct acacagagaa actatctaaa aaataattac taactactta      60
   ataggagatt ggatgttaag atctggtcac taagaggcag aattgagatt cgaaccagta     120
   ttttctacct ggtatgtttt aaattgcagt aaggatctaa gtgtagatat ataataataa     180
   gattctattg atctctgcaa caacagagag tgttagattt gtttggaaaa aaatattatc     240
   agccaacatc ttctaccatt tcagtatagc acagagtacc cacccatatc tccccaccca     300
   tcccccatac cagactggtt attgattttc atggtgactg gcctgagaag attaaaaaaa     360
   gtaatgctac cttattggga gtgtcccatg gaccaagata gcaactgtca tagctaccgt     420
   cacactgctt tgatcaagaa gaccctttga ggaactgaaa acagaacctt aggcacatct     480
   gttgctttcg ctcccatcct cctccaacag cctgggtggt gcactccaca ccctttcaag     540
   tttccaaagc ctcatacacc tgctccctac cccagcacct ggccaaggct gtatccagca     600
   ctgggatgaa aatgataccc cacctccatc ttgtttgata ttactctatc tcaagcccca     660
   ggttagtccc cagtcccaat gcttttgcac agtcaaaact caacttggaa taatcagtat     720
   ccttgaagag ttctgatatg gtcactgggc ccatatacca tgtaagacat gtggaaaaga     780
   tgtttcatgg ggcccagaca cgttctag                                       808
<210> 15
   <211> 60
   <212> DNA
   <213> Mus musculus
<400> 15
   aagcagccct caggcagagg ataaaagctc acactaactg agaagctcca tcctcttctc      60
<210> 16
   <211> 60
   <212> DNA
   <213> Mus musculus
<400> 16
   aattaggcca ccctcatcac atgaaaacca gcccagagtg actctagcag tgggatcctg 60
<210> 17
   <211> 60
   <212> DNA
   <213> Mus musculus
<400> 17
   catgtgcgac tgtgatgatt aatataggga tatccacacc aaacatcata tgagccctat 60
<210> 18
   <211> 60
   <212> DNA
   <213> Mus musculus
<400> 18
   aacatgagtc tgtgattata aatacagaga tatccatacc aaacaactta tgagcactgt 60
<210> 19
   <211> 338
   <212> DNA
   <213> Homo sapiens
<220>
   <221> misc feature
   <222> (16)..(16)
   <223> n = unknown
<400> 19
   atcccaaaat ctgtcnttga tccaggatca cactcatctc tcagaccagc tccttcagca 60
   catctcttta cctggaagaa gaggactctg ggcttggaga ggggagcccc caagaagaga 120
   actgagttct caaagggcac agccagcatt ctcctcccag ggtgagctca aaagactggc 180
   gcctctctca tcccttttca ctgctccgta caaacgcacc acccccatgc aaatcctcac 240
   ttaggcgccc acaggaagcc accacacatt tccttaaatt caggtccaac tcataaggga 300
   aatgctttct gagagtcatg gatctcatgt gcaagaaa 338
<210> 20
   <211> 255
   <212> DNA
   <213> Homo sapiens
<400> 20
<210> 21
   <211> 238
   <212> DNA
   <213> Homo sapiens
<400> 21
<210> 22
   <211> 244
   <212> DNA
   <213> Homo sapiens
<400> 22
   gatgggtagg ggatgcgtgt cctctaacag gattacgtct tgaaccctca gcttctacaa      60
   ttgtgtcgtc catgtgtcat gtatttgctc tttctcatcc tgggtcagga attgggctat     120
   taaatagcat ccttcatgaa tatgcaaata actgaggtga atatagatat ctgtgtgccc     180
   tgagagcatc acccaaaaac cacacccctc cttgggagaa tcccctagat cacagctcct     240
   cacc                                                                  244
<210> 23
   <211> 36
   <212> PRT
   <213> Homo sapiens
<400> 23
   Pro Asn Lys Gly Ser Thr Thr Ser Gly Thr Thr Arg Leu Leu Ser Gly
   1               5                   10                  15
   His Thr Cys Phe Thr Leu Thr Gly Leu Leu Gly Thr Leu Val Thr Met
               20                  25                  30
   Gly Leu Leu Thr
           35
<210> 24
   <211> 111
   <212> DNA
   <213> Homo sapiens
<400> 24
   ccaaataaag gaagtggaac cacttcaggt actacccgtc ttctatctgg gcacacgtgt 60
   ttcacgttga caggtttgct tgggacgcta gtaaccatgg gcttgctgac t 111
<210> 25
   <211> 33
   <212> PRT
   <213> Sus scrofa
<400> 25
   Cys Arg Thr Asn Tyr Gly Tyr Ser Ala Ala Pro Ser Leu His Leu Pro
   1 5 10 15
   Pro Gly Ser Leu Leu Ala Ser Leu Val Pro Leu Leu Leu Leu Ser Leu
   20 25 30
   Pro
<210> 26
   <211> 99
   <212> DNA
   <213> Sus scrofa
<400> 26
   tgccggacga attacggcta ctcagccgcc cccagcctcc acctcccgcc gggctcgctg 60
   ctggcctccc tcgtgcccct cctcctcctc agtcttccg 99
<210> 27
   <211> 30
   <212> PRT
   <213> Rattus rattus
<400> 27
   Ala Ser Ser Gln Ser Tyr Arg Met Thr Trp Asn Ile Leu Tyr Thr Leu
   1 5 10 15
   Leu Ile Ser Met Thr Thr Leu Phe Gln Ile Ser Thr Lys Glu
   20 25 30
<210> 28
   <211> 90
   <212> DNA
   <213> Rattus rattus
<400> 28
   gcatcgtctc agagctacag gatgacctgg aacatactct atacactgtt aatcagcatg      60
   actactttat tccaaatatc taccaaggag                                       90
<210> 29
   <211> 47
   <212> PRT
   <213> Mus musculus
<400> 29
   Ser Ser Asn Lys Ser Ile Ser Val Tyr Arg Asp Lys Leu Val Lys Cys
   1               5                   10                  15
   Gly Gly Ile Ser Leu Leu Val Gln Asn Thr Ser Trp Met Leu Leu Leu
               20                  25                  30
   Leu Leu Ser Leu Ser Leu Leu Gln Ala Leu Asp Phe Ile Ser Leu
           35                  40                  45
<210> 30
   <211> 135
   <212> DNA
   <213> Mus musculus
<400> 30
   agctccaata aaagtatcag tgtgtataga gacaagctgg tcaagtgtgg cggcataagc      60
   ctgctggttc agaacacatc ctggatgctg ctgctgctgc tttccctctc cctcctccaa     120
   gccctggact tcatt                                                      135
<210> 31
   <211> 37
   <212> PRT
   <213> Mus musculus
<400> 31
   Pro Glu Asp Pro Pro Asp Ser Lys Asn Thr Leu Val Leu Phe Gly Ala
   1               5                   10                  15
   Gly Phe Gly Ala Val Ile Thr Val Val Val Ile Val Val Ile Ile Lys
               20                  25                  30
   Cys Phe Cys Lys His
           35
<210> 32
   <211> 111
   <212> DNA
   <213> Mus musculus
<400> 32
   ccagaagacc ctcctgatag caagaacaca cttgtgctct ttggggcagg attcggcgca      60
   gtaataacag tcgtcgtcat cgttgtcatc atcaaatgct tctgtaagca c              111
<210> 33
   <211> 25
   <212> PRT
   <213> Homo sapiens
<400> 33
   Met Gly Ile Gln Gly Gly Ser Val Leu Phe Gly Leu Leu Leu Val Leu
   1               5                   10                  15
   Ala Val Phe Cys His Ser Gly His Ser
               20                  25
<210> 34
   <211> 75
   <212> DNA
   <213> Homo sapiens
<400> 34
   atgggaatcc aaggagggtc tgtcctgttc gggctgctgc tcgtcctggc tgtcttctgc      60
   cattcaggtc atagc                                                       75
<210> 35
   <211> 39
   <212> DNA
   <213> Artificial Sequence
<220>
<223> Oligonucleotide primer
<400> 35
   ccggaattca atttgagatt gtgtgtgaga tctcaggag 39
<210> 36
   <211> 39
   <212> DNA
   <213> Artificial Sequence
<220>
<223> Oligonucleotide primer
<400> 36
   ccggaattca tagacagcgc aggtgaggga cagggtctc 39
<210> 37
   <211> 39
   <212> DNA
   <213> Artificial Sequence
<220>
<223> Oligonucleotide primer
<400> 37
   ccggaattcc tgagaactca gttctcttcc tgtggcctc 39
<210> 38
   <211> 39
   <212> DNA
   <213> Artificial Sequence
<220>
<223> Oligonucleotide primer
<400> 38
   ccggaattca atttgagatt gtgtgtgaga tctcaggag 39
<210> 39
   <211> 39
   <212> DNA
   <213> Artificial Sequence
<220>
<223> Oligonucleotide primer
<400> 39
   cccaagcttt cctgttacaa catccatgga gatattttg 39
<210> 40
   <211> 39
   <212> DNA
   <213> Artificial Sequence
<220>
<223> Oligonucleotide primer
<400> 40
   ccggaattct gaattgcaag aacataccct agggtgtgc 39
<210> 41
   <211> 40
   <212> DNA
   <213> Artificial Sequence
<220>
<223> Oligonucleotide primer
<400> 41
   ccggaattct agggcaaaca gaggccagat gtttgaggag 40
<210> 42
   <211> 39
   <212> DNA
   <213> Artificial Sequence
<220>
<223> Oligonucleotide primer
<400> 42
   ccggaattca atttaacagc ataaaaacga tcagtccaa 39
<210> 43
   <211> 39
   <212> DNA
   <213> Artificial Sequence
<220>
<223> Oligonucleotide primer
<400> 43
   ccggaattcc gtgtttctgg agcagggcat ggctttggg 39
<210> 44
   <211> 39
   <212> DNA
<213> Artificial Sequence
<220>
<223> Oligonucleotide primer
<400> 44
   ccggaattcg ttgggttccc agtgtaggtg atgatccat 39
<210> 45
   <211> 39
   <212> DNA
   <213> Artificial Sequence
<220>
<223> Oligonucleotide primer
<400> 45
   ccggaattct cccaggaagt gggttatttt taaatagta 39
<210> 46
   <211> 39
   <212> DNA
   <213> Artificial Sequence
<220>
<223> Oligonucleotide primer
<400> 46
   ccggaattca ctatagtcac ctcagttaat tgcatattc 39
<210> 47
   <211> 39
   <212> DNA
   <213> Artificial Sequence
<220>
<223> Oligonucleotide primer
<400> 47
   cccaagcttg acttccttta aaaatatcta aaataagta 39
<210> 48
   <211> 39
   <212> DNA
   <213> Artificial Sequence
<220>
<223> Oligonucleotide primer
<400> 48
   ccggaattcg gttctcatta caacatccag tttgataaa 39
<210> 49
   <211> 39
   <212> DNA
   <213> Artificial Sequence
<220>
<223> Oligonucleotide primer
<400> 49
   ccggaattcc tccaagaaaa gatctcatgc atcaccagg 39
<210> 50
   <211> 39
   <212> DNA
   <213> Artificial Sequence
<220>
<223> Oligonucleotide primer
<400> 50
   cccaagctta atttagtttt cacggggcat ctgcagggt 39
<210> 51
   <211> 39
   <212> DNA
   <213> Artificial Sequence
<220>
<223> Oligonucleotide primer
<400> 51
   cccaagcttt gcacacccta gggtatgttc ttgcaattc 39
<210> 52
   <211> 39
   <212> DNA
<213> Artificial Sequence
<220>
<223> Oligonucleotide primer
<400> 52
   cccaagcttc ccaaagccat gccctgctcc agaaacacg 39
<210> 53
   <211> 39
   <212> DNA
   <213> Artificial Sequence
<220>
<223> Oligonucleotide primer
<400> 53
   ccggaattcc cataatatgt gaatgcgtta tttagggaa 39
<210> 54
   <211> 29 <212> DNA
   <213> Artificial Sequence
<220>
<223> Oligonucleotide primer
<400> 54
   cccaagcttc atgttccacg cattacgtc 29
<210> 55
   <211> 29
   <212> DNA
   <213> Artificial Sequence
<220>
<223> Oligonucleotide primer
<400> 55
   cccaagctta agagtgtttg ggttcaccg 29
<210> 56
   <211> 39
   <212> DNA
   <213> Artificial Sequence
<220>
<223> Oligonucleotide primer
<400> 56
   cccaagcttt taactcagga ggactcaata caccctgga 39
<210> 57
   <211> 39
   <212> DNA
   <213> Artificial Sequence
<220>
<223> Oligonucleotide primer
<400> 57
   cccaagctta aacaatacct acaaattcag aagctcttt 39
<210> 58
   <211> 38
   <212> DNA
   <213> Artificial Sequence
<220>
<223> Oligonucleotide primer
<400> 58
   cccaagctta agtcttctgg ttacaccttc accattat 38
<210> 59
   <211> 39
   <212> DNA
   <213> Artificial Sequence
<220>
<223> Oligonucleotide primer
<400> 59
   cccaagctta ctctcttccc tctgtgacta gagctctgt 39
<210> 60
   <211> 39
   <212> DNA
   <213> Artificial Sequence
<220>
<223> Oligonucleotide primer
<400> 60
   cccaagcttt cagcttctac agttgtgtca cccatgtgt 39
<210> 61
   <211> 39
   <212> DNA
   <213> Artificial Sequence
<220>
<223> Oligonucleotide primer
<400> 61
   cccaagcttt gcagagttca ctgggtttcc taaaggcaa 39
<210> 62
   <211> 39
   <212> DNA
   <213> Artificial Sequence
<220>
<223> Oligonucleotide primer
<400> 62
   cccaagcttt caccacaaag gaactttcat ctctcctgg 39
<210> 63
   <211> 39
   <212> DNA
   <213> Artificial Sequence
<220>
<223> Oligonucleotide primer
<400> 63
   cccaagcttt ttcacacaga aatgtttaga ggtcaggcc 39
<210> 64
   <211> 39
   <212> DNA
   <213> Artificial Sequence
<220>
<223> Oligonucleotide primer
<400> 64
   cccaagcttt gcacacccta gggtatgttc ttgcaattc 39
<210> 65
   <211> 39
   <212> DNA
   <213> Artificial Sequence
<220>
<223> Oligonucleotide primer
<400> 65
   cccaagcttc ccaaagccat gccctgctcc agaaacacg 39
<210> 66
   <211> 33
   <212> DNA
   <213> Artificial Sequence
<220>
<223> Oligonucleotide primer
<400> 66
   cccaagctta tgatgtaacc ctcattggcc tca 33
<210> 67
   <211> 33
   <212> DNA
   <213> Artificial Sequence
<220>
<223> Oligonucleotide primer
<400> 67
   ccggaattcg agactccaag aaaagatctc atg 33
<210> 68
   <211> 33
   <212> DNA
   <213> Artificial Sequence
<220>
<223> Oligonucleotide primer
<400> 68
   cccaagctta tgttcttgca attcagcgga gga 33
<210> 69
   <211> 33
   <212> DNA
   <213> Artificial Sequence
<220>
<223> Oligonucleotide primer
<400> 69
   ccggaattct gtgtgagatc tcaggagaag gta 33
<210> 70
   <211> 39
   <212> DNA
   <213> Artificial Sequence
<220>
<223> Oligonucleotide primer
<400> 70
   cccaagcttc ggccccgatg cgggactgcg ttttgacca 39
<210> 71
   <211> 39
   <212> DNA
   <213> Artificial Sequence
<220>
<223> Oligonucleotide primer
<400> 71
   ccggaattca taacaagcta atttaaaaaa ctttttgaa 39
<210> 72
   <211> 39 <212> DNA
   <213> Artificial Sequence
<220>
<223> Oligonucleotide primer
<400> 72
   cccaagcttg cacagacggg aggtacggta tggacgtct 39
<210> 73
   <211> 39
   <212> DNA
   <213> Artificial Sequence
<220>
<223> Oligonucleotide primer
<400> 73
   ccggaattca aaaaaataaa cttgatttat gatggtcaa 39
<210> 74
   <211> 39
   <212> DNA
   <213> Artificial Sequence
<220>
<223> Oligonucleotide primer
<400> 74
   ccggaattcc gcggtgacct gcttcctgcc acctgctgt 39
<210> 75
   <211> 29
   <212> DNA
   <213> Artificial Sequence
<220>
<223> Oligonucleotide primer
<400> 75
   ccggaattca gttagtgcag ccaagccct 29
<210> 76
   <211> 39 <212> DNA
   <213> Artificial Sequence
<220>
<223> Oligonucleotide primer
<400> 76
   ccggaattca aaaggcaagt ggacttcggt gcttacctg 39
<210> 77
   <211> 36
   <212> DNA
   <213> Artificial Sequence
<220>
<223> Oligonucleotide primer
<400> 77
   cccaagcttc agctcagctc agttcagttc agccct 36
<210> 78
   <211> 39
   <212> DNA
   <213> Artificial Sequence
<220>
<223> Oligonucleotide primer
<400> 78
   cccaagctta tgcgagggtc tggacggctg aggaccccc 39
<210> 79
   <211> 39 <212> DNA
   <213> Artificial Sequence
<220>
<223> Oligonucleotide primer
<400> 79
   ccggaattca tgcggcaagg gttgcggacc gctggctgg 39
<210> 80
   <211> 29
   <212> DNA
   <213> Artificial Sequence
<220>
<223> Oligonucleotide primer
<400> 80
   ccggaattcg cccagcccag cctagctca 29
<210> 81
   <211> 29
   <212> DNA
   <213> Artificial Sequence
<220>
<223> Oligonucleotide primer
<400> 81
   cccaagcttt tatcaactgc tagtttgtg 29
<210> 82
   <211> 36
   <212> DNA
   <213> Artificial Sequence
<220>
<223> Oligonucleotide primer
<400> 82
   ccggaattca gggctgaact gaactgagct gagctg 36
<210> 83
   <211> 35
   <212> DNA
   <213> Artificial Sequence
<220>
<223> Oligonucleotide primer
<400> 83
   cggctgatat ctgggagcct ctgtggattt tccga 35
<210> 84
   <211> 31
   <212> DNA
   <213> Artificial Sequence
<220>
<223> Oligonucleotide primer
<400> 84
   agccggatat cgcccagccc agcctagctc a 31
<210> 85
   <211> 24
   <212> DNA
<213> Artificial Sequence
<220>
<223> Oligonucleotide primer
<400> 85
   gaaagttaaa tgggagtgac ccag 24
<210> 86
   <211> 24
   <212> DNA
   <213> Artificial Sequence
<220>
<223> Oligonucleotide primer
<400> 86
   gagtgaccat cgcacccttg acag 24
<210> 87
   <211> 10826
   <212> DNA
   <213> Artificial Sequence
<220>
<223> Plasmid 3kb15a-7-4T
<400> 87
   ctaaattgta agcgttaata ttttgttaaa attcgcgtta aatttttgtt aaatcagctc     60
   attttttaac caataggccg aaatcggcaa aatcccttat aaatcaaaag aatagaccga    120
   gatagggttg agtgttgttc cagtttggaa caagagtcca ctattaaaga acgtggactc    180
   caacgtcaaa gggcgaaaaa ccgtctatca gggcgatggc ccactacgtg aaccatcacc    240
   ctaatcaagt tttttggggt cgaggtgccg taaagcacta aatcggaacc ctaaagggag    300
   cccccgattt agagcttgac ggggaaagcc ggcgaacgtg gcgagaaagg aagggaagaa    360
   agcgaaagga gcgggcgcta gggcgctggc aagtgtagcg gtcacgctgc gcgtaaccac    420
   cacacccgcc gcgcttaatg cgccgctaca gggcgcgtcc cattcgccat tcaggctgcg    480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tgggtaccgg 660
   gccccccctc gaggtcgacg gtatcgataa gcttgatatc tgggagcctc tgtggatttt 720
   ccgatgcctt tggaaaatgg gactcaggtt gggtgcgtct gatggagtaa ctgagcctgg 780
   gggcttgggg agccacattt ggacgagatg cctgaacaaa ccaggggtct tagtgatggc 840
   tgaggaatgt gtctcaggag cggtgtctgt aggactgcaa gatcgctgca cagcagcgaa 900
   tcgtgaaata ttttctttag aattatgagg tgcgctgtgt gtcaacctgc atcttaaatt 960
   ctttattggc tggaaagaga actgtcggag tgggtgaatc cagccaggag ggacgcgtag 1020
   ccccggtctt gatgagagca gggttggggg caggggtagc ccagaaacgg tggctgccgt 1080
   cctgacaggg gcttagggag gctccaggac ctcagtgcct tgaagctggt ttccatgaga 1140
   aaaggattgt ttatcttagg aggcatgctt actgttaaaa gacaggatat gtttgaagtg 1200
   gcttctgaga aaaatggtta agaaaattat gacttaaaaa tgtgagagat tttcaagtat 1260
   attaattttt ttaactgtcc aagtatttga aattcttatc atttgattaa cacccatgag 1320
   tgatatgtgt ctggaattga ggccaaagca agctcagcta agaaatacta gcacagtgct 1380
   gtcggccccg atgcgggact gcgttttgac catcataaat caagtttatt tttttaatta 1440
   attgagcgaa gctggaagca gatgatgaat tagagtcaag atggctgcat gggggtctcc 1500
   ggcacccaca gcaggtggca ggaagcaggt caccgcgaga gtctatttta ggaagcaaaa 1560
   aaacacaatt ggtaaattta tcacttctgg ttgtgaagag gtggttttgc ccaggcccag 1620
   atctgaaagt gctctactga gcaaaacaac acctggacaa tttgcgtttc taaaataagg 1680
   cgaggctgac cgaaactgaa aaggcttttt ttaactatct gaatttcatt tccaatctta 1740
   gcttatcaac tgctagtttg tgcaaacagc atatcaactt ctaaactgca ttcattttta 1800
   aagtaagatg tttaagaaat taaacagtct tagggagagt ttatgactgt attcaaaaag 1860
   ttttttaaat tagcttgtta tcccttcatg tgataattaa tctcaaatac tttttcgata 1920
   cctcagagca ttattttcat aatgactgtg ttcacaatct ttttaggtta actcgttttc 1980
   tctttgtgat taaggagaaa cactttgata ttctgataga gtggccttca ttttagtatt 2040
   tttcaagacc acttttcaac tactcacttt aggataagtt ttaggaaaaa tgtgcatcat 2100
   tatcctgaat tatttcagtt aagcatgtta gttggtggca taagagaaaa ctcaatcaga 2160
   tagtgctgaa gacaggactg tggagacacc ttagaaggac agattctgtt ccgaatcacc 2220
   gatgcggcgt cagcaggact ggcctagcgg aggctctggg agggtggctg ccaggcccgg 2280
   cctgggctct gggtctcccc ggactaccca gagctgggat gcgtggcttc tgctgccggg 2340
   ccgactggct gctcaggccc cagcccttgt taatggactt ggaggaatga ttccatgcca 2400
   aagctttgca aggctcgcag tgaccaggcg cccgacatgg taagagacag gcagccgccg 2460
   ctgctgcatt tgcttctctt aaaactttgt atttgacgtc ttatttccac tagaagggga 2520
   actggtctta attgcttgat gaagagcagg agactcattt atgtgagtct tttgagtgac 2580
   cattgtctgg gtcactccca tttaactttc cctaaagccc atttgaagga gaggtcgcac 2640
   gagctgctcc acaacctctg aatggggatg gcatgggtaa tgatgcttga gaacatacca 2700
   agtcccactg gcatcgccct tgtctaagtc attgactgta ggtcatcatc gcacccttga 2760
   aagtagccca tgccttccaa agcgatttat ggtaaatggc agaattttaa gtggcaaatt 2820
   cagataaaat gcatttcttg gttgtttcca atgatgactg ttatctagag ggaatttaaa 2880
   ggcaggggtt tactgcagac tcagaaggga ggggatgctc cgggaaggtg gaggctctga 2940
   gcatctcaat accctcctct tggtgcagaa gatatgctgc cacttctaga gcaaggggac 3000
   ctgctcattt ttatcacagc acaggctcct aaattcttgg tctcattctc aagatgtttt 3060
   aatgacttta aagcagcaaa gaaatattcc acccaggtag tggagggtgg taatgattgg 3120
   taatgctttg gaaccaaaac ccaggtggcg ctggggcagg actgcaggga actggggtat 3180
   caagtagagg gagacaaaag atggaagcca gcctggctgt gcaggaaccc ggcaatgaga 3240
   tggctttagc tgagacaagc aggtctggtg ggctgaccat ttctggccat gacaactcca 3300
   tccagttttc agaaatggac tcagatgggc aaaactgacc taagttgacc tagactaaac 3360
   aaggccgaac tgggctgagc tgagctgaac tgggctgagt tgaactgggc tgagctgagc 3420
   tgagctgagc tgggctaagt tgcaccaggt gagctgagtt gagctgggct tggctgcatt 3480
   aaggtgggct gagctgggca gggctgggct gaattgagct gggctgggct gagctaggct 3540
   gggctgggcg atatcgaatt cctgcagccc gggggatccg cccccatcga taatttagtt 3600
   ttcacggggc atctgcaggg ttccaaagtg agaccaggtg aattcaatgt gcatgcactt 3660
   cccaagtgtc cacttgtatt ctgtttcttt acttctgttt acagaaagta gacacatatt 3720
   cagtcttagt accagtgtag ggagcgcttt ccatgagatg gataccagaa aaaaatggca 3780
   aacatgggat ccgttaatat aaaaattagc cacgatgtat atatatatat gtgtgtgtgt 3840
   gtgtgtgtgt gtgtgtacac acgcgcgcgc atgtgtgagt tgaatagcag agttggagtg 3900
   ggtttctatc cacatgtacc tgcacctgca ggtattctca ggtgccataa tcaactgtag 3960
   gaccctaaag gaaataagag tctcccctca acccctgaag agtgtttggg ttcaccgtgt 4020
   gtccaatgat tctgtgcctc ttgagctcca ggaaagggct ccctggtgat gcatgagatc 4080
   ttttcttgga gtctctctgc agagttcact gggtttccta aaggcaattc actatttcaa 4140
   aagatggtgt gaggagcatg tggtgtccct aaaggagaat tctgagccag ggcacaacca 4200
   ctttatactg agctggatac actggtagga atatactctg tcagctcaga tagaaacctc 4260
   cctgcatggt tggggctggg ctgcaggggg cgctccggat acacccagca caggctcccg 4320
   ccccagagca ggtgcacagg aggctgggga gaggttcctc ccagggcctg ggacttcctt 4380
   taaaaatatc taaaataagt atttcacaaa gactgctgaa gtttgtataa atatctattc 4440
   aattgtgagc atttatcaaa ctggatgttg taatgagaac cacttttaca atggggattt 4500
   caaactctgc tggaggtcag gaagagatcc tttcttataa ataaatgcaa tttttggata 4560
   aacacagtca ttccctaaat aacgcattca catattatgg tctagaaatg atgcaagttg 4620
   accctgagac agtcaaatgt ggtttcaaag tgaggtgctg tccttgagga gcttgttctc 4680
   cagtggggga agctctgtca acacagagtt cagggatgtg taggggacac atggcctcta 4740
   acaggattac ggcttgaacc ctcagcttct acagttgtgt cacccatgtg tctgtttctc 4800
   atactgggtc aggaattggg ctattaaata gcatccttca tgaatatgca attaactgag 4860
   gtgactatag tatctccgtt ccctgagagc ctcacccaac aaccacaccc ctcctctgga 4920
   gaagccccta gatcacagct cctcaccatg gactggacct gaaggatcct cttcttgatg 4980
   gcagcagcaa caggtaaggg gctccccagt ctcagggctg aggaagaaac caggccagtc 5040
   atgtgagact tcacccactc ttgtgtccac tccacaggtg cccactccct gcagctggtg 5100
   cagtctgggc ctgaggtgaa gaagcctggg gcctcagtga aggtctccta taagtcttct 5160
   ggttacacct tcaccatcta tggtatgaat tgggtatgat agacccctgg acagggcttt 5220
   gagtggatgt gatggatcat cacctacact gggaacccaa cgtataccca cggcttcaca 5280
   ggatggtttg tcttctccat ggacacgtct gtcagcacgg cgtgtcttca gatcagcagc 5340
   ctaaaggctg aggacacggc cgagtattac tgtgcgaagt acacagtgtg gaaacccaca 5400
   tcccgagagt ttcagaaagc ctgaggaagg aggcagctgt gctgagctga ggcagtggta 5460
   cagcagtttt ctgaacttcc atagtatctc attttgcatt gagttccgct ttaatattag 5520
   ccaagaatat gggatagacg ggtgctccta agagatcctt aacttgccca ttttgatggg 5580
   ttttcccaaa gacgtgagaa gccacttttt tcgcaaagca tcccaaagcc atgccctgct 5640
   ccagaaacac gtgtatccat ttcctggtct ttgattaact gacaaactct catcagcgca 5700
   cctgggctaa tttcacatca ggtagaaata tgtgctttaa agcaaggcta acgttgtaat 5760
   agcaattcct gcttaataac cttcagcatt gttgttgtgt gctccatcaa ctaattacgt 5820
   tagttcaagg ttctcaatgg gagtttctaa taaatataag ggatgtatag aagttcccct 5880
   aattaaaaca attgtgaaga caacctcagt gttcaaccat atttcaaccc ttcaccacaa 5940
   aggaactttc atctctcctg gaagttgggt tcattttcaa attagttttt ttattttaat 6000
   atctcaagat tattgtatgt gactatttta gcagaaagtg aattatggga acttgaacta 6060
   accaactgaa aatacattca gaactaatta aacaagatgc cagaatgtga ttggctccag 6120
   gcattttaaa ttcaacaggt tatgtaacca ggctttaaat ttgcacatct tcgtgttacc 6180
   ttcatgacac agtcaactcc cattatgtaa gaaatggtga gtgcattccc aagggtcttg 6240
   cacagttata aaaatagact tgatgaggtg aggagttgtt taaattcccc tctgaagaag 6300
   cagcatcaac ccaacaaacc actctcttcc ctctgtgact agagctctgt cacaggccac 6360
   atggacctaa atccttgatg gagattacag gactacgtaa attggactga tcgtttttat 6420
   gctgttaaat taataggtga gtctgcactc cagcctgggc aacagaataa tcttgtctgt 6480
   aaaatacaaa agaaagataa attaatagat actgactttg acatttcgga taataatatt 6540
   ttcataaacc gaatttaatt atacccacat tgttacctac accttcactg aaaagttcct 6600
   agttatgttg agttccatca acactccaca tgttcaaatc tggacatcca agagagtcta 6660
   gagaataaaa cgcaatgagg gcagtgaaac ttgcgtatat tcagcacctc ttaactcagg 6720
   aggactcaat acaccctgga acactctgct tttctgaatg gctcacaatg actccagctc 6780
   actctccaac ctcctcaaac atctggcctc tgtttgccct aagttcacgc tctgctctta 6840
   gtctatgttc tgaagtcttt gtagaggtga aaatgagctg tcagatggat cttccttctc 6900
   actgcaacat ggaatttgct atttcactta atgaccactc tttccacaat ggttgatttc 6960
   ttttggcctg ttcattactg gtgattttca agggaatctc agttgaatct ttactgtttt 7020
   gcattttgtc tccatgacaa tgttgggaag tttttcttct agcagcataa catgatctag 7080
   tgacctgaca catttgcagc aaacaatacc tacaaattca gaagctcttt ggtttctttt 7140
   ccacgaaata taattcttgc tcttctgtgt atgagcacat cctagcatcc ctgtacacac 7200
   ccaggtagat gtaaacacgc cgatgaaata ttccctgtaa ataaaaaaag tatctcagtt 7260
   tctctcaatg ttcataattc tcctgagggt gaggaaggta cttctgggtc tgctcaaaca 7320
   aatggcccag agaccacctg gtaggtaggt aaggagctca cctcgctctg gatattgagt 7380
   ctgtctcttt ccctctgtcg tctcatagaa ggccagccca cttgttcagc tcctaagaag 7440
   agagcccagg tttatccaga ttatacaaca caaccagctt ctgatgactc tcctgttaca 7500
   acatccatgg agatattttg tgtattatat aattcaccaa actaatgtga aatgcccaag 7560
   ttgcaatact gcacacccta gggtatgttc ttgcaattca gcggaggaga aattctttca 7620
   gagacagatg gatctgaatt ggtaaatatg tgggtacgaa ttctgggttt gagtgtcatt 7680
   gtccagccat gtttcacagg tgtgacctgt cagggaagaa ccagagttcc ttgttctctc 7740
   agagggtaga gctcacagag gtcctctctg gttcccagga aaggtaattt cactaatctt 7800
   ggtgatgaga ctatcctcca gtgctgatgt actatagagt tttcatctga agctgtcact 7860
   gctatcccca atgtacatct tttcacacag aaatgtttag aggtcaggcc atattctcag 7920
   ggttacacat tgagaaggat ggagatatat tctactacct tctcctgaga tctcacacac 7980
   aatctcaaat ttcaaaaggt ctcagaaggg cagctctcag gtactattta aaaataaccc 8040
   acttcctggg acaggtagca tccttctaac catgatggat gttctgaact acagtacaca 8100
   ttgcatggat ccaggtttgt ctcaattcac tgtgattatt acactcagca gctgtttcaa 8160
   tatgtctgaa ggggtaaatg acaatttagg tgacctgggt gtatggttgg tgttatatga 8220
   atctttaaat gtagaacagt attaactgta ttccaaaatc tgtctttgat ccatgatcac 8280
   acttgtctcc cagaccagct ccttcagcac atttcctacc tggaagaaga ggactctggg 8340
   tttggtgagg ggaggccaca ggaagagaac tgagttctca gagggcacag ccagcataca 8400
   cctcccaggg tgagcccaaa agactggggc ctccctcatc cctttttacc tatccataca 8460
   aaggcaccac ccacatgcaa atcctcactt aggcacccac aggaaatgac tacacatttc 8520
   cttaaattca gggtccagct cacatgggaa gtgctttctg agagtcatgg acctcctgca 8580
   caagaacggt accgggctag agcggccgcc accgcggtgg agctccagct tttgttccct 8640
   ttagtgaggg ttaattgcgc gcttggcgta atcatggtca tagctgtttc ctgtgtgaaa 8700
   ttgttatccg ctcacaattc cacacaacat acgagccgga agcataaagt gtaaagcctg 8760
   gggtgcctaa tgagtgagct aactcacatt aattgcgttg cgctcactgc ccgctttcca 8820
   gtcgggaaac ctgtcgtgcc agctgcatta atgaatcggc caacgcgcgg ggagaggcgg 8880
   tttgcgtatt gggcgctctt ccgcttcctc gctcactgac tcgctgcgct cggtcgttcg 8940
   gctgcggcga gcggtatcag ctcactcaaa ggcggtaata cggttatcca cagaatcagg 9000
   ggataacgca ggaaagaaca tgtgagcaaa aggccagcaa aaggccagga accgtaaaaa 9060
   ggccgcgttg ctggcgtttt tccataggct ccgcccccct gacgagcatc acaaaaatcg 9120
   acgctcaagt cagaggtggc gaaacccgac aggactataa agataccagg cgtttccccc 9180
   tggaagctcc ctcgtgcgct ctcctgttcc gaccctgccg cttaccggat acctgtccgc 9240
   ctttctccct tcgggaagcg tggcgctttc tcatagctca cgctgtaggt atctcagttc 9300
   ggtgtaggtc gttcgctcca agctgggctg tgtgcacgaa ccccccgttc agcccgaccg 9360
   ctgcgcctta tccggtaact atcgtcttga gtccaacccg gtaagacacg acttatcgcc 9420
   actggcagca gccactggta acaggattag cagagcgagg tatgtaggcg gtgctacaga 9480
   gttcttgaag tggtggccta actacggcta cactagaagg acagtatttg gtatctgcgc 9540
   tctgctgaag ccagttacct tcggaaaaag agttggtagc tcttgatccg gcaaacaaac 9600
   caccgctggt agcggtggtt tttttgtttg caagcagcag attacgcgca gaaaaaaagg 9660
   atctcaagaa gatcctttga tcttttctac ggggtctgac gctcagtgga acgaaaactc 9720
   acgttaaggg attttggtca tgagattatc aaaaaggatc ttcacctaga tccttttaaa 9780
   ttaaaaatga agttttaaat caatctaaag tatatatgag taaacttggt ctgacagtta 9840
   ccaatgctta atcagtgagg cacctatctc agcgatctgt ctatttcgtt catccatagt 9900
   tgcctgactc cccgtcgtgt agataactac gatacgggag ggcttaccat ctggccccag 9960
   tgctgcaatg ataccgcgag acccacgctc accggctcca gatttatcag caataaacca 10020
   gccagccgga agggccgagc gcagaagtgg tcctgcaact ttatccgcct ccatccagtc 10080
   tattaattgt tgccgggaag ctagagtaag tagttcgcca gttaatagtt tgcgcaacgt 10140
   tgttgccatt gctacaggca tcgtggtgtc acgctcgtcg tttggtatgg cttcattcag 10200
   ctccggttcc caacgatcaa ggcgagttac atgatccccc atgttgtgca aaaaagcggt 10260
   tagctccttc ggtcctccga tcgttgtcag aagtaagttg gccgcagtgt tatcactcat 10320
   ggttatggca gcactgcata attctcttac tgtcatgcca tccgtaagat gcttttctgt 10380
   gactggtgag tactcaacca agtcattctg agaatagtgt atgcggcgac cgagttgctc 10440
   ttgcccggcg tcaatacggg ataataccgc gccacatagc agaactttaa aagtgctcat 10500
   cattggaaaa cgttcttcgg ggcgaaaact ctcaaggatc ttaccgctgt tgagatccag 10560
   ttcgatgtaa cccactcgtg cacccaactg atcttcagca tcttttactt tcaccagcgt 10620
   ttctgggtga gcaaaaacag gaaggcaaaa tgccgcaaaa aagggaataa gggcgacacg 10680
   gaaatgttga atactcatac tcttcctttt tcaatattat tgaagcattt atcagggtta 10740
   ttgtctcatg agcggataca tatttgaatg tatttagaaa aataaacaaa taggggttcc 10800
   gcgcacattt ccccgaaaag tgccac 10826
<210> 88
   <211> 42
   <212> DNA
   <213> Artificial Sequence
<220>
<223> Oligonucleotide Primer
<400> 88
   gacggtatcg ataagcttga tatcgaattc ctgcagcccg gg 42
<210> 89
   <211> 42
   <212> DNA
   <213> Artificial Sequence
<220>
<223> Oligonucleotide primer
<400> 89
   gacctcctgc acaagaacgg taccgggcta gagcggccgc ca 42
<210> 90
   <211> 25
   <212> DNA
   <213> Artificial Sequence
<220>
<223> Oligonucleotide primer
<400> 90
   cagtgctgca atgataccgc gagac 25
<210> 91
   <211> 13722
   <212> DNA
   <213> Artificial Sequence
<220>
<223> Plasmid KW2
<400> 91
   cagtgtggtt ttgcaagagg aagcaaaaag cctctccacc caggcctgga atgtttccac 60
   ccaatgtcga gcagtgtggt tttgcaagag gaagcaaaaa gcctctccac ccaggcctgg 120
   aatgtttcca cccaatgtcg agcaaacccc gcccagcgtc ttgtcattgg cgaattcgaa 180
   cacgcagatg cagtcggggc ggcgcggtcc caggtccact tcgcatatta aggtgacgcg 240
   tgtggcctcg aacaccgagc gaccctgcag ccaatatggg atcggccatt gaacaagatg 300
   gattgcacgc aggttctccg gccgcttggg tggagaggct attcggctat gactgggcac 360
   aacagacaat cggctgctct gatgccgccg tgttccggct gtcagcgcag gggcgcccgg 420
   ttctttttgt caagaccgac ctgtccggtg ccctgaatga actgcaggac gaggcagcgc 480
   ggctatcgtg gctggccacg acgggcgttc cttgcgcagc tgtgctcgac gttgtcactg 540
   aagcgggaag ggactggctg ctattgggcg aagtgccggg gcaggatctc ctgtcatctc 600
   accttgctcc tgccgagaaa gtatccatca tggctgatgc aatgcggcgg ctgcatacgc 660
   ttgatccggc tacctgccca ttcgaccacc aagcgaaaca tcgcatcgag cgagcacgta 720
   ctcggatgga agccggtctt gtcgatcagg atgatctgga cgaagagcat caggggctcg 780
   cgccagccga actgttcgcc aggctcaagg cgcgcatgcc cgacggcgag gatctcgtcg 840
   tgacccatgg cgatgcctgc ttgccgaata tcatggtgga aaatggccgc ttttctggat 900
   tcatcgactg tggccggctg ggtgtggcgg accgctatca ggacatagcg ttggctaccc 960
   gtgatattgc tgaagagctt ggcggcgaat gggctgaccg cttcctcgtg ctttacggta 1020
   tcgccgctcc cgattcgcag cgcatcgcct tctatcgcct tcttgacgag ttcttctgag 1080
   gggatcggca ataaaaagac agaataaaac gcacgggtgt tgggtcgttt gttcggatcc 1140
   gtcgacttta tcatcatcat ctttataatc tttatcatca tcatctttat aatcgttatg 1200
   tcctaatttc gaaggcactt gggagtagct agccaccaca gactcgtgct gctcaacggt 1260
   cttccaatcg tcacttatgt tcagcttttc catacgcaaa tgatgaaagt gaagttctgg 1320
   aagctttacc tctttcttcg acttgtaagt tgtttccatg aagcaagaaa gatgacctcc 1380
   gtcagccagc ataagtgcgg gggtatcgcg aagcagaagt ccaccatctc gtgggatgac 1440
   tgtttcagtt gatggctccc atccacaggt cttcttttgc atcactggac cgtttgcggg 1500
   aaaattggtg ccaaggactt taactttgca gatgatgcaa tcaccctgca aacttgtttc 1560
   ttgggtagct gaaagcactc ctccatcttc ataggtgctt actctttccc aagaaaaacc 1620
   accaggtaga gactgcttaa agaagtcagg aatttctttg gggtacttgg cgaagacctt 1680
   gatgccatac tgaaaggcgt gtgacagaat gtcaaaagca aatggcagag gacctccttc 1740
   agttattgta atattcaggc tttgggtgcc ttcgtatggt tttccctctc cttttccagt 1800
   gcacttgaag gcgtggtagt taacactacc ctccatagaa agctgaacgc gcatggtttc 1860
   cttgatggaa ggatacatgg taccatgact ctcagaaagc acttcccatg tgagctggac 1920
   cctgaattta aggaaatgtg tagtcatttc ctgtgggtgc ctaagtgagg atttgcatgt 1980
   gggtggtgcc tttgtatgga taggtaaaaa gggatgaggg aggccccagt cttttgggct 2040
   caccctggga ggtgtatgct ggctgtgccc tctgagaact cagttctctt cctgtggcct 2100
   cccctcacca aacccagagt cctcttcttc caggtaggaa atgtgctgaa ggagctggtc 2160
   tgggagacaa gtgtgatcat ggatcaaaga cagattttgg aatacagtta atactgttct 2220
   acatttaaag attcatataa caccaaccat acacccaggt cacctaaatt gtcatttacc 2280
   ccttcagaca tattgaaaca gctgctgagt gtaataatca cagtgaattg agacaaacct 2340
   ggatccatgc aatgtgtact gtagttcaga acatccatca tggttagaag gatgctacct 2400
   gtcccaggaa gtgggttatt tttaaatagt acctgagagc tgcccttctg agaccttttg 2460
   aaatttgaga ttgtgtgtga gatctcagga gaaggtagta gaatatatct ccatccttct 2520
   caatgtgtaa ccctgagaat atggcctgac ctctaaacat ttctgtgtga aaagatgtac 2580
   attggggata gcagtgacag cttcagatga aaactctata gtacatcagc actggaggat 2640
   agtctcatca ccaagattag tgaaattacc tttcctggga accagagagg acctctgtga 2700
   gctctaccct ctgagagaac aaggaactct ggttcttccc tgacaggtca cacctgtgaa 2760
   acatggctgg acaatgacac tcaaacccag aattcgtacc cacatattta ccaattcaga 2820
   tccatctgtc tctgaaagaa tttctcctcc gctgaattgc aagaacatac cctagggtgt 2880
   gcagtattgc aacttgggca tttcacatta gtttggtgaa ttatataata cacaaaatat 2940
   ctccatggat gttgtaacag gagagtcatc agaagctggt tgtgttgtat aatctggata 3000
   aacctgggct ctcttcttag gagctgaaca agtgggctgg ccttctatga gacgacagag 3060
   ggaaagagac agactcaata tccagagcga ggtgagctcc ttacctacct accaggtggt 3120
   ctctgggcca tttgtttgag cagacccaga agtaccttcc tcaccctcag gagaattatg 3180
   aacattgaga gaaactgaga tacttttttt atttacaggg aatatttcat cggcgtgttt 3240
   acatctacct gggtgtgtac agggatgcta ggatgtgctc atacacagaa gagcaagaat 3300
   tatatttcgt ggaaagaaaa ccaaagagct tctgaatttg taggtattgt ttgctgcaaa 3360
   tgtgtcaggt cactagatca tgttatgctg ctagaagaaa aacttcccaa cattgtcatg 3420
   gagacaaaat gcaaaacagt aaagattcaa ctgagattcc cttgaaaatc accagtaatg 3480
   aacaggccaa aagaaatcaa ccattgtgga aagagtggtc attaagtgaa atagcaaatt 3540
   ccatgttgca gtgagaagga agatccatct gacagctcat tttcacctct acaaagactt 3600
   cagaacatag actaagagca gagcgtgaac ttagggcaaa cagaggccag atgtttgagg 3660
   aggttggaga gtgagctgga gtcattgtga gccattcaga aaagcagagt gttccagggt 3720
   gtattgagtc ctcctgagtt aagaggtgct gaatatacgc aagtttcact gccctcattg 3780
   cgttttattc tctagactct cttggatgtc cagatttgaa catgtggagt gttgatggaa 3840
   ctcaacataa ctaggaactt ttcagtgaag gtgtaggtaa caatgtgggt ataattaaat 3900
   tcggtttatg aaaatattat tatccgaaat gtcaaagtca gtatctatta atttatcttt 3960
   cttttgtatt ttacagacaa gattattctg ttgcccaggc tggagtgcag actcacctat 4020
   taatttaaca gcataaaaac gatcagtcca atttacgtag tcctgtaatc tccatcaagg 4080
   atttaggtcc atgtggcctg tgacagagct ctagtcacag agggaagaga gtggtttgtt 4140
   gggttgatgc tgcttcttca gaggggaatt taaacaactc ctcacctcat caagtctatt 4200
   tttataactg tgcaagaccc ttgggaatgc actcaccatt tcttacataa tgggagttga 4260
   ctgtgtcatg aaggtaacac gaagatgtgc aaatttaaag cctggttaca taacctgttg 4320
   aatttaaaat gcctggagcc aatcacattc tggcatcttg tttaattagt tctgaatgta 4380
   ttttcagttg gttagttcaa gttcccataa ttcactttct gctaaaatag tcacatacaa 4440
   taatcttgag atattaaaat aaaaaaacta atttgaaaat gaacccaact tccaggagag 4500
   atgaaagttc ctttgtggtg aagggttgaa atatggttga acactgaggt tgtcttcaca 4560
   attgttttaa ttaggggaac ttctatacat cccttatatt tattagaaac tcccattgag 4620
   aaccttgaac taacgtaatt agttgatgga gcacacaaca acaatgctga aggttattaa 4680
   gcaggaattg ctattacaac gttagccttg ctttaaagca catatttcta cctgatgtga 4740
   aattagccca ggtgcgctga tgagagtttg tcagttaatc aaagaccagg aaatggatac 4800
   acgtgtttct ggagcagggc atggctttgg gatgctttgc gaaaaaagtg gcttctcacg 4860
   tctttgggaa aacccatcaa aatgggcaag ttaaggatct cttaggagca cccgtctatc 4920
   ccatattctt ggctaatatt aaagcggaac tcaatgcaaa atgagatact atggaagttc 4980
   agaaaactgc tgtaccactg cctcagctca gcacagctgc ctccttcctc aggctttctg 5040
   aaactctcgg gatgtgggtt tccacactgt gtacttcgca cagtaatact cggccgtgtc 5100
   ctcagccttt aggctgctga tctgaagaca cgccgtgctg acagacgtgt ccatggagaa 5160
   gacaaaccat cctgtgaagc cgtgggtata cgttgggttc ccagtgtagg tgatgatcca 5220
   tcacatccac tcaaagccct gtccaggggt ctatcatacc caattcatac catagatggt 5280
   gaaggtgtaa ccagaagact tataggagac cttcactgag gccccaggct tcttcacctc 5340
   aggcccagac tgcaccagct gcagggagtg ggcacctgtg gagtggacac aagagtgggt 5400
   gaagtctcac atgactggcc tggtttcttc ctcagccctg agactgggga gccccttacc 5460
   tgttgctgct gccatcaaga agaggatcct tcaggtccag tccatggtga ggagctgtga 5520
   tctaggggct tctccagagg aggggtgtgg ttgttgggtg aggctctcag ggaacggaga 5580
   tactatagtc acctcagtta attgcatatt catgaaggat gctatttaat agcccaattc 5640
   ctgacccagt atgagaaaca gacacatggg tgacacaact gtagaagctg agggttcaag 5700
   ccgtaatcct gttagaggcc atgtgtcccc tacacatccc tgaactctgt gttgacagag 5760
   cttcccccac tggagaacaa gctcctcaag gacagcacct cactttgaaa ccacatttga 5820
   ctgtctcagg gtcaacttgc atcatttcta gaccataata tgtgaatgcg ttatttaggg 5880
   aatgactgtg tttatccaaa aattgcattt atttataaga aaggatctct tcctgacctc 5940
   cagcagagtt tgaaatcccc attgtaaaag tggttctcat tacaacatcc agtttgataa 6000
   atgctcacaa ttgaatagat atttatacaa acttcagcag tctttgtgaa atacttattt 6060
   tagatatttt taaaggaagt cccaggccct gggaggaacc tctccccagc ctcctgtgca 6120
   cctgctctgg ggcgggagcc tgtgctgggt gtatccggag cgccccctgc agcccagccc 6180
   caaccatgca gggaggtttc tatctgagct gacagagtat attcctacca gtgtatccag 6240
   ctcagtataa agtggttgtg ccctggctca gaattctcct ttagggacac cacatgctcc 6300
   tcacaccatc ttttgaaata gtgaattgcc tttaggaaac ccagtgaact ctgcagagag 6360
   actccaagaa aagatctcat gcatcaccag ggagcccttt cctggagctc aagaggcaca 6420
   gaatcattgg acacacggtg aacccaaaca ctcttcaggg gttgagggga gactcttatt 6480
   tcctttaggg tcctacagtt gattatggca cctgagaata cctgcaggtg caggtacatg 6540
   tggatagaaa cccactccaa ctctgctatt caactcacac atgcgcgcgc gtgtgtacac 6600
   acacacacac acacacacac acatatatat atatacatcg tggctaattt ttatattaac 6660
   ggatcccatg tttgccattt ttttctggta tccatctcat ggaaagcgct ccctacactg 6720
   gtactaagac tgaatatgtg tctactttct gtaaacagaa gtaaagaaac agaatacaag 6780
   tggacacttg ggaagtgcat gcacattgaa ttcacctggt ctcactttgg aaccctgcag 6840
   atgccccgtg aaaactaaat tatcgattgc cagccctggg accgaacccc gcgtttatga 6900
   acaaacgacc caacacccgt gcgttttatt ctgtcttttt attgccgtca tagcgcgggt 6960
   tccttccggt attgtctcct tccgtgtttc agttagcctc ccccatctcc cgggcaaacg 7020
   tgcgcgccag gtcgcagatc gtcggtatgg agcctggggt ggtgacgtgg gtctggacca 7080
   tcccggaggt aagttgcagc agggcgtccc ggtagccggc gggcgattgg tcgtaatcca 7140
   ggataaagac gtgcatggga cggaggcgtt tggccaagac gtccaaggcc caggcaaaca 7200
   cgttatacag gtcgccgttg ggggccagca actcgggggc ccgaaacagg gtaaataacg 7260
   tgtccccgat atggggtcgt gggcccgcgt tgctctgggg ctcggcaccc tggggcggca 7320
   cggccgtccc cgaaagctgt ccccaatcct cccgccacga cccgccgccc tgcagatacc 7380
   gcaccgtatt ggcaagcagc ccgtaaacgc ggcgaatcgc ggccagcata gccaggtcaa 7440
   gccgctcgcc ggggcgctgg cgtttggcca ggcggtcgat gtgtctgtcc tccggaaggg 7500
   cccccaacac gatgtttgtg ccgggcaagg tcggcgggat gagggccacg aacgccagca 7560
   cggcctgggg ggtcatgctg cccataaggt atcgcgcggc cgggtagcac aggagggcgg 7620
   cgatgggatg gcggtcgaag atgagggtga gggccggggg cggggcatgt gagctcccag 7680
   cctccccccc gatatgagga gccagaacgg cgtcggtcac ggcataaggc atgcccattg 7740
   ttatctgggc gcttgtcatt accaccgccg cgtccccggc cgatatctca ccctggtcga 7800
   ggcggtgttg tgtggtgtag atgttcgcga ttgtctcgga agcccccagc acctgccagt 7860
   aagtcatcgg ctcgggtacg tagacgatat cgtcgcgcga acccagggcc accagcagtt 7920
   gcgtggtggt ggttttcccc atcccgtgag gaccgtctat ataaacccgc agtagcgtgg 7980
   gcattttctg ctccaggcgg acttccgtgg cttcttgctg ccggcgaggg cgcaacgccg 8040
   tacgtcggtt gctatggccg cgagaacgcg cagcctggtc gaacgcagac gcgtgttgat 8100
   ggcaggggta cgaagccata cgcgcttcta caaggcgctt gccgaagagg tgcgggagtt 8160
   tcacgccacc aagatctgcg gcacgctgtt gacgctgtta agcgggtcgc tgcaggtcga 8220
   aaggcccgga gatgaggaag aggagaacag cgcggcagac gtgcgctttt gaagcgtgca 8280
   gaatgccggg cctccggagg accttcgggc gcccgccccg cccctgagcc cgcccctgag 8340
   cccgcccccg gacccacccc ttcccagcct ctgagcccag aaagcgaagg agcaaagctg 8400
   ctattggccg ctgccccaaa ggcctacccg cttccattgc tcagcggtgc tgtccatctg 8460
   cacgagacta gtgagacgtg ctacttccat ttgtcacgtc ctgcacgacg cgagctgcgg 8520
   ggcggggggg aacttcctga ctaggggagg agtagaaggt ggcgcgaagg ggccaccaaa 8580
   gaacggagcc ggttggcgcc taccggtgga tgtggaatgt gtgcgaggcc agaggccact 8640
   tgtgtagcgc caagtgccca gcggggctgc taaagcgcat gctccagact gcaagcttgg 8700
   cgtaatcatg gtcatagctg tttcctgtgt gaaattgtta tccgctcaca attccacaca 8760
   acatacgagc cggaagcata aagtgtaaag cctggggtgc ctaatgagtg aggtaactca 8820
   cattaattgc gttgcgctca ctgcccgctt tccagtcggg aaacctgtcg tgccagctgc 8880
   attaatgaat cggccaacgc gcggggagag gcggtttgcg tattggcgct cttccgcttc 8940
   ctcgctcact gactcgctgc gctcggtcgt tcggctgcgg cgagcggtat cagctcactc 9000
   aaaggcggta atacggttat ccacagaatc aggggataac gcaggaaaga acatgtgagc 9060
   aaaaggccag caaaaggcca ggaaccgtaa aaaggccgcg ttgctggcgt ttttccatag 9120
   gctccgcccc cctgacgagc atcacaaaaa tcgacgctca agtcagaggt ggcgaaaccc 9180
   gacaggacta taaagatacc aggcgtttcc ccctggaagc tccctcgtgc gctctcctgt 9240
   tccgaccctg ccgcttaccg gatacctgtc cgcctttctc ccttcgggaa gcgtggcgct 9300
   ttctcaatgc tcacgctgta ggtatctcag ttcggtgtag gtcgttcgct ccaagctggg 9360
   ctgtgtgcac gaaccccccg ttcagcccga ccgctgcgcc ttatccggta actatcgtct 9420
   tgagtccaac ccggtaagac acgacttatc gccactggca gcagccactg gtaacaggat 9480
   tagcagagcg aggtatgtag gcggtgctac agagttcttg aagtggtggc ctaactacgg 9540
   ctacactaga aggacagtat ttggtatctg cgctctgctg aagccagtta ccttcggaaa 9600
   aagagttggt agctcttgat ccggcaaaca aaccaccgct ggtagcggtg gtttttttgt 9660
   ttgcaagcag cagattacgc gcagaaaaaa aggatctcaa gaagatcctt tgatcttttc 9720
   tacggggtct gacgctcagt ggaacgaaaa ctcacgttaa gggattttgg tcatgagatt 9780
   atcaaaaagg atcttcacct agatcctttt aaattaaaaa tgaagtttta aatcaatcta 9840
   aagtatatat gagtaaactt ggtctgacag ttaccaatgc ttaatcagtg aggcacctat 9900
   ctcagcgatc tgtctatttc gttcatccat agttgcctga ctccccgtcg tgtagataac 9960
   tacgatacgg gagggcttac catctggccc cagtgctgca atgataccgc gagacccacg 10020
   ctcaccggct ccagatttat cagcaataaa ccagccagcc ggaagggccg agcgcagaag 10080
   tggtcctgca actttatccg cctccatcca gtctattaat tgttgccggg aagctagagt 10140
   aagtagttcg ccagttaata gtttgcgcaa cgttgttgcc attgctacag gcatcgtggt 10200
   gtcacgctcg tcgtttggta tggcttcatt cagctccggt tcccaacgat caaggcgagt 10260
   tacatgatcc cccatgttgt gcaaaaaagc ggttagctcc ttcggtcctc cgatcgttgt 10320
   cagaagtaag ttggccgcag tgttatcact catggttatg gcagcactgc ataattctct 10380
   tactgtcatg ccatccgtaa gatgcttttc tgtgactggt gagtactcaa ccaagtcatt 10440
   ctgagaatag tgtatgcggc gaccgagttg ctcttgcccg gcgtcaatac gggataatac 10500
   cgcgccacat agcagaactt taaaagtgct catcattgga aaacgttctt cggggcgaaa 10560
   actctcaagg atcttaccgc tgttgagatc cagttcgatg taacccactc gtgcacccaa 10620
   ctgatcttca gcatctttta ctttcaccag cgtttctggg tgagcaaaaa caggaaggca 10680
   aaatgccgca aaaaagggaa taagggcgac acggaaatgt tgaatactca tactcttcct 10740
   ttttcaatat tattgaagca tttatcaggg ttattgtctc atgagcggat acatatttga 10800
   atgtatttag aaaaataaac aaataggggt tccgcgcaca tttccccgaa aagtgccacc 10860
   tgacgtcgcc cagcccagcc tagctcagcc cagcccagct caattcagcc cagccctgcc 10920
   cagctcagcc caccttaatg cagccaagcc cagctcaact cagctcacct ggtgcaactt 10980
   agcccagctc agctcagctc agctcagccc agttcaactc agcccagttc agctcagctc 11040
   agcccagttc ggccttgttt agtctaggtc aacttaggtc agttttgccc atctgagtcc 11100
   atttctgaaa actggatgga gttgtcatgg ccagaaatgg tcagcccacc agacctgctt 11160
   gtctcagcta aagccatctc attgccgggt tcctgcacag ccaggctggc ttccatcttt 11220
   tgtctccctc tacttgatac cccagttccc tgcagtcctg ccccagcgcc acctgggttt 11280
   tggttccaaa gcattaccaa tcattaccac cctccactac ctgggtggaa tatttctttg 11340
   ctgctttaaa gtcattaaaa catcttgaga atgagaccaa gaatttagga gcctgtgctg 11400
   tgataaaaat gagcaggtcc ccttgctcta gaagtggcag catatcttct gcaccaagag 11460
   gagggtattg agatgctcag agcctccacc ttcccggagc atcccctccc ttctgagtct 11520
   gcagtaaacc cctgccttta aattccctct agataacagt catcattgga aacaaccaag 11580
   aaatgcattt tatctgaatt tgccacttaa aattctgcca tttaccataa atcgctttgg 11640
   aaggcatggg ctactttcaa gggtgcgatg atgacctaca gtcaatgact tagacaaggg 11700
   cgatgccagt gggacttggt atgttctcaa gcatcattac ccatgccatc cccattcaga 11760
   ggttgtggag cagctcgtgc gacctctcct tcaaatgggc tttagggaaa gttaaatggg 11820
   agtgacccag acaatggtca ctcaaaagac tcacataaat gagtctcctg ctcttcatca 11880
   agcaattaag accagttccc cttctagtgg aaataagacg tcaaatacaa agttttaaga 11940
   gaagcaaatg cagcagcggc ggctgcctgt ctcttaccat gtcgggcgcc tggtcactgc 12000
   gagccttgca aagctttggc atggaatcat tcctccaagt ccattaacaa gggctggggc 12060
   ctgagcagcc agtcggcccg gcagcagaag ccacgcatcc cagctctggg tagtccgggg 12120
   agacccagag cccaggccgg gcctggcagc caccctccca gagcctccgc taggccagtc 12180
   ctgctgacgc cgcatcggtg attcggaaca gaatctgtcc ttctaaggtg tctccacagt 12240
   cctgtcttca gcactatctg attgagtttt ctcttatgcc accaactaac atgcttaact 12300
   gaaataattc aggataatga tgcacatttt tcctaaaact tatcctaaag tgagtagttg 12360
   aaaagtggtc ttgaaaaata ctaaaatgaa ggccactcta tcagaatatc aaagtgtttc 12420
   tccttaatca caaagagaaa acgagttaac ctaaaaagat tgtgaacaca gtcattatga 12480
   aaataatgct ctgaggtatc gaaaaagtat ttgagattaa ttatcacatg aagggataac 12540
   aagctaattt aaaaaacttt ttgaatacag tcataaactc tccctaagac tgtttaattt 12600
   cttaaacatc ttactttaaa aatgaatgca gtttagaagt tgatatgctg tttgcacaaa 12660
   ctagcagttg ataagctaag attggaaatg aaattcagat agttaaaaaa agccttttca 12720
   gtttcggtca gcctcgcctt attttagaaa cgcaaattgt ccaggtgttg ttttgctcag 12780
   tagagcactt tcagatctgg gcctgggcaa aaccacctct tcacaaccag aagtgataaa 12840
   tttaccaatt gtgttttttt gcttcctaaa atagactctc gcggtgacct gcttcctgcc 12900
   acctgctgtg ggtgccggag acccccatgc agccatcttg actctaattc atcatctgct 12960
   tccagcttcg ctcaattaat taaaaaaata aacttgattt atgatggtca aaacgcagtc 13020
   ccgcatcggg gccgacagca ctgtgctagt atttcttagc tgagcttgct ttggcctcaa 13080
   ttccagacac atatcactca tgggtgttaa tcaaatgata agaatttcaa atacttggac 13140
   agttaaaaaa attaatatac ttgaaaatct ctcacatttt taagtcataa ttttcttaac 13200
   catttttctc agaagccact tcaaacatat cctgtctttt aacagtaagc atgcctccta 13260
   agataaacaa tccttttctc atggaaacca gcttcaaggc actgaggtcc tggagcctcc 13320
   ctaagcccct gtcaggacgg cagccaccgt ttctgggcta cccctgcccc caaccctgct 13380
   ctcatcaaga ccggggctac gcgtccctcc tggctggatt cacccactcc gacagttctc 13440
   tttccagcca ataaagaatt taagatgcag gttgacacac agcgcacctc ataattctaa 13500
   agaaaatatt tcacgattcg ctgctgtgca gcgatcttgc agtcctacag acaccgctcc 13560
   tgagacacat tcctcagcca tcactaagac ccctggtttg ttcaggcatc tcgtccaaat 13620
   gtggctcccc aagcccccag gctcagttac tccatcagac gcacccaacc tgagtcccat 13680
   tttccaaagg catcggaaaa tccacagagg ctcccactcg ag 13722
<210> 92
   <211> 33
   <212> DNA
   <213> Artificial Sequence
<220>
<223> Oligonucleotide primer
<400> 92
   ccgctcgaga tgtatccttc catcaaggaa acc 33
<210> 93
   <211> 69
   <212> DNA
   <213> Artificial Sequence
<220>
<223> Oligonucleotide primer
<400> 93
   tctagattat tatttatcat catcatcttt ataatcttta tcatcatcat ctttataatc 60
   agcggccgc 69
<210> 94
   <211> 31
   <212> DNA
   <213> Artificial Sequence
<220>
<223> Oligonucleotide primer
<400> 94
   ctagtctaga ttattattta tcatcatcat c 31
<210> 95
   <211> 33
   <212> DNA
   <213> Artificial Sequence
<220>
<223> Oligonucleotide primer
<400> 95
   gttatgtcct aatttcgaag gcacttggga gta 33
<210> 96
   <211> 30
   <212> PRT
   <213> Homo sapiens
<400> 96
   Met Lys Thr Pro Trp Lys Val Leu Leu Gly Leu Leu Gly Ala Ala Ala
   1 5 10 15
   Leu Val Thr Ile Ile Thr Val Pro Val Val Leu Leu Asn Lys
   20 25 30
<210> 97
   <211> 90 <212> DNA
   <213> Homo sapiens
<400> 97
   atgaagacac cgtggaaggt tctcctggga ctgctgggtg ctgctgcgct tgtcaccatc 60
   atcaccgtgc ccgtggttct gctgaacaaa 90
<210> 98
   <211> 50
   <212> DNA
   <213> Artificial Sequence
<220>
<223> Oligonucleotide primer
<400> 98
   cgtgcccgtg gttctgctga acaaaatgta tccttccatc aaggaaacca 50
<210> 99
   <211> 51
   <212> DNA
   <213> Artificial Sequence
<220>
<223> Oligonucleotide primer
<400> 99
   ccgtgcccgt ggttctgctg aacaaagtgt atccttccat caaggaaacc a 51
<210> 100
   <211> 52
   <212> DNA
   <213> Artificial Sequence
<220>
<223> Oligonucleotide primer
<400> 100
   tgctgctgcg cttgtcacca tcatcaccgt gcccgtggtt ctgctgaaca aa 52
<210> 101
   <211> 51
   <212> DNA
   <213> Artificial Sequence
<220>
<223> Oligonucleotide primer
<400> 101
   ggaaggttct cctgggactg ctgggtgctg ctgcgcttgt caccatcatc a 51
<210> 102
   <211> 47
   <212> DNA
   <213> Artificial Sequence
<220>
<223> Oligonucleotide primer
<400> 102
   ccggaattca tgaagacacc gtggaaggtt ctcctgggac tgctggg 47
<210> 103
   <211> 49
   <212> DNA
   <213> Artificial Sequence
<220>
<223> Oligonucleotide primer
<400> 103
   gactagttta ttatttatca tcatcatctt tataatcttt atcatcatc 49
<210> 104
   <211> 3735 <212> DNA
   <213> Artificial Sequence
<220>
<223> Plasmid pME18sCD26asfp499
<400> 104
   aagcttggct gtggaatgtg tgtcagttag ggtgtggaaa gtccccaggc tccccagcag     60
   gcagaagtat gcaaagcatg catctcaatt agtcagcaac catagtcccg cccctaactc    120
   cgcccatccc gcccctaact ccgcccagtt ccgcccattc tccgccccat ggctgactaa    180
   ttttttttat ttatgcagag gccgaggccg cctcggcctc tgagctattc cagaagtagt    240
   gaggaggctt ttttggaggc ctaggctttt gcaaaaagct cctcgatcga ggggctcgca    300
   tctctccttc acgcgcccgc cgccctacct gaggccgcca tccacgccgg ttgagtcgcg    360
   ttctgccgcc tcccgcctgt ggtgcctcct gaactgcgtc cgccgtctag gtaagtttaa    420
   agctcaggtc gagaccgggc ctttgtccgg cgctcccttg gagcctacct agactcagcc    480
   ggctctccac gctttgcctg accctgcttg ctcaactcta cgtctttgtt tcgttttctg    540
   ttctgcgccg ttacagatcc aagctctgaa aaaccagaaa gttaactggt aagtttagtc    600
   tttttgtctt ttatttcagg tcccggatcc ggtggtggtg caaatcaaag aactgctcct    660
   cagtggatgt tgcctttact tctaggcctg tacggaagtg ttacttctgc tctaaaagct    720
   gcggaattca tgaagacacc gtggaaggtt ctcctgggac tgctgggtgc tgctgcgctt    780
   gtcaccatca tcaccgtgcc cgtggttctg ctgaacaaaa tgtatccttc catcaaggaa    840
   accatgcgcg ttcagctttc tatggagggt agtgttaact accacgcctt caagtgcact    900
   ggaaaaggag agggaaaacc atacgaaggc acccaaagcc tgaatattac aataactgaa    960
   ggaggtcctc tgccatttgc ttttgacatt ctgtcacacg cctttcagta tggcatcaag   1020
   gtcttcgcca agtaccccaa agaaattcct gacttcttta agcagtctct acctggtggt   1080
   ttttcttggg aaagagtaag cacctatgaa gatggaggag tgctttcagc tacccaagaa   1140
   acaagtttgc agggtgattg catcatctgc aaagttaaag tccttggcac caattttccc   1200
   gcaaacggtc cagtgatgca aaagaagacc tgtggatggg agccatcaac tgaaacagtc   1260
   atcccacgag atggtggact tctgcttcgc gatacccccg cacttatgct ggctgacgga   1320
   ggtcatcttt cttgcttcat ggaaacaact tacaagtcga agaaagaggt aaagcttcca   1380
   gaacttcact ttcatcattt gcgtatggaa aagctgaaca taagtgacga ttggaagacc   1440
   gttgagcagc acgagtctgt ggtggctagc tactcccaag tgccttcgaa attaggacat   1500
   aacgcggccg ctgattataa agatgatgat gataaagatt ataaagatga tgatgataaa   1560
   taataaacta gtctagagaa aaaacctccc acacctcccc ctgaacctga aacataaaat   1620
   gaatgcaatt gttgttgtta acttgtttat tgcagcttat aatggttaca aataaagcaa   1680
   tagcatcaca aatttcacaa ataaagcatt tttttcactg cattctagtt gtggtttgtc   1740
   caaactcatc aatgtatctt atcatgtctg gatccccggg taccgagctc gaattaattc   1800
   ctcttccgct tcctcgctca ctgactcgct gcgctcggtc gttcggctgc ggcgagcggt 1860
   atcagctcac tcaaaggcgg taatacggtt atccacagaa tcaggggata acgcaggaaa 1920
   gaacatgtga gcaaaaggcc agcaaaaggc caggaaccgt aaaaaggccg cgttgctggc 1980
   gtttttccat aggctccgcc cccctgacga gcatcacaaa aatcgacgct caagtcagag 2040
   gtggcgaaac ccgacaggac tataaagata ccaggcgttt ccccctggaa gctccctcgt 2100
   gcgctctcct gttccgaccc tgccgcttac cggatacctg tccgcctttc tcccttcggg 2160
   aagcgtggcg ctttctcata gctcacgctg taggtatctc agttcggtgt aggtcgttcg 2220
   ctccaagctg ggctgtgtgc acgaaccccc cgttcagccc gaccgctgcg ccttatccgg 2280
   taactatcgt cttgagtcca acccggtaag acacgactta tcgccactgg cagcagccac 2340
   tggtaacagg attagcagag cgaggtatgt aggcggtgct acagagttct tgaagtggtg 2400
   gcctaactac ggctacacta gaagaacagt atttggtatc tgcgctctgc tgaagccagt 2460
   taccttcgga aaaagagttg gtagctcttg atccggcaaa caaaccaccg ctggtagcgg 2520
   tggttttttt gtttgcaagc agcagattac gcgcagaaaa aaaggatctc aagaagatcc 2580
   tttgatcttt tctacggggt ctgacgctca gtggaacgaa aactcacgtt aagggatttt 2640
   ggtcatgaga ttatcaaaaa ggatcttcac ctagatcctt ttaaattaaa aatgaagttt 2700
   taaatcaatc taaagtatat atgagtaaac ttggtctgac agttaccaat gcttaatcag 2760
   tgaggcacct atctcagcga tctgtctatt tcgttcatcc atagttgcct gactccccgt 2820
   cgtgtagata actacgatac gggagggctt accatctggc cccagtgctg caatgatacc 2880
   gcgagaccca cgctcaccgg ctccagattt atcagcaata aaccagccag ccggaagggc 2940
   cgagcgcaga agtggtcctg caactttatc cgcctccatc cagtctatta attgttgccg 3000
   ggaagctaga gtaagtagtt cgccagttaa tagtttgcgc aacgttgttg ccattgctac 3060
   aggcatcgtg gtgtcacgct cgtcgtttgg tatggcttca ttcagctccg gttcccaacg 3120
   atcaaggcga gttacatgat cccccatgtt gtgcaaaaaa gcggttagct ccttcggtcc 3180
   tccgatcgtt gtcagaagta agttggccgc agtgttatca ctcatggtta tggcagcact 3240
   gcataattct cttactgtca tgccatccgt aagatgcttt tctgtgactg gtgagtactc 3300
   aaccaagtca ttctgagaat agtgtatgcg gcgaccgagt tgctcttgcc cggcgtcaat 3360
   acgggataat accgcgccac atagcagaac tttaaaagtg ctcatcattg gaaaacgttc 3420
   ttcggggcga aaactctcaa ggatcttacc gctgttgaga tccagttcga tgtaacccac 3480
   tcgtgcaccc aactgatctt cagcatcttt tactttcacc agcgtttctg ggtgagcaaa 3540
   aacaggaagg caaaatgccg caaaaaaggg aataagggcg acacggaaat gttgaatact 3600
   catactcttc ctttttcaat attattgaag catttatcag ggttattgtc tcatgagcgg 3660
   atacatattt gaatgtattt agaaaaataa acaaataggg gttccgcgca catttccccg 3720
   aaaagtgcca cctgc 3735
<210> 105
   <211> 28
   <212> DNA
   <213> Artificial Sequence
<220>
<223> Oligonucleotide primer
<400> 105
   atggacagcc tcttgatgaa ccggagga 28
<210> 106
   <211> 25
   <212> DNA
   <213> Artificial Sequence
<220>
<223> Oligonucleotide primer
<400> 106
   caaagtccca aagtacgaaa tgcgt 25
<210> 107
   <211> 596
   <212> DNA
   <213> Homo sapiens
<400> 107
   atggacagcc tcttgatgaa ccggaggaag tttctttacc aattcaaaaa tgtccgctgg      60
   gctaagggtc ggcgtgagac ctacctgtgc tacgtagtga agaggcgtga cagtgctaca     120
   tccttttcac tggactttgg ttatcttcgc aataagaacg gctgccacgt ggaattgctc     180
   ttcctccgct acatctcgga ctgggaccta gaccctggcc gctgctaccg cgtcacctgg     240
   ttcacctcct ggagcccctg ctacgactgt gcccgacatg tggccgactt tctgcgaggg     300
   aaccccaacc tcagtctgag gatcttcacc gcgcgcctct acttctgtga ggaccgcaag     360
   gctgagcccg aggggctgcg gcggctgcac cgcgccgggg tgcaaatagc catcatgacc     420
   ttcaaagatt atttttactg ctggaatact tttgtagaaa accatgaaag aactttcaaa     480
   gcctgggaag ggctgcatga aaattcagtt cgtctctcca gacagcttcg gcgcatcctt     540
   ttgcccctgt atgaggttga tgacttacga gacgcatttc gtactttggg actttg         596
<210> 108
   <211> 33
   <212> DNA
   <213> Artificial Sequence
<220>
<223> Oligonucleotide primer
<400> 108
   ccctcgagat ggacagcctc ttgatgaacc gga 33
<210> 109
   <211> 33
   <212> DNA
   <213> Artificial Sequence
<220>
<223> Oligonucleotide primer
<400> 109
   gctctagaca aagtcccaaa gtacgaaatg cgt 33
<210> 110
   <211> 12990
   <212> DNA
   <213> Artificial Sequence
<220>
<223> Plasmid KW3
<400> 110
   gtcgacggta ccatgactct cagaaagcac ttcccatgtg agctggaccc tgaatttaag      60
   gaaatgtgta gtcatttcct gtgggtgcct aagtgaggat ttgcatgtgg gtggtgcctt     120
   tgtatggata ggtaaaaagg gatgagggag gccccagtct tttgggctca ccctgggagg     180
   tgtatgctgg ctgtgccctc tgagaactca gttctcttcc tgtggcctcc cctcaccaaa     240
   cccagagtcc tcttcttcca ggtaggaaat gtgctgaagg agctggtctg ggagacaagt     300
   gtgatcatgg atcaaagaca gattttggaa tacagttaat actgttctac atttaaagat     360
   tcatataaca ccaaccatac acccaggtca cctaaattgt catttacccc ttcagacata     420
   ttgaaacagc tgctgagtgt aataatcaca gtgaattgag acaaacctgg atccatgcaa     480
   tgtgtactgt agttcagaac atccatcatg gttagaagga tgctacctgt cccaggaagt     540
   gggttatttt taaatagtac ctgagagctg cccttctgag accttttgaa atttgagatt     600
   gtgtgtgaga tctcaggaga aggtagtaga atatatctcc atccttctca atgtgtaacc     660
   ctgagaatat ggcctgacct ctaaacattt ctgtgtgaaa agatgtacat tggggatagc     720
   agtgacagct tcagatgaaa actctatagt acatcagcac tggaggatag tctcatcacc 780
   aagattagtg aaattacctt tcctgggaac cagagaggac ctctgtgagc tctaccctct 840
   gagagaacaa ggaactctgg ttcttccctg acaggtcaca cctgtgaaac atggctggac 900
   aatgacactc aaacccagaa ttcgtaccca catatttacc aattcagatc catctgtctc 960
   tgaaagaatt tctcctccgc tgaattgcaa gaacataccc tagggtgtgc agtattgcaa 1020
   cttgggcatt tcacattagt ttggtgaatt atataataca caaaatatct ccatggatgt 1080
   tgtaacagga gagtcatcag aagctggttg tgttgtataa tctggataaa cctgggctct 1140
   cttcttagga gctgaacaag tgggctggcc ttctatgaga cgacagaggg aaagagacag 1200
   actcaatatc cagagcgagg tgagctcctt acctacctac caggtggtct ctgggccatt 1260
   tgtttgagca gacccagaag taccttcctc accctcagga gaattatgaa cattgagaga 1320
   aactgagata ctttttttat ttacagggaa tatttcatcg gcgtgtttac atctacctgg 1380
   gtgtgtacag ggatgctagg atgtgctcat acacagaaga gcaagaatta tatttcgtgg 1440
   aaagaaaacc aaagagcttc tgaatttgta ggtattgttt gctgcaaatg tgtcaggtca 1500
   ctagatcatg ttatgctgct agaagaaaaa cttcccaaca ttgtcatgga gacaaaatgc 1560
   aaaacagtaa agattcaact gagattccct tgaaaatcac cagtaatgaa caggccaaaa 1620
   gaaatcaacc attgtggaaa gagtggtcat taagtgaaat agcaaattcc atgttgcagt 1680
   gagaaggaag atccatctga cagctcattt tcacctctac aaagacttca gaacatagac 1740
   taagagcaga gcgtgaactt agggcaaaca gaggccagat gtttgaggag gttggagagt 1800
   gagctggagt cattgtgagc cattcagaaa agcagagtgt tccagggtgt attgagtcct 1860
   cctgagttaa gaggtgctga atatacgcaa gtttcactgc cctcattgcg ttttattctc 1920
   tagactctct tggatgtcca gatttgaaca tgtggagtgt tgatggaact caacataact 1980
   aggaactttt cagtgaaggt gtaggtaaca atgtgggtat aattaaattc ggtttatgaa 2040
   aatattatta tccgaaatgt caaagtcagt atctattaat ttatctttct tttgtatttt 2100
   acagacaaga ttattctgtt gcccaggctg gagtgcagac tcacctatta atttaacagc 2160
   ataaaaacga tcagtccaat ttacgtagtc ctgtaatctc catcaaggat ttaggtccat 2220
   gtggcctgtg acagagctct agtcacagag ggaagagagt ggtttgttgg gttgatgctg 2280
   cttcttcaga ggggaattta aacaactcct cacctcatca agtctatttt tataactgtg 2340
   caagaccctt gggaatgcac tcaccatttc ttacataatg ggagttgact gtgtcatgaa 2400
   ggtaacacga agatgtgcaa atttaaagcc tggttacata acctgttgaa tttaaaatgc 2460
   ctggagccaa tcacattctg gcatcttgtt taattagttc tgaatgtatt ttcagttggt 2520
   tagttcaagt tcccataatt cactttctgc taaaatagtc acatacaata atcttgagat 2580
   attaaaataa aaaaactaat ttgaaaatga acccaacttc caggagagat gaaagttcct 2640
   ttgtggtgaa gggttgaaat atggttgaac actgaggttg tcttcacaat tgttttaatt 2700
   aggggaactt ctatacatcc cttatattta ttagaaactc ccattgagaa ccttgaacta 2760
   acgtaattag ttgatggagc acacaacaac aatgctgaag gttattaagc aggaattgct 2820
   attacaacgt tagccttgct ttaaagcaca tatttctacc tgatgtgaaa ttagcccagg 2880
   tgcgctgatg agagtttgtc agttaatcaa agaccaggaa atggatacac gtgtttctgg 2940
   agcagggcat ggctttggga tgctttgcga aaaaagtggc ttctcacgtc tttgggaaaa 3000
   cccatcaaaa tgggcaagtt aaggatctct taggagcacc cgtctatccc atattcttgg 3060
   ctaatattaa agcggaactc aatgcaaaat gagatactat ggaagttcag aaaactgctg 3120
   taccactgcc tcagctcagc acagctgcct ccttcctcag gctttctgaa actctcggga 3180
   tgtgggtttc cacactgtgt acttcgcaca gtaatactcg gccgtgtcct cagcctttag 3240
   gctgctgatc tgaagacacg ccgtgctgac agacgtgtcc atggagaaga caaaccatcc 3300
   tgtgaagccg tgggtatacg ttgggttccc agtgtaggtg atgatccatc acatccactc 3360
   aaagccctgt ccaggggtct atcataccca attcatacca tagatggtga aggtgtaacc 3420
   agaagactta taggagacct tcactgaggc cccaggcttc ttcacctcag gcccagactg 3480
   caccagctgc agggagtggg cacctgtgga gtggacacaa gagtgggtga agtctcacat 3540
   gactggcctg gtttcttcct cagccctgag actggggagc cccttacctg ttgctgctgc 3600
   catcaagaag aggatccttc aggtccagtc catggtgagg agctgtgatc taggggcttc 3660
   tccagaggag gggtgtggtt gttgggtgag gctctcaggg aacggagata ctatagtcac 3720
   ctcagttaat tgcatattca tgaaggatgc tatttaatag cccaattcct gacccagtat 3780
   gagaaacaga cacatgggtg acacaactgt agaagctgag ggttcaagcc gtaatcctgt 3840
   tagaggccat gtgtccccta cacatccctg aactctgtgt tgacagagct tcccccactg 3900
   gagaacaagc tcctcaagga cagcacctca ctttgaaacc acatttgact gtctcagggt 3960
   caacttgcat catttctaga ccataatatg tgaatgcgtt atttagggaa tgactgtgtt 4020
   tatccaaaaa ttgcatttat ttataagaaa ggatctcttc ctgacctcca gcagagtttg 4080
   aaatccccat tgtaaaagtg gttctcatta caacatccag tttgataaat gctcacaatt 4140
   gaatagatat ttatacaaac ttcagcagtc tttgtgaaat acttatttta gatattttta 4200
   aaggaagtcc caggccctgg gaggaacctc tccccagcct cctgtgcacc tgctctgggg 4260
   cgggagcctg tgctgggtgt atccggagcg ccccctgcag cccagcccca accatgcagg 4320
   gaggtttcta tctgagctga cagagtatat tcctaccagt gtatccagct cagtataaag 4380
   tggttgtgcc ctggctcaga attctccttt agggacacca catgctcctc acaccatctt 4440
   ttgaaatagt gaattgcctt taggaaaccc agtgaactct gcagagagac tccaagaaaa 4500
   gatctcatgc atcaccaggg agccctttcc tggagctcaa gaggcacaga atcattggac 4560
   acacggtgaa cccaaacact cttcaggggt tgaggggaga ctcttatttc ctttagggtc 4620
   ctacagttga ttatggcacc tgagaatacc tgcaggtgca ggtacatgtg gatagaaacc 4680
   cactccaact ctgctattca actcacacat gcgcgcgcgt gtgtacacac acacacacac 4740
   acacacacac atatatatat atacatcgtg gctaattttt atattaacgg atcccatgtt 4800
   tgccattttt ttctggtatc catctcatgg aaagcgctcc ctacactggt actaagactg 4860
   aatatgtgtc tactttctgt aaacagaagt aaagaaacag aatacaagtg gacacttggg 4920
   aagtgcatgc acattgaatt cacctggtct cactttggaa ccctgcagat gccccgtgaa 4980
   aactaaatta tcgattgcca gccctgggac cgaaccccgc gtttatgaac aaacgaccca 5040
   acacccgtgc gttttattct gtctttttat tgccgtcata gcgcgggttc cttccggtat 5100
   tgtctccttc cgtgtttcag ttagcctccc ccatctcccg ggcaaacgtg cgcgccaggt 5160
   cgcagatcgt cggtatggag cctggggtgg tgacgtgggt ctggaccatc ccggaggtaa 5220
   gttgcagcag ggcgtcccgg tagccggcgg gcgattggtc gtaatccagg ataaagacgt 5280
   gcatgggacg gaggcgtttg gccaagacgt ccaaggccca ggcaaacacg ttatacaggt 5340
   cgccgttggg ggccagcaac tcgggggccc gaaacagggt aaataacgtg tccccgatat 5400
   ggggtcgtgg gcccgcgttg ctctggggct cggcaccctg gggcggcacg gccgtccccg 5460
   aaagctgtcc ccaatcctcc cgccacgacc cgccgccctg cagataccgc accgtattgg 5520
   caagcagccc gtaaacgcgg cgaatcgcgg ccagcatagc caggtcaagc cgctcgccgg 5580
   ggcgctggcg tttggccagg cggtcgatgt gtctgtcctc cggaagggcc cccaacacga 5640
   tgtttgtgcc gggcaaggtc ggcgggatga gggccacgaa cgccagcacg gcctgggggg 5700
   tcatgctgcc cataaggtat cgcgcggccg ggtagcacag gagggcggcg atgggatggc   5760
   ggtcgaagat gagggtgagg gccgggggcg gggcatgtga gctcccagcc tcccccccga   5820
   tatgaggagc cagaacggcg tcggtcacgg cataaggcat gcccattgtt atctgggcgc 5880
   ttgtcattac caccgccgcg tccccggccg atatctcacc ctggtcgagg cggtgttgtg 5940
   tggtgtagat gttcgcgatt gtctcggaag cccccagcac ctgccagtaa gtcatcggct 6000
   cgggtacgta gacgatatcg tcgcgcgaac ccagggccac cagcagttgc gtggtggtgg 6060
   ttttccccat cccgtgagga ccgtctatat aaacccgcag tagcgtgggc attttctgct 6120
   ccaggcggac ttccgtggct tcttgctgcc ggcgagggcg caacgccgta cgtcggttgc 6180
   tatggccgcg agaacgcgca gcctggtcga acgcagacgc gtgttgatgg caggggtacg 6240
   aagccatacg cgcttctaca aggcgcttgc cgaagaggtg cgggagtttc acgccaccaa 6300
   gatctgcggc acgctgttga cgctgttaag cgggtcgctg caggtcgaaa ggcccggaga 6360
   tgaggaagag gagaacagcg cggcagacgt gcgcttttga agcgtgcaga atgccgggcc 6420
   tccggaggac cttcgggcgc ccgccccgcc cctgagcccg cccctgagcc cgcccccgga 6480
   cccacccctt cccagcctct gagcccagaa agcgaaggag caaagctgct attggccgct 6540
   gccccaaagg cctacccgct tccattgctc agcggtgctg tccatctgca cgagactagt 6600
   gagacgtgct acttccattt gtcacgtcct gcacgacgcg agctgcgggg cgggggggaa 6660
   cttcctgact aggggaggag tagaaggtgg cgcgaagggg ccaccaaaga acggagccgg 6720
   ttggcgccta ccggtggatg tggaatgtgt gcgaggccag aggccacttg tgtagcgcca 6780
   agtgcccagc ggggctgcta aagcgcatgc tccagactgc aagcttggcg taatcatggt 6840
   catagctgtt tcctgtgtga aattgttatc cgctcacaat tccacacaac atacgagccg 6900
   gaagcataaa gtgtaaagcc tggggtgcct aatgagtgag gtaactcaca ttaattgcgt 6960
   tgcgctcact gcccgctttc cagtcgggaa acctgtcgtg ccagctgcat taatgaatcg 7020
   gccaacgcgc ggggagaggc ggtttgcgta ttggcgctct tccgcttcct cgctcactga 7080
   ctcgctgcgc tcggtcgttc ggctgcggcg agcggtatca gctcactcaa aggcggtaat 7140
   acggttatcc acagaatcag gggataacgc aggaaagaac atgtgagcaa aaggccagca 7200
   aaaggccagg aaccgtaaaa aggccgcgtt gctggcgttt ttccataggc tccgcccccc 7260
   tgacgagcat cacaaaaatc gacgctcaag tcagaggtgg cgaaacccga caggactata 7320
   aagataccag gcgtttcccc ctggaagctc cctcgtgcgc tctcctgttc cgaccctgcc 7380
   gcttaccgga tacctgtccg cctttctccc ttcgggaagc gtggcgcttt ctcaatgctc 7440
   acgctgtagg tatctcagtt cggtgtaggt cgttcgctcc aagctgggct gtgtgcacga 7500
   accccccgtt cagcccgacc gctgcgcctt atccggtaac tatcgtcttg agtccaaccc 7560
   ggtaagacac gacttatcgc cactggcagc agccactggt aacaggatta gcagagcgag 7620
   gtatgtaggc ggtgctacag agttcttgaa gtggtggcct aactacggct acactagaag 7680
   gacagtattt ggtatctgcg ctctgctgaa gccagttacc ttcggaaaaa gagttggtag 7740
   ctcttgatcc ggcaaacaaa ccaccgctgg tagcggtggt ttttttgttt gcaagcagca 7800
   gattacgcgc agaaaaaaag gatctcaaga agatcctttg atcttttcta cggggtctga 7860
   cgctcagtgg aacgaaaact cacgttaagg gattttggtc atgagattat caaaaaggat 7920
   cttcacctag atccttttaa attaaaaatg aagttttaaa tcaatctaaa gtatatatga 7980
   gtaaacttgg tctgacagtt accaatgctt aatcagtgag gcacctatct cagcgatctg 8040
   tctatttcgt tcatccatag ttgcctgact ccccgtcgtg tagataacta cgatacggga 8100
   gggcttacca tctggcccca gtgctgcaat gataccgcga gacccacgct caccggctcc 8160
   agatttatca gcaataaacc agccagccgg aagggccgag cgcagaagtg gtcctgcaac 8220
   tttatccgcc tccatccagt ctattaattg ttgccgggaa gctagagtaa gtagttcgcc 8280
   agttaatagt ttgcgcaacg ttgttgccat tgctacaggc atcgtggtgt cacgctcgtc 8340
   gtttggtatg gcttcattca gctccggttc ccaacgatca aggcgagtta catgatcccc 8400
   catgttgtgc aaaaaagcgg ttagctcctt cggtcctccg atcgttgtca gaagtaagtt 8460
   ggccgcagtg ttatcactca tggttatggc agcactgcat aattctctta ctgtcatgcc 8520
   atccgtaaga tgcttttctg tgactggtga gtactcaacc aagtcattct gagaatagtg 8580
   tatgcggcga ccgagttgct cttgcccggc gtcaatacgg gataataccg cgccacatag 8640
   cagaacttta aaagtgctca tcattggaaa acgttcttcg gggcgaaaac tctcaaggat 8700
   cttaccgctg ttgagatcca gttcgatgta acccactcgt gcacccaact gatcttcagc 8760
   atcttttact ttcaccagcg tttctgggtg agcaaaaaca ggaaggcaaa atgccgcaaa 8820
   aaagggaata agggcgacac ggaaatgttg aatactcata ctcttccttt ttcaatatta 8880
   ttgaagcatt tatcagggtt attgtctcat gagcggatac atatttgaat gtatttagaa 8940
   aaataaacaa ataggggttc cgcgcacatt tccccgaaaa gtgccacctg acgtcgccca 9000
   gcccagccta gctcagccca gcccagctca attcagccca gccctgccca gctcagccca 9060
   ccttaatgca gccaagccca gctcaactca gctcacctgg tgcaacttag cccagctcag 9120
   ctcagctcag ctcagcccag ttcaactcag cccagttcag ctcagctcag cccagttcgg 9180
   ccttgtttag tctaggtcaa cttaggtcag ttttgcccat ctgagtccat ttctgaaaac 9240
   tggatggagt tgtcatggcc agaaatggtc agcccaccag acctgcttgt ctcagctaaa 9300
   gccatctcat tgccgggttc ctgcacagcc aggctggctt ccatcttttg tctccctcta 9360
   cttgataccc cagttccctg cagtcctgcc ccagcgccac ctgggttttg gttccaaagc 9420
   attaccaatc attaccaccc tccactacct gggtggaata tttctttgct gctttaaagt 9480
   cattaaaaca tcttgagaat gagaccaaga atttaggagc ctgtgctgtg ataaaaatga 9540
   gcaggtcccc ttgctctaga agtggcagca tatcttctgc accaagagga gggtattgag 9600
   atgctcagag cctccacctt cccggagcat cccctccctt ctgagtctgc agtaaacccc 9660
   tgcctttaaa ttccctctag ataacagtca tcattggaaa caaccaagaa atgcatttta 9720
   tctgaatttg ccacttaaaa ttctgccatt taccataaat cgctttggaa ggcatgggct 9780
   actttcaagg gtgcgatgat gacctacagt caatgactta gacaagggcg atgccagtgg 9840
   gacttggtat gttctcaagc atcattaccc atgccatccc cattcagagg ttgtggagca 9900
   gctcgtgcga cctctccttc aaatgggctt tagggaaagt taaatgggag tgacccagac 9960
   aatggtcact caaaagactc acataaatga gtctcctgct cttcatcaag caattaagac 10020
   cagttcccct tctagtggaa ataagacgtc aaatacaaag ttttaagaga agcaaatgca 10080
   gcagcggcgg ctgcctgtct cttaccatgt cgggcgcctg gtcactgcga gccttgcaaa 10140
   gctttggcat ggaatcattc ctccaagtcc attaacaagg gctggggcct gagcagccag 10200
   tcggcccggc agcagaagcc acgcatccca gctctgggta gtccggggag acccagagcc 10260
   caggccgggc ctggcagcca ccctcccaga gcctccgcta ggccagtcct gctgacgccg 10320
   catcggtgat tcggaacaga atctgtcctt ctaaggtgtc tccacagtcc tgtcttcagc 10380
   actatctgat tgagttttct cttatgccac caactaacat gcttaactga aataattcag 10440
   gataatgatg cacatttttc ctaaaactta tcctaaagtg agtagttgaa aagtggtctt 10500
   gaaaaatact aaaatgaagg ccactctatc agaatatcaa agtgtttctc cttaatcaca 10560
   aagagaaaac gagttaacct aaaaagattg tgaacacagt cattatgaaa ataatgctct 10620
   gaggtatcga aaaagtattt gagattaatt atcacatgaa gggataacaa gctaatttaa 10680
   aaaacttttt gaatacagtc ataaactctc cctaagactg tttaatttct taaacatctt 10740
   actttaaaaa tgaatgcagt ttagaagttg atatgctgtt tgcacaaact agcagttgat 10800
   aagctaagat tggaaatgaa attcagatag ttaaaaaaag ccttttcagt ttcggtcagc 10860
   ctcgccttat tttagaaacg caaattgtcc aggtgttgtt ttgctcagta gagcactttc 10920
   agatctgggc ctgggcaaaa ccacctcttc acaaccagaa gtgataaatt taccaattgt 10980
   gtttttttgc ttcctaaaat agactctcgc ggtgacctgc ttcctgccac ctgctgtggg 11040
   tgccggagac ccccatgcag ccatcttgac tctaattcat catctgcttc cagcttcgct 11100
   caattaatta aaaaaataaa cttgatttat gatggtcaaa acgcagtccc gcatcggggc 11160
   cgacagcact gtgctagtat ttcttagctg agcttgcttt ggcctcaatt ccagacacat 11220
   atcactcatg ggtgttaatc aaatgataag aatttcaaat acttggacag ttaaaaaaat 11280
   taatatactt gaaaatctct cacattttta agtcataatt ttcttaacca tttttctcag 11340
   aagccacttc aaacatatcc tgtcttttaa cagtaagcat gcctcctaag ataaacaatc 11400
   cttttctcat ggaaaccagc ttcaaggcac tgaggtcctg gagcctccct aagcccctgt 11460
   caggacggca gccaccgttt ctgggctacc cctgccccca accctgctct catcaagacc 11520
   ggggctacgc gtccctcctg gctggattca cccactccga cagttctctt tccagccaat  11580
   aaagaattta agatgcaggt tgacacacag cgcacctcat aattctaaag aaaatatttc  11640
   acgattcgct gctgtgcagc gatcttgcag tcctacagac accgctcctg agacacattc  11700
   ctcagccatc actaagaccc ctggtttgtt caggcatctc gtccaaatgt ggctccccaa  11760
   gcccccaggc tcagttactc catcagacgc acccaacctg agtcccattt tccaaaggca  11820
   tcggaaaatc cacagaggct cccactcgag cagtgtggtt ttgcaagagg aagcaaaaag  11880
   cctctccacc caggcctgga atgtttccac ccaatgtcga gcagtgtggt tttgcaagag  11940
   gaagcaaaaa gcctctccac ccaggcctgg aatgtttcca cccaatgtcg agcaaacccc  12000
   gcccagcgtc ttgtcattgg cgaattcgaa cacgcagatg cagtcggggc ggcgcggtcc  12060
   caggtccact tcgcatatta aggtgacgcg tgtggcctcg aacaccgagc gaccctgcag  12120
   ccaatatggg atcggccatt gaacaagatg gattgcacgc aggttctccg gccgcttggg  12180
   tggagaggct attcggctat gactgggcac aacagacaat cggctgctct gatgccgccg  12240
   tgttccggct gtcagcgcag gggcgcccgg ttctttttgt caagaccgac ctgtccggtg  12300
   ccctgaatga actgcaggac gaggcagcgc ggctatcgtg gctggccacg acgggcgttc  12360
   cttgcgcagc tgtgctcgac gttgtcactg aagcgggaag ggactggctg ctattgggcg  12420
   aagtgccggg gcaggatctc ctgtcatctc accttgctcc tgccgagaaa gtatccatca  12480
   tggctgatgc aatgcggcgg ctgcatacgc ttgatccggc tacctgccca ttcgaccacc  12540
   aagcgaaaca tcgcatcgag cgagcacgta ctcggatgga agccggtctt gtcgatcagg  12600
   atgatctgga cgaagagcat caggggctcg cgccagccga actgttcgcc aggctcaagg  12660
   cgcgcatgcc cgacggcgag gatctcgtcg tgacccatgg cgatgcctgc ttgccgaata  12720
   tcatggtgga aaatggccgc ttttctggat tcatcgactg tggccggctg ggtgtggcgg  12780
   accgctatca ggacatagcg ttggctaccc gtgatattgc tgaagagctt ggcggcgaat  12840
   gggctgaccg cttcctcgtg ctttacggta tcgccgctcc cgattcgcag cgcatcgcct  12900
   tctatcgcct tcttgacgag ttcttctgag gggatcggca ataaaaagac agaataaaac  12960
   gcacgggtgt tgggtcgttt gttcggatcc                                  12990
<210> 111
   <211> 33
   <212> DNA
   <213> Artificial Sequence
<220>
   <223> Oligonucleotide primer
<400> 111
   ccgctcgagt gggagcctct gtggattttc cga 33
<210> 112
   <211> 32
   <212> DNA
   <213> Artificial Sequence
<220>
<223> Oligonucleotide primer
<400> 112
   tgaccggacg tcgcccagcc cagcctagct ca 32
<210> 113
   <211> 48
   <212> DNA
   <213> Artificial Sequence
<220>
<223> Oligonucleotide primer
<400> 113
   cccaagctta tggactggac ctggaggatc ctcttcttgg tggcagca 48
<210> 114
   <211> 51 <212> DNA
   <213> Artificial Sequence
<220>
   <223> Oligonucleotide primer
<400> 114
   cccaagctta tggacacact ttgctccacg ctcctgctgc tgaccatccc t 51
<210> 115
   <211> 48
   <212> DNA
   <213> Artificial Sequence
<220>
   <223> Oligonucleotide primer
<400> 115
   cccaagctta tggagtttgg gctgagctgg gttttccttg ttgctatt 48
<210> 116
   <211> 48
   <212> DNA
   <213> Artificial Sequence
<220>
   <223> Oligonucleotide primer
<400> 116
   cccaagctta tgaaacacct gtggttcttc ctcctgctgg tggcagct 48
<210> 117
   <211> 51
   <212> DNA
   <213> Artificial Sequence
<220>
   <223> Oligonucleotide primer
<400> 117
   cccaagctta tggggtcaac cgccatcctc gccctcctcc tggctgttct c 51
<210> 118
   <211> 51
   <212> DNA
   <213> Artificial Sequence
<220>
   <223> Oligonucleotide primer
<400> 118
   cccaagctta tgtctgtctc cttcctcatc ttcctgcccg tgctgggcct c 51
<210> 119
   <211> 51
   <212> DNA
   <213> Artificial Sequence
<220>
   <223> Oligonucleotide primer
<400> 119
   cccaagctta tggactggac ctggaggatc ctcttcttgg tggcagcagc a 51
<210> 120
   <211> 39
   <212> DNA
   <213> Artificial Sequence
<220>
   <223> Oligonucleotide primer
<400> 120
   cccaagcttc cccaagcttc ccaggtgcag ctacagcag 39

### SEQUENCE LISTING

<110> Diatech Pty Ltd
<120> In vivo affinity maturation scheme
<130> AHB/FP6309611
<140> EP 03779576.2
   <141> 2003-12-18
<150> PCT/AU2003/001697
   <151> 2003-12-18
<150> AU 200295381
   <151> 2002-12-18
<160> 120
<170> PatentIn version 3.1
<210> 1
   <211> 8699
   <212> DNA
   <213> Homo sapiens
<220>
   <221> misc_feature
   <222> (2697)..(2697)
   <223> n = unknown
<220>
   <221> misc_feature
   <222> (4014)..(4014)
   <223> n = unknown
<400> 1
   gcttcattgt tccacgcatt acgtctcacc agtttagtca atatggatta aatatgagag     60
   tgtggcaatt cgcaaactct atctgaggag gaaaatcgga taaaaaatgt tatgaaaaat    120
   aaagcaattt gaagcctctg acttcagcaa cttcaccact aatgaaatga tgtaaccctc    180
   attggcctca aatttagttt tcacggggca tctgcagggt tccaaagtga gaccaggtga    240
   attcaatgtg catgcacttc ccaagtgtcc acttgtattc tgtttcttta cttctgttta    300
   cagaaagtag acacatattc agtcttagta ccagtgtagg gagcgctttc catgagatgg    360
   ataccagaaa aaaatggcaa acatgggatc cgttaatata aaaattagcc acgatgtata    420
   tatatatatg tgtgtgtgtg tgtgtgtgtg tgtgtacaca cgcgcgcgca tgtgtgagtt    480
   gaatagcaga gttggagtgg gtttctatcc acatgtacct gcacctgcag gtattctcag    540
   gtgccataat caactgtagg accctaaagg aaataagagt ctcccctcaa cccctgaaga    600
   gtgtttgggt tcaccgtgtg tccaatgatt ctgtgcctct tgagctccag gaaagggctc    660
   cctggtgatg catgagatct tttcttggag tctctctgca gagttcactg ggtttcctaa    720
   aggcaattca ctatttcaaa agatggtgtg aggagcatgt ggtgtcccta aaggagaatt    780
   ctgagccagg gcacaaccac tttatactga gctggataca ctggtaggaa tatactctgt    840
   cagctcagat agaaacctcc ctgcatggtt ggggctgggc tgcagggggc gctccggata    900
   cacccagcac aggctcccgc cccagagcag gtgcacagga ggctggggag aggttcctcc    960
   cagggcctgg gacttccttt aaaaatatct aaaataagta tttcacaaag actgctgaag   1020
   tttgtataaa tatctattca attgtgagca tttatcaaac tggatgttgt aatgagaacc   1080
   acttttacaa tggggatttc aaactctgct ggaggtcagg aagagatcct ttcttataaa   1140
   taaatgcaat ttttggataa acacagtcat tccctaaata acgcattcac atattatggt   1200
   ctagaaatga tgcaagttga ccctgagaca gtcaaatgtg gtttcaaagt gaggtgctgt   1260
   ccttgaggag cttgttctcc agtgggggaa gctctgtcaa cacagagttc agggatgtgt   1320
   aggggacaca tggcctctaa caggattacg gcttgaaccc tcagcttcta cagttgtgtc   1380
   acccatgtgt ctgtttctca tactgggtca ggaattgggc tattaaatag catccttcat   1440
   gaatatgcaa ttaactgagg tgactatagt atctctgtac cctgaaagca tcacccaaca   1500
   acaacatccc tccttgggag aatcccctag agcacagctc ctcaccatgg actggacctg   1560
   gagcatcctc ttcttggtgg cagcagcaac aggtaagggg ctccccagtc tcggggttga   1620
   ggcagaaacc aggccactca agtgaggctt tacccacccc tgtgtcctct ccacaggtac   1680
   ctactcccag gtgcagctgg tgcagtctgg ccatgaggtg aagcagcctg gggcctcagt   1740
   gaaggtctcc tgcaagtctt ctggttacac cttcaccatc tatggtatga attgggtatg   1800
   atagacccct ggacagggct ttgagtggat gtgatggatc atcacctaca ctgggaaccc   1860
   aacgtatacc cacggcttca caggatggtt tgtcttctcc atggacacgt ctgtcagcac   1920
   ggcgtgtctt cagatcagca gcctaaaggc tgaggacacg gccgagtatt actgtgcgaa   1980
   gtacacagtg tggaaaccca catcccgaga gtttcagaaa gcctgaggaa ggaggcagct   2040
   gtgctgagct gaggcagtgg tacagcagtt ttctgaactt ccatagtatc tcattttgca   2100
   ttgagttccg ctttaatatt agccaagaat atgggataga cgggtgctcc taagagatcc   2160
   ttaacttgcc cattttgatg ggttttccca aagacgtgag aagccacttt tttcgcaaag   2220
   catcccaaag ccatgccctg ctccagaaac acgtgtatcc atttcctggt ctttgattaa   2280
   ctgacaaact ctcatcagcg cacctgggct aatttcacat caggtagaaa tatgtgcttt   2340
   aaagcaaggc taacgttgta atagcaattc ctgcttaata accttcagca ttgttgttgt   2400
   gtgctccatc aactaattac gttagttcaa ggttctcaat gggagtttct aataaatata   2460
   agggatgtat agaagttccc ctaattaaaa caattgtgaa gacaacctca gtgttcaacc 2520
   atatttcaac ccttcaccac aaaggaactt tcatctctcc tggaagttgg gttcattttc 2580
   aaattagttt ttttatttta atatctcaag attattgtat gtgactattt tagcagaaag 2640
   tgaattatgg gaacttgaac taaccaactg aaaatacatt cagaactaat taaacangat 2700
   gccagaatgt gattggctcc aggcatttta aattcaacag gttatgtaac caggctttaa 2760
   atttgcacat cttcgtgtta ccttcatgac acagtcaact cccattatgt aagaaatggt 2820
   gagtgcattc ccaagggtct tgcacagtta taaaaataga cttgatgagg tgaggagttg 2880
   tttaaattcc cctctgaaga agcagcatca acccaacaaa ccactctctt ccctctgtga 2940
   ctagagctct gtcacaggcc acatggacct aaatccttga tggagattac aggactacgt 3000
   aaattggact gatcgttttt atgctgttaa attaataggt gagtctgcac tccagcctgg 3060
   gcaacagaat aatcttgtct gtaaaataca aaagaaagat aaattaatag atactgactt 3120
   tgacatttcg gataataata ttttcataaa ccgaatttaa ttatacccac attgttacct 3180
   acaccttcac tgaaaagttc ctagttatgt tgagttccat caacactcca catgttcaaa 3240
   tctggacatc caagagagtc tagagaataa aacgcaatga gggcagtgaa acttgcgtat 3300
   attcagcacc tcttaactca ggaggactca atacaccctg gaacactctg cttttctgaa 3360
   tggctcacaa tgactccagc tcactctcca acctccgcaa acatctggcc tctgtttgcc 3420
   ctaagttcac gctctgctct tagtctatgt tctgaagtct ttgtagaggt gaaaatgagc 3480
   tgtcagatgg atcttccttc tcactgcaac atggaatttg ctatttcact taatgaccac 3540
   tctttccaca atggttgatt tcttttggcc tgttcattac tggtgatttt caagggaatc 3600
   tcagttgaat ctttactgtt ttgcattttg tctccatgac aatgttggga agtttttctt 3660
   ctagcagcat aacatgatct agtgacctga cacatttgca gcaaacaata cctacaaatt 3720
   cagaagctct ttggttttct ttccacgaaa tataattctt gctcttctgt gtatgagcac 3780
   atcctagcat ccctgtacac acccaggtag atgtctacac gccgatgaaa tattccctgt 3840
   aaataaaaaa agtatctcag tttctctcaa tgttcataat tctcctgagg gtgaggaagg 3900
   tacttctggg tctgctcaaa caaatggccc agagaccacc tggtaggtag gtaaggagct 3960
   cacctcgctc tggatattga gtctgtctct ttccctctgt cgtctcatag aagnccagcc 4020
   cacttgttca gctcctaaga agagagccca ggtttatcca gattatacaa cacaaccagc 4080
   ttctgatgac tctcctgtta caacatccat ggagatattt tgtgtattat ataattcacc 4140
   aaactaatgt gaaatgccca agttgcaata ctgcacaccc tagggtatgt tcttgcaatt 4200
   cagcggagga gaaattcttt cagagacaga tggatctgaa ttggtaaata tgtgggtacg 4260
   aattctgggc ttgagtgtca ttgtccagcc atgtttcaca ggtgtgacct gtcagggaag 4320
   aaccagagtt ccttgttctc tcagagggta gagctcacag aggtcctctc tggttcccag 4380
   gaaaggtaat ttcactaatc ttggtgatga gactatcctc cagtgctgat gtactataga 4440
   gttttcatct gaagctgtca ctgctatccc caatgtacat cttttcacac agaaatgttt 4500
   agaggtcagg ccatattctc agggttacac attgagaagg atggagatat attctactac 4560
   cttctcctga gatctcacac acaatctcaa atttcaaaag gtctcagaag ggcagctctc 4620
   aggtactatt taaaaataac ccacttcctg ggacaggtag catccttcta accatgatgg 4680
   atgttctgaa ctacagtaca cattgcatgg atccaggttt gtctcaattc actgtgatta 4740
   ttacactcag cagctgtttc aatatgtctg aaggggtaaa tgacaattta ggtgacctgg 4800
   gtgtatggtt ggtgttatat gaatctttaa atgtagaaca gtattaactg tattccaaaa 4860
   tctgtctttg atccatgatc acacttgtct cccagaccag ctccttcagc acatttccta 4920
   cctggaagaa gaggactctg ggtttggtga ggggaggcca caggaagaga actgagttct 4980
   cagagggcac agccagcata cacctcccag ggtgagccca aaagactggg gcctccctca 5040
   tcccttttta cctatccata caaaggcacc acccacatgc aaatcctcac ttaggcaccc 5100
   acaggaaatg actacacatt tccttaaatt cagggtccag ctcacatggg aagtgctttc 5160
   tgagagtcat ggacctcctg cacaagaaca tgaaacacct gtggttcttc ctcctcctgg 5220
   tggcagctcc cagatgtgag tgtctcagga atgcggatat gaagatatga gatgctgcct 5280
   ctgatcccag ggctcactgt gggtttttct gttcacaggg gtcctgtccc aggtgcagct 5340
   acagcagtgg ggcgcaggac tgttgaagcc ttcggagacc ctgtccctca cctgcggtgt 5400
   ttatggtggg tccttcagtg gttactactg gagctggatc cgccagcccc cagggaaggg 5460
   gctggagtgg attggggaaa tcaatcatag tggaagcacc aactacaacc cgtccctcaa 5520
   gagtcgagtc accatatcag tagacacgtc caagaagcag ctctccctga agttgagctc 5580
   tgtgaacgcc gcggacacgg ctgtgtatta ctgtgcgaga gttattacta gggcgagtcc 5640
   tggcacagac gggaggtacg gtatggacgt ctggggccaa gggaccacgg tcaccgtctc 5700
   ctcaggtgag aatggccact ctagggcctc tgttctctgc tactgcctgt ggggtttcct 5760
   gagcattgca ggttggtcct cggggcatgt tccgagggga cctgggcgga ctggccagga 5820
   ggggacgggc actggggtgc cttgaggatc tgggagcctc tgtggatttt ccgatgcctt 5880
   tggaaaatgg gactcaggtt gggtgcgtct gatggagtaa ctgagcctgg gggcttgggg 5940
   agccacattt ggacgagatg cctgaacaaa ccaggggtct tagtgatggc tgaggaatgt 6000
   gtctcaggag cggtgtctgt aggactgcaa gatcgctgca cagcagcgaa tcgtgaaata 6060
   ttttctttag aattatgagg tgcgctgtgt gtcaacctgc atcttaaatt ctttattggc 6120
   tggaaagaga actgtcggag tgggtgaatc cagccaggag ggacgcgtag ccccggtctt 6180
   gatgagagca gggttggggg caggggtagc ccagaaacgg tggctgccgt cctgacaggg 6240
   gcttagggag gctccaggac ctcagtgcct tgaagctggt ttccatgaga aaaggattgt 6300
   ttatcttagg aggcatgctt actgttaaaa gacaggatat gtttgaagtg gcttctgaga 6360
   aaaatggtta agaaaattat gacttaaaaa tgtgagagat tttcaagtat attaattttt 6420
   ttaactgtcc aagtatttga aattcttatc atttgattaa cacccatgag tgatatgtgt 6480
   ctggaattga ggccaaagca agctcagcta agaaatacta gcacagtgct gtcggccccg 6540
   atgcgggact gcgttttgac catcataaat caagtttatt tttttaatta attgagcgaa 6600
   gctggaagca gatgatgaat tagagtcaag atggctgcat gggggtctcc ggcacccaca 6660
   gcaggtggca ggaagcaggt caccgcgaga gtctatttta ggaagcaaaa aaacacaatt 6720
   ggtaaattta tcacttctgg ttgtgaagag gtggttttgc ccaggcccag atctgaaagt 6780
   gctctactga gcaaaacaac acctggacaa tttgcgtttc taaaataagg cgaggctgac 6840
   cgaaactgaa aaggcttttt ttaactatct gaatttcatt tccaatctta gcttatcaac 6900
   tgctagtttg tgcaaacagc atatcaactt ctaaactgca ttcattttta aagtaagatg 6960
   tttaagaaat taaacagtct tagggagagt ttatgactgt attcaaaaag ttttttaaat 7020
   tagcttgtta tcccttcatg tgataattaa tctcaaatac tttttcgata cctcagagca 7080
   ttattttcat aatgactgtg ttcacaatct ttttaggtta actcgttttc tctttgtgat 7140
   taaggagaaa cactttgata ttctgataga gtggccttca ttttagtatt tttcaagacc 7200
   acttttcaac tactcacttt aggataagtt ttaggaaaaa tgtgcatcat tatcctgaat 7260
   tatttcagtt aagcatgtta gttggtggca taagagaaaa ctcaatcaga tagtgctgaa 7320
   gacaggactg tggagacacc ttagaaggac agattctgtt ccgaatcacc gatgcggcgt 7380
   cagcaggact ggcctagcgg aggctctggg agggtggctg ccaggcccgg cctgggctct 7440
   gggtctcccc ggactaccca gagctgggat gcgtggcttc tgctgccggg ccgactggct 7500
   gctcaggccc cagcccttgt taatggactt ggaggaatga ttccatgcca aagctttgca 7560
   aggctcgcag tgaccaggcg cccgacatgg taagagacag gcagccgccg ctgctgcatt 7620
   tgcttctctt aaaactttgt atttgacgtc ttatttccac tagaagggga actggtctta 7680
   attgcttgat gaagagcagg agactcattt atgtgagtct tttgagtgac cattgtctgg 7740
   gtcactccca tttaactttc cctaaagccc atttgaagga gaggtcgcac gagctgctcc 7800
   acaacctctg aatggggatg gcatgggtaa tgatgcttga gaacatacca agtcccactg 7860
   gcatcgccct tgtctaagtc attgactgta ggtcatcatc gcacccttga aagtagccca 7920
   tgccttccaa agcgatttat ggtaaatggc agaattttaa gtggcaaatt cagataaaat 7980
   gcatttcttg gttgtttcca atgatgactg ttatctagag ggaatttaaa ggcaggggtt 8040
   tactgcagac tcagaaggga ggggatgctc cgggaaggtg gaggctctga gcatctcaat 8100
   accctcctct tggtgcagaa gatatgctgc cacttctaga gcaaggggac ctgctcattt 8160
   ttatcacagc acaggctcct aaattcttgg tctcattctc aagatgtttt aatgacttta 8220
   aagcagcaaa gaaatattcc acccaggtag tggagggtgg taatgattgg taatgctttg 8280
   gaaccaaaac ccaggtggcg ctggggcagg actgcaggga actggggtat caagtagagg 8340
   gagacaaaag atggaagcca gcctggctgt gcaggaaccc ggcaatgaga tggctttagc 8400
   tgagacaagc aggtctggtg ggctgaccat ttctggccat gacaactcca tccagttttc 8460
   agaaatggac tcagatgggc aaaactgacc taagttgacc tagactaaac aaggccgaac 8520
   tgggctgagc tgagctgaac tgggctgagt tgaactgggc tgagctgagc tgagctgagc 8580
   tgggctaagt tgcaccaggt gagctgagtt gagctgggct tggctgcatt aaggtgggct 8640
   gagctgggca gggctgggct gaattgagct gggctgggct gagctaggct gggctgggc 8699
<210> 2
   <211> 338
   <212> DNA
   <213> Home sapiens
<220>
   <221> misc_feature
   <222> (54)..(54)
   <223> n = unknown
<220>
   <221> misc_feature
   <222> (86)..(86)

   <223> n = unknown
<400> 2
   taagtgaatc ctggtgtgtc tgaactcaag tgattgttac attaagctgc tgtnccaatc     60
   tgtttcctca cctgggaaaa gagganccag gacatagtga gttgaggccc caggaagata    120
   actgaattct cagagggcac agccagcatc ctcctcccag ggagagtcta aaagactggg    180
   gcctccctca tcccttttca cctgtccata cagaggcacc acccacatgc aaatctcact    240
   taggcaccca cagaaaacca ccacacattt ccttaaattc agggtcctgc tcacatggga    300
   aatactttct gagagtcctg gacctcctgt gcaagaac                            338
<210> 3
   <211> 339
   <212> DNA
   <213> Homo sapiens
<400> 3
   taagtgaatc ctggtgtgtc tgaactcaca tgattgttac attaagctgc tgttgcaatc     60
   tgtttcctca cctgggaaaa gaggagccag gacatagtga gttgaggccc caggaagata    120
   actgaattct cagagggcac aaccagcatc ctccttgcca gggagagcct aaaagactgg    180
   ggcctccctc atcccttttc acctctccat acagaggcac cacccacatg caaatctcac    240
   ttaggcaccc aagggaaacc atcacacatt tccttaaatt cagggtcctg ctcacatggg    300
   aaatactttc tgagagctct ggacctcctg tgcaagaac                           339
<210> 4
   <211> 338
   <212> DNA
   <213> Homo sapiens
<220>
   <221> misc_feature
   <222> (106)..(106)
   <223> n = unknown
<400> 4
   attccaaaat ctgtctttga tccatgatca cacttgtctc ccagaccagc tccttcagca      60
   catttcctac ctggaagaag aggactctgg gtttggtgag gggagnccac aggaagagaa     120
   ctgagttctc agagggcaca gccagcatac acctcccagg gtgagcccaa aagactgggg     180
   cctccctcat ccctttttac ctatccatac aaaggcacca cccacatgca aatcctcact      240
   taggcaccca caggaaatga ctacacattt ccttaaattc agggtccagc tcacatggga     300
   agtgctttct gagagtcatg gacctcctgc acaagaac                             338
<210> 5
   <211> 339
   <212> DNA
   <213> Homo sapiens
<400> 5
   attccaaaat ctgtccctga tccaagatca cactgatctc ccagagcagc atcttcagca      60
   catttcccta cctggaagaa gaggactatg ggcttggtaa ggggaggcca caggaagaga     120
   actgagttct cagagggcac agccagcttc ctactcccag ggcaagccca aaagactggg     180
   gcctccctcc tcccttttca cctgtccata caaagtcacc gcccacatgc aaatcctcac     240
   ttaggcacct acaggaaacc agcacacatt tccttaaatt tgggatccag ctcacatggg     300
   aaatactttc tgagactcat gggcctcctg cacaagaac                            339
<210> 6
   <211> 338
   <212> DNA
   <213> Homo sapiens
<220>
   <221> misc_feature
   <222> (16)..(16)
   <223> n = unknown
<400> 6
   atcccaaaat ctgtcnttga tccaggatca cactcatctc tcagaccagc tccttcagca 60
   catctcttta cctggaagaa gaggactctg ggcttggaga ggggagcccc caagaagaga 120
   actgagttct caaagggcac agccagcatt ctcctcccag ggtgagctca aaagactggc 180
   gcctctctca tcccttttca ctgctccgta caaacgcacc acccccatgc aaatcctcac 240
   ttaggcgccc acaggaagcc accacacatt tccttaaatt caggtccaac tcataaggga 300
   aatgctttct gagagtcatg gatctcatgt gcaagaaa 338
<210> 7
   <211> 334
   <212> DNA
   <213> Artificial Sequence
<220>
<223> Consensus sequence for human immunoglobulin heavy chain promoter for VH4 alleles
<400> 7
   atccaaaatc tgtctctgat ccaagatcac acttatctcc agagcagctc ttcagcacat 60
   ttccttacct ggaagaagag gactctgggc ttggtgaggg gaggccccag gaagagaact 120
   gagttctcag agggcacagc cagcatcctc ctcccagggg agcccaaaag actggggcct 180
   ccctcatccc ttttcacctt ccatacaaag gcaccaccca catgcaaatc ctcacttagg 240
   cacccacagg aaaccaccac acatttcctt aaattcaggg tccagctcac atgggaaata 300
   ctttctgaga gtcatggacc tcctgtgcaa gaac 334
<210> 8
   <211> 3693
   <212> DNA
   <213> Artificial Sequence
<220>
<223> Plasmid pME18Sasfp499
<400> 8
   aagcttggct gtggaatgtg tgtcagttag ggtgtggaaa gtccccaggc tccccagcag    60
   gcagaagtat gcaaagcatg catctcaatt agtcagcaac catagtcccg cccctaactc   120
   cgcccatccc gcccctaact ccgcccagtt ccgcccattc tccgccccat ggctgactaa   180
   ttttttttat ttatgcagag gccgaggccg cctcggcctc tgagctattc cagaagtagt   240
   gaggaggctt ttttggaggc ctaggctttt gcaaaaagct cctcgatcga ggggctcgca   300
   tctctccttc acgcgcccgc cgccctacct gaggccgcca tccacgccgg ttgagtcgcg   360
   ttctgccgcc tcccgcctgt ggtgcctcct gaactgcgtc cgccgtctag gtaagtttaa   420
   agctcaggtc gagaccgggc ctttgtccgg cgctcccttg gagcctacct agactcagcc   480
   ggctctccac gctttgcctg accctgcttg ctcaactcta cgtctttgtt tcgttttctg   540
   ttctgcgccg ttacagatcc aagctctgaa aaaccagaaa gttaactggt aagtttagtc   600
   tttttgtctt ttatttcagg tcccggatcc ggtggtggtg caaatcaaag aactgctcct   660
   cagtggatgt tgcctttact tctaggcctg tacggaagtg ttacttctgc tctaaaagct   720
   gcgctcgaga tgtatccttc catcaaggaa accatgcgcg ttcagctttc tatggagggt   780
   agtgttaact accacgcctt caagtgcact ggaaaaggag agggaaaacc atacgaaggc   840
   acccaaagcc tgaatattac aataactgaa ggaggtcctc tgccatttgc ttttgacatt   900
   ctgtcacacg cctttcagta tggcatcaag gtcttcgcca agtaccccaa agaaattcct   960
   gacttcttta agcagtctct acctggtggt ttttcttggg aaagagtaag cacctatgaa  1020
   gatggaggag tgctttcagc tacccaagaa acaagtttgc agggtgattg catcatctgc  1080
   aaagttaaag tccttggcac caattttccc gcaaacggtc cagtgatgca aaagaagacc  1140
   tgtggatggg agccatcaac tgaaacagtc atcccacgag atggtggact tctgcttcgc  1200
   gatacccccg cacttatgct ggctgacgga ggtcatcttt cttgcttcat ggaaacaact  1260
   tacaagtcga agaaagaggt aaagcttcca gaacttcact ttcatcattt gcgtatggaa  1320
   aagctgaaca taagtgacga ttggaagacc gttgagcagc acgagtctgt ggtggctagc  1380
   tactcccaag tgccttcgaa attaggacat aacgcggccg ctgattataa agatgatgat   1440
   gataaagatt ataaagatga tgatgataaa gattataaag atgatgatga taaagattat   1500
   aaagatgatg atgataaata ataaactagt ctagagaaaa aacctcccac acctccccct   1560
   gaacctgaaa cataaaatga atgcaattgt tgttgttaac ttgtttattg cagcttataa   1620
   tggttacaaa taaagcaata gcatcacaaa tttcacaaat aaagcatttt tttcactgca   1680
   ttctagttgt ggtttgtcca aactcatcaa tgtatcttat catgtctgga tccccgggta   1740
   ccgagctcga attaattcct cttccgcttc ctcgctcact gactcgctgc gctcggtcgt   1800
   tcggctgcgg cgagcggtat cagctcactc aaaggcggta atacggttat ccacagaatc   1860
   aggggataac gcaggaaaga acatgtgagc aaaaggccag caaaaggcca ggaaccgtaa   1920
   aaaggccgcg ttgctggcgt ttttccatag gctccgcccc cctgacgagc atcacaaaaa   1980
   tcgacgctca agtcagaggt ggcgaaaccc gacaggacta taaagatacc aggcgtttcc   2040
   ccctggaagc tccctcgtgc gctctcctgt tccgaccctg ccgcttaccg gatacctgtc   2100
   cgcctttctc ccttcgggaa gcgtggcgct ttctcatagc tcacgctgta ggtatctcag   2160
   ttcggtgtag gtcgttcgct ccaagctggg ctgtgtgcac gaaccccccg ttcagcccga   2220
   ccgctgcgcc ttatccggta actatcgtct tgagtccaac ccggtaagac acgacttatc   2280
   gccactggca gcagccactg gtaacaggat tagcagagcg aggtatgtag gcggtgctac   2340
   agagttcttg aagtggtggc ctaactacgg ctacactaga agaacagtat ttggtatctg   2400
   cgctctgctg aagccagtta ccttcggaaa aagagttggt agctcttgat ccggcaaaca   2460
   aaccaccgct ggtagcggtg gtttttttgt ttgcaagcag cagattacgc gcagaaaaaa   2520
   aggatctcaa gaagatcctt tgatcttttc tacggggtct gacgctcagt ggaacgaaaa   2580
   ctcacgttaa gggattttgg tcatgagatt atcaaaaagg atcttcacct agatcctttt   2640
   aaattaaaaa tgaagtttta aatcaatcta aagtatatat gagtaaactt ggtctgacag   2700
   ttaccaatgc ttaatcagtg aggcacctat ctcagcgatc tgtctatttc gttcatccat   2760
   agttgcctga ctccccgtcg tgtagataac tacgatacgg gagggcttac catctggccc   2820
   cagtgctgca atgataccgc gagacccacg ctcaccggct ccagatttat cagcaataaa   2880
   ccagccagcc ggaagggccg agcgcagaag tggtcctgca actttatccg cctccatcca   2940
   gtctattaat tgttgccggg aagctagagt aagtagttcg ccagttaata gtttgcgcaa   3000
   cgttgttgcc attgctacag gcatcgtggt gtcacgctcg tcgtttggta tggcttcatt   3060
   cagctccggt tcccaacgat caaggcgagt tacatgatcc cccatgttgt gcaaaaaagc   3120
   ggttagctcc ttcggtcctc cgatcgttgt cagaagtaag ttggccgcag tgttatcact 3180
   catggttatg gcagcactgc ataattctct tactgtcatg ccatccgtaa gatgcttttc 3240
   tgtgactggt gagtactcaa ccaagtcatt ctgagaatag tgtatgcggc gaccgagttg 3300
   ctcttgcccg gcgtcaatac gggataatac cgcgccacat agcagaactt taaaagtgct 3360
   catcattgga aaacgttctt cggggcgaaa actctcaagg atcttaccgc tgttgagatc 3420
   cagttcgatg taacccactc gtgcacccaa ctgatcttca gcatctttta ctttcaccag 3480
   cgtttctggg tgagcaaaaa caggaaggca aaatgccgca aaaaagggaa taagggcgac 3540
   acggaaatgt tgaatactca tactcttcct ttttcaatat tattgaagca tttatcaggg 3600
   ttattgtctc atgagcggat acatatttga atgtatttag aaaaataaac aaataggggt 3660
   tccgcgcaca tttccccgaa aagtgccacc tgc 3693
<210> 9
   <211> 581
   <212> DNA
<213> Homo sapiens
<400> 9
   cggccccgat gcgggactgc gttttgacca tcataaatca agtttatttt tttaattaat 60
   tgagcgaagc tggaagcaga tgatgaatta gagtcaagat ggctgcatgg gggtctccgg 120
   cacccacagc aggtggcagg aagcaggtca ccgcgagagt ctattttagg aagcaaaaaa 180
   acacaattgg taaatttatc acttctggtt gtgaagaggt ggttttgccc aggcccagat 240
   ctgaaagtgc tctactgagc aaaacaacac ctggacaatt tgcgtttcta aaataaggcg 300
   aggctgaccg aaactgaaaa ggcttttttt aactatctga atttcatttc caatcttagc 360
   ttatcaactg ctagtttgtg caaacagcat atcaacttct aaactgcatt catttttaaa 420
   gtaagatgtt taagaaatta aacagtctta gggagagttt atgactgtat tcaaaaagtt 480
   ttttaaatta gcttgttatc ccttcatgtg taattaatct caaatacttt ttcgatacct 540
   cagagcatta ttttcataat gactgtgttc acaatctttt t 581
<210> 10
   <211> 582
   <212> DNA
   <213> Homo sapiens
<400> 10
   cggccccgat gcgggactgc gttttgacca tcataaatca agtttatttt tttaattaat     60
   tgagcgaagc tggaagcaga tgatgaatta gagtcaagat ggctgcatgg gggtctccgg    120
   cacccacagc aggtggcagg aagcaggtca ccgcgagagt ctattttagg aagcaaaaaa    180
   acacaattgg taaatttatc acttctggtt gtgaagaggt ggttttgccc aggcccagat    240
   ctgaaagtgc tctactgagc aaaacaacac ctggacaatt tgcgtttcta aaataaggcg    300
   aggctgaccg aaactgaaaa ggcttttttt aactatctga atttcatttc caatcttagc    360
   ttatcaactg ctagtttgtg caaacagcat atcaacttct aaactgcatt catttttaaa    420
   gtaagatgtt taagaaatta aacagtctta gggagagttt atgactgtat tcaaaaagtt    480
   ttttaaatta gcttgttatc ccttcatgtg ataattaatc tcaaatactt tttcgatacc    540
   tcagagcatt attttcataa tgactgtgtt cacaatcttt tt                       582
<210> 11
   <211> 666
   <212> DNA
   <213> Ovis aries
<400> 11
   ctgcgaatac cgagacgggg cctctcaaag ccacccctga tagtctggaa aattgaaact     60
   ttaaaaagag agatgtttaa agtattttaa atttttatca tttaattaac aactgcgaat    120
   catggctttg gagagttgag taagagtttg gctgaaaagt actaactagg ttccatcggc    180
   cctcggcccc aattcagggc tgttttgaga ataataaatt cagcttattt ttttaatgta    240
   attggtggtg ccgagttagt caagatggcc acgggccaga ctgaccacct gcagcaggtg    300
   gcaggaagca tgtccacttg agagtctgtt tttggaagca agaaaaaaca gttggtaaat    360
   ttatcgcttc tggtttccaa aaggtggttt gcggctggtt ttgcccagcc ccacagaacc    420
   gaaagtgttc cactgagcac aacagcacct ggctaatttg catttctaaa ataaggcgca    480
   gatgctgacc gaaactggaa ggttcctctt ctaactattt gagttaactt cagctttagc    540
   ttatcaactg ctcacttatc ttcattttca aagtcgatgt ttaagaaagc cacctgtctc    600
   gggtgcactg tctcggtgca ttgctgcact ctctgatgag ccgtccttca aggtggttga    660
   gctgag                                                               666
<210> 12
   <211> 1067
   <212> DNA
   <213> Mus musculus
<400> 12
   ctagatactg agttctggtt ctaataactg gctcctgtac tgatggatgg gtcctgacta 60
   gtcattgggc cctgatcctc aacattgact tcaaaacctg aactctagcc ccatgcctca 120
   ttcacattag gatgatccct acaggggatt cctgcagaag attccagaat ccccacaaca 180
   ctgttcacac actgggctgc aactgggaca gtgacccttt tgactcatag gacttgccag 240
   gcacagaggc acagaatgga gacaaagcaa gcccaggacc ctggagatgg agcctctggt 300
   ggggtctaca gatgtggggt cagcatcgta gggaggtttg cagggcaggt gtggggcagg 360
   gcagaggtag tcatgcttat agatactatt tttctctcct ctggagcctc ctttgtctat 420
   cacctgctgt cctgggatct ctatctgggg tcaacaatgt ttgcagtaca ggtgtggggg 480
   tagggcaggg atgctcacat tagcaacttg tttttctctc ttctgaagtc tctgttgtct 540
   atcacctgct gaaacattca aagcagctct cagctgaggg cagctgagtc atcctgagcc 600
   tgtctcagca caggtgcccc aaaccagagc tactgttctg agaatcacat cacactggac 660
   caggccaggt gggcctggga catggatgag gggtgggagc caggggagcc tgccaggggc 720
   tgaggaggcc ccaaccccca ctacccaagg ccatccacac ctgtgcctta gtgaggccat 780
   gttctgtccc aatgagaaca agtccaatta agattaagta tggtcttccc aggactatcc 840
   agagctaagg ggtgtcagcc agggacaacc cagaccagcc tgaggtcagc cagcatcacc 900
   caaggccaca cagctattct ggctagagga ctagatagct agctcatcga ggccctggag 960
   atgcagaatg gaagagttta tccctgccag acagggctca tcagaaaggc aggtatctca 1020
   ctacacatga cctccctgaa tatttcccag agtccagttg gttctag 1067
<210> 13
   <211> 221
   <212> DNA
   <213> Mus musculus
<400> 13
   agtcaagatg gccgatcaga accagaacac ctgcagcagc tggcaggaag caggtcatgt 60
   ggcaaggcta tttggggaag ggaaaataaa accactaggt aaacttgtag ctgtggtttg 120
   aagaagtggt tttgaaacac tctgtccagc cccaccaaac cgaaagtcca ggctgagcaa 180
   aacaccacct gggtaatttg catttctaaa ataagttgag g 221
<210> 14
   <211> 808
   <212> DNA
   <213> Mus musculus
<400> 14
   agctcaaacc agcttaggct acacagagaa actatctaaa aaataattac taactactta 60
   ataggagatt ggatgttaag atctggtcac taagaggcag aattgagatt cgaaccagta 120
   ttttctacct ggtatgtttt aaattgcagt aaggatctaa gtgtagatat ataataataa 180
   gattctattg atctctgcaa caacagagag tgttagattt gtttggaaaa aaatattatc 240
   agccaacatc ttctaccatt tcagtatagc acagagtacc cacccatatc tccccaccca 300
   tcccccatac cagactggtt attgattttc atggtgactg gcctgagaag attaaaaaaa 360
   gtaatgctac cttattggga gtgtcccatg gaccaagata gcaactgtca tagctaccgt 420
   cacactgctt tgatcaagaa gaccctttga ggaactgaaa acagaacctt aggcacatct 480
   gttgctttcg ctcccatcct cctccaacag cctgggtggt gcactccaca ccctttcaag 540
   tttccaaagc ctcatacacc tgctccctac cccagcacct ggccaaggct gtatccagca 600
   ctgggatgaa aatgataccc cacctccatc ttgtttgata ttactctatc tcaagcccca 660
   ggttagtccc cagtcccaat gcttttgcac agtcaaaact caacttggaa taatcagtat 720
   ccttgaagag ttctgatatg gtcactgggc ccatatacca tgtaagacat gtggaaaaga 780
   tgtttcatgg ggcccagaca cgttctag 808
<210> 15
   <211> 60
   <212> DNA
   <213> Mus musculus
<400> 15
   aagcagccct caggcagagg ataaaagctc acactaactg agaagctcca tcctcttctc 60
<210> 16
   <211> 60
   <212> DNA
   <213> Mus musculus
<400> 16
   aattaggcca ccctcatcac atgaaaacca gcccagagtg actctagcag tgggatcctg 60
<210> 17
   <211> 60
   <212> DNA
   <213> Mus musculus
<400> 17
   catgtgcgac tgtgatgatt aatataggga tatccacacc aaacatcata tgagccctat 60
<210> 18
   <211> 60
   <212> DNA
   <213> Mus musculus
<400> 18
   aacatgagtc tgtgattata aatacagaga tatccatacc aaacaactta tgagcactgt 60
<210> 19
   <211> 338
   <212> DNA
   <213> Homo sapiens
<220>
   <221> misc_feature
   <222> (16)..(16)
   <223> n = unknown
<400> 19 <210> 20
   <211> 255
   <212> DNA
   <213> Homo sapiens
<400> 20 <210> 21
   <211> 238
   <212> DNA
   <213> Homo sapiens
<400> 21
<210> 22
   <211> 244
   <212> DNA
<213> Homo sapiens
<400> 22
   gatgggtagg ggatgcgtgt cctctaacag gattacgtct tgaaccctca gcttctacaa 60
   ttgtgtcgtc catgtgtcat gtatttgctc tttctcatcc tgggtcagga attgggctat 120
   taaatagcat ccttcatgaa tatgcaaata actgaggtga atatagatat ctgtgtgccc 180
   tgagagcatc acccaaaaac cacacccctc cttgggagaa tcccctagat cacagctcct 240
   cacc 244
<210> 23
   <211> 36
   <212> PRT
   <213> Homo sapiens
<400> 23
   Pro Asn Lys Gly Ser Thr Thr Ser Gly Thr Thr Arg Leu Leu Ser Gly
   1 5 10 15
   His Thr Cys Phe Thr Leu Thr Gly Leu Leu Gly Thr Leu Val Thr Met
   20 25 30
   Gly Leu Leu Thr
   35
<210> 24
   <211> 111
   <212> DNA
   <213> Homo sapiens
<400> 24
   ccaaataaag gaagtggaac cacttcaggt actacccgtc ttctatctgg gcacacgtgt 60
   ttcacgttga caggtttgct tgggacgcta gtaaccatgg gcttgctgac t 111
<210> 25
   <211> 33
   <212> PRT
   <213> Sus scrofa
<400> 25
   Cys Arg Thr Asn Tyr Gly Tyr Ser Ala Ala Pro Ser Leu His Leu Pro
   1 5 10 15
   Pro Gly Ser Leu Leu Ala Ser Leu Val Pro Leu Leu Leu Leu Ser Leu
   20 25 30
   Pro
<210> 26
   <211> 99
   <212> DNA
   <213> Sus scrofa
<400> 26
   tgccggacga attacggcta ctcagccgcc cccagcctcc acctcccgcc gggctcgctg 60
   ctggcctccc tcgtgcccct cctcctcctc agtcttccg 99
<210> 27
   <211> 30
   <212> PRT
   <213> Rattus rattus
<400> 27
   Ala Ser Ser Gln Ser Tyr Arg Met Thr Trp Asn Ile Leu Tyr Thr Leu
   1               5                   10                  15
   Leu Ile Ser Met Thr Thr Leu Phe Gln Ile Ser Thr Lys Glu
               20                  25                  30
<210> 28
   <211> 90
   <212> DNA
   <213> Rattus rattus
<400> 28
   gcatcgtctc agagctacag gatgacctgg aacatactct atacactgtt aatcagcatg     60
   actactttat tccaaatatc taccaaggag                                      90
<210> 29
   <211> 47
   <212> PRT
   <213> Mus musculus
<400> 29
   Ser Ser Asn Lys Ser Ile Ser Val Tyr Arg Asp Lys Leu Val Lys Cys
   1               5                   10                  15
   Gly Gly Ile Ser Leu Leu Val Gln Asn Thr Ser Trp Met Leu Leu Leu
               20                  25                  30
   Leu Leu Ser Leu Ser Leu Leu Gln Ala Leu Asp Phe Ile Ser Leu
           35                  40                  45
<210> 30
   <211> 135
   <212> DNA
   <213> Mus musculus
<400> 30
   agctccaata aaagtatcag tgtgtataga gacaagctgg tcaagtgtgg cggcataagc 60
   ctgctggttc agaacacatc ctggatgctg ctgctgctgc tttccctctc cctcctccaa 120
   gccctggact tcatt 135
<210> 31
   <211> 37
   <212> PRT
   <213> Mus musculus
<400> 31
   Pro Glu Asp Pro Pro Asp Ser Lys Asn Thr Leu Val Leu Phe Gly Ala
   1 5 10 15
   Gly Phe Gly Ala Val Ile Thr Val Val Val Ile Val Val Ile Ile Lys
   20 25 30
   Cys Phe Cys Lys His
   35
<210> 32
   <211> 111
   <212> DNA
   <213> Mus musculus
<400> 32
   ccagaagacc ctcctgatag caagaacaca cttgtgctct ttggggcagg attcggcgca 60
   gtaataacag tcgtcgtcat cgttgtcatc atcaaatgct tctgtaagca c 111
<210> 33
   <211> 25
   <212> PRT
   <213> Homo sapiens
<400> 33
   Met Gly Ile Gln Gly Gly Ser Val Leu Phe Gly Leu Leu Leu Val Leu
   1 5 10 15
   Ala Val Phe Cys His Ser Gly His Ser
   20 25
<210> 34
   <211> 75
   <212> DNA
   <213> Homo sapiens
<400> 34
   atgggaatcc aaggagggtc tgtcctgttc gggctgctgc tcgtcctggc tgtcttctgc 60
   cattcaggtc atagc 75
<210> 35
   <211> 39
   <212> DNA
   <213> Artificial Sequence
<220>
<223> Oligonucleotide primer
<400> 35
   ccggaattca atttgagatt gtgtgtgaga tctcaggag 39
<210> 36
   <211> 39
   <212> DNA
   <213> Artificial Sequence
<220>
<223> Oligonucleotide primer
<400> 36
   ccggaattca tagacagcgc aggtgaggga cagggtctc 39
<210> 37
   <211> 39
   <212> DNA
   <213> Artificial Sequence
<220>
<223> Oligonucleotide primer
<400> 37
   ccggaattcc tgagaactca gttctcttcc tgtggcctc 39
<210> 38
   <211> 39
   <212> DNA
   <213> Artificial Sequence
<220>
<223> Oligonucleotide primer
<400> 38
   ccggaattca atttgagatt gtgtgtgaga tctcaggag 39
<210> 39
   <211> 39
   <212> DNA
   <213> Artificial Sequence
<220>
<223> Oligonucleotide primer
<400> 39
   cccaagcttt cctgttacaa catccatgga gatattttg 39
<210> 40
   <211> 39
   <212> DNA
   <213> Artificial Sequence
<220>
<223> Oligonucleotide primer
<400> 40
   ccggaattct gaattgcaag aacataccct agggtgtgc 39
<210> 41
   <211> 40
   <212> DNA
   <213> Artificial Sequence
<220>
<223> Oligonucleotide primer
<400> 41
   ccggaattct agggcaaaca gaggccagat gtttgaggag 40
<210> 42
   <211> 39
   <212> DNA
   <213> Artificial Sequence
<220>
<223> Oligonucleotide primer
<400> 42
   ccggaattca atttaacagc ataaaaacga tcagtccaa 39
<210> 43
   <211> 39
   <212> DNA
   <213> Artificial Sequence
<220>
<223> Oligonucleotide primer
<400> 43
   ccggaattcc gtgtttctgg agcagggcat ggctttggg 39
<210> 44
   <211> 39
   <212> DNA
   <213> Artificial Sequence
<220>
<223> Oligonucleotide primer
<400> 44
   ccggaattcg ttgggttccc agtgtaggtg atgatccat 39
<210> 45
   <211> 39
   <212> DNA
   <213> Artificial Sequence
<220>
<223> Oligonucleotide primer
<400> 45
   ccggaattct cccaggaagt gggttatttt taaatagta 39
<210> 46
   <211> 39
   <212> DNA
   <213> Artificial Sequence
<220>
<223> Oligonucleotide primer
<400> 46
   ccggaattca ctatagtcac ctcagttaat tgcatattc 39
<210> 47
   <211> 39 <212> DNA
   <213> Artificial Sequence
<220>
<223> Oligonucleotide primer
<400> 47
   cccaagcttg acttccttta aaaatatcta aaataagta 39
<210> 48
   <211> 39
   <212> DNA
<213> Artificial Sequence
<220>
<223> Oligonucleotide primer
<400> 48
   ccggaattcg gttctcatta caacatccag tttgataaa 39
<210> 49
   <211> 39
   <212> DNA
   <213> Artificial Sequence
<220>
<223> Oligonucleotide primer
<400> 49
   ccggaattcc tccaagaaaa gatctcatgc atcaccagg 39
<210> 50
   <211> 39
   <212> DNA
   <213> Artificial Sequence
<220>
<223> Oligonucleotide primer
<400> 50
   cccaagctta atttagtttt cacggggcat ctgcagggt 39
<210> 51
   <211> 39
   <212> DNA
   <213> Artificial Sequence
<220>
<223> Oligonucleotide primer
<400> 51
   cccaagcttt gcacacccta gggtatgttc ttgcaattc 39
<210> 52
   <211> 39
   <212> DNA
   <213> Artificial Sequence
<220>
<223> Oligonucleotide primer
<400> 52
<210> 53 <211> 39
   <212> DNA
   <213> Artificial Sequence
<220>
<223> Oligonucleotide primer
<400> 53
   ccggaattcc cataatatgt gaatgcgtta tttagggaa 39
<210> 54
   <211> 29
   <212> DNA
   <213> Artificial Sequence
<220>
<223> Oligonucleotide primer
<400> 54
   cccaagcttc atgttccacg cattacgtc 29
<210> 55
   <211> 29
   <212> DNA
   <213> Artificial Sequence
<220>
<223> Oligonucleotide primer
<400> 55
   cccaagctta agagtgtttg ggttcaccg 29
<210> 56
   <211> 39
   <212> DNA
   <213> Artificial Sequence
<220>
<223> Oligonucleotide primer
<400> 56
   cccaagcttt taactcagga ggactcaata caccctgga 39
<210> 57
   <211> 39
   <212> DNA
   <213> Artificial Sequence
<220>
<223> Oligonucleotide primer
<400> 57
   cccaagctta aacaatacct acaaattcag aagctcttt 39
<210> 58
   <211> 38
   <212> DNA
   <213> Artificial Sequence
<220>
<223> Oligonucleotide primer
<400> 58
   cccaagctta agtcttctgg ttacaccttc accattat 38
<210> 59
   <211> 39
   <212> DNA
   <213> Artificial Sequence
<220>
<223> Oligonucleotide primer
<400> 59
   cccaagctta ctctcttccc tctgtgacta gagctctgt 39
<210> 60
   <211> 39
   <212> DNA
   <213> Artificial Sequence
<220>
<223> Oligonucleotide primer
<400> 60
   cccaagcttt cagcttctac agttgtgtca cccatgtgt 39
<210> 61
   <211> 39 <212> DNA
   <213> Artificial Sequence
<220>
<223> Oligonucleotide primer
<400> 61
   cccaagcttt gcagagttca ctgggtttcc taaaggcaa 39
<210> 62
   <211> 39
   <212> DNA
   <213> Artificial Sequence
<220>
<223> Oligonucleotide primer
<400> 62
   cccaagcttt caccacaaag gaactttcat ctctcctgg 39
<210> 63
   <211> 39
   <212> DNA
   <213> Artificial Sequence
<220>
<223> Oligonucleotide primer
<400> 63
   cccaagcttt ttcacacaga aatgtttaga ggtcaggcc 39
<210> 64
   <211> 39
   <212> DNA
   <213> Artificial Sequence
<220>
<223> Oligonucleotide primer
<400> 64
   cccaagcttt gcacacccta gggtatgttc ttgcaattc 39
<210> 65
   <211> 39
   <212> DNA
   <213> Artificial Sequence
<220>
<223> Oligonucleotide primer
<400> 65
   cccaagcttc ccaaagccat gccctgctcc agaaacacg 39
<210> 66
   <211> 33
   <212> DNA
   <213> Artificial Sequence
<220>
<223> Oligonucleotide primer
<400> 66
   cccaagctta tgatgtaacc ctcattggcc tca 33
<210> 67
   <211> 33
   <212> DNA
   <213> Artificial Sequence
<220>
<223> Oligonucleotide primer
<400> 67
   ccggaattcg agactccaag aaaagatctc atg 33
<210> 68
   <211> 33
   <212> DNA
   <213> Artificial Sequence
<220>
<223> Oligonucleotide primer
<400> 68
   cccaagctta tgttcttgca attcagcgga gga 33
<210> 69
   <211> 33
   <212> DNA
   <213> Artificial Sequence
<220>
<223> Oligonucleotide primer
<400> 69
   ccggaattct gtgtgagatc tcaggagaag gta 33
<210> 70
   <211> 39
   <212> DNA
   <213> Artificial Sequence
<220>
<223> Oligonucleotide primer
<400> 70
   cccaagcttc ggccccgatg cgggactgcg ttttgacca 39
<210> 71
   <211> 39
   <212> DNA
   <213> Artificial Sequence
<220>
<223> Oligonucleotide primer
<400> 71
   ccggaattca taacaagcta atttaaaaaa ctttttgaa 39
<210> 72
   <211> 39
   <212> DNA
   <213> Artificial Sequence
<220>
<223> Oligonucleotide primer
<400> 72
   cccaagcttg cacagacggg aggtacggta tggacgtct 39
<210> 73
   <211> 39
   <212> DNA
   <213> Artificial Sequence
<220>
<223> Oligonucleotide primer
<400> 73
   ccggaattca aaaaaataaa cttgatttat gatggtcaa 39
<210> 74
   <211> 39
   <212> DNA
   <213> Artificial Sequence
<220>
<223> Oligonucleotide primer
<400> 74
   ccggaattcc gcggtgacct gcttcctgcc acctgctgt 39
<210> 75
   <211> 29
   <212> DNA
   <213> Artificial Sequence
<220>
<223> Oligonucleotide primer
<400> 75
   ccggaattca gttagtgcag ccaagccct 29
<210> 76
   <211> 39
   <212> DNA
   <213> Artificial Sequence
<220>
<223> Oligonucleotide primer
<400> 76
   ccggaattca aaaggcaagt ggacttcggt gcttacctg 39
<210> 77
   <211> 36
   <212> DNA
   <213> Artificial Sequence
<220>
<223> Oligonucleotide primer
<400> 77
   cccaagcttc agctcagctc agttcagttc agccct 36
<210> 78
   <211> 39
   <212> DNA
   <213> Artificial Sequence
<220>
<223> Oligonucleotide primer
<400> 78
   cccaagctta tgcgagggtc tggacggctg aggaccccc 39
<210> 79
   <211> 39
   <212> DNA
   <213> Artificial Sequence
<220>
<223> Oligonucleotide primer
<400> 79
   ccggaattca tgcggcaagg gttgcggacc gctggctgg 39
<210> 80
   <211> 29
   <212> DNA
   <213> Artificial Sequence
<220>
<223> Oligonucleotide primer
<400> 80
   ccggaattcg cccagcccag cctagctca 29
<210> 81
   <211> 29
   <212> DNA
   <213> Artificial Sequence
<220>
<223> Oligonucleotide primer
<400> 81
   cccaagcttt tatcaactgc tagtttgtg 29
<210> 82
   <211> 36
   <212> DNA
   <213> Artificial Sequence
<220>
<223> Oligonucleotide primer
<400> 82
   ccggaattca gggctgaact gaactgagct gagctg 36
<210> 83
   <211> 35
   <212> DNA
   <213> Artificial Sequence
<220>
<223> Oligonucleotide primer
<400> 83
   cggctgatat ctgggagcct ctgtggattt tccga 35
<210> 84
   <211> 31
   <212> DNA
   <213> Artificial Sequence
<220>
<223> Oligonucleotide primer
<400> 84
   agccggatat cgcccagccc agcctagctc a 31
<210> 85
   <211> 24
   <212> DNA
   <213> Artificial Sequence
<220>
<223> Oligonucleotide primer
<400> 85
   gaaagttaaa tgggagtgac ccag 24
<210> 86
   <211> 24
   <212> DNA
   <213> Artificial Sequence
<220>
<223> Oligonucleotide primer
<400> 86
   gagtgaccat cgcacccttg acag 24
<210> 87
   <211> 10826
   <212> DNA
   <213> Artificial Sequence
<220>
<223> Plasmid 3kb15a-7-4T
<400> 87
   ctaaattgta agcgttaata ttttgttaaa attcgcgtta aatttttgtt aaatcagctc     60
   attttttaac caataggccg aaatcggcaa aatcccttat aaatcaaaag aatagaccga    120
   gatagggttg agtgttgttc cagtttggaa caagagtcca ctattaaaga acgtggactc    180
   caacgtcaaa gggcgaaaaa ccgtctatca gggcgatggc ccactacgtg aaccatcacc    240
   ctaatcaagt tttttggggt cgaggtgccg taaagcacta aatcggaacc ctaaagggag    300
   cccccgattt agagcttgac ggggaaagcc ggcgaacgtg gcgagaaagg aagggaagaa    360
   agcgaaagga gcgggcgcta gggcgctggc aagtgtagcg gtcacgctgc gcgtaaccac    420
   cacacccgcc gcgcttaatg cgccgctaca gggcgcgtcc cattcgccat tcaggctgcg    480
   caactgttgg gaagggcgat cggtgcgggc ctcttcgcta ttacgccagc tggcgaaagg    540
   gggatgtgct gcaaggcgat taagttgggt aacgccaggg ttttcccagt cacgacgttg    600
   taaaacgacg gccagtgagc gcgcgtaata cgactcacta tagggcgaat tgggtaccgg    660
   gccccccctc gaggtcgacg gtatcgataa gcttgatatc tgggagcctc tgtggatttt    720
   ccgatgcctt tggaaaatgg gactcaggtt gggtgcgtct gatggagtaa ctgagcctgg    780
   gggcttgggg agccacattt ggacgagatg cctgaacaaa ccaggggtct tagtgatggc    840
   tgaggaatgt gtctcaggag cggtgtctgt aggactgcaa gatcgctgca cagcagcgaa    900
   tcgtgaaata ttttctttag aattatgagg tgcgctgtgt gtcaacctgc atcttaaatt    960
   ctttattggc tggaaagaga actgtcggag tgggtgaatc cagccaggag ggacgcgtag   1020
   ccccggtctt gatgagagca gggttggggg caggggtagc ccagaaacgg tggctgccgt   1080
   cctgacaggg gcttagggag gctccaggac ctcagtgcct tgaagctggt ttccatgaga   1140
   aaaggattgt ttatcttagg aggcatgctt actgttaaaa gacaggatat gtttgaagtg   1200
   gcttctgaga aaaatggtta agaaaattat gacttaaaaa tgtgagagat tttcaagtat   1260
   attaattttt ttaactgtcc aagtatttga aattcttatc atttgattaa cacccatgag   1320
   tgatatgtgt ctggaattga ggccaaagca agctcagcta agaaatacta gcacagtgct   1380
   gtcggccccg atgcgggact gcgttttgac catcataaat caagtttatt tttttaatta   1440
   attgagcgaa gctggaagca gatgatgaat tagagtcaag atggctgcat gggggtctcc   1500
   ggcacccaca gcaggtggca ggaagcaggt caccgcgaga gtctatttta ggaagcaaaa   1560
   aaacacaatt ggtaaattta tcacttctgg ttgtgaagag gtggttttgc ccaggcccag   1620
   atctgaaagt gctctactga gcaaaacaac acctggacaa tttgcgtttc taaaataagg   1680
   cgaggctgac cgaaactgaa aaggcttttt ttaactatct gaatttcatt tccaatctta   1740
   gcttatcaac tgctagtttg tgcaaacagc atatcaactt ctaaactgca ttcattttta   1800
   aagtaagatg tttaagaaat taaacagtct tagggagagt ttatgactgt attcaaaaag   1860
   ttttttaaat tagcttgtta tcccttcatg tgataattaa tctcaaatac tttttcgata   1920
   cctcagagca ttattttcat aatgactgtg ttcacaatct ttttaggtta actcgttttc   1980
   tctttgtgat taaggagaaa cactttgata ttctgataga gtggccttca ttttagtatt   2040
   tttcaagacc acttttcaac tactcacttt aggataagtt ttaggaaaaa tgtgcatcat   2100
   tatcctgaat tatttcagtt aagcatgtta gttggtggca taagagaaaa ctcaatcaga   2160
   tagtgctgaa gacaggactg tggagacacc ttagaaggac agattctgtt ccgaatcacc   2220
   gatgcggcgt cagcaggact ggcctagcgg aggctctggg agggtggctg ccaggcccgg   2280
   cctgggctct gggtctcccc ggactaccca gagctgggat gcgtggcttc tgctgccggg   2340
   ccgactggct gctcaggccc cagcccttgt taatggactt ggaggaatga ttccatgcca   2400
   aagctttgca aggctcgcag tgaccaggcg cccgacatgg taagagacag gcagccgccg   2460
   ctgctgcatt tgcttctctt aaaactttgt atttgacgtc ttatttccac tagaagggga   2520
   actggtctta attgcttgat gaagagcagg agactcattt atgtgagtct tttgagtgac   2580
   cattgtctgg gtcactccca tttaactttc cctaaagccc atttgaagga gaggtcgcac   2640
   gagctgctcc acaacctctg aatggggatg gcatgggtaa tgatgcttga gaacatacca   2700
   agtcccactg gcatcgccct tgtctaagtc attgactgta ggtcatcatc gcacccttga   2760
   aagtagccca tgccttccaa agcgatttat ggtaaatggc agaattttaa gtggcaaatt   2820
   cagataaaat gcatttcttg gttgtttcca atgatgactg ttatctagag ggaatttaaa   2880
   ggcaggggtt tactgcagac tcagaaggga ggggatgctc cgggaaggtg gaggctctga   2940
   gcatctcaat accctcctct tggtgcagaa gatatgctgc cacttctaga gcaaggggac   3000
   ctgctcattt ttatcacagc acaggctcct aaattcttgg tctcattctc aagatgtttt   3060
   aatgacttta aagcagcaaa gaaatattcc acccaggtag tggagggtgg taatgattgg   3120
   taatgctttg gaaccaaaac ccaggtggcg ctggggcagg actgcaggga actggggtat   3180
   caagtagagg gagacaaaag atggaagcca gcctggctgt gcaggaaccc ggcaatgaga   3240
   tggctttagc tgagacaagc aggtctggtg ggctgaccat ttctggccat gacaactcca   3300
   tccagttttc agaaatggac tcagatgggc aaaactgacc taagttgacc tagactaaac   3360
   aaggccgaac tgggctgagc tgagctgaac tgggctgagt tgaactgggc tgagctgagc   3420
   tgagctgagc tgggctaagt tgcaccaggt gagctgagtt gagctgggct tggctgcatt   3480
   aaggtgggct gagctgggca gggctgggct gaattgagct gggctgggct gagctaggct   3540
   gggctgggcg atatcgaatt cctgcagccc gggggatccg cccccatcga taatttagtt   3600
   ttcacggggc atctgcaggg ttccaaagtg agaccaggtg aattcaatgt gcatgcactt   3660
   cccaagtgtc cacttgtatt ctgtttcttt acttctgttt acagaaagta gacacatatt   3720
   cagtcttagt accagtgtag ggagcgcttt ccatgagatg gataccagaa aaaaatggca   3780
   aacatgggat ccgttaatat aaaaattagc cacgatgtat atatatatat gtgtgtgtgt   3840
   gtgtgtgtgt gtgtgtacac acgcgcgcgc atgtgtgagt tgaatagcag agttggagtg   3900
   ggtttctatc cacatgtacc tgcacctgca ggtattctca ggtgccataa tcaactgtag   3960
   gaccctaaag gaaataagag tctcccctca acccctgaag agtgtttggg ttcaccgtgt   4020
   gtccaatgat tctgtgcctc ttgagctcca ggaaagggct ccctggtgat gcatgagatc 4080
   ttttcttgga gtctctctgc agagttcact gggtttccta aaggcaattc actatttcaa 4140
   aagatggtgt gaggagcatg tggtgtccct aaaggagaat tctgagccag ggcacaacca 4200
   ctttatactg agctggatac actggtagga atatactctg tcagctcaga tagaaacctc 4260
   cctgcatggt tggggctggg ctgcaggggg cgctccggat acacccagca caggctcccg 4320
   ccccagagca ggtgcacagg aggctgggga gaggttcctc ccagggcctg ggacttcctt 4380
   taaaaatatc taaaataagt atttcacaaa gactgctgaa gtttgtataa atatctattc 4440
   aattgtgagc atttatcaaa ctggatgttg taatgagaac cacttttaca atggggattt 4500
   caaactctgc tggaggtcag gaagagatcc tttcttataa ataaatgcaa tttttggata 4560
   aacacagtca ttccctaaat aacgcattca catattatgg tctagaaatg atgcaagttg 4620
   accctgagac agtcaaatgt ggtttcaaag tgaggtgctg tccttgagga gcttgttctc 4680
   cagtggggga agctctgtca acacagagtt cagggatgtg taggggacac atggcctcta 4740
   acaggattac ggcttgaacc ctcagcttct acagttgtgt cacccatgtg tctgtttctc 4800
   atactgggtc aggaattggg ctattaaata gcatccttca tgaatatgca attaactgag 4860
   gtgactatag tatctccgtt ccctgagagc ctcacccaac aaccacaccc ctcctctgga 4920
   gaagccccta gatcacagct cctcaccatg gactggacct gaaggatcct cttcttgatg 4980
   gcagcagcaa caggtaaggg gctccccagt ctcagggctg aggaagaaac caggccagtc 5040
   atgtgagact tcacccactc ttgtgtccac tccacaggtg cccactccct gcagctggtg 5100
   cagtctgggc ctgaggtgaa gaagcctggg gcctcagtga aggtctccta taagtcttct 5160
   ggttacacct tcaccatcta tggtatgaat tgggtatgat agacccctgg acagggcttt 5220
   gagtggatgt gatggatcat cacctacact gggaacccaa cgtataccca cggcttcaca 5280
   ggatggtttg tcttctccat ggacacgtct gtcagcacgg cgtgtcttca gatcagcagc 5340
   ctaaaggctg aggacacggc cgagtattac tgtgcgaagt acacagtgtg gaaacccaca 5400
   tcccgagagt ttcagaaagc ctgaggaagg aggcagctgt gctgagctga ggcagtggta 5460
   cagcagtttt ctgaacttcc atagtatctc attttgcatt gagttccgct ttaatattag 5520
   ccaagaatat gggatagacg ggtgctccta agagatcctt aacttgccca ttttgatggg 5580
   ttttcccaaa gacgtgagaa gccacttttt tcgcaaagca tcccaaagcc atgccctgct 5640
   ccagaaacac gtgtatccat ttcctggtct ttgattaact gacaaactct catcagcgca 5700
   cctgggctaa tttcacatca ggtagaaata tgtgctttaa agcaaggcta acgttgtaat 5760
   agcaattcct gcttaataac cttcagcatt gttgttgtgt gctccatcaa ctaattacgt 5820
   tagttcaagg ttctcaatgg gagtttctaa taaatataag ggatgtatag aagttcccct 5880
   aattaaaaca attgtgaaga caacctcagt gttcaaccat atttcaaccc ttcaccacaa 5940
   aggaactttc atctctcctg gaagttgggt tcattttcaa attagttttt ttattttaat 6000
   atctcaagat tattgtatgt gactatttta gcagaaagtg aattatggga acttgaacta 6060
   accaactgaa aatacattca gaactaatta aacaagatgc cagaatgtga ttggctccag 6120
   gcattttaaa ttcaacaggt tatgtaacca ggctttaaat ttgcacatct tcgtgttacc 6180
   ttcatgacac agtcaactcc cattatgtaa gaaatggtga gtgcattccc aagggtcttg 6240
   cacagttata aaaatagact tgatgaggtg aggagttgtt taaattcccc tctgaagaag 6300
   cagcatcaac ccaacaaacc actctcttcc ctctgtgact agagctctgt cacaggccac 6360
   atggacctaa atccttgatg gagattacag gactacgtaa attggactga tcgtttttat 6420
   gctgttaaat taataggtga gtctgcactc cagcctgggc aacagaataa tcttgtctgt 6480
   aaaatacaaa agaaagataa attaatagat actgactttg acatttcgga taataatatt 6540
   ttcataaacc gaatttaatt atacccacat tgttacctac accttcactg aaaagttcct 6600
   agttatgttg agttccatca acactccaca tgttcaaatc tggacatcca agagagtcta 6660
   gagaataaaa cgcaatgagg gcagtgaaac ttgcgtatat tcagcacctc ttaactcagg 6720
   aggactcaat acaccctgga acactctgct tttctgaatg gctcacaatg actccagctc 6780
   actctccaac ctcctcaaac atctggcctc tgtttgccct aagttcacgc tctgctctta 6840
   gtctatgttc tgaagtcttt gtagaggtga aaatgagctg tcagatggat cttccttctc 6900
   actgcaacat ggaatttgct atttcactta atgaccactc tttccacaat ggttgatttc 6960
   ttttggcctg ttcattactg gtgattttca agggaatctc agttgaatct ttactgtttt 7020
   gcattttgtc tccatgacaa tgttgggaag tttttcttct agcagcataa catgatctag 7080
   tgacctgaca catttgcagc aaacaatacc tacaaattca gaagctcttt ggtttctttt 7140
   ccacgaaata taattcttgc tcttctgtgt atgagcacat cctagcatcc ctgtacacac 7200
   ccaggtagat gtaaacacgc cgatgaaata ttccctgtaa ataaaaaaag tatctcagtt 7260
   tctctcaatg ttcataattc tcctgagggt gaggaaggta cttctgggtc tgctcaaaca 7320
   aatggcccag agaccacctg gtaggtaggt aaggagctca cctcgctctg gatattgagt 7380
   ctgtctcttt ccctctgtcg tctcatagaa ggccagccca cttgttcagc tcctaagaag 7440
   agagcccagg tttatccaga ttatacaaca caaccagctt ctgatgactc tcctgttaca 7500
   acatccatgg agatattttg tgtattatat aattcaccaa actaatgtga aatgcccaag 7560
   ttgcaatact gcacacccta gggtatgttc ttgcaattca gcggaggaga aattctttca 7620
   gagacagatg gatctgaatt ggtaaatatg tgggtacgaa ttctgggttt gagtgtcatt 7680
   gtccagccat gtttcacagg tgtgacctgt cagggaagaa ccagagttcc ttgttctctc 7740
   agagggtaga gctcacagag gtcctctctg gttcccagga aaggtaattt cactaatctt 7800
   ggtgatgaga ctatcctcca gtgctgatgt actatagagt tttcatctga agctgtcact 7860
   gctatcccca atgtacatct tttcacacag aaatgtttag aggtcaggcc atattctcag 7920
   ggttacacat tgagaaggat ggagatatat tctactacct tctcctgaga tctcacacac 7980
   aatctcaaat ttcaaaaggt ctcagaaggg cagctctcag gtactattta aaaataaccc 8040
   acttcctggg acaggtagca tccttctaac catgatggat gttctgaact acagtacaca 8100
   ttgcatggat ccaggtttgt ctcaattcac tgtgattatt acactcagca gctgtttcaa 8160
   tatgtctgaa ggggtaaatg acaatttagg tgacctgggt gtatggttgg tgttatatga 8220
   atctttaaat gtagaacagt attaactgta ttccaaaatc tgtctttgat ccatgatcac 8280
   acttgtctcc cagaccagct ccttcagcac atttcctacc tggaagaaga ggactctggg 8340
   tttggtgagg ggaggccaca ggaagagaac tgagttctca gagggcacag ccagcataca 8400
   cctcccaggg tgagcccaaa agactggggc ctccctcatc cctttttacc tatccataca 8460
   aaggcaccac ccacatgcaa atcctcactt aggcacccac aggaaatgac tacacatttc 8520
   cttaaattca gggtccagct cacatgggaa gtgctttctg agagtcatgg acctcctgca 8580
   caagaacggt accgggctag agcggccgcc accgcggtgg agctccagct tttgttccct 8640
   ttagtgaggg ttaattgcgc gcttggcgta atcatggtca tagctgtttc ctgtgtgaaa 8700
   ttgttatccg ctcacaattc cacacaacat acgagccgga agcataaagt gtaaagcctg 8760
   gggtgcctaa tgagtgagct aactcacatt aattgcgttg cgctcactgc ccgctttcca 8820
   gtcgggaaac ctgtcgtgcc agctgcatta atgaatcggc caacgcgcgg ggagaggcgg 8880
   tttgcgtatt gggcgctctt ccgcttcctc gctcactgac tcgctgcgct cggtcgttcg 8940
   gctgcggcga gcggtatcag ctcactcaaa ggcggtaata cggttatcca cagaatcagg 9000
   ggataacgca ggaaagaaca tgtgagcaaa aggccagcaa aaggccagga accgtaaaaa 9060
   ggccgcgttg ctggcgtttt tccataggct ccgcccccct gacgagcatc acaaaaatcg 9120
   acgctcaagt cagaggtggc gaaacccgac aggactataa agataccagg cgtttccccc 9180
   tggaagctcc ctcgtgcgct ctcctgttcc gaccctgccg cttaccggat acctgtccgc 9240
   ctttctccct tcgggaagcg tggcgctttc tcatagctca cgctgtaggt atctcagttc 9300
   ggtgtaggtc gttcgctcca agctgggctg tgtgcacgaa ccccccgttc agcccgaccg 9360
   ctgcgcctta tccggtaact atcgtcttga gtccaacccg gtaagacacg acttatcgcc 9420
   actggcagca gccactggta acaggattag cagagcgagg tatgtaggcg gtgctacaga 9480
   gttcttgaag tggtggccta actacggcta cactagaagg acagtatttg gtatctgcgc 9540
   tctgctgaag ccagttacct tcggaaaaag agttggtagc tcttgatccg gcaaacaaac 9600
   caccgctggt agcggtggtt tttttgtttg caagcagcag attacgcgca gaaaaaaagg 9660
   atctcaagaa gatcctttga tcttttctac ggggtctgac gctcagtgga acgaaaactc 9720
   acgttaaggg attttggtca tgagattatc aaaaaggatc ttcacctaga tccttttaaa 9780
   ttaaaaatga agttttaaat caatctaaag tatatatgag taaacttggt ctgacagtta 9840
   ccaatgctta atcagtgagg cacctatctc agcgatctgt ctatttcgtt catccatagt 9900
   tgcctgactc cccgtcgtgt agataactac gatacgggag ggcttaccat ctggccccag 9960
   tgctgcaatg ataccgcgag acccacgctc accggctcca gatttatcag caataaacca 10020
   gccagccgga agggccgagc gcagaagtgg tcctgcaact ttatccgcct ccatccagtc 10080
   tattaattgt tgccgggaag ctagagtaag tagttcgcca gttaatagtt tgcgcaacgt 10140
   tgttgccatt gctacaggca tcgtggtgtc acgctcgtcg tttggtatgg cttcattcag 10200
   ctccggttcc caacgatcaa ggcgagttac atgatccccc atgttgtgca aaaaagcggt 10260
   tagctccttc ggtcctccga tcgttgtcag aagtaagttg gccgcagtgt tatcactcat 10320
   ggttatggca gcactgcata attctcttac tgtcatgcca tccgtaagat gcttttctgt 10380
   gactggtgag tactcaacca agtcattctg agaatagtgt atgcggcgac cgagttgctc 10440
   ttgcccggcg tcaatacggg ataataccgc gccacatagc agaactttaa aagtgctcat 10500
   cattggaaaa cgttcttcgg ggcgaaaact ctcaaggatc ttaccgctgt tgagatccag 10560
   ttcgatgtaa cccactcgtg cacccaactg atcttcagca tcttttactt tcaccagcgt 10620
   ttctgggtga gcaaaaacag gaaggcaaaa tgccgcaaaa aagggaataa gggcgacacg 10680
   gaaatgttga atactcatac tcttcctttt tcaatattat tgaagcattt atcagggtta 10740
   ttgtctcatg agcggataca tatttgaatg tatttagaaa aataaacaaa taggggttcc 10800
   gcgcacattt ccccgaaaag tgccac 10826
<210> 88
   <211> 42
   <212> DNA
   <213> Artificial Sequence
<220>
<223> Oligonucleotide Primer
<400> 88
   gacggtatcg ataagcttga tatcgaattc ctgcagcccg gg 42
<210> 89
   <211> 42
   <212> DNA
   <213> Artificial Sequence
<220>
<223> Oligonucleotide primer
<400> 89
   gacctcctgc acaagaacgg taccgggcta gagcggccgc ca 42
<210> 90
<211> 25
   <212> DNA
   <213> Artificial Sequence
<220>
<223> Oligonucleotide primer
<400> 90
   cagtgctgca atgataccgc gagac 25
<210> 91
   <211> 13722
   <212> DNA
   <213> Artificial Sequence
<220>
<223> Plasmid KW2
<400> 91
   cagtgtggtt ttgcaagagg aagcaaaaag cctctccacc caggcctgga atgtttccac     60
   ccaatgtcga gcagtgtggt tttgcaagag gaagcaaaaa gcctctccac ccaggcctgg    120
   aatgtttcca cccaatgtcg agcaaacccc gcccagcgtc ttgtcattgg cgaattcgaa    180
   cacgcagatg cagtcggggc ggcgcggtcc caggtccact tcgcatatta aggtgacgcg    240
   tgtggcctcg aacaccgagc gaccctgcag ccaatatggg atcggccatt gaacaagatg    300
   gattgcacgc aggttctccg gccgcttggg tggagaggct attcggctat gactgggcac    360
   aacagacaat cggctgctct gatgccgccg tgttccggct gtcagcgcag gggcgcccgg    420
   ttctttttgt caagaccgac ctgtccggtg ccctgaatga actgcaggac gaggcagcgc   480
   ggctatcgtg gctggccacg acgggcgttc cttgcgcagc tgtgctcgac gttgtcactg   540
   aagcgggaag ggactggctg ctattgggcg aagtgccggg gcaggatctc ctgtcatctc   600
   accttgctcc tgccgagaaa gtatccatca tggctgatgc aatgcggcgg ctgcatacgc   660
   ttgatccggc tacctgccca ttcgaccacc aagcgaaaca tcgcatcgag cgagcacgta   720
   ctcggatgga agccggtctt gtcgatcagg atgatctgga cgaagagcat caggggctcg   780
   cgccagccga actgttcgcc aggctcaagg cgcgcatgcc cgacggcgag gatctcgtcg   840
   tgacccatgg cgatgcctgc ttgccgaata tcatggtgga aaatggccgc ttttctggat   900
   tcatcgactg tggccggctg ggtgtggcgg accgctatca ggacatagcg ttggctaccc   960
   gtgatattgc tgaagagctt ggcggcgaat gggctgaccg cttcctcgtg ctttacggta  1020
   tcgccgctcc cgattcgcag cgcatcgcct tctatcgcct tcttgacgag ttcttctgag  1080
   gggatcggca ataaaaagac agaataaaac gcacgggtgt tgggtcgttt gttcggatcc  1140
   gtcgacttta tcatcatcat ctttataatc tttatcatca tcatctttat aatcgttatg  1200
   tcctaatttc gaaggcactt gggagtagct agccaccaca gactcgtgct gctcaacggt  1260
   cttccaatcg tcacttatgt tcagcttttc catacgcaaa tgatgaaagt gaagttctgg  1320
   aagctttacc tctttcttcg acttgtaagt tgtttccatg aagcaagaaa gatgacctcc  1380
   gtcagccagc ataagtgcgg gggtatcgcg aagcagaagt ccaccatctc gtgggatgac  1440
   tgtttcagtt gatggctccc atccacaggt cttcttttgc atcactggac cgtttgcggg  1500
   aaaattggtg ccaaggactt taactttgca gatgatgcaa tcaccctgca aacttgtttc  1560
   ttgggtagct gaaagcactc ctccatcttc ataggtgctt actctttccc aagaaaaacc  1620
   accaggtaga gactgcttaa agaagtcagg aatttctttg gggtacttgg cgaagacctt  1680
   gatgccatac tgaaaggcgt gtgacagaat gtcaaaagca aatggcagag gacctccttc  1740
   agttattgta atattcaggc tttgggtgcc ttcgtatggt tttccctctc cttttccagt  1800
   gcacttgaag gcgtggtagt taacactacc ctccatagaa agctgaacgc gcatggtttc  1860
   cttgatggaa ggatacatgg taccatgact ctcagaaagc acttcccatg tgagctggac  1920
   cctgaattta aggaaatgtg tagtcatttc ctgtgggtgc ctaagtgagg atttgcatgt  1980
   gggtggtgcc tttgtatgga taggtaaaaa gggatgaggg aggccccagt cttttgggct  2040
   caccctggga ggtgtatgct ggctgtgccc tctgagaact cagttctctt cctgtggcct  2100
   cccctcacca aacccagagt cctcttcttc caggtaggaa atgtgctgaa ggagctggtc 2160
   tgggagacaa gtgtgatcat ggatcaaaga cagattttgg aatacagtta atactgttct 2220
   acatttaaag attcatataa caccaaccat acacccaggt cacctaaatt gtcatttacc 2280
   ccttcagaca tattgaaaca gctgctgagt gtaataatca cagtgaattg agacaaacct 2340
   ggatccatgc aatgtgtact gtagttcaga acatccatca tggttagaag gatgctacct 2400
   gtcccaggaa gtgggttatt tttaaatagt acctgagagc tgcccttctg agaccttttg 2460
   aaatttgaga ttgtgtgtga gatctcagga gaaggtagta gaatatatct ccatccttct 2520
   caatgtgtaa ccctgagaat atggcctgac ctctaaacat ttctgtgtga aaagatgtac 2580
   attggggata gcagtgacag cttcagatga aaactctata gtacatcagc actggaggat 2640
   agtctcatca ccaagattag tgaaattacc tttcctggga accagagagg acctctgtga 2700
   gctctaccct ctgagagaac aaggaactct ggttcttccc tgacaggtca cacctgtgaa 2760
   acatggctgg acaatgacac tcaaacccag aattcgtacc cacatattta ccaattcaga 2820
   tccatctgtc tctgaaagaa tttctcctcc gctgaattgc aagaacatac cctagggtgt 2880
   gcagtattgc aacttgggca tttcacatta gtttggtgaa ttatataata cacaaaatat 2940
   ctccatggat gttgtaacag gagagtcatc agaagctggt tgtgttgtat aatctggata 3000
   aacctgggct ctcttcttag gagctgaaca agtgggctgg ccttctatga gacgacagag 3060
   ggaaagagac agactcaata tccagagcga ggtgagctcc ttacctacct accaggtggt 3120
   ctctgggcca tttgtttgag cagacccaga agtaccttcc tcaccctcag gagaattatg 3180
   aacattgaga gaaactgaga tacttttttt atttacaggg aatatttcat cggcgtgttt 3240
   acatctacct gggtgtgtac agggatgcta ggatgtgctc atacacagaa gagcaagaat 3300
   tatatttcgt ggaaagaaaa ccaaagagct tctgaatttg taggtattgt ttgctgcaaa 3360
   tgtgtcaggt cactagatca tgttatgctg ctagaagaaa aacttcccaa cattgtcatg 3420
   gagacaaaat gcaaaacagt aaagattcaa ctgagattcc cttgaaaatc accagtaatg 3480
   aacaggccaa aagaaatcaa ccattgtgga aagagtggtc attaagtgaa atagcaaatt 3540
   ccatgttgca gtgagaagga agatccatct gacagctcat tttcacctct acaaagactt 3600
   cagaacatag actaagagca gagcgtgaac ttagggcaaa cagaggccag atgtttgagg 3660
   aggttggaga gtgagctgga gtcattgtga gccattcaga aaagcagagt gttccagggt 3720
   gtattgagtc ctcctgagtt aagaggtgct gaatatacgc aagtttcact gccctcattg 3780
   cgttttattc tctagactct cttggatgtc cagatttgaa catgtggagt gttgatggaa 3840
   ctcaacataa ctaggaactt ttcagtgaag gtgtaggtaa caatgtgggt ataattaaat 3900
   tcggtttatg aaaatattat tatccgaaat gtcaaagtca gtatctatta atttatcttt 3960
   cttttgtatt ttacagacaa gattattctg ttgcccaggc tggagtgcag actcacctat 4020
   taatttaaca gcataaaaac gatcagtcca atttacgtag tcctgtaatc tccatcaagg 4080
   atttaggtcc atgtggcctg tgacagagct ctagtcacag agggaagaga gtggtttgtt 4140
   gggttgatgc tgcttcttca gaggggaatt taaacaactc ctcacctcat caagtctatt 4200
   tttataactg tgcaagaccc ttgggaatgc actcaccatt tcttacataa tgggagttga 4260
   ctgtgtcatg aaggtaacac gaagatgtgc aaatttaaag cctggttaca taacctgttg 4320
   aatttaaaat gcctggagcc aatcacattc tggcatcttg tttaattagt tctgaatgta 4380
   ttttcagttg gttagttcaa gttcccataa ttcactttct gctaaaatag tcacatacaa 4440
   taatcttgag atattaaaat aaaaaaacta atttgaaaat gaacccaact tccaggagag 4500
   atgaaagttc ctttgtggtg aagggttgaa atatggttga acactgaggt tgtcttcaca 4560
   attgttttaa ttaggggaac ttctatacat cccttatatt tattagaaac tcccattgag 4620
   aaccttgaac taacgtaatt agttgatgga gcacacaaca acaatgctga aggttattaa 4680
   gcaggaattg ctattacaac gttagccttg ctttaaagca catatttcta cctgatgtga 4740
   aattagccca ggtgcgctga tgagagtttg tcagttaatc aaagaccagg aaatggatac 4800
   acgtgtttct ggagcagggc atggctttgg gatgctttgc gaaaaaagtg gcttctcacg 4860
   tctttgggaa aacccatcaa aatgggcaag ttaaggatct cttaggagca cccgtctatc 4920
   ccatattctt ggctaatatt aaagcggaac tcaatgcaaa atgagatact atggaagttc 4980
   agaaaactgc tgtaccactg cctcagctca gcacagctgc ctccttcctc aggctttctg 5040
   aaactctcgg gatgtgggtt tccacactgt gtacttcgca cagtaatact cggccgtgtc 5100
   ctcagccttt aggctgctga tctgaagaca cgccgtgctg acagacgtgt ccatggagaa 5160
   gacaaaccat cctgtgaagc cgtgggtata cgttgggttc ccagtgtagg tgatgatcca 5220
   tcacatccac tcaaagccct gtccaggggt ctatcatacc caattcatac catagatggt 5280
   gaaggtgtaa ccagaagact tataggagac cttcactgag gccccaggct tcttcacctc 5340
   aggcccagac tgcaccagct gcagggagtg ggcacctgtg gagtggacac aagagtgggt 5400
   gaagtctcac atgactggcc tggtttcttc ctcagccctg agactgggga gccccttacc 5460
   tgttgctgct gccatcaaga agaggatcct tcaggtccag tccatggtga ggagctgtga 5520
   tctaggggct tctccagagg aggggtgtgg ttgttgggtg aggctctcag ggaacggaga 5580
   tactatagtc acctcagtta attgcatatt catgaaggat gctatttaat agcccaattc 5640
   ctgacccagt atgagaaaca gacacatggg tgacacaact gtagaagctg agggttcaag 5700
   ccgtaatcct gttagaggcc atgtgtcccc tacacatccc tgaactctgt gttgacagag 5760
   cttcccccac tggagaacaa gctcctcaag gacagcacct cactttgaaa ccacatttga 5820
   ctgtctcagg gtcaacttgc atcatttcta gaccataata tgtgaatgcg ttatttaggg 5880
   aatgactgtg tttatccaaa aattgcattt atttataaga aaggatctct tcctgacctc 5940
   cagcagagtt tgaaatcccc attgtaaaag tggttctcat tacaacatcc agtttgataa 6000
   atgctcacaa ttgaatagat atttatacaa acttcagcag tctttgtgaa atacttattt 6060
   tagatatttt taaaggaagt cccaggccct gggaggaacc tctccccagc ctcctgtgca 6120
   cctgctctgg ggcgggagcc tgtgctgggt gtatccggag cgccccctgc agcccagccc 6180
   caaccatgca gggaggtttc tatctgagct gacagagtat attcctacca gtgtatccag 6240
   ctcagtataa agtggttgtg ccctggctca gaattctcct ttagggacac cacatgctcc 6300
   tcacaccatc ttttgaaata gtgaattgcc tttaggaaac ccagtgaact ctgcagagag 6360
   actccaagaa aagatctcat gcatcaccag ggagcccttt cctggagctc aagaggcaca 6420
   gaatcattgg acacacggtg aacccaaaca ctcttcaggg gttgagggga gactcttatt 6480
   tcctttaggg tcctacagtt gattatggca cctgagaata cctgcaggtg caggtacatg 6540
   tggatagaaa cccactccaa ctctgctatt caactcacac atgcgcgcgc gtgtgtacac 6600
   acacacacac acacacacac acatatatat atatacatcg tggctaattt ttatattaac 6660
   ggatcccatg tttgccattt ttttctggta tccatctcat ggaaagcgct ccctacactg 6720
   gtactaagac tgaatatgtg tctactttct gtaaacagaa gtaaagaaac agaatacaag 6780
   tggacacttg ggaagtgcat gcacattgaa ttcacctggt ctcactttgg aaccctgcag 6840
   atgccccgtg aaaactaaat tatcgattgc cagccctggg accgaacccc gcgtttatga 6900
   acaaacgacc caacacccgt gcgttttatt ctgtcttttt attgccgtca tagcgcgggt 6960
   tccttccggt attgtctcct tccgtgtttc agttagcctc ccccatctcc cgggcaaacg 7020
   tgcgcgccag gtcgcagatc gtcggtatgg agcctggggt ggtgacgtgg gtctggacca 7080
   tcccggaggt aagttgcagc agggcgtccc ggtagccggc gggcgattgg tcgtaatcca 7140
   ggataaagac gtgcatggga cggaggcgtt tggccaagac gtccaaggcc caggcaaaca 7200
   cgttatacag gtcgccgttg ggggccagca actcgggggc ccgaaacagg gtaaataacg 7260
   tgtccccgat atggggtcgt gggcccgcgt tgctctgggg ctcggcaccc tggggcggca 7320
   cggccgtccc cgaaagctgt ccccaatcct cccgccacga cccgccgccc tgcagatacc 7380
   gcaccgtatt ggcaagcagc ccgtaaacgc ggcgaatcgc ggccagcata gccaggtcaa 7440
   gccgctcgcc ggggcgctgg cgtttggcca ggcggtcgat gtgtctgtcc tccggaaggg 7500
   cccccaacac gatgtttgtg ccgggcaagg tcggcgggat gagggccacg aacgccagca 7560
   cggcctgggg ggtcatgctg cccataaggt atcgcgcggc cgggtagcac aggagggcgg 7620
   cgatgggatg gcggtcgaag atgagggtga gggccggggg cggggcatgt gagctcccag 7680
   cctccccccc gatatgagga gccagaacgg cgtcggtcac ggcataaggc atgcccattg 7740
   ttatctgggc gcttgtcatt accaccgccg cgtccccggc cgatatctca ccctggtcga 7800
   ggcggtgttg tgtggtgtag atgttcgcga ttgtctcgga agcccccagc acctgccagt 7860
   aagtcatcgg ctcgggtacg tagacgatat cgtcgcgcga acccagggcc accagcagtt 7920
   gcgtggtggt ggttttcccc atcccgtgag gaccgtctat ataaacccgc agtagcgtgg 7980
   gcattttctg ctccaggcgg acttccgtgg cttcttgctg ccggcgaggg cgcaacgccg 8040
   tacgtcggtt gctatggccg cgagaacgcg cagcctggtc gaacgcagac gcgtgttgat 8100
   ggcaggggta cgaagccata cgcgcttcta caaggcgctt gccgaagagg tgcgggagtt 8160
   tcacgccacc aagatctgcg gcacgctgtt gacgctgtta agcgggtcgc tgcaggtcga 8220
   aaggcccgga gatgaggaag aggagaacag cgcggcagac gtgcgctttt gaagcgtgca 8280
   gaatgccggg cctccggagg accttcgggc gcccgccccg cccctgagcc cgcccctgag 8340
   cccgcccccg gacccacccc ttcccagcct ctgagcccag aaagcgaagg agcaaagctg 8400
   ctattggccg ctgccccaaa ggcctacccg cttccattgc tcagcggtgc tgtccatctg 8460
   cacgagacta gtgagacgtg ctacttccat ttgtcacgtc ctgcacgacg cgagctgcgg 8520
   ggcggggggg aacttcctga ctaggggagg agtagaaggt ggcgcgaagg ggccaccaaa 8580
   gaacggagcc ggttggcgcc taccggtgga tgtggaatgt gtgcgaggcc agaggccact 8640
   tgtgtagcgc caagtgccca gcggggctgc taaagcgcat gctccagact gcaagcttgg 8700
   cgtaatcatg gtcatagctg tttcctgtgt gaaattgtta tccgctcaca attccacaca 8760
   acatacgagc cggaagcata aagtgtaaag cctggggtgc ctaatgagtg aggtaactca 8820
   cattaattgc gttgcgctca ctgcccgctt tccagtcggg aaacctgtcg tgccagctgc 8880
   attaatgaat cggccaacgc gcggggagag gcggtttgcg tattggcgct cttccgcttc 8940
   ctcgctcact gactcgctgc gctcggtcgt tcggctgcgg cgagcggtat cagctcactc 9000
   aaaggcggta atacggttat ccacagaatc aggggataac gcaggaaaga acatgtgagc 9060
   aaaaggccag caaaaggcca ggaaccgtaa aaaggccgcg ttgctggcgt ttttccatag 9120
   gctccgcccc cctgacgagc atcacaaaaa tcgacgctca agtcagaggt ggcgaaaccc 9180
   gacaggacta taaagatacc aggcgtttcc ccctggaagc tccctcgtgc gctctcctgt 9240
   tccgaccctg ccgcttaccg gatacctgtc cgcctttctc ccttcgggaa gcgtggcgct 9300
   ttctcaatgc tcacgctgta ggtatctcag ttcggtgtag gtcgttcgct ccaagctggg 9360
   ctgtgtgcac gaaccccccg ttcagcccga ccgctgcgcc ttatccggta actatcgtct 9420
   tgagtccaac ccggtaagac acgacttatc gccactggca gcagccactg gtaacaggat 9480
   tagcagagcg aggtatgtag gcggtgctac agagttcttg aagtggtggc ctaactacgg 9540
   ctacactaga aggacagtat ttggtatctg cgctctgctg aagccagtta ccttcggaaa 9600
   aagagttggt agctcttgat ccggcaaaca aaccaccgct ggtagcggtg gtttttttgt 9660
   ttgcaagcag cagattacgc gcagaaaaaa aggatctcaa gaagatcctt tgatcttttc 9720
   tacggggtct gacgctcagt ggaacgaaaa ctcacgttaa gggattttgg tcatgagatt 9780
   atcaaaaagg atcttcacct agatcctttt aaattaaaaa tgaagtttta aatcaatcta 9840
   aagtatatat gagtaaactt ggtctgacag ttaccaatgc ttaatcagtg aggcacctat 9900
   ctcagcgatc tgtctatttc gttcatccat agttgcctga ctccccgtcg tgtagataac 9960
   tacgatacgg gagggcttac catctggccc cagtgctgca atgataccgc gagacccacg 10020
   ctcaccggct ccagatttat cagcaataaa ccagccagcc ggaagggccg agcgcagaag 10080
   tggtcctgca actttatccg cctccatcca gtctattaat tgttgccggg aagctagagt 10140
   aagtagttcg ccagttaata gtttgcgcaa cgttgttgcc attgctacag gcatcgtggt 10200
   gtcacgctcg tcgtttggta tggcttcatt cagctccggt tcccaacgat caaggcgagt 10260
   tacatgatcc cccatgttgt gcaaaaaagc ggttagctcc ttcggtcctc cgatcgttgt 10320
   cagaagtaag ttggccgcag tgttatcact catggttatg gcagcactgc ataattctct 10380
   tactgtcatg ccatccgtaa gatgcttttc tgtgactggt gagtactcaa ccaagtcatt 10440
   ctgagaatag tgtatgcggc gaccgagttg ctcttgcccg gcgtcaatac gggataatac 10500
   cgcgccacat agcagaactt taaaagtgct catcattgga aaacgttctt cggggcgaaa 10560
   actctcaagg atcttaccgc tgttgagatc cagttcgatg taacccactc gtgcacccaa 10620
   ctgatcttca gcatctttta ctttcaccag cgtttctggg tgagcaaaaa caggaaggca 10680
   aaatgccgca aaaaagggaa taagggcgac acggaaatgt tgaatactca tactcttcct 10740
   ttttcaatat tattgaagca tttatcaggg ttattgtctc atgagcggat acatatttga 10800
   atgtatttag aaaaataaac aaataggggt tccgcgcaca tttccccgaa aagtgccacc 10860
   tgacgtcgcc cagcccagcc tagctcagcc cagcccagct caattcagcc cagccctgcc 10920
   cagctcagcc caccttaatg cagccaagcc cagctcaact cagctcacct ggtgcaactt 10980
   agcccagctc agctcagctc agctcagccc agttcaactc agcccagttc agctcagctc 11040
   agcccagttc ggccttgttt agtctaggtc aacttaggtc agttttgccc atctgagtcc 11100
   atttctgaaa actggatgga gttgtcatgg ccagaaatgg tcagcccacc agacctgctt 11160
   gtctcagcta aagccatctc attgccgggt tcctgcacag ccaggctggc ttccatcttt 11220
   tgtctccctc tacttgatac cccagttccc tgcagtcctg ccccagcgcc acctgggttt 11280
   tggttccaaa gcattaccaa tcattaccac cctccactac ctgggtggaa tatttctttg 11340
   ctgctttaaa gtcattaaaa catcttgaga atgagaccaa gaatttagga gcctgtgctg 11400
   tgataaaaat gagcaggtcc ccttgctcta gaagtggcag catatcttct gcaccaagag 11460
   gagggtattg agatgctcag agcctccacc ttcccggagc atcccctccc ttctgagtct 11520
   gcagtaaacc cctgccttta aattccctct agataacagt catcattgga aacaaccaag 11580
   aaatgcattt tatctgaatt tgccacttaa aattctgcca tttaccataa atcgctttgg 11640
   aaggcatggg ctactttcaa gggtgcgatg atgacctaca gtcaatgact tagacaaggg 11700
   cgatgccagt gggacttggt atgttctcaa gcatcattac ccatgccatc cccattcaga 11760
   ggttgtggag cagctcgtgc gacctctcct tcaaatgggc tttagggaaa gttaaatggg 11820
   agtgacccag acaatggtca ctcaaaagac tcacataaat gagtctcctg ctcttcatca 11880
   agcaattaag accagttccc cttctagtgg aaataagacg tcaaatacaa agttttaaga 11940
   gaagcaaatg cagcagcggc ggctgcctgt ctcttaccat gtcgggcgcc tggtcactgc 12000
   gagccttgca aagctttggc atggaatcat tcctccaagt ccattaacaa gggctggggc 12060
   ctgagcagcc agtcggcccg gcagcagaag ccacgcatcc cagctctggg tagtccgggg 12120
   agacccagag cccaggccgg gcctggcagc caccctccca gagcctccgc taggccagtc 12180
   ctgctgacgc cgcatcggtg attcggaaca gaatctgtcc ttctaaggtg tctccacagt 12240
   cctgtcttca gcactatctg attgagtttt ctcttatgcc accaactaac atgcttaact 12300
   gaaataattc aggataatga tgcacatttt tcctaaaact tatcctaaag tgagtagttg 12360
   aaaagtggtc ttgaaaaata ctaaaatgaa ggccactcta tcagaatatc aaagtgtttc 12420
   tccttaatca caaagagaaa acgagttaac ctaaaaagat tgtgaacaca gtcattatga 12480
   aaataatgct ctgaggtatc gaaaaagtat ttgagattaa ttatcacatg aagggataac 12540
   aagctaattt aaaaaacttt ttgaatacag tcataaactc tccctaagac tgtttaattt 12600
   cttaaacatc ttactttaaa aatgaatgca gtttagaagt tgatatgctg tttgcacaaa  12660
   ctagcagttg ataagctaag attggaaatg aaattcagat agttaaaaaa agccttttca  12720
   gtttcggtca gcctcgcctt attttagaaa cgcaaattgt ccaggtgttg ttttgctcag  12780
   tagagcactt tcagatctgg gcctgggcaa aaccacctct tcacaaccag aagtgataaa  12840
   tttaccaatt gtgttttttt gcttcctaaa atagactctc gcggtgacct gcttcctgcc  12900
   acctgctgtg ggtgccggag acccccatgc agccatcttg actctaattc atcatctgct  12960
   tccagcttcg ctcaattaat taaaaaaata aacttgattt atgatggtca aaacgcagtc  13020
   ccgcatcggg gccgacagca ctgtgctagt atttcttagc tgagcttgct ttggcctcaa  13080
   ttccagacac atatcactca tgggtgttaa tcaaatgata agaatttcaa atacttggac  13140
   agttaaaaaa attaatatac ttgaaaatct ctcacatttt taagtcataa ttttcttaac  13200
   catttttctc agaagccact tcaaacatat cctgtctttt aacagtaagc atgcctccta  13260
   agataaacaa tccttttctc atggaaacca gcttcaaggc actgaggtcc tggagcctcc  13320
   ctaagcccct gtcaggacgg cagccaccgt ttctgggcta cccctgcccc caaccctgct  13380
   ctcatcaaga ccggggctac gcgtccctcc tggctggatt cacccactcc gacagttctc  13440
   tttccagcca ataaagaatt taagatgcag gttgacacac agcgcacctc ataattctaa  13500
   agaaaatatt tcacgattcg ctgctgtgca gcgatcttgc agtcctacag acaccgctcc  13560
   tgagacacat tcctcagcca tcactaagac ccctggtttg ttcaggcatc tcgtccaaat  13620
   gtggctcccc aagcccccag gctcagttac tccatcagac gcacccaacc tgagtcccat  13680
   tttccaaagg catcggaaaa tccacagagg ctcccactcg ag                     13722
<210> 92
   <211> 33
   <212> DNA
   <213> Artificial Sequence
<220>
<223> Oligonucleotide primer
<400> 92
   ccgctcgaga tgtatccttc catcaaggaa acc 33
<210> 93
   <211> 69
   <212> DNA
   <213> Artificial Sequence
<220>
<223> Oligonucleotide primer
<400> 93
   tctagattat tatttatcat catcatcttt ataatcttta tcatcatcat ctttataatc     60
   agcggccgc                                                             69
<210> 94
   <211> 31
   <212> DNA
   <213> Artificial Sequence
<220>
<223> Oligonucleotide primer
<400> 94
   ctagtctaga ttattattta tcatcatcat c 31
<210> 95
   <211> 33 <212> DNA
   <213> Artificial Sequence
<220>
<223> Oligonucleotide primer
<400> 95
   gttatgtcct aatttcgaag gcacttggga gta 33
<210> 96
   <211> 30
   <212> PRT
   <213> Homo sapiens
<400> 96
   Met Lys Thr Pro Trp Lys Val Leu Leu Gly Leu Leu Gly Ala Ala Ala
   1               5                   10                  15
   Leu Val Thr Ile Ile Thr Val Pro Val Val Leu Leu Asn Lys
               20                  25                  30
<210> 97
   <211> 90
   <212> DNA
   <213> Homo sapiens
<400> 97
   atgaagacac cgtggaaggt tctcctggga ctgctgggtg ctgctgcgct tgtcaccatc 60
   atcaccgtgc ccgtggttct gctgaacaaa 90
<210> 98
   <211> 50
   <212> DNA
   <213> Artificial Sequence
<220>
<223> Oligonucleotide primer
<400> 98
   cgtgcccgtg gttctgctga acaaaatgta tccttccatc aaggaaacca 50
<210> 99
   <211> 51
   <212> DNA
<213> Artificial Sequence
<220>
<223> Oligonucleotide primer
<400> 99
   ccgtgcccgt ggttctgctg aacaaagtgt atccttccat caaggaaacc a 51
<210> 100
   <211> 52
   <212> DNA
   <213> Artificial Sequence
<220>
<223> Oligonucleotide primer
<400> 100
   tgctgctgcg cttgtcacca tcatcaccgt gcccgtggtt ctgctgaaca aa 52
<210> 101
   <211> 51
   <212> DNA
   <213> Artificial Sequence
<220>
<223> Oligonucleotide primer
<400> 101
   ggaaggttct cctgggactg ctgggtgctg ctgcgcttgt caccatcatc a 51
<210> 102
   <211> 47
   <212> DNA
   <213> Artificial Sequence
<220>
<223> Oligonucleotide primer
<400> 102
   ccggaattca tgaagacacc gtggaaggtt ctcctgggac tgctggg 47
<210> 103
   <211> 49
   <212> DNA
   <213> Artificial Sequence
<220>
<223> Oligonucleotide primer
<400> 103
   gactagttta ttatttatca tcatcatctt tataatcttt atcatcatc 49
<210> 104
   <211> 3735
   <212> DNA
   <213> Artificial Sequence
<220>
<223> Plasmid pME18sCD26asfp499
<400> 104
   aagcttggct gtggaatgtg tgtcagttag ggtgtggaaa gtccccaggc tccccagcag      60
   gcagaagtat gcaaagcatg catctcaatt agtcagcaac catagtcccg cccctaactc     120
   cgcccatccc gcccctaact ccgcccagtt ccgcccattc tccgccccat ggctgactaa     180
   ttttttttat ttatgcagag gccgaggccg cctcggcctc tgagctattc cagaagtagt      240
   gaggaggctt ttttggaggc ctaggctttt gcaaaaagct cctcgatcga ggggctcgca     300
   tctctccttc acgcgcccgc cgccctacct gaggccgcca tccacgccgg ttgagtcgcg     360
   ttctgccgcc tcccgcctgt ggtgcctcct gaactgcgtc cgccgtctag gtaagtttaa     420
   agctcaggtc gagaccgggc ctttgtccgg cgctcccttg gagcctacct agactcagcc     480
   ggctctccac gctttgcctg accctgcttg ctcaactcta cgtctttgtt tcgttttctg     540
   ttctgcgccg ttacagatcc aagctctgaa aaaccagaaa gttaactggt aagtttagtc     600
   tttttgtctt ttatttcagg tcccggatcc ggtggtggtg caaatcaaag aactgctcct     660
   cagtggatgt tgcctttact tctaggcctg tacggaagtg ttacttctgc tctaaaagct     720
   gcggaattca tgaagacacc gtggaaggtt ctcctgggac tgctgggtgc tgctgcgctt     780
   gtcaccatca tcaccgtgcc cgtggttctg ctgaacaaaa tgtatccttc catcaaggaa     840
   accatgcgcg ttcagctttc tatggagggt agtgttaact accacgcctt caagtgcact    900
   ggaaaaggag agggaaaacc atacgaaggc acccaaagcc tgaatattac aataactgaa    960
   ggaggtcctc tgccatttgc ttttgacatt ctgtcacacg cctttcagta tggcatcaag   1020
   gtcttcgcca agtaccccaa agaaattcct gacttcttta agcagtctct acctggtggt   1080
   ttttcttggg aaagagtaag cacctatgaa gatggaggag tgctttcagc tacccaagaa   1140
   acaagtttgc agggtgattg catcatctgc aaagttaaag tccttggcac caattttccc   1200
   gcaaacggtc cagtgatgca aaagaagacc tgtggatggg agccatcaac tgaaacagtc   1260
   atcccacgag atggtggact tctgcttcgc gatacccccg cacttatgct ggctgacgga   1320
   ggtcatcttt cttgcttcat ggaaacaact tacaagtcga agaaagaggt aaagcttcca   1380
   gaacttcact ttcatcattt gcgtatggaa aagctgaaca taagtgacga ttggaagacc   1440
   gttgagcagc acgagtctgt ggtggctagc tactcccaag tgccttcgaa attaggacat   1500
   aacgcggccg ctgattataa agatgatgat gataaagatt ataaagatga tgatgataaa   1560
   taataaacta gtctagagaa aaaacctccc acacctcccc ctgaacctga aacataaaat   1620
   gaatgcaatt gttgttgtta acttgtttat tgcagcttat aatggttaca aataaagcaa   1680
   tagcatcaca aatttcacaa ataaagcatt tttttcactg cattctagtt gtggtttgtc   1740
   caaactcatc aatgtatctt atcatgtctg gatccccggg taccgagctc gaattaattc   1800
   ctcttccgct tcctcgctca ctgactcgct gcgctcggtc gttcggctgc ggcgagcggt   1860
   atcagctcac tcaaaggcgg taatacggtt atccacagaa tcaggggata acgcaggaaa   1920
   gaacatgtga gcaaaaggcc agcaaaaggc caggaaccgt aaaaaggccg cgttgctggc   1980
   gtttttccat aggctccgcc cccctgacga gcatcacaaa aatcgacgct caagtcagag   2040
   gtggcgaaac ccgacaggac tataaagata ccaggcgttt ccccctggaa gctccctcgt   2100
   gcgctctcct gttccgaccc tgccgcttac cggatacctg tccgcctttc tcccttcggg   2160
   aagcgtggcg ctttctcata gctcacgctg taggtatctc agttcggtgt aggtcgttcg   2220
   ctccaagctg ggctgtgtgc acgaaccccc cgttcagccc gaccgctgcg ccttatccgg   2280
   taactatcgt cttgagtcca acccggtaag acacgactta tcgccactgg cagcagccac   2340
   tggtaacagg attagcagag cgaggtatgt aggcggtgct acagagttct tgaagtggtg   2400
   gcctaactac ggctacacta gaagaacagt atttggtatc tgcgctctgc tgaagccagt   2460
   taccttcgga aaaagagttg gtagctcttg atccggcaaa caaaccaccg ctggtagcgg   2520
   tggttttttt gtttgcaagc agcagattac gcgcagaaaa aaaggatctc aagaagatcc   2580
   tttgatcttt tctacggggt ctgacgctca gtggaacgaa aactcacgtt aagggatttt 2640
   ggtcatgaga ttatcaaaaa ggatcttcac ctagatcctt ttaaattaaa aatgaagttt 2700
   taaatcaatc taaagtatat atgagtaaac ttggtctgac agttaccaat gcttaatcag 2760
   tgaggcacct atctcagcga tctgtctatt tcgttcatcc atagttgcct gactccccgt 2820
   cgtgtagata actacgatac gggagggctt accatctggc cccagtgctg caatgatacc 2880
   gcgagaccca cgctcaccgg ctccagattt atcagcaata aaccagccag ccggaagggc 2940
   cgagcgcaga agtggtcctg caactttatc cgcctccatc cagtctatta attgttgccg 3000
   ggaagctaga gtaagtagtt cgccagttaa tagtttgcgc aacgttgttg ccattgctac 3060
   aggcatcgtg gtgtcacgct cgtcgtttgg tatggcttca ttcagctccg gttcccaacg 3120
   atcaaggcga gttacatgat cccccatgtt gtgcaaaaaa gcggttagct ccttcggtcc 3180
   tccgatcgtt gtcagaagta agttggccgc agtgttatca ctcatggtta tggcagcact 3240
   gcataattct cttactgtca tgccatccgt aagatgcttt tctgtgactg gtgagtactc 3300
   aaccaagtca ttctgagaat agtgtatgcg gcgaccgagt tgctcttgcc cggcgtcaat 3360
   acgggataat accgcgccac atagcagaac tttaaaagtg ctcatcattg gaaaacgttc 3420
   ttcggggcga aaactctcaa ggatcttacc gctgttgaga tccagttcga tgtaacccac 3480
   tcgtgcaccc aactgatctt cagcatcttt tactttcacc agcgtttctg ggtgagcaaa 3540
   aacaggaagg caaaatgccg caaaaaaggg aataagggcg acacggaaat gttgaatact 3600
   catactcttc ctttttcaat attattgaag catttatcag ggttattgtc tcatgagcgg 3660
   atacatattt gaatgtattt agaaaaataa acaaataggg gttccgcgca catttccccg 3720
   aaaagtgcca cctgc 3735
<210> 105
   <211> 28
   <212> DNA
   <213> Artificial Sequence
<220>
<223> Oligonucleotide primer
<400> 105
   atggacagcc tcttgatgaa ccggagga 28
<210> 106
   <211> 25
   <212> DNA
   <213> Artificial Sequence
<220>
<223> Oligonucleotide primer
<400> 106
   caaagtccca aagtacgaaa tgcgt 25
<210> 107
   <211> 596
   <212> DNA
   <213> Homo sapiens
<400> 107
   atggacagcc tcttgatgaa ccggaggaag tttctttacc aattcaaaaa tgtccgctgg     60
   gctaagggtc ggcgtgagac ctacctgtgc tacgtagtga agaggcgtga cagtgctaca    120
   tccttttcac tggactttgg ttatcttcgc aataagaacg gctgccacgt ggaattgctc    180
   ttcctccgct acatctcgga ctgggaccta gaccctggcc gctgctaccg cgtcacctgg    240
   ttcacctcct ggagcccctg ctacgactgt gcccgacatg tggccgactt tctgcgaggg    300
   aaccccaacc tcagtctgag gatcttcacc gcgcgcctct acttctgtga ggaccgcaag    360
   gctgagcccg aggggctgcg gcggctgcac cgcgccgggg tgcaaatagc catcatgacc    420
   ttcaaagatt atttttactg ctggaatact tttgtagaaa accatgaaag aactttcaaa    480
   gcctgggaag ggctgcatga aaattcagtt cgtctctcca gacagcttcg gcgcatcctt    540
   ttgcccctgt atgaggttga tgacttacga gacgcatttc gtactttggg actttg        596
<210> 108
   <211> 33
   <212> DNA
   <213> Artificial Sequence
<220>
<223> Oligonucleotide primer
<400> 108
   ccctcgagat ggacagcctc ttgatgaacc gga 33
<210> 109
   <211> 33
   <212> DNA
   <213> Artificial Sequence
<220>
<223> Oligonucleotide primer
<400> 109
   gctctagaca aagtcccaaa gtacgaaatg cgt 33
<210> 110
   <211> 12990
   <212> DNA
   <213> Artificial Sequence
<220>
<223> Plasmid KW3
<400> 110
   gtcgacggta ccatgactct cagaaagcac ttcccatgtg agctggaccc tgaatttaag 60
   gaaatgtgta gtcatttcct gtgggtgcct aagtgaggat ttgcatgtgg gtggtgcctt 120
   tgtatggata ggtaaaaagg gatgagggag gccccagtct tttgggctca ccctgggagg 180
   tgtatgctgg ctgtgccctc tgagaactca gttctcttcc tgtggcctcc cctcaccaaa 240
   cccagagtcc tcttcttcca ggtaggaaat gtgctgaagg agctggtctg ggagacaagt 300
   gtgatcatgg atcaaagaca gattttggaa tacagttaat actgttctac atttaaagat 360
   tcatataaca ccaaccatac acccaggtca cctaaattgt catttacccc ttcagacata 420
   ttgaaacagc tgctgagtgt aataatcaca gtgaattgag acaaacctgg atccatgcaa 480
   tgtgtactgt agttcagaac atccatcatg gttagaagga tgctacctgt cccaggaagt 540
   gggttatttt taaatagtac ctgagagctg cccttctgag accttttgaa atttgagatt 600
   gtgtgtgaga tctcaggaga aggtagtaga atatatctcc atccttctca atgtgtaacc 660
   ctgagaatat ggcctgacct ctaaacattt ctgtgtgaaa agatgtacat tggggatagc 720
   agtgacagct tcagatgaaa actctatagt acatcagcac tggaggatag tctcatcacc 780
   aagattagtg aaattacctt tcctgggaac cagagaggac ctctgtgagc tctaccctct 840
   gagagaacaa ggaactctgg ttcttccctg acaggtcaca cctgtgaaac atggctggac 900
   aatgacactc aaacccagaa ttcgtaccca catatttacc aattcagatc catctgtctc 960
   tgaaagaatt tctcctccgc tgaattgcaa gaacataccc tagggtgtgc agtattgcaa 1020
   cttgggcatt tcacattagt ttggtgaatt atataataca caaaatatct ccatggatgt 1080
   tgtaacagga gagtcatcag aagctggttg tgttgtataa tctggataaa cctgggctct 1140
   cttcttagga gctgaacaag tgggctggcc ttctatgaga cgacagaggg aaagagacag 1200
   actcaatatc cagagcgagg tgagctcctt acctacctac caggtggtct ctgggccatt 1260
   tgtttgagca gacccagaag taccttcctc accctcagga gaattatgaa cattgagaga 1320
   aactgagata ctttttttat ttacagggaa tatttcatcg gcgtgtttac atctacctgg 1380
   gtgtgtacag ggatgctagg atgtgctcat acacagaaga gcaagaatta tatttcgtgg   1440
   aaagaaaacc aaagagcttc tgaatttgta ggtattgttt gctgcaaatg tgtcaggtca   1500
   ctagatcatg ttatgctgct agaagaaaaa cttcccaaca ttgtcatgga gacaaaatgc   1560
   aaaacagtaa agattcaact gagattccct tgaaaatcac cagtaatgaa caggccaaaa   1620
   gaaatcaacc attgtggaaa gagtggtcat taagtgaaat agcaaattcc atgttgcagt   1680
   gagaaggaag atccatctga cagctcattt tcacctctac aaagacttca gaacatagac   1740
   taagagcaga gcgtgaactt agggcaaaca gaggccagat gtttgaggag gttggagagt   1800
   gagctggagt cattgtgagc cattcagaaa agcagagtgt tccagggtgt attgagtcct   1860
   cctgagttaa gaggtgctga atatacgcaa gtttcactgc cctcattgcg ttttattctc   1920
   tagactctct tggatgtcca gatttgaaca tgtggagtgt tgatggaact caacataact   1980
   aggaactttt cagtgaaggt gtaggtaaca atgtgggtat aattaaattc ggtttatgaa   2040
   aatattatta tccgaaatgt caaagtcagt atctattaat ttatctttct tttgtatttt   2100
   acagacaaga ttattctgtt gcccaggctg gagtgcagac tcacctatta atttaacagc   2160
   ataaaaacga tcagtccaat ttacgtagtc ctgtaatctc catcaaggat ttaggtccat   2220
   gtggcctgtg acagagctct agtcacagag ggaagagagt ggtttgttgg gttgatgctg   2280
   cttcttcaga ggggaattta aacaactcct cacctcatca agtctatttt tataactgtg   2340
   caagaccctt gggaatgcac tcaccatttc ttacataatg ggagttgact gtgtcatgaa   2400
   ggtaacacga agatgtgcaa atttaaagcc tggttacata acctgttgaa tttaaaatgc   2460
   ctggagccaa tcacattctg gcatcttgtt taattagttc tgaatgtatt ttcagttggt   2520
   tagttcaagt tcccataatt cactttctgc taaaatagtc acatacaata atcttgagat   2580
   attaaaataa aaaaactaat ttgaaaatga acccaacttc caggagagat gaaagttcct   2640
   ttgtggtgaa gggttgaaat atggttgaac actgaggttg tcttcacaat tgttttaatt   2700
   aggggaactt ctatacatcc cttatattta ttagaaactc ccattgagaa ccttgaacta   2760
   acgtaattag ttgatggagc acacaacaac aatgctgaag gttattaagc aggaattgct   2820
   attacaacgt tagccttgct ttaaagcaca tatttctacc tgatgtgaaa ttagcccagg   2880
   tgcgctgatg agagtttgtc agttaatcaa agaccaggaa atggatacac gtgtttctgg   2940
   agcagggcat ggctttggga tgctttgcga aaaaagtggc ttctcacgtc tttgggaaaa   3000
   cccatcaaaa tgggcaagtt aaggatctct taggagcacc cgtctatccc atattcttgg   3060
   ctaatattaa agcggaactc aatgcaaaat gagatactat ggaagttcag aaaactgctg   3120
   taccactgcc tcagctcagc acagctgcct ccttcctcag gctttctgaa actctcggga 3180
   tgtgggtttc cacactgtgt acttcgcaca gtaatactcg gccgtgtcct cagcctttag 3240
   gctgctgatc tgaagacacg ccgtgctgac agacgtgtcc atggagaaga caaaccatcc 3300
   tgtgaagccg tgggtatacg ttgggttccc agtgtaggtg atgatccatc acatccactc 3360
   aaagccctgt ccaggggtct atcataccca attcatacca tagatggtga aggtgtaacc 3420
   agaagactta taggagacct tcactgaggc cccaggcttc ttcacctcag gcccagactg 3480
   caccagctgc agggagtggg cacctgtgga gtggacacaa gagtgggtga agtctcacat 3540
   gactggcctg gtttcttcct cagccctgag actggggagc cccttacctg ttgctgctgc 3600
   catcaagaag aggatccttc aggtccagtc catggtgagg agctgtgatc taggggcttc 3660
   tccagaggag gggtgtggtt gttgggtgag gctctcaggg aacggagata ctatagtcac 3720
   ctcagttaat tgcatattca tgaaggatgc tatttaatag cccaattcct gacccagtat 3780
   gagaaacaga cacatgggtg acacaactgt agaagctgag ggttcaagcc gtaatcctgt 3840
   tagaggccat gtgtccccta cacatccctg aactctgtgt tgacagagct tcccccactg 3900
   gagaacaagc tcctcaagga cagcacctca ctttgaaacc acatttgact gtctcagggt 3960
   caacttgcat catttctaga ccataatatg tgaatgcgtt atttagggaa tgactgtgtt 4020
   tatccaaaaa ttgcatttat ttataagaaa ggatctcttc ctgacctcca gcagagtttg 4080
   aaatccccat tgtaaaagtg gttctcatta caacatccag tttgataaat gctcacaatt 4140
   gaatagatat ttatacaaac ttcagcagtc tttgtgaaat acttatttta gatattttta 4200
   aaggaagtcc caggccctgg gaggaacctc tccccagcct cctgtgcacc tgctctgggg 4260
   cgggagcctg tgctgggtgt atccggagcg ccccctgcag cccagcccca accatgcagg 4320
   gaggtttcta tctgagctga cagagtatat tcctaccagt gtatccagct cagtataaag 4380
   tggttgtgcc ctggctcaga attctccttt agggacacca catgctcctc acaccatctt 4440
   ttgaaatagt gaattgcctt taggaaaccc agtgaactct gcagagagac tccaagaaaa 4500
   gatctcatgc atcaccaggg agccctttcc tggagctcaa gaggcacaga atcattggac 4560
   acacggtgaa cccaaacact cttcaggggt tgaggggaga ctcttatttc ctttagggtc 4620
   ctacagttga ttatggcacc tgagaatacc tgcaggtgca ggtacatgtg gatagaaacc 4680
   cactccaact ctgctattca actcacacat gcgcgcgcgt gtgtacacac acacacacac 4740
   acacacacac atatatatat atacatcgtg gctaattttt atattaacgg atcccatgtt 4800
   tgccattttt ttctggtatc catctcatgg aaagcgctcc ctacactggt actaagactg 4860
   aatatgtgtc tactttctgt aaacagaagt aaagaaacag aatacaagtg gacacttggg 4920
   aagtgcatgc acattgaatt cacctggtct cactttggaa ccctgcagat gccccgtgaa 4980
   aactaaatta tcgattgcca gccctgggac cgaaccccgc gtttatgaac aaacgaccca 5040
   acacccgtgc gttttattct gtctttttat tgccgtcata gcgcgggttc cttccggtat 5100
   tgtctccttc cgtgtttcag ttagcctccc ccatctcccg ggcaaacgtg cgcgccaggt 5160
   cgcagatcgt cggtatggag cctggggtgg tgacgtgggt ctggaccatc ccggaggtaa 5220
   gttgcagcag ggcgtcccgg tagccggcgg gcgattggtc gtaatccagg ataaagacgt 5280
   gcatgggacg gaggcgtttg gccaagacgt ccaaggccca ggcaaacacg ttatacaggt 5340
   cgccgttggg ggccagcaac tcgggggccc gaaacagggt aaataacgtg tccccgatat 5400
   ggggtcgtgg gcccgcgttg ctctggggct cggcaccctg gggcggcacg gccgtccccg 5460
   aaagctgtcc ccaatcctcc cgccacgacc cgccgccctg cagataccgc accgtattgg 5520
   caagcagccc gtaaacgcgg cgaatcgcgg ccagcatagc caggtcaagc cgctcgccgg 5580
   ggcgctggcg tttggccagg cggtcgatgt gtctgtcctc cggaagggcc cccaacacga 5640
   tgtttgtgcc gggcaaggtc ggcgggatga gggccacgaa cgccagcacg gcctgggggg 5700
   tcatgctgcc cataaggtat cgcgcggccg ggtagcacag gagggcggcg atgggatggc 5760
   ggtcgaagat gagggtgagg gccgggggcg gggcatgtga gctcccagcc tcccccccga 5820
   tatgaggagc cagaacggcg tcggtcacgg cataaggcat gcccattgtt atctgggcgc 5880
   ttgtcattac caccgccgcg tccccggccg atatctcacc ctggtcgagg cggtgttgtg 5940
   tggtgtagat gttcgcgatt gtctcggaag cccccagcac ctgccagtaa gtcatcggct 6000
   cgggtacgta gacgatatcg tcgcgcgaac ccagggccac cagcagttgc gtggtggtgg 6060
   ttttccccat cccgtgagga ccgtctatat aaacccgcag tagcgtgggc attttctgct 6120
   ccaggcggac ttccgtggct tcttgctgcc ggcgagggcg caacgccgta cgtcggttgc 6180
   tatggccgcg agaacgcgca gcctggtcga acgcagacgc gtgttgatgg caggggtacg 6240
   aagccatacg cgcttctaca aggcgcttgc cgaagaggtg cgggagtttc acgccaccaa 6300
   gatctgcggc acgctgttga cgctgttaag cgggtcgctg caggtcgaaa ggcccggaga 6360
   tgaggaagag gagaacagcg cggcagacgt gcgcttttga agcgtgcaga atgccgggcc 6420
   tccggaggac cttcgggcgc ccgccccgcc cctgagcccg cccctgagcc cgcccccgga 6480
   cccacccctt cccagcctct gagcccagaa agcgaaggag caaagctgct attggccgct 6540
   gccccaaagg cctacccgct tccattgctc agcggtgctg tccatctgca cgagactagt 6600
   gagacgtgct acttccattt gtcacgtcct gcacgacgcg agctgcgggg cgggggggaa 6660
   cttcctgact aggggaggag tagaaggtgg cgcgaagggg ccaccaaaga acggagccgg 6720
   ttggcgccta ccggtggatg tggaatgtgt gcgaggccag aggccacttg tgtagcgcca 6780
   agtgcccagc ggggctgcta aagcgcatgc tccagactgc aagcttggcg taatcatggt 6840
   catagctgtt tcctgtgtga aattgttatc cgctcacaat tccacacaac atacgagccg 6900
   gaagcataaa gtgtaaagcc tggggtgcct aatgagtgag gtaactcaca ttaattgcgt 6960
   tgcgctcact gcccgctttc cagtcgggaa acctgtcgtg ccagctgcat taatgaatcg 7020
   gccaacgcgc ggggagaggc ggtttgcgta ttggcgctct tccgcttcct cgctcactga 7080
   ctcgctgcgc tcggtcgttc ggctgcggcg agcggtatca gctcactcaa aggcggtaat 7140
   acggttatcc acagaatcag gggataacgc aggaaagaac atgtgagcaa aaggccagca 7200
   aaaggccagg aaccgtaaaa aggccgcgtt gctggcgttt ttccataggc tccgcccccc 7260
   tgacgagcat cacaaaaatc gacgctcaag tcagaggtgg cgaaacccga caggactata 7320
   aagataccag gcgtttcccc ctggaagctc cctcgtgcgc tctcctgttc cgaccctgcc 7380
   gcttaccgga tacctgtccg cctttctccc ttcgggaagc gtggcgcttt ctcaatgctc 7440
   acgctgtagg tatctcagtt cggtgtaggt cgttcgctcc aagctgggct gtgtgcacga 7500
   accccccgtt cagcccgacc gctgcgcctt atccggtaac tatcgtcttg agtccaaccc 7560
   ggtaagacac gacttatcgc cactggcagc agccactggt aacaggatta gcagagcgag 7620
   gtatgtaggc ggtgctacag agttcttgaa gtggtggcct aactacggct acactagaag 7680
   gacagtattt ggtatctgcg ctctgctgaa gccagttacc ttcggaaaaa gagttggtag 7740
   ctcttgatcc ggcaaacaaa ccaccgctgg tagcggtggt ttttttgttt gcaagcagca 7800
   gattacgcgc agaaaaaaag gatctcaaga agatcctttg atcttttcta cggggtctga 7860
   cgctcagtgg aacgaaaact cacgttaagg gattttggtc atgagattat caaaaaggat 7920
   cttcacctag atccttttaa attaaaaatg aagttttaaa tcaatctaaa gtatatatga 7980
   gtaaacttgg tctgacagtt accaatgctt aatcagtgag gcacctatct cagcgatctg 8040
   tctatttcgt tcatccatag ttgcctgact ccccgtcgtg tagataacta cgatacggga 8100
   gggcttacca tctggcccca gtgctgcaat gataccgcga gacccacgct caccggctcc 8160
   agatttatca gcaataaacc agccagccgg aagggccgag cgcagaagtg gtcctgcaac 8220
   tttatccgcc tccatccagt ctattaattg ttgccgggaa gctagagtaa gtagttcgcc 8280
   agttaatagt ttgcgcaacg ttgttgccat tgctacaggc atcgtggtgt cacgctcgtc 8340
   gtttggtatg gcttcattca gctccggttc ccaacgatca aggcgagtta catgatcccc 8400
   catgttgtgc aaaaaagcgg ttagctcctt cggtcctccg atcgttgtca gaagtaagtt 8460
   ggccgcagtg ttatcactca tggttatggc agcactgcat aattctctta ctgtcatgcc 8520
   atccgtaaga tgcttttctg tgactggtga gtactcaacc aagtcattct gagaatagtg 8580
   tatgcggcga ccgagttgct cttgcccggc gtcaatacgg gataataccg cgccacatag 8640
   cagaacttta aaagtgctca tcattggaaa acgttcttcg gggcgaaaac tctcaaggat 8700
   cttaccgctg ttgagatcca gttcgatgta acccactcgt gcacccaact gatcttcagc 8760
   atcttttact ttcaccagcg tttctgggtg agcaaaaaca ggaaggcaaa atgccgcaaa 8820
   aaagggaata agggcgacac ggaaatgttg aatactcata ctcttccttt ttcaatatta 8880
   ttgaagcatt tatcagggtt attgtctcat gagcggatac atatttgaat gtatttagaa 8940
   aaataaacaa ataggggttc cgcgcacatt tccccgaaaa gtgccacctg acgtcgccca 9000
   gcccagccta gctcagccca gcccagctca attcagccca gccctgccca gctcagccca 9060
   ccttaatgca gccaagccca gctcaactca gctcacctgg tgcaacttag cccagctcag 9120
   ctcagctcag ctcagcccag ttcaactcag cccagttcag ctcagctcag cccagttcgg 9180
   ccttgtttag tctaggtcaa cttaggtcag ttttgcccat ctgagtccat ttctgaaaac 9240
   tggatggagt tgtcatggcc agaaatggtc agcccaccag acctgcttgt ctcagctaaa 9300
   gccatctcat tgccgggttc ctgcacagcc aggctggctt ccatcttttg tctccctcta 9360
   cttgataccc cagttccctg cagtcctgcc ccagcgccac ctgggttttg gttccaaagc 9420
   attaccaatc attaccaccc tccactacct gggtggaata tttctttgct gctttaaagt 9480
   cattaaaaca tcttgagaat gagaccaaga atttaggagc ctgtgctgtg ataaaaatga 9540
   gcaggtcccc ttgctctaga agtggcagca tatcttctgc accaagagga gggtattgag 9600
   atgctcagag cctccacctt cccggagcat cccctccctt ctgagtctgc agtaaacccc 9660
   tgcctttaaa ttccctctag ataacagtca tcattggaaa caaccaagaa atgcatttta 9720
   tctgaatttg ccacttaaaa ttctgccatt taccataaat cgctttggaa ggcatgggct 9780
   actttcaagg gtgcgatgat gacctacagt caatgactta gacaagggcg atgccagtgg 9840
   gacttggtat gttctcaagc atcattaccc atgccatccc cattcagagg ttgtggagca 9900
   gctcgtgcga cctctccttc aaatgggctt tagggaaagt taaatgggag tgacccagac 9960
   aatggtcact caaaagactc acataaatga gtctcctgct cttcatcaag caattaagac 10020
   cagttcccct tctagtggaa ataagacgtc aaatacaaag ttttaagaga agcaaatgca 10080
   gcagcggcgg ctgcctgtct cttaccatgt cgggcgcctg gtcactgcga gccttgcaaa 10140
   gctttggcat ggaatcattc ctccaagtcc attaacaagg gctggggcct gagcagccag 10200
   tcggcccggc agcagaagcc acgcatccca gctctgggta gtccggggag acccagagcc 10260
   caggccgggc ctggcagcca ccctcccaga gcctccgcta ggccagtcct gctgacgccg 10320
   catcggtgat tcggaacaga atctgtcctt ctaaggtgtc tccacagtcc tgtcttcagc 10380
   actatctgat tgagttttct cttatgccac caactaacat gcttaactga aataattcag 10440
   gataatgatg cacatttttc ctaaaactta tcctaaagtg agtagttgaa aagtggtctt 10500
   gaaaaatact aaaatgaagg ccactctatc agaatatcaa agtgtttctc cttaatcaca 10560
   aagagaaaac gagttaacct aaaaagattg tgaacacagt cattatgaaa ataatgctct 10620
   gaggtatcga aaaagtattt gagattaatt atcacatgaa gggataacaa gctaatttaa 10680
   aaaacttttt gaatacagtc ataaactctc cctaagactg tttaatttct taaacatctt 10740
   actttaaaaa tgaatgcagt ttagaagttg atatgctgtt tgcacaaact agcagttgat 10800
   aagctaagat tggaaatgaa attcagatag ttaaaaaaag ccttttcagt ttcggtcagc 10860
   ctcgccttat tttagaaacg caaattgtcc aggtgttgtt ttgctcagta gagcactttc 10920
   agatctgggc ctgggcaaaa ccacctcttc acaaccagaa gtgataaatt taccaattgt 10980
   gtttttttgc ttcctaaaat agactctcgc ggtgacctgc ttcctgccac ctgctgtggg 11040
   tgccggagac ccccatgcag ccatcttgac tctaattcat catctgcttc cagcttcgct 11100
   caattaatta aaaaaataaa cttgatttat gatggtcaaa acgcagtccc gcatcggggc 11160
   cgacagcact gtgctagtat ttcttagctg agcttgcttt ggcctcaatt ccagacacat 11220
   atcactcatg ggtgttaatc aaatgataag aatttcaaat acttggacag ttaaaaaaat 11280
   taatatactt gaaaatctct cacattttta agtcataatt ttcttaacca tttttctcag 11340
   aagccacttc aaacatatcc tgtcttttaa cagtaagcat gcctcctaag ataaacaatc 11400
   cttttctcat ggaaaccagc ttcaaggcac tgaggtcctg gagcctccct aagcccctgt 11460
   caggacggca gccaccgttt ctgggctacc cctgccccca accctgctct catcaagacc 11520
   ggggctacgc gtccctcctg gctggattca cccactccga cagttctctt tccagccaat 11580
   aaagaattta agatgcaggt tgacacacag cgcacctcat aattctaaag aaaatatttc 11640
   acgattcgct gctgtgcagc gatcttgcag tcctacagac accgctcctg agacacattc 11700
   ctcagccatc actaagaccc ctggtttgtt caggcatctc gtccaaatgt ggctccccaa 11760
   gcccccaggc tcagttactc catcagacgc acccaacctg agtcccattt tccaaaggca 11820
   tcggaaaatc cacagaggct cccactcgag cagtgtggtt ttgcaagagg aagcaaaaag 11880
   cctctccacc caggcctgga atgtttccac ccaatgtcga gcagtgtggt tttgcaagag 11940
   gaagcaaaaa gcctctccac ccaggcctgg aatgtttcca cccaatgtcg agcaaacccc 12000
   gcccagcgtc ttgtcattgg cgaattcgaa cacgcagatg cagtcggggc ggcgcggtcc 12060
   caggtccact tcgcatatta aggtgacgcg tgtggcctcg aacaccgagc gaccctgcag 12120
   ccaatatggg atcggccatt gaacaagatg gattgcacgc aggttctccg gccgcttggg 12180
   tggagaggct attcggctat gactgggcac aacagacaat cggctgctct gatgccgccg 12240
   tgttccggct gtcagcgcag gggcgcccgg ttctttttgt caagaccgac ctgtccggtg 12300
   ccctgaatga actgcaggac gaggcagcgc ggctatcgtg gctggccacg acgggcgttc 12360
   cttgcgcagc tgtgctcgac gttgtcactg aagcgggaag ggactggctg ctattgggcg 12420
   aagtgccggg gcaggatctc ctgtcatctc accttgctcc tgccgagaaa gtatccatca 12480
   tggctgatgc aatgcggcgg ctgcatacgc ttgatccggc tacctgccca ttcgaccacc 12540
   aagcgaaaca tcgcatcgag cgagcacgta ctcggatgga agccggtctt gtcgatcagg 12600
   atgatctgga cgaagagcat caggggctcg cgccagccga actgttcgcc aggctcaagg 12660
   cgcgcatgcc cgacggcgag gatctcgtcg tgacccatgg cgatgcctgc ttgccgaata 12720
   tcatggtgga aaatggccgc ttttctggat tcatcgactg tggccggctg ggtgtggcgg 12780
   accgctatca ggacatagcg ttggctaccc gtgatattgc tgaagagctt ggcggcgaat 12840
   gggctgaccg cttcctcgtg ctttacggta tcgccgctcc cgattcgcag cgcatcgcct 12900
   tctatcgcct tcttgacgag ttcttctgag gggatcggca ataaaaagac agaataaaac 12960
   gcacgggtgt tgggtcgttt gttcggatcc 12990
<210> 111
   <211> 33
   <212> DNA
   <213> Artificial Sequence
<220>
   <223> Oligonucleotide primer
<400> 111
   ccgctcgagt gggagcctct gtggattttc cga 33
<210> 112
   <211> 32
   <212> DNA
   <213> Artificial Sequence
<220>
   <223> Oligonucleotide primer
<400> 112
   tgaccggacg tcgcccagcc cagcctagct ca 32
<210> 113
   <211> 48
   <212> DNA
   <213> Artificial Sequence
<220>
   <223> Oligonucleotide primer
<400> 113
   cccaagctta tggactggac ctggaggatc ctcttcttgg tggcagca 48
<210> 114
   <211> 51
   <212> DNA
   <213> Artificial Sequence
<220>
   <223> Oligonucleotide primer
<400> 114
   cccaagctta tggacacact ttgctccacg ctcctgctgc tgaccatccc t 51
<210> 115
   <211> 48
   <212> DNA
   <213> Artificial Sequence
<220>
<223> Oligonucleotide primer
<400> 115
   cccaagctta tggagtttgg gctgagctgg gttttccttg ttgctatt 48
<210> 116
   <211> 48
   <212> DNA
   <213> Artificial Sequence
<220>
   <223> Oligonucleotide primer
<400> 116
   cccaagctta tgaaacacct gtggttcttc ctcctgctgg tggcagct 48
<210> 117
   <211> 51
   <212> DNA
   <213> Artificial Sequence
<220>
   <223> Oligonucleotide primer
<400> 117
   cccaagctta tggggtcaac cgccatcctc gccctcctcc tggctgttct c 51
<210> 118
   <211> 51
   <212> DNA
   <213> Artificial Sequence
<220>
   <223> Oligonucleotide primer
<400> 118
   cccaagctta tgtctgtctc cttcctcatc ttcctgcccg tgctgggcct c 51
<210> 119
   <211> 51
   <212> DNA
   <213> Artificial Sequence
<220>
   <223> Oligonucleotide primer
<400> 119
   cccaagctta tggactggac ctggaggatc ctcttcttgg tggcagcagc a 51
<210> 120
   <211> 39 <212> DNA
   <213> Artificial Sequence
<220>
   <223> Oligonucleotide primer
<400> 120
   cccaagcttc cccaagcttc ccaggtgcag ctacagcag 39

## Description

### Field of the Invention

The present invention relates to the field of evolution of nucleic acids *in vivo* and provides methods and compositions for introducing diversity into gene products. The present invention allows generation of new sequences that have desirable properties by virtue of high frequency mutation events within a cell. The high frequency mutation of a polynucleotide sequence results in the production of a large population of new sequence variants. Appropriate selection and/or screening permits identification and isolation of mutant forms of the polynucleotide sequence as well as products resulting from expression of the mutant sequences.

### Background of the Invention

*In vitro* evolution of proteins involves generating diversity by introducing mutations into known gene sequences to produce a library of mutant sequences, translating the sequences to produce a very large number of mutant gene products, which are then selected for the desired properties. Such schemes can be divided into two distinct groups; i) partial *in vitro* methods in which the mutated target protein is displayed on the surface of the either phage, bacteria or yeast but the mutation step is performed outside the cell and ii) entirely *in vitro* methods in which the target is displayed as part of a ribosome quaternary complex or polysome and all other steps including mutation are performed in a cell free environment. These processes have the potential for generating proteins with improved diagnostic and therapeutic utilities. Unfortunately, however, the potential of such processes has been limited by deficiencies in methods currently available for mutation, library generation and display of correctly folded proteins.

For example, in the case of partial *in vitro* methods, the DNA must be synthesised *in vitro* or extracted from the cells for mutagenesis. Although various mutagenesis approaches (including error prone PCR, DNA shuffling, chain shuffling and site directed mutagenesis) have been successfully used to generate mutant libraries, some of this diversity is lost due to limitations in subsequent transformation efficiency. Consequently, the generation of large libraries (e.g. beyond a library size of 10¹⁰) of unique individual genes and their encoded proteins has proven difficult particularly with phage display systems. A further disadvantage is that methods which utilise phage display systems require several sequential steps of mutation, amplification, selection and further mutation. Given that extraction and reintroduction of DNA into the cell is required for these systems, their potential to generate large diversity in the target gene library is further restricted.

To circumvent this problem, entirely *in vitro* methods such as continuous *in vitro* evolution (CIVE) have been developed and are described, for example, in WO 99/58661. In theory any diversity created through mutagenesis in these systems is not lost. In the case of CIVE, a mutating enzyme is used to introduce nucleic acid base changes into the target sequence. The only factor limiting diversity here is the mutation rate of this enzyme. Reports of mutation rates using other *in vitro* mutation methods such as error prone PCR, DNA shuffling (sexual PCR), chain shuffling and site directed mutagenesis over selected CDRs which can be used in this scheme vary significantly. Despite using different mechanisms all these approaches operate in an artificial environment in which only defined components required for these processes are present. It is possible there may be additional unknown factors involved, which are not supplied. Furthermore, this cell-free environment lacks the secretory and post-translational machinery required to produce a correctly folded and processed protein. As a result, this restricts the type of targets which can be "evolved" in these systems and allows incorrectly folded, unmodified mutant proteins which have no functional relevance in a clinical setting to be selected. Bacterial and phage display also have the same associated problems.

*In vivo* evolution of proteins involves the same steps and principles as previously described for in vitro evolution (i.e. mutation, display, selection and amplification). However, in vivo systems overcome many of the problems associated with the in vitro approaches. One *in vivo* cyclical procedure that has been reported involves *Escherichia coli* mutator cells that were used as a vehicle for mutation of recombinant antibody genes. The *E.coli* mutator cells, MUTD5-FIT which carried a mutated DNAQ gene were used as the source of the S-30 extracts and therefore allowed mutations to be introduced into DNA during replication as a result of proofreading errors. However, mutation rates were low compared to the required rate. For example, to mutate 20 residues with the complete permutation of 20 amino acid requires a library size of 1x10²⁶, an extremely difficult task with currently available phage library display methodology. Obviously, the disadvantages with using bacteria and phage in terms of transformation efficiencies, and protein folding etc. make this a less desirable scheme.

WO 00/22111 describes a number of different processes for introducing heterologous sequences into hypermutating cells, most of which result in multiple copies of the heterologous sequence either integrated into the host genome or as episomes. WO 00/22111 does not mention the integration of a single copy of the heterologous sequence into the genome of the hypermutating cell.

Yélamos, J. et al., Nature, Vol. 376, 1995, describes integration of transgene constructs into the immunoglobulin kappa light chain gene of a mouse genome. Yélamos, J. *et al.* does not mention the importance of integration of a single copy of the heterologous sequence into the genome of the hypermutating cell for the purpose of facilitating recovery of the mutant target nucleic acid sequence of interest.

US 5,885,827 describes a method for performing saturation mutagenesis on a target gene which involves introducing the target gene into a hypermutating cell, the target gene being present on a self-replicating vector containing immunoglobulin enhancer fragments that effect hypermutation.

In view of the above it is clear that the current affinity maturation schemes are somewhat limited in their ability to generate and select functionally superior binders.

### Summary of the invention

The present inventors have now developed a novel process for the *in vivo* evolution of gene products. This process generates mutants of target nucleic acid sequences by somatic hypermutation, yielding mutant products capable of undergoing any post-translational modifications that may be required for biological activity. A selection system particular to the properties of the target product is then utilized to identify desirable mutants.

Accordingly, in a first aspect, the present invention provides a method for producing and selecting a gene product with desired characteristics, the method comprising
(i) introducing into a hypermutating cell a target nucleic acid molecule encoding a gene product such that a single copy of the target nucleic acid molecule is integrated into an immunoglobulin locus of the genome of the hypermutating cell, wherein the target nucleic acid molecule is introduced into the cell by way of an integration vector comprising a sequence homologous to a region upstream of a rearranged V allele and a sequence homologous to a region downstream of a rearranged V allele;
(ii) culturing the hypermutating cell such that the target nucleic acid molecule undergoes hypermutation during DNA and/or RNA synthesis, giving rise to a population of cells expressing mutant gene products; and
(iii) selecting a mutant gene product with desired characteristics.

The term "immunoglobulin locus" refers to a variable region of an antibody molecule or all or a portion of a regulatory nucleotide sequence that controls expression of an antibody molecule. Immunoglobulin loci for heavy chains may include but are not limited to all or a portion of the V, D, J, and switch regions (including intervening sequences called introns) and flanking sequences associated with or adjacent to the particular heavy chain constant region gene expressed by the antibody-producing cell to be transfected and may include regions located within or downstream of the constant region (including introns). Immunoglobulin loci for light chains may include but are not limited to the V and J regions, their upstream flanking sequences, and intervening sequences (introns), associated with or adjacent to the light chain constant region gene expressed by the antibody-producing cell to be transfected and may include regions located within or downstream of the constant region (including introns). Immunoglobulin loci for heavy chain variable regions may include but are not limited to all or a portion of the V, D, and J regions (including introns) and flanking sequences associated with or adjacent to the particular variable region gene expressed by the antibody-producing cell to be transfected. Immunoglobulin loci for light chain variable regions may include but are not limited to the V and J region (including introns) and flanking sequences associated with or adjacent to the light chain variable region gene expressed by the antibody-producing cell to be transfected.

In the human, the immunoglobulin heavy chain (IgH) locus is located on chromosome 14. In the 5'-3' direction of transcription, the locus comprises a large cluster of variable region genes (V_{H}), the diversity (D) region genes, followed by the joining (J_{H}) region genes and the constant (C_{H}) gene cluster. The size of the locus is estimated to be about from 1,500 to about 2,500 kilobases (kb). During B-cell development, discontinuous gene segments from the germ line IgH locus are juxtaposed by means of a physical rearrangement of the DNA. In order for a functional heavy chain Ig polypeptide to be produced, three discontinuous DNA segments, from the V_{H}, D, and J_{H} regions must be joined in a specific sequential fashion; first D to J_{H} then V_{H} to DJ_{H}, generating the functional unit V_{H}DJ_{H}. Once a V_{H}DJ_{H} has been formed, specific heavy chains are produced following transcription of the Ig locus, utilizing as a template the specific V_{H}DJ_{H}C_{H} unit comprising exons and introns.

There are two loci for immunoglobulin light chains (IgL), the kappa locus on human chromosome 2 and the lambda locus on human chromosome 22. The organization of the IgL loci is similar to that of the IgH locus, except that the D region is not present. Following IgH rearrangement, rearrangement of a light chain locus is similarly accomplished by V_{L} to J_{L} joining of the kappa or lambda chain. The sizes of the lambda and kappa loci are each approximately 1000 kb to 2000 kb. Expression of rearranged IgH and an Ig kappa or Ig lambda light chain in a particular B-cell allows for the generation of antibody molecules.

In a further preferred embodiment of the invention the immunoglobulin locus is a rearranged V_{H}4 gene. In a further preferred embodiment, the immunoglobulin locus is a rearranged VH₄-₃₄ allele.

By "hypermutation" we mean a mechanism by which mutagenesis occurs at a rate approaching that naturally occurring in the immunoglobulin variable region, which is preferably in the range of 10⁻⁴ to 10⁻³/base pair/generation/cell but more preferably in the range of 5x10⁻⁵ to 5x10⁻⁴/base pair/generation/cell.

A "hypermutating cell" is a cell or cell line containing hypermutation elements.

By "hypermutation elements" we mean an intronic enhancer (Ei), matrix attachment regions (MAR), and a 3' enhancer. The intronic enhancer may be, for example, E mu or E kappa. The 3' enhancer may be, for example, a 3' kappa enhancer or a 3'H enhancer.

A "matrix attachment region" (MAR) is defined by its ability to bind to the nuclear matrix. Matrix attachment region sequences flank the IgH intronic enhancer.

In one embodiment the hypermutating cell is an immunoglobulin-expressing cell which is capable of expressing at least one immunoglobulin V gene. A V gene may be a variable light chain (V_{L}) or a variable heavy chain (V_{H}) gene, and may be produced as part of an entire immunoglobulin molecule. Preferred hypermutating cells for use in the present invention are derived from B-cell lines. Lymphoma cells may be used for the isolation of constitutively hypermutating cell lines for use in the present invention.

In a preferred embodiment of the present invention, following integration into the immunoglobulin locus of the hypermutating cell, the target nucleic acid molecule is located in proximity to one or more endogenous hypermutation elements. Preferably, the immunoglobulin locus is a V_{H} gene and the target nucleic acid molecule is located in proximity to an endogenous intronic enhancer, and endogenous matrix attachment regions.

The phrase "located in proximity to" means that hypermutation elements are located close enough to the target nucleic acid molecule to effect hypermutation of the target nucleic acid molecule.

In an alternative embodiment of the invention, following integration into the immunoglobulin locus of the hypermutating cell, the target nucleic acid molecule is located in proximity to at least one exogenous hypermutation element. For example, the target nucleic acid molecule may be located in proximity to an exogenous intronic enhancer, an exogenous matrix attachment region and/or an exogenous 3' kappa enhancer. Any one or more of these exogenous elements may be integrated into the immunoglobulin locus simultaneously with the target nucleic acid molecule.

A suitable exogenous "intronic enhancer" may be, for example, the *Xbal-EcoRI* fragment described in Grosschedl et al (1985) Cell Vol 41:885-897, the intronic enhancer described in Rabbitts et al (1983) Nature 306 (5945):806-809, or the intronic enhancer described in Ravetch et al (1981) Cell 27 (3 Pt 2): 583-591; or can be one or more sub-fragments thereof determined to have hypermutation activity.

A suitable exogenous "3' kappa enhancer" is the ScaI-XbaI fragment described in Meyer et al (1989) EMBO Journal Vol. 8, no. 7 p. 1959-1964 and can be one or more sub-fragments determined to have hypermutation activity.

Hypermutation-competent fragments of exogenous intronic enhancers or the 3' kappa enhancer can be identified in a number of ways. One way is to perform deletional analysis by constructing hypermutation cassettes containing various enhancer deletion mutants and a reporter gene. The hypermutation efficency of the enhancer deletion mutant can be assessed by determining the rate of mutation of the reporter gene. Deletion mutants can be prepared in a variety of ways. Oligonucleotides can be designed containing fragment sequences to be tested. Alternatively, a more random approach is to linearize the expression vector by restriction digest within an enhancer, followed by subsequent exonuclease treatment and religation. Yet another method is to simply use restriction digests to remove sections of DNA.

It is preferred that following integration, a 3' enhancer and/or an intronic enhancer are positioned at a location 3' of the target nucleic acid sequence. It is further preferred that the intronic enhancer be located in greater proximity to the target gene than the 3' enhancer. The 5' end of the intronic enhancer is preferably positioned up to 3 kb 3' of the 3' end of the target gene, preferably less than 2 kb, more preferably less than 1 kb, and most preferably immediately adjacent to the target nucleic acid sequence. The intronic enhancer can be positioned greater than 3 kb 3' of the target gene, but this is less preferred. The 3' enhancer is preferably located up to 20 kb and preferably 5-15 kb 3' of the intronic enhancer. The 3' enhancer can be located as close as 1 kb 3' of the intronic enhancer, but this is less preferred. In another embodiment, the 3' enhancer fragment is located 5' relative to the target gene. The intronic enhancer can also be positioned 5' relative to the target gene, although this embodiment is less preferred.

In a further preferred embodiment, the enhancers are present in a genomic orientation. The enhancer sequence present in the genomic immunoglobulin gene is present in a "genomic orientation". If it is flipped in the construct so that it now appears in a 3' to 5' orientation (as opposed to the 5' to 3' orientation in the native genomic configuration), it is present in the "reverse orientation". However, the 3' enhancer can be present in reverse orientation. The enhancer can also be present in reverse orientation, but this is less preferred.

In a further preferred embodiment of the first aspect, following integration of the target nucleic acid molecule into the immunoglobulin locus, the target nucleic acid molecule is operatively linked to a promoter. Preferably, the target nucleic acid molecule is located downstream of the promoter and upstream of an intronic enhancer.

The term "promoter" is well-known in the art and encompasses nucleic acid regions ranging in size and complexity from minimal promoters to promoters including upstream elements and enhancers.

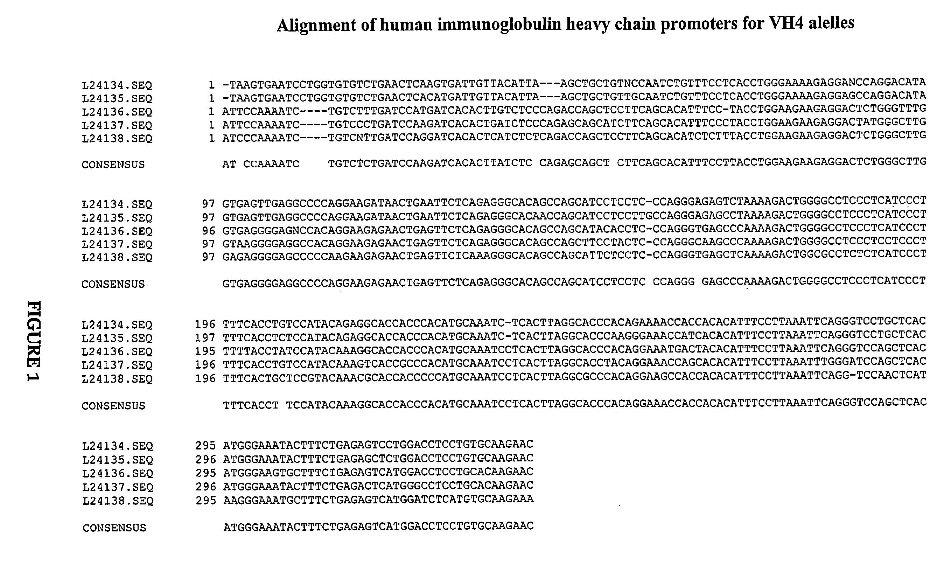

In one preferred embodiment of the invention, the promoter is a naturally occurring promoter that exists within the immunoglobulin locus. In one embodiment, the promoter is an immunoglobulin heavy or light chain promoter. Preferably, the promoter is an immunoglobulin heavy chain promoter of a V_{H}4 allele. There is strong conservation between the promoter sequences of the V_{H}4 alleles (Figure 1). A wide range of hypermutating cell lines (including RAMOS and BL2 cell lines) carry rearranged V_{H}4 alleles.

Alternatively, the promoter may be a heterologous or exogenous promoter selected from those which are functional in mammalian cells, although prokaryotic promoters and promoters functional in other eukaryotic cells may be used. The promoter may be derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of α-actin, β-actin, tubulin) or, alternatively, a tissue-specific manner (such as promoters of the genes for pyruvate kinase). They may also be promoters that respond to specific stimuli, for example promoters that bind steroid hormone receptors. Viral promoters may also be used, for example the Moloney murine leukaemia virus long terminal repeat (MMLV LTR) promoter, the rous sarcoma virus (RSV) LTR promoter or the human cytomegalovirus (CMV) IE promoter. In a preferred embodiment, the promoter is an immunoglobulin promoter sequence such as a murine immunoglobulin promoter sequence or a human immunoglobulin heavy chain promoter.

The promoter region of mammalian/human genes can contain several regulatory elements in the DNA sequences, and span several hundred bases or more, it is generally observed that one of these elements, designated 'TATA box" sequence, in eukaryotic promoter regions is usually found approximately 300 bases or more upstream of the translation initiation site (start) sequence, ATG. Rearrangements which bring the V gene promoter into closer proximity to the ATG translation start signal also brings the promoter closer to the enhancer which is 3' and located in the C region. This activates the promoter which indicates that the close proximity to the enhancer may affect the rate at which the DNA in the VH locus is mutated.

The present inventors have found that in a RAMOS RA-1 cell line in which the rearrangement generates a functional VH4-34 allele, the promoter is significantly closer to the translation initiation start site than in other mammalian/human genes (based upon the number of nucleotides between the 3' end of the "TATA box" and the initiation codon of the immunoglobulin leader sequence). This proximity of the promoter to the start codon and the three dimensional structure of the promoter caused significant difficulties in cloning this region from RAMOS RA-1.

Accordingly, in a further preferred embodiment of the invention, following integration of the target nucleic acid molecule into the immunoglobulin locus, the target nucleic acid molecule is located within close proximity to the promoter. Preferably, the initiation codon of the target nucleic acid molecule is located within 500 bp of the 3' end of the promoter, more preferably within 200 bp of the 3' end of the promoter, more preferably within 100 bp of the 3' end of the promoter and more preferably within 20 bp of the 3' end of the promoter.

It may also be advantageous for the promoters to be inducible so that the levels of expression of the heterologous gene can be regulated during the life-time of the cell. Inducible means that the levels of expression obtained using the promoter can be regulated.

In addition, any of these promoters may be modified by the addition of further regulatory sequences, for example enhancer sequences. Chimeric promoters may also be used comprising sequence elements from two or more different promoters described above.

In a further preferred embodiment of the present invention, the target nucleic acid molecule is introduced into the cell by way of an integration vector comprising a sequence homologous to a region of at least 500 bp, more preferably at least 2 kb, more preferably approximately 5 kb upstream of a rearranged V gene and a sequence homologous to a region of at least 500 bp, more preferably at least 2 kb, more preferably approximately 5kb downstream of the same rearranged V gene. Preferably, the sequence homologous to a region downstream of the rearranged V gene comprises an intronic enhancer and matrix attachment regions or portions thereof. It is preferred that the upstream and downstream homologous sequences are at least 500 bp in length, more preferably about 5 kb in length.

A "target nucleic acid molecule" can be any nucleic acid molecule of interest (including DNA and RNA molecules) encoding a gene product where diversification of the gene product is desired.

The "gene product" may be any biologically active molecule of interest. For example, the gene product may be a catalytic molecule such as a ribozyme, a DNAzyme, an LNAzyme or an RNAi/siRNA molecule. Alternatively, the gene product may be an antibody or fragment thereof, an enzyme, a hormone, a receptor, a cell surface molecule, a viral protein, transcription factor or any other biologically active polypeptide.

The selected mutant target sequence may be recycled through the methods of the present invention in order to introduce further diversification. Accordingly, in a further preferred embodiment of the first and second aspects, at least steps (ii) and (iii) of the method are repeated.

The hypermutating cell may be a mammalian, avian, yeast, fungi, insect or bacterial cell. In a preferred embodiment, the hypermutating cell is a mammalian cell. The mammalian hypermutating cell may be selected from the group consisting of RAMOS, BL2, BL41, BL70 and Nalm.

The method of the present invention may include further steps to increase the rate of mutation of the target nucleic acid sequence. For example, the hypermutating cell may be cultured in the presence of chemical mutagens. Suitable chemical mutagens include, for example, sodium bisulfite, nitrous acid, hydroxylamine, hydrazine or formic acid. Other agents which are analogues of nucleotide or nucleoside precursors include nitrosoguanidine, ribavirin, 5-bromouracil, 2-aminopurine, 5-formyl uridine, isoguanosine, acridine and of N⁴-aminocytidine, N¹-methyl-N⁴-aminocytidine, 3,N⁴-ethenocytidine, 3-methylcytidine, 5-hydroxycytidine, N⁴-dimethylcytidine, 5-(2-hydroxyethyl)cytidine, 5-chlorocytidine, 5-bromocytidine, N⁴-methyl-N.sup.4-aminocytidine, 5-aminocytidine, 5-nitrosocytidine, 5-(hydroxyalkyl)-cytidine, 5-(thioalkyl)-cytidine and cytidine glycol, 5-hydroxyuridine, 3-hydroxyethyluridine, 3-methyluridine, O²-methyluridine, O²-ethyluridine, 5-aminouridine, O⁴-methyluridine, O⁴-ethyluridine, O⁴-isobutyluridine, O⁴-alkyluridine, 5-nitrosouridine, 5-(hydroxyalkyl)-uridine, and 5-(thioalkyl)-uridine, 1,N⁶-ethenoadenosine, 3-methyladenosine, and N⁶-methyladenosine, 8-hydroxyguanosine, O⁶-methylguanosine, O⁶-ethylguanosine, O⁶-isopropylguanosine, 3,N²-ethenoguanosine, O⁶-alkylguanosine, 8-oxo-guanosine, 2,N³-ethenoguanosine, and 8-aminoguanosineas well as derivatives/analogues thereof. Examples of suitable nucleoside precursors, and synthesis thereof, are described in further detail in USSN 20030119764. Generally, these agents are added to the replication or transcription reaction thereby mutating the sequence. Intercalating agents such as proflavine, acriflavine, quinacrine and the like can also be used.

Random mutagenesis of the target nucleic acid molecule can also be achieved by irradiation with X-rays or ultraviolet light.

Antigen stimulation of the hypermutating cells, or exposure of the cells to interleukins (such as IL-2, IL-4 or IL-10) or CD40 ligand or B cell activating factor (BAFF) may also be used to increase the mutation frequency.

In one preferred embodiment, the level or activity of activation-induced cytidine deaminase (AID) within the hypermutating cell is increased. This may be achieved, for example, by increasing expression levels of AID within the hypermutating cells. For example, the hypermutating cells may be transfected with plasmid vectors which encode and express AID.

It will be appreciated by those skilled in the art that any process of selecting a mutant product can be used in the methods of the present invention.

In one embodiment, selection can be achieved by binding to a target molecule or by measurement of a biological response affected by the mutant product.

In another example, if the product of interest is an agent that promotes or reduces cell growth or division, the selection process can involve exposing mutant products to a population of cells and monitoring the biological responses of those cells.

In another example, if the mutant product is a receptor ligand, the process can involve exposing mutant proteins to cells expressing the receptor and monitoring a biological response effected by signalling of the receptor.

In one embodiment, the mutant product is selected by way of an assay performed within the hypermutating cell. For example, if the target nucleotide sequence encodes an enzyme, the assay may simply measure enzymatic activity.

Alternatively , the assay performed within the hypermutating cell may be a protein-fragment complementation assay (PCA). PCAs rely on the complementation of enzyme fragments fused to interacting proteins that reconstitute enzymatic activity once dimerised. Examples of PCA protocols are described in Michnick (2001) Current opinion in structural biology 11:472-477; Wehrman et al. (2002). PNAS 99(6):3469-3474; and Galarneau et al. (2002). Nature 20:616-622.

Suitable enzyme fragments may be derived, for example, from β-lactamase. Studies using the (β-lactamase system in both bacteria and mammalian cells have successfully validated it as a suitable reporter system to detect protein-protein interactions inside the cell.

The target nucleic acid molecule may therefore encode a fusion protein comprising a binding partner and a (β-lactamase fragment. Its binding partner may be introduced into the cell as a fusion protein comprising a complementary β-lactamase fragment. Selection occurs inside the cell with the binding of the target protein to its cognate partner which is detected by (β-lactamase activity on a substrate supplied.

If selection assays such as PCAs are used, it is not necessary to display the target polypeptide on the surface of the hypermutating cell. These selection assays are particularly advantageous for targets which are naturally found intracellularly.

In an alternative embodiment of the present invention, the target nucleic acid molecule is linked to a sequence encoding an anchor domain such that following expression, the mutant gene product is displayed on the surface of the hypermutating cell. Examples of suitable anchor domains include attachment signals from glycosylphosphatidylinositol (GPI) anchored membrane proteins or transmembrane domains of other cell surface proteins.

If the gene product to be displayed is not normally found on the cell surface, it is preferred that the target nucleic acid molecule is also linked to a sequence encoding an N-terminal signal peptide (or a leader peptide). This signal peptide facilitates targeting of the gene product to the plasma membrane.

Following display on the surface of the hypermutating cell, the mutant gene product may be selected by detecting binding of a binding partner to the mutant gene product. This may involve, for example, labelling cells with a detectable marker such as a fluorescent dye and allowing binding to occur between the mutant protein on the cell surface and its binding partner. If the binding partner has been immobilized on a plate, a suitable detection system, such as a fluorimeter, can be used to identify wells containing a mutant of interest.

Alternatively, the binding partner may be labelled with a fluorescent tag, and cells expressing a mutant gene product of interest may be sorted using flow cytometric techniques. The binding partner may be selected from the group consisting of an antibody, receptor, hormone, enzyme, cell surface molecule, transcription factor, DNA or RNA molecule.

In another embodiment of the invention, the target gene product is expressed in soluble form.

To prevent repeated mutation after selection, hypermutation may be arrested prior to culturing the selected cells. This can be accomplished in a number of ways, including fusion to a myeloma or repression of an inducible promoter.

In a further preferred embodiment of the invention, the mutant nucleic acid sequence encoding the selected gene product is recovered from the hypermutating cell. This recovery can be achieved by amplification of the mutant nucleic acid sequence in whole or in part by polymerase chain reaction, using oligonucleotides that will anneal to locations outside the region of hypermutation or within the target sequence itself. Alternatively, the mutant nucleic acid sequence may be amplified using RT-PCR. The mutant nucleic acid sequence can then be subcloned for other purposes, such as expression, purification, or characterization.

The conditioned media from the transfected cells can be concentrated if desired and applied to the selection system. Specific binders can be identified directly or indirectly, for example by antibody recognition of either the target gene product itself or an attached tag sequence. The mutant gene products of interest can then be further characterized by a number of protein chemistry techniques such as micro-sequencing.

In a third aspect the present invention provides a vector for targeted integration into an immunoglobulin locus of a hypermutating cell, the vector comprising a sequence homologous to a region upstream of a rearranged V gene of the hypermutating cell, a sequence homologous to a region downstream of a rearranged V gene of the hypermutating cell and a site for insertion of a target nucleic acid molecule, wherein the region upstream of the rearranged VH gene of the hypermutating cell is a region within nucleotides 1 to 5190 of SEQ ID NO:1. Preferably, the region is at least 500 bp, more preferably at least 2 kb, more preferably about 5 kb within nucleotides 1 to 5190 of SEQ ID NO:1. In a further preferred embodiment the region upstream of the rearranged V gene of the hypermutating cell comprises nucleotides 191 to 5190 of SEQ ID NO:1.

In a further preferred embodiment of the third aspect, the region downstream of the rearranged VH gene of the hypermutating cell is a region within nucleotides 5709 to 8699 of SEQ ID NO:1. Preferably, the region is at least 500 bp, more preferably at least 3 kb, 5709 to 8699 of SEQ ID NO:1. In a further preferred embodiment the region downstream of the rearranged VH gene of the hypermutating cell comprises nucleotides 5709 to 8634 of SEQ ID NO:1.

In a further preferred embodiment of the third aspect, the vector further comprises a selectable marker. The term "selection marker" or "selectable marker" includes both positive and negative selection markers. A "positive selection marker" is a nucleic acid sequence that allows the survival of cells containing the positive selection marker under growth conditions that kill or prevent growth of cells lacking the marker. An example of a positive selection marker is a nucleic acid sequence which promotes expression of the neomycin resistance gene, or the kanamycin resistance gene. Cells not containing the neomycin resistance gene are selected against by application of G418, whereas cells expressing the neomycin resistance gene are not harmed by G418 (positive selection). A "negative selection marker" is a nucleic acid sequence that kills, prevents growth of or otherwise selects against cells containing the negative selection marker, usually upon application of an appropriate exogenous agent. An example of a negative selection marker is a nucleic acid sequence which promotes expression of the thymidine kinase gene of herpes simplex virus (HSV-TK). Cells expressing HSV-TK are selected against by application of ganciclovir or 1-2'-deoxy-2'fluoro-b-D-arabinofuranosyl-5-iodouracil (FIAU); negative selection), whereas cells not expressing the gene are relatively unharmed by ganciclovir or FIAU.

In a further preferred embodiment the vector encodes an anchor molecule suitable for display of the protein encoded by the target nucleic acid molecule.

In a further preferred embodiment the vector comprises a target nucleic acid molecule with an epitope tag(s) (for example, two flag tags).

In a further preferred embodiment, the vector for targeted integration comprises a sequence as set out in SEQ ID NO:110.

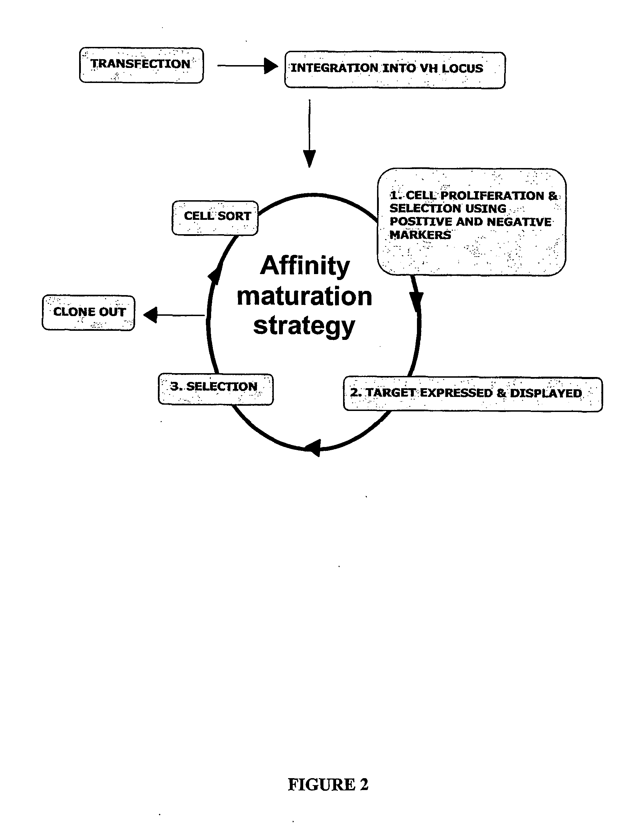

The present invention provides a novel approach for generating diversity in a gene product (see Figure 2). An important feature of the present invention is that the target nucleic acid molecule is integrated into the immunoglobulin locus of a host cell genome. This integration is achieved by including sequences homologous to regions upstream and downstream of the rearranged V gene in the integration vector. This directed integration ensures that the target nucleic acid molecule is positioned in proximity to elements that effect hypermutation (eg. in proximity to elements such as an intronic enhancer, matrix attachment regions and/or a 3' enhancer) following integration.

The integration process also ensures that only one copy of the target nucleic acid molecule is present in each cell. This facilitates the recovery of mutant target nucleic acid sequences of interest following the selection process by, for example, PCR or RT-PCR. In contrast, methods which involve the introduction of a target nucleic acid on a self replication vector or on a vector which integrates randomly into the host genome are likely to result in multiple mutated copies of target nucleic acid molecule in the host cell. This makes it difficult to determine which sequence encodes the mutant gene product which has been selected for its desired properties.

It will be appreciated that the methods of the present invention may be used for a variety of purposes. For example, the methods of the present invention can be used to effect affinity maturation of antibodies. In one aspect, the invention may be applied toward improving the affinity of antibodies from "naive," i.e., non-immune, phage human antibody libraries. Such libraries already exist and yield antibodies to any antigen. However, since they are made from non-immunized individuals, their affinities are low. In another aspect of the invention, the affinity of antibodies that were generated by conventional hybridoma techniques can be improved by applying a high rate mutagenesis system of the invention to the isolated target nucleic acid encoding for the initial low-affinity antibody. These enhanced-affinity antibodies can be utilized as improvements over many antibody-based diagnostics and therapeutics currently available.

The methods of the present invention allow a very large library of peptides and single-chain antibodies to be screened and the polynucleotide sequence encoding the desired peptide(s) or single-chain antibodies to be selected. The pool of polynucleotides can then be isolated and shuffled to recombine combinatorially the amino acid sequence of the selected peptide(s) (or predetermine portions thereof) or single-chain antibodies (or just V_{H}, V_{L}, or CDR portions thereof). Using these methods, one can identify a peptide or single-chain antibody as having a desired binding affinity for a molecule and can exploit the process of the invention to converge rapidly to a desired high-affinity peptide or scFv. The peptide or antibody can then be synthesized in bulk by conventional means for any suitable use (e.g., as a therapeutic or diagnostic agent).

The mutagenesis system can also be used to effect receptor or ligand modification. In one aspect, the invention can generate a ligand or receptor with enhanced binding characteristics for its corresponding receptor or ligand. In another aspect, the mutagenesis system can be used to generate an inhibitor of functional receptor-ligand interaction by creating a ligand or receptor that still binds, but does not elicit a functional response. In yet another aspect of the invention, multiple biologically active variants of a target protein can be identified and recovered, thereby providing a means to study structure-function relationships of the protein. Additionally, species diversity can be investigated by comparing results obtained by selections utilizing receptors or other molecules from different species.

A receptor or ligand can be modified such that it can still bind, but does not signal any more. Alternatively, a better signalling ligand can be selected, which would provide a lower effective dosage of a pharmacologically active therapeutic.

The mutagenesis system of the present invention may also be used, for example, on a target such as caspase, an initiation factor target involved in a novel survival mechanism. This involves a cascade of essentially signalling reactions on the route to programmed cell death (apoptosis). Caspase-3 once activated binds to, and cleaves (activates) the 'cell death' proteins (including Id3). *In vivo* mutation and expression of mutated caspases, especially caspase-3, would have an effect on apoptosis. Therefore caspase 3 would be a preferred target molecule. This could be relevant to diagnostics in cell signal transduction for monitoring and detection of cancer, and with potential therapeutic outcomes.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### A Brief Description of the Figures

**Figure 1****:** Alignment of human immunoglobulin heavy chain promoters for VH4 alleles. Sequences provided as SEQ ID NO's 2 to 7.
**Figure 2****:** Schematic representation of a preferred method of the present invention.
**Figure 3****:** Schematic representation of the gene targeting region - the preferred site for direct insertion of the target nucleic acid into a rearranged V gene.
**Figure 4****:** Schematic representation of vector 3kb15a-7-4T.
**Figure 5****:** Schematic representation of vector KW2.
**Figure 6****:** Schematic representation of vector for targeted integration, KW3.
**Figure 7****:** RAMOS cells stained with CFSE showing successive divisions on each day.
**Figure 8****:** Schematic representation of vector pME18SasFP499.
**Figure 9****:** The effect of DNA amount and electroporation parameters on transfection of RAMOS RA-1 cells with pME18SEGFP.
**Figure 10****:** Comparison of RAMOS RA-1 cells transfected with pME18sEGFP, pME18sasFP499 or mock transfected.
**Figure 11: (A):** Quantum Simply Cellular Beads stained with mouse anti-human IgM-Alexa 488. (**B**) RAMOS cells stained with mouse anti-human IgM-Alexa 488. (**C**) Quantitation of IgM molecules on the surface of RAMOS RA-1 cells.
**Figure 12****:** Comparison of IgM expression on RAMOS RA-1 cells of different passage number. (**A**) RAMOS RA-1 passage **1** (**B**) RAMOS RA-1 passage 14.
**Figure 13****:** IgM expression on RAMOS RA-1 samples from different sources.
**Figure 14****:** Schematic representation of sequential overlap extension PCR.
**Figure 15****:** Schematic representation of mammalian expression vector pME18sCD26asfp499.
**Figure 16****:** Comparison of expression of asFP499 with and without the CD26 anchor in RAMOS RA-1.
**Figure 17****:** Comparison of expression of asFP499 with and without the CD26 anchor in HEK 293 T.

### Key to the Sequence Listing

SEQ ID NO:1 - Heavy chain locus of Ramos RA-1 cells.
SEQ ID NO:2 - Sequence of promoter region of a VH4 allele.
SEQ ID NO:3 - Sequence of promoter region of a VH4 allele.
SEQ ID NO:4 - Sequence of promoter region of a VH4 allele.
SEQ ID NO:5 - Sequence of promoter region of a VH4 allele.
SEQ ID NO:6 - Sequence of promoter region of a VH4 allele.
SEQ ID NO:7 - Consensus sequence of SEQ ID NO's 2 to 6.
SEQ ID NO: 8 - Plasmid pME18SasFP499.
SEQ ID NO:9 - Sequence of enhancer of human immunoglobulin D segment locus.
SEQ ID NO:10 - Sequence of enhancer of human immunoglobulin heavy locus on chromsome 14.
SEQ ID NO:11 - Sequence of sheep immunoglobulin heavy chain 5' intronic enhancer.
SEQ ID NO:12 - Sequence of mouse 3' IgH regulatory enhancer.
SEQ ID NO:13 - Sequence of murine IgH enhancer.
SEQ ID NO:14 - Sequence of mouse 3' kappa enhancer.
SEQ ID NO:15 - Promoter sequence of mouse immunoglobulin VH gene.
SEQ ID NO: 16 - Promoter sequence of mouse immunoglobulin V1 gene.
SEQ ID NO:17 - Promoter sequence of mouse immunoglobulin mu heavy chain gene.
SEQ ID NO:18 - Promoter sequence of mouse immunoglobulin VH gene.
SEQ ID NO:19 - Homo sapiens germline IgH chain (ProV4-39) gene fragment.
SEQ ID NO:20 - Homo sapiens germline IgH chain (ProV3-30) gene fragment.
SEQ ID NO:21 - Homo sapiens germline IgH chain (ProV3-9) gene fragment.
SEQ ID NO:22 - Homo sapiens germline IgH chain (ProV1-18) gene fragment.
SEQ ID NO:23 - GPI signal from human decay-accelerating factor.
SEQ ID NO:24 - Polynucleotide encoding SEQ ID NO:23.
SEQ ID NO:25 - GPI signal from porcine membrane dipeptidase.
SEQ ID NO:26 - Polynucleotide encoding SEQ ID NO:25.
SEQ ID NO:27 - GPI signal from rat ceruloplasmin.
SEQ ID NO:28 - Polynucleotide encoding SEQ ID NO:27.
SEQ ID NO:29 - GPI signal from mouse Thy-1.
SEQ ID NO:30 - Polynucleotide encoding SEQ ID NO:29.
SEQ ID NO:31 - Transmembrane domain of murine B7-1.
SEQ ID NO:32 - Polynucleotide encoding SEQ ID NO:31.
SEQ ID NO:33 - Signal sequence of CD59.
SEQ ID NO:34 - Polynucleotide encoding SEQ ID NO:33.
SEQ ID NO's 35 to 86, 88 to 90, 92 to 103, 105, 106, 108 to120 - Oligonucleotide primers.
SEQ ID NO:87 - Plasmid 3kb15a-7-4T.
SEQ ID NO:91 - Plasmid KW2.
SEQ ID NO:104 - Plasmid pME18sCD26asFP499.
SEQ ID NO:107 - Sequence of coding region of AICDA cDNA.
SEQ ID NO:110 - Plasmid KW3.

### Detailed Description of the Invention

### General Techniques

The present invention is performed without undue experimentation using, unless otherwise indicated, conventional techniques of molecular biology, microbiology, virology, recombinant DNA technology, peptide synthesis in solution, solid phase peptide synthesis, and immunology. Such procedures are described, for example, in the following texts that are incorporated by reference:
1. Sambrook, Fritsch & Maniatis, Molecular Cloning: A Laboratory Manual, Cold Spring Harbor Laboratories, New York, Second Edition (1989), whole of Vols I, II, and III;
2. DNA Cloning: A Practical Approach, Vols. I and II (D. N. Glover, ed., 1985), IRL Press, Oxford, whole of text;
3. Oligonucleotide Synthesis: A Practical Approach (M. J. Gait, ed., 1984) IRL Press, Oxford, whole of text, and particularly the papers therein by Gait, ppl-22; Atkinson *et al.,* pp35-81; Sproat *et al.,* pp 83-115; and Wu *et al.,* pp 135-151;
4. Nucleic Acid Hybridization: A Practical Approach (B. D. Hames & S. J. Higgins, eds., 1985) IRL Press, Oxford, whole of text;
5. Animal Cell Culture: Practical Approach, Third Edition (John R.W. Masters, ed., 2000), ISBN 0199637970, whole of text;
6. Immobilized Cells and Enzymes: A Practical Approach (1986) IRL Press, Oxford, whole of text;
7. Perbal, B., A Practical Guide to Molecular Cloning (1984);
8. Methods In Enzymology (S. Colowick and N. Kaplan, eds., Academic Press, Inc.), whole of series;
9. J.F. Ramalho Ortigão, "The Chemistry of Peptide Synthesis" In: Knowledge database of Access to Virtual Laboratory website (Interactiva, Germany);
10. Sakakibara, D., Teichman, J., Lien, E. Land Fenichel, R.L. (1976). Biochem. Biophys. Res. Commun. 73 336-342
11. Merrifield, R.B. (1963). J. Am. Chem. Soc. 85, 2149-2154.
12. Barany, G. and Merrifield, R.B. (1979) in The Peptides (Gross, E. and Meienhofer, J. eds.), vol. 2, pp. 1-284, Academic Press, New York.
13. Wünsch, E., ed. (1974) Synthese von Peptiden in Houben-Weyls Metoden der Organischen Chemie (Müler, E., ed.), vol. 15, 4th edn., Parts 1 and 2, Thieme, Stuttgart.
14. Bodanszky, M. (1984) Principles of Peptide Synthesis, Springer-Verlag, Heidelberg.
15. Bodanszky, M. & Bodanszky, A. (1984) The Practice of Peptide Synthesis, Springer- Verlag, Heidelberg.
16. Bodanszky, M. (1985) Int. J. Peptide Protein Res. 25, 449-474.

### Hypemutating cells

In the context of the present invention, the hypermutating cells may be bacterial, yeast, avian, fungal, insect or mammalian cells. Examples of suitable hypermutating cells are described below.

### Bacterial hypermutating strains :

(i) *Epicurian coli* mutator strain XL1-Red (triple DNA repair deficient - mutD, mutS, mut T) by Stratagene;
(ii) *Escherichia coli* mutator strain MutD5 (MutD5-FIT, mutated DNAQ gene) Irving RA, Kortt AA, Hudson PJ (1996). Immunotechnology 2(2) 127-43;
(iii) *Escherichia coli* strain FC40. Foster PL (2000) Bioessays 22(12) : 1067-74 and Powell SC & Wartwell RM (2001) Mutation Research 473 (2) 219-28;
(iv) Serogroup B meningococcal strains BF18, BF21 (defect in methyl - directed mismatch repair - lack DNA adenine methyltransferase (Dam) activity). Bucci et al. (1999) Mol Cell 3 (4) 435-45.

### Yeast hypermutating strains:

(i) *Saccharomyces cerevisiae* strain *(mlh1* Δ mutant). Shcherbakova & Kunkel (1999). Molecular and Cellular Biology 19(4) 3177-3183.
(ii) *Saccharomyces cerevisiae* strain DAG60 *(msh2* mutant). (deficient in mismatch repair system). Drotschmann et al. (1999) Proc. Natl Acad Sci USA 96 2970-2975.

### Mammalian hypermutating strains:

(i) Human B cell lines from Burkitt's lymphoma strains : RAMOS, BL2, BL41, BL70, Nalm-6. Sale & Neuberger (1998) Immunity 9 (6) 859-869.
(ii) BL2 : Denepouxs et al. (1997) Immunity 6(1) 35-46 and Poltorasky et al. (2001) Proc Natl Acad Sci USA 98 (14) 7976-81
(iii) Human pre B cell line strain 18.81. Bachl et al. (2001) Journal Immunology 166 (8) 5051-7.

### Intronic Enhancers

Examples of suitable exogenous intronic enhancers for use in the present invention include the following:
1) 1) DNA sequence of the human immunoglobulin D segment locus (Rabbitts et al (1983) Nature 306 (5945), 806-809):
2) Homo sapiens immunoglobulin heavy locus (IGH.1@) on chromosome 14 (Ravetch et al (1981) Cell 27 (3 Pt 2), 583-591):
3) Ovis aries immunoglobulin heavy chain 5' intronic enhancer (Dufour et al (1996) J. Immunol 156:2163-2170):
4) Mouse 3' IgH regulatory enhancers (C alpha3'E and hs3) (Saleque et al (1997) J. Immunol. 158 (10), 4780-4787):
5) Murine IgH enhancer (Kadesch et al (1986) Nucleic Acids Res. 14 (20), 8209-8221):

### 3' kappa enhancers

An example of a suitable exogenous 3' kappa enhancer for use in the present invention is as follows.
1) Murine 3' kappa enhancer (Meyer and Neuberger (1989) EMBO J. 8 (7), 1959-1964):

### Promoter sequences

Examples of suitable exogenous promoter sequences for use in the present invention include:
1) (Kataoka et al (1982). J Biol. Chem. 257 : 2777-285):
   5' AAGCAGCCCTCAGGCAGAGGATAAAAGCTCACACTAACTGAGAAGC TCCATCCTCTTCTC 3' (SEQ ID NO:15)
2) (Clarke et al (1982). Nucleic Acids Res. 10 : 7731):
   5' AATTAGGCCACCCTCATCACATGAAAACCAGCCCAGAGTGACTCTAG CAGTGGGATCCTG 3' (SEQ ID NO:16)
3) Grosschedl and Baltimore D., (1985). Cell 41: 885-897):
   5' CATGTGCGACTGTGATGATTAATATAGGGATATCCACACCAAACATC ATATGAGCCCTAT 3' (SEQ ID NO:17)
4) (Schiff et al (1986). J. Exp. Med. 163 : 573-687):
   5' AACATGAGTCTGTGATTATAAATACAGAGATATCCATACCAAACAAC TTATGAGCACTGT 3' (SEQ ID NO:18)

Murine B lymphocyte VH promoters (eg. V1 Ig VH promoter , BGL1 VH promoter) may also be used in the methods of the present invention.

### Human Immunoglobulin heavy chain promoters

The following human heavy chain promoters are described in in Haino et al (1994) J. Biol. Chem. 269 (4), 2619-2626:
1) Homo sapiens germline IGH chain (ProV4-39) gene fragment:
2) Homo sapiens germline IGH chain (ProV3-30) gene fragment:
3) Homo sapiens germline IGH chain (ProV3-9) gene fragment:

### CCCTGCAGCTCTGGGAGAGGAGCCCCAGCCCTGAGATTCCCAGGTGTT TCCATTCAGTGATCAGCACTGAACACAGAGGACTCACC 3' (SEQ ID NO:21)

4) Homo sapiens germline IGH chain (ProV1-18) gene fragment:

### Methods for selection of nucleic acids or proteins/peptides with an altered phenotype

The terms "altered phenotype", "desired activity" and "altered activity" are generally used interchangeably herein.

In particular embodiments of the present invention, the mutated nucleic acid, or gene product encoded thereby, is subjected to an assay for identifying an altered phenotype. Suitable procedures for identifying altered phenotypes include, but are not limited to, those described below.

### Protein/peptide display and selection

In one embodiment of the invention, proteins encoded by nucleic acids obtained using the methods of the invention are displayed on the surface of the hypermutating cells.

One well-known peptide display method involves the presentation of a peptide sequence on the surface of a filamentous bacteriophage, typically as a fusion with a bacteriophage coat protein. The bacteriophage library can be incubated with an immobilized, predetermined macromolecule or small molecule (e.g., a receptor) so that bacteriophage particles which present a peptide sequence that binds to the immobilized macromolecule can be differentially partitioned from those that do not present peptide sequences that bind to the predetermined macromolecule. The bacteriophage particles (i.e., library members) which are bound to the immobilized macromolecule are then recovered and replicated to amplify the selected bacteriophage subpopulation for a subsequent round of affinity enrichment and phage replication. After several rounds of affinity enrichment and phage replication, the bacteriophage library members that are thus selected are isolated and the nucleotide sequence encoding the displayed peptide sequence is determined, thereby identifying the sequence(s) of peptides that bind to the predetermined macromolecule (e.g., receptor). Such methods are further described in WO 91/17271, WO 91/18980, WO 91/19818 and WO 93/08278.

WO 93/08278 describes a recombinant DNA method for the display of peptide ligands that involves the production of a library of fusion proteins with each fusion protein composed of a first polypeptide portion, typically comprising a variable sequence, that is available for potential binding to a predetermined macromolecule, and a second polypeptide portion that binds to DNA, such as the DNA vector encoding the individual fusion protein. When transformed host cells are cultured under conditions that allow for expression of the fusion protein, the fusion protein binds to the DNA vector encoding it. Upon lysis of the host cell, the fusion protein/vector DNA complexes can be screened against a predetermined macromolecule in much the same way as bacteriophage particles are screened in the phage-based display system, with the replication and sequencing of the DNA vectors in the selected fusion protein/vector DNA complexes serving as the basis for identification of the selected library peptide sequence(s).

The displayed protein/peptide sequences can be of varying lengths, typically from 3-5000 amino acids long or longer, frequently from 5-100 amino acids long, and often from about 8-15 amino acids long. A library can comprise library members having varying lengths of displayed peptide sequence, or may comprise library members having a fixed length of displayed peptide sequence. Portions or all of the displayed peptide sequence(s) can be random, pseudorandom, defined set kernal, fixed, or the like. The display methods include methods for display of single-chain antibodies, such as nascent scFv on polysomes or scFv displayed on phage, which enable large-scale screening of scFv libraries having broad diversity of variable region sequences and binding specificities.

Another method of display is bacterial surface display. The protein of interest is expressed on the bacterial cell surface as a fusion with one of the following proteins, OmpA, LamB, PhoE, FliC, PALD, and EaeA intimin. Alternatively it may be expressed in the periplasm (periplasmic expression with cytometric screening, (PECS)) (Chen *et al.,* 2001). Whilst experiments demonstrate expression on the bacterial cell surface, this display system is not operating as well or used as frequently as the yeast surface display. The nature of a prokaryotic system itself may explain why the system is not always preferred. Firstly, bacteria lack the protein folding and post-translational modification machinery required for the presentation of an eukaryotic protein. Secondly, the protein of interest needs to be expressed in a soluble form. Thirdly, steric interference from the bacterial lipopolysaccharide layer can potentially impede binding to larger macromolecular antigens. Despite being a single cell system (one plasmid to each bacterium), capable of displaying thousands of copies of the protein of interest on the cell surface and being amenable to screening using flow cytometry, this system offers no additional advantages over the yeast surface display system.

A further method of display is yeast surface display. The protein of interest is fused to an alpha agglutinin subunit called Aga2p and expressed on the surface of the yeast cell. This form of display appears to be very successful and offers several advantages over other display systems. These include; correct protein folding and secretion (homologous to that in mammalian cells), display of large numbers of protein on yeast cell surface, single cell system (i.e. each yeast cell contains only one plasmid copy), system is amenable to screening using flow cytometry which offers finer quantitative discrimination between mutants and the dissociation constant (K_{D}) can be estimated *in situ* in the display format without having to subclone. The major disadvantage of this system is that the library size is restricted due to low transfection efficiency. To date no one has managed to overcome this limitation of the system.

Proteins can be anchored to the surface of mammalian cells via a number of different anchors including, but not limited to, Type I transmembrane domains (TM I), type II transmembrane (TM II) and glycosylphosphatydlinisitol (GPI).

Examples of suitable GPI anchor attachment signals include:
(i) GPI signal from human decay-accelerating factor (DAF). Caras IW, Weddell GN, Davits MA, Nussenzweig W, Martin DW. (1987) Science 238(4831):1280-3 Accession No.: AY055758
   Peptide sequence: PNKGSTTSGTTRLLSGHTCFTLTGLLGTLVTMGLLT (SEQ ID NO:23)
   Nucleotide sequence: 1158-1268
(ii) GPI signal from porcine membrane dipeptidase. Hooper NM, Low MG, Turner AJ. (1987) Biochem. J. 244(2):465-9
   Accession No.: E04233
   Peptide sequence: CRTNYGYSAAPSLHLPPGSLLASLVPLLLLSLP (SEQ ID NO:25)
   Nucleotide sequence: 1248-1346
(iii) GPI signal from rat ceruloplasmin. Patel BN, Dunn RJ & David S. (2000) J. Biol. Chem. 275(6):4305-10
   Accession No.: AF202115
   Peptide sequence: ASSQSYRMTWNILYTLLISMTTLFQISTKE (SEQ ID NO:27)
   Nucleotide sequence: 3161-3252
(iv) GPI signal from mouse Thy-1. Bernasconi E, Fasel N & Wittek R. (1996) J. Cell Sci. 109(6):1195-201
   Peptide sequence: Nucleotide sequence:
(v) Dictyostelium discoideum protein 1I. Stevens BA, White IJ, Hames BD Hooper NM. (2001) Biochimica et Biophysica Acta 1511: 317-329.
(vi) Plasmodium falciparum merozoite surface protein-1. Burghas PA, Gerold P, Pan W, Schwartz RT, Lingelbach K, Burjard H. (1999) Molecular and Biochemical Parisitology 104:171-183.

An example of a suitable transmembrane domain for use as an anchor domain in the present invention is:
(i) The transmembrane domain of murine B7-1. Chou W., Liao K., Jiang S.Y., Yeh M.Y. and Roffler S.R.(1999) Biotechnol Bioeng. 1999 Oct 20;65(2):160-9.
   Accession No.:AH0046553
   Peptide sequence: PEDPPDSKNTLVLFGAGFGAVITVVVIVVIIKCFCKH (SEQ ID NO:31)
   Nucleotide sequence: 171-281:

Other suitable examples include those provided in Table 1.

**Table 1 : Examples of anchors suitable for surface display:**

| Anchor type | Molecule name | Accession No. |
|---|---|---|
| <Transmembrane type I (TM I) | CD1a | X04450 (gi 32495) |
| | CD68 | BC05557(gi 33869409) |
| Transmembrane type II (TM II) | CD10 | Y00811 (gi 29625) |
| | CD13 | X13276 (gi 28677) |
| | CD26 | M74777 (gi 180082) |
| Glycosylphosphatydlinisitol (GPI) | CD14 | X113334 (gi 29740) |
| | CD24 | M58664 (gi 180167) |
| | CD48 | M59904 (gi 180138) |
| | CDw52 | X62466 (gi 29645) |
| | CD55 | M31516 (gi 181467) |
| | CD59 | X16447(gi 29805) |
| | CD67 | X52378 (gi 29918) |

### Signal peptide sequences

For TM1 and GPI anchors, an N-terminal signal sequence as well as a C-terminal signal sequence is preferably be added to the polypeptide if it is not normally found on the cell surface. For TM2 the signal and anchor sequence are one and the same and are added to the N-terminus of the polypeptide. To achieve adequate levels of surface expression, it may be necessary to mutate the initiation codon of the target gene so that only chimeric proteins consisting of the signal and/or anchor fused to the target protein are produced.

An example of an appropriate signal sequence is the signal sequence of CD59:
Accession No.:X16447 (gi 180082)
Peptide sequence: MGIQGGSVLFGLLLVLAVFCHSGHS (SEQ ID NO:33) Nucleotide sequence: 64-138

Proteins/peptides encoded by mutant nucleic acids obtained using the methods of the invention can be used in a number of yeast based methods to detect protein-protein interactions. One well known system is the yeast two-hybrid system (Fields and Song 1989) which has been used to identify interacting proteins and to isolate the corresponding encoding genes. In this system, prototrophic selectable markers which allow positive growth selection are used as reporter genes to facilitate identification of protein-protein interactions. Related systems which may be employed include the yeast three-hybrid system (Licitra and Liu 1996) and the yeast reverse two-hybrid system (Vidal et al. 1996). Such procedures are known to those skilled in the art.

### Examples

### Example 1: Defining a region in RAMOS cells for integration of target genes

### Methods & Materials

### Cell line and cell culture conditions

The RAMOS strain RA 1 was obtained from the American Tissue Culture Collection (ATCC-CLR-1596). This strain is IgM positive and expresses the interleukin 4 (IL-4) and CD23 receptors. Cells were maintained in RPMI 1640 medium (Gibco BRL) supplemented with 10% heat inactivated fetal calf serum (FCS) and penicillin (100 U / ml) and streptomycin (100 µg / ml), and incubated at 37 °C with 5% CO₂.

### Extraction of DNA from cells

Cells were harvested and centrifuged at 1500 rpm and resuspended in PBS. DNA was extracted from cells using a Genoprep DNA isolation kit (Scientifix, Australia) according to manufacturer's instructions. Briefly, after removing the supernatant 375 µl of lysis and binding solution was added to cells (5 x 10⁵), together with 20 µl Genoprep DNA magnetic beads. This mixture was vortexed for five seconds then incubated at room temperature for a minimum of ten minutes on a rocker, or rotating wheel. Beads with attached DNA were collected using a magnet, the supernatant was removed and 450 µl of washing solution was added. The mixture was subsequently vortexed for five seconds and the beads were collected as described previously. This washing procedure was repeated twice, with the final wash solution being 70 % ethanol. Beads were resuspended in 450 µl of 70 % ethanol and transferred to a new tube. After removing the supernatant, 450 µl of sterile water was added to the beads and removed immediately. The beads were then resuspended in 200 µl of sterile water and incubated at 70 °C for two minutes to elute the DNA. The beads were collected again using a magnet and the supernatent containing the eluted DNA was transferred to a new tube.

The quantity and quality of isolated DNA was determined by spectrometry and electrophoresis. A culture containing 5 x 10⁵ cells consistently yielded 10 ng / µl of DNA. This DNA migrated as a single band at approximately 23 kbp on a 0.9 % gel indicating that the genomic DNA was intact.

### Sequencing of the 5' region upstream of the rearranged VH allele

The homologous sequence upstream of the site of integration chosen for the vector corresponds to a ~ 5kb fragment between the VH₇₋₃₅ and VH₄-₃₄ alleles of the immunoglobulin heavy chain locus of the RAMOS cell line (corresponding to the sequence gi 4512287 nucleotides 54521- 59517).

RAMOS RA-1 genomic DNA was prepared using the Genoprep DNA isolation kit (Scientifix, Australia). Platimun PfX DNA polymerase (GibcoBRL, Life Technologies) was used to amplify fragments varying from 300bp to 1000 bp from genomic DNA. The reaction included 1 X PfX amplification buffer, 50 mM Magnesium sulfate, 1 X PCR enhancer solution, forward primer (10 pM), reverse primer (10 pM), dNTPs (10 mM each) template DNA (100 ng), platinum PfX DNA polymerase (0.6 U) and sterile water in a final volume of 20 µl. Cycling conditions were as follows; one cycle of 95°C for 5 minutes, 30 cycles of 95°C for seconds, 60°C for 30 seconds, 68°C for one and a half seconds, and one cycle of 72°C for 7 minutes. Annealing temperatures and extension times for some primer sets varied, ranging from 55°C to 65°C and one minute to two and a half minutes respectively. Primers were designed based on the human germline DNA for immunoglobulin heavy-chain variable region, complete sequence gi 4512287 nucleotides 54786 to 59721 (Table 2). A second PCR reaction was performed using 0.5 / 20 µl of the first PCR as a template to gain sufficient DNA for cloning.

PCR products were run on 1.0 % agarose gels and DNA was extracted using Nucleospin Extraction Kit (Nagel-Macherey, Germany) according to manufacturer's instructions. The purified DNA was digested with restriction enzymes EcoR I and Hind III. Digested products were cleaned up and concentrated using phenol extraction followed by ethanol precipitation. These products were ligated into into pBluescript SK + (Stratagene, Texas, USA) and transformed into *Eschericlzia coli* XL1 Blue electro-competent bacteria. Minipreps were prepared from 5 ml overnight cultures using QIAprep miniprep spin kit (Qiagen, CA, USA) and sequenced using an ABI 373 DNA sequencer with primers T3 and T7.

Sequences were analysed using BLAST program (NBCI, http://www.ncbi.nlm.nih.gov/BLAST/) and assembled in Clone Manager Suite 7 (Scientific and Educational Software). The assembled sequence is set out as nucleotides 1 to 5190 of SEQ ID NO:1. This sequence shares 99 % similarity with the published sequence gi 4512287 nucleotides 54521-59517.

### Cloning of the 5kb fragment upstream of the rearranged VH allele from genomic DNA

Genomic DNA was prepared using Genoprep DNA isolation kit (Scientifix, Australia). Platinum PfX DNA polymerase (Invitrogen, CA. USA ) was used to amplify the 5 kb fragment from genomic DNA. The reaction included 1 X PfX amplification buffer, 50 mM magnesium sulfate, 1X PCR enhancer solution, forward primer 8771 (5'CCATCGATAATTTAGTTTTCACGGGGCATCTGCAGGGT 3') 10 pM, reverse primer 8872 (5' GGGGTACCGTTCTTGTGCAGGAGGTCCATGACTCTCAG 3') 10 pM, dNTPs (10 mM each) template DNA (100 ng), platinum PtX DNA polymerase (0.6 U) and sterile water in a final volume of 20 µl. Cycling conditions were as follows ; one cycle of 94 °C for 15 seconds, fifteen cycles of 94°C for 10 seconds, 68°C for 3.5 minutes, 15 cycles of 94°C for 10 seconds, 68°C for 3.5 minutes with and an extra 15 seconds added each cycle, and one cycle of 72°C for 7 minutes. A second PCR reaction was performed using 0.5 / 20.0 µl of the first PCR reaction as a template to gain sufficient DNA for detection using ethidium bromide.

PCR products were run on a 0.9 % agarose gel and DNA was extracted using Gel Extraction Kit (Qiagen, CA, USA). Purified DNA was cloned into pPCRScript using the PCR-Script Amp cloning kit (Stratagene, Texas, USA) at the Srf I site. The resulting construct was referred to as 5kbPCRScript 15a-7 (7971 bp). This construct was sequenced using primers in Table 3.

**Table 2: Primers used for genomic PCR to sequence the 5' region upstream of the rearranged VH allele (VH₄₋₃₄) in RAMOS RA-1**

| Primer Name | Sequence | Homologous sequence in gi AB019439 |
|---|---|---|
| 8444 | | NT 58890 - 58920 |
| 8436 | | NT 59702 - 59731 |
| 8438 | | NT 59281 - 59310 |
| 8440 | | NT 58890 - 58920 |
| 8441 | | NT 58420 - 58450 |
| 8442 | | NT 58500 - 58530 |
| 8464 | | NT 57720 - 57750 |
| 8466 | | NT 57330 - 57360 |
| 8470 | | NT 56550 - 56580 |
| 8472 | | NT 56160 - 56190 |
| 8550 | | NT 58951- 58981 |
| 8553 | | NT 55770 - 55800 |
| 8554 | | NT 55300 - 55330 |
| 8555 | | NT 55380 - 55410 |
| 8557 | | NT 54990 - 55020 |
| 8558 | | NT 54520 - 54550 |
| 8606 | | NT 58500 - 58530 |
| 8607 | | NT 56550 - 56580 |
| 8608 | | NT 55500 - 55529 |
| 8687 | 5' CCCAAGCTTCATGTTCCACGCATTACGTC 3' (SEQ ID NO:54) | NT 54336 - 54355 |
| 8689 | | NT 54927 - 54946 |

**Table 3 : Primers used for sequencing clones containing the 5 kb region upstream of the rearranged VH allele (VH₄₋₃₄) in RAMOS RA-1**

| Primer Name | Sequence | Homologous sequence in gi AB019439 |
|---|---|---|
| 8445 | | NT 57640-57670 |
| 8443 | | NT 58030-58060 |
| 8471 | | NT 56080-56110 |
| 8465 | | NT 57250-57280 |
| 8473 | | NT 55690-55720 |
| 8609 | | NT 55027-55056 |
| 8467 | | NT 56860-56890 |
| 8439 | | NT 58810-58840 |
| 8606 | | NT 58500- 58530 |
| 8551 | | NT 56550- 56580 |
| 0003 | | NT 54497-54520 |
| 0006 | | NT 55001-55024 |
| 0007 | | NT 58514-58537 |
| 0008 | | NT 58885-58908 |

### PCR amplification of rearranged VH, D and JH segments from genomic DNA

Genomic DNA was prepared using the Genoprep DNA isolation kit (Scientifix, Australia). Platinum PfX DNA polymerase (Invitrogen, CA, USA) was used to amplify the rearranged VH, D and JH genes from genomic DNA. The PCR reaction included 1X PfX amplification buffer, 50 mM magnesium sulfate, 1X PCR enhancer solution, forward primer (10 pM), reverse primer (10 pM), dNTPs (10 mM each), template (100 ng), platinum PtX DNA polymerase (0.6 U) and sterile water in a final volume of 20 µl. Cycling conditions were as follows; one cycle of 95°C for 5 minutes, 30 cycles of 95°C for 30 seconds, 65°C for 30 seconds, 68°C for 2.5 minutes, and one cycle of 72°C for 7 minutes. Primers used were specific for each of the seven VH family leader sequences together with a previously described consensus JH primer, JOL48, that anneals to all six human JH segments (Table 4). A second PCR reaction was performed using 0.5 / 20 µl of the first PCR as template to gain sufficient DNA for detection using ethidium bromide.

PCR products were run on 1.5 % agarose gels and DNA was extracted from bands using Nucleospin Extraction Kit (Nagel-Macherey, Germany) according to manufacturer's instructions. A third PCR was performed on the purified DNA as described above. Products were cloned into pBluescript SK + (Stratagene, Texas, USA) using EcoR I and Hind III sites and sequenced as previously described.

**Table 4 : Primers for PCR amplification of rearranged VH, D and JH segments from genomic DNA.**

| Primer Name | Sequence | Specificity |
|---|---|---|
| 8111 | | VH1 leader |
| 8116 | | VH2 leader |
| 8112 | | VH3 leader |
| 8113 | | VH4 leader |
| 8118 | | VH5 leader |
| 8114 | | VH6 leader |
| 8115 | | H7 leader |
| N8336 (JOL48) | | Consensus JH |

**Table 5 : Primer sequences used for genomic PCR to sequence the 3 kb region downstream of the rearranged VH allele (VH₄₋₃₄) in RAMOS RA-1**

| Primer Name | Sequence | Homologous sequence in NCBI database |
|---|---|---|
| 8604 | | Gi 34819 NT 1- 30 |
| 8605 | | Gi 34819 NT 450 - 500 |
| 8559 | | Gi 33100 NT 460- 490 |
| 8562 | | Gi 33100 NT 705- 734 |
| 8603 | | Gi 34819 NT 126- 155 |
| 8692 | | Gi 33101 NT 301- 320 |
| 8695 | | Gi 188910 NT 945- 974 |
| 8697 | | Gi 188910 NT 4-30 |
| 8508 | | Gi 188910 NT 370- 400 |
| 8696 | | Gi 188910 NT 715- 744 |
| 8694 | 5' CCGGAATTCGCCCAGCCCAGCCTAGCTCA 3' (SEQ ID NO:80) | Gi 33101 NT 770- 790 |
| 8691 | 5' CCCAAGCTTTTATCAACTGCTAGTTTGTG 3' (SEQ ID NO:81) | Gi 34819 NT 361- 381 |
| 9090 | | Gi 188910 NT 1-30 |
| 9879 | | Gi 33100 NT 1-24 |
| 9805 | | Gi 33101 NT 770- 790 |
| 0002 | 5' GAAAGTTAAATGGGAGTGACCCAG 3' (SEQ ID NO:85) | GI 29502084 NT 962860 - 962884 |
| 0021 | 5' GAGTGACCATCGCACCCTTGACAG 3' (SEQ ID NO:86) | Gi 29502084 |

### Identification of rearranged VDJ segment in RAMOS RA-1

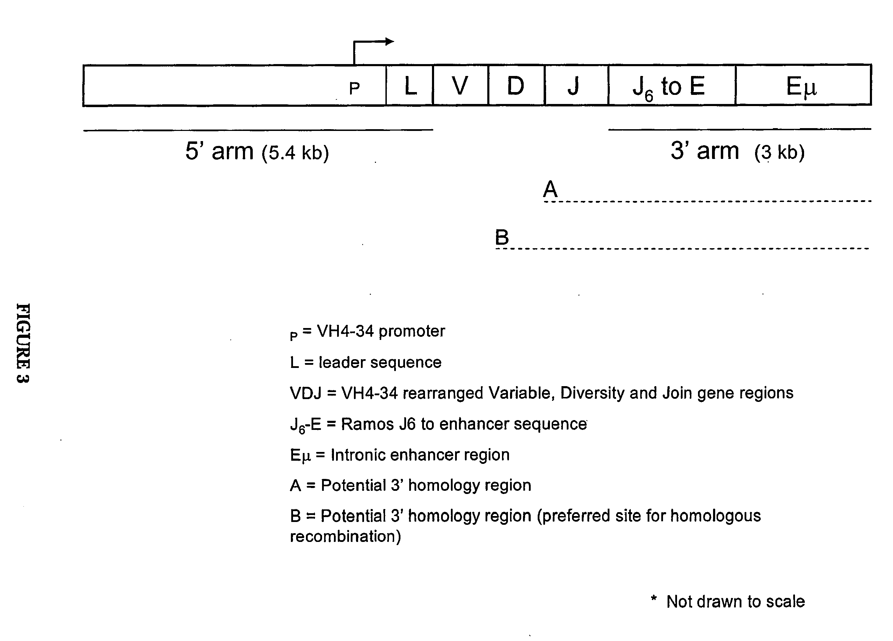

The sequenced VH allele for RAMOS RA-1 was identified as VH₄₋₃₄ (DP 63) using V-Base (http://www.mrc-cpe.cam.ac.uk/vbase). A schematic diagram of the gene targeting region is shown in Figure 3 and the genomic sequence is set out in SEQ ID NO:1. The immunoglobulin heavy chain promoter shown in Figure 3 corresponds to nucleotides 4852 - 5190 of SEQ ID NO:1. The leader sequence (including intron 1) shown in Figure 3 corresponds to nucleotides 5191 - 5329 of SEQ ID NO:1. The VDJ segment shown in Figure 3 corresponds to nucleotides 5330 - 5708 of SEQ ID NO:1.

The consensus nucleotide sequence differs from the published VH₄₋₃₄ sequence (V - Base) by six nucleotides only (C₆₈->G, C₇₂->T, C₂₂₈->G, T₂₃₂->C, C₂₄₄->T, C₂₄₈->A) of which four were coding changes (A₂₃->G; N₇₆->K, F₇₈->L, T₈₃->N). The sequence was further classified as VH₄₋₃₄ subgroup 2 (Journal of Molecular Biology, 1987, Vol 195, 761-768) using the Kabat database (Kabat et al., 1991, Sequences of proteins of immunological interest, vol 1, 5th edition). The C₆₈-> G mutation in framework 1 and N₇₆- > K mutation in framework 4 were unique to RAMOS RA-1 and occurred in otherwise conserved residues in the other four members of the group (Lee et al. 1987, Journal of Immunology, 142, 4054-4061, Sanz et al. 1988, Clinical Experimental Immunology 71, 508-516).

The nucleotide sequence generated from RAMOS DNA corresponding to the D allele differed significantly from the published alleles in V Base. Although no significant homology was identified, the closest related sequence similarity (and sequence length) was to D₃₋₁₆ (Corbett, S et al, Journal of Molecular Biology, 270, 587-597).

The sequenced JH allele for RAMOS RA-1 was identified as JH6b (Mattila et al. 1995) using V-Base. No nucleotide base changes were detected between the consensus sequence and the published JH_{6b} sequence.

### Sequencing of the 3' region downstream of the rearranged VH allele

The homologous sequence downstream of the site of integration chosen for the vector corresponds to a ~ 3kb region between the rearranged VJD genes through to the mu enhancer of the human immunoglobulin heavy chain locus of the RAMOS cell line (corresponding to sequence gi 29502084 nucleotides 960091 - 962947).

The region 3' downstream of the rearranged VDJ segment was sequenced using methods previously described. Primers were designed based on published sequences, human immunoglobulin heavy chain enhancer on chromosome 14 (gi 34819), human J6 to enhancer DNA of the immunoglobulin heavy-chain gene (gi 33100), human (AW-Ramos) translocated t(8;14) c-myc oncogene, exon 1 (gi 188910) and human mu switch DNA of the immunoglobulin heavy-chain gene locus (gi 33101)(Table 5).

Sequences were analysed using BLAST program (NBCI, http://www.ncbi.nlm.nih.gov/BLAST/) and assembled in Clone Manager Suite 7 (Scientific and Educational Software). The assembled sequence is set out as nucleotides 5709 to 8634 of SEQ ID NO:1. This sequence shares 98 % similarity with the published sequence gi 29502084 nucleotides 960091-962947.

### Cloning of the 3kb fragment downstream of the rearranged VH allele from genomic DNA

The 3 kb fragment was amplified from genomic DNA extracted from RAMOS RA-1 cells using Platinum PfX DNA polymerase (Invitrogen, CA, USA). The PCR reaction was the same as previously described except the forward primer 9779 (5' CCGCTCGAGTGGGAGCCTCTGTGGATTTTCCGA 3') (SEQ ID NO:111) and reverse primer 9801 (5' TGACCGGACGTCGCCCAGCCCAGCCTAGCTCA 3') (SEQ ID NO:112) were used and the cycling conditions were as follows ; one cycle of 94°C for 15 seconds, fifteen cycles of 94°C for 10 seconds, 68°C for 2 minutes, 15 cycles of 94°C for 10 seconds, 68°C for 2 minutes with and an extra 15 seconds added each cycle, and one cycle of 72°C for 7 minutes. A second PCR reaction was performed using 0.5 / 20.0 µl of the first PCR reaction as a template to gain sufficient DNA for detection using ethidium bromide.

The 3 kb fragment was cloned into pPCRScript using the PCR-Script Amp cloning kit (Stratagene, Texas, USA) at the Srf I site. The resulting construct was referred to as 3kbPCRScript 10-1-3 (5822 bp). This construct was sequenced using primers in Table 5.

The size and GC rich stretches present in the 5 kb and 3 kb homology regions gave rise to structural regions which caused difficulties in cloning and thereafter problems with stability of the cloned regions.

### Example 2: Design and construction of a vector for integration into the rearranged VH allele, VH4-34 in RAMOS RA-1

### 1) Construction of Vector For Integration

A 3 kb fragment, containing sequence homologous to the region downstream of the rearranged allele Volt.34 was amplified from the construct 3kbPCRScript 10-1-3 with the forward primer 9879 (5' CGGGTGATATGTGGGAGCCTCTGTGGATTTTCCGA 3') (SEQ ID NO:83) and the reverse primer 9805 (5' AGCCGGATATCGCCCAGCCCAGCCTAGCTCA 3') (SEQ ID NO:84) using Platinum PfX DNA polymerase (Invitrogen, CA. USA). PCR products were purified using QIAquick PCR Purification Kit (Qiagen^{™}) according to manufacturer's instructions then digested with EcoRV and ethanol precipitated. Digested fragments were ligated into construct 5kbPCRScript 15a-7 containing the 5 kb sequence upstream of the rearranged VH allele. The DNA was transformed into Esherichia coli and grown at 37 °C overnight.

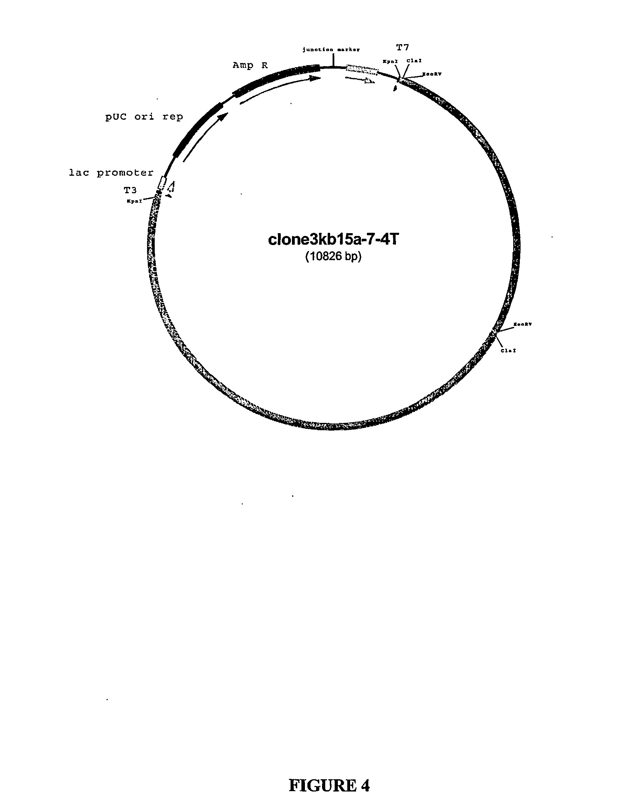

Bacterial colonies were screened by Southern blotting and probed using ³²P labeled oligonucleotides 8604 (5' CCCAAGCTTCGGCCCCGATGCGGGACTGCGTTTTGACCA 3') (SEQ ID NO:70) to detect the 3 kb fragment and a pool of labeled oligonucleotides 8687 (5' CCCAAGCTTCATGTTCCACGCATTACGTC 3') (SEQ ID NO:54), 8440 (5' CCGGAATTCAATTTGAGATTGTGTGTGAGATCTCAGGAG 3') (SEQ ID NO:38) and 8472 (5' CCGGAATTCGTTGGGTTCCCAGTGTAGGTGATGATCCAT 3') (SEQ ID NO:44) to detect the 5 kb fragment. Colonies that were positive for both fragments were subcultured and DNA was extracted using QIAprep Miniprep kit (Qiagen^{™}). Clones were analysed by diagnostic restriction enzymes and PCR for each fragment and sequenced using an ABI 373 DNA sequencer with T7 and T3 primers. This new construct is referred to as 3kb15a-7- 4T is 10 826 bp long (Figure 4). The sequence of is shown in SEQ ID NO:87.

### 2) Transfection of 3kb15a-7-4T into RAMOS and screening for integration

Clone 3kb15a-7-4T (5 µg) is transfected into 3 x 10⁶ RAMOS RA-1 cells using the following protocol ; cells are centrifuged to remove spent media then resuspended at 1 x 10⁶ cells / ml in RPMI containing DEAE Dextran (25 µg / ml). Resuspended cells (1 ml) are transferred into electroporation cuvettes (4 mm gap) and DNA is added. The cuvette containing the cell / DNA mixture is then incubated at 37°C for 10 minutes. Cells are then pulsed twice at 550 V and 25 µFd capacitance, using a Gene Pulser (BioRad). Following a 10 minute incubation on ice, the cells are removed from the cuvette and transferred to T₂₅ flasks containing 9 ml of RPMI + 15 % FCS. Flasks are incubated at 37°C for at least 24 hours before the efficiency of transfection is assessed. Mock transfected cells are used as controls.

Three days after transfection, cells are stained for surface IgM and sorted by flow cytometry into an IgM positive population and IgM negative population. Prior to this experiment the background level of IgM negative cells is determined.

Genomic DNA is extracted from IgM negative cells using the Genoprep DNA isolation kit (Scientifx, Australia) according the manufacturers' instructions. The DNA is then digested using Xba I enzyme and run on a 0.6 % agarose gel. DNA is transferred onto nitrocellulose membrane using standard methods for Southern blotting and probed with ³²P labeled oligonucleotides 0041 (5' GACGGTATCGATAAGCTTGATATCGAATTCCTGCAGCCCGGG 3') (SEQ ID NO:88) and 0042 (5' GACCTCCTGCACAAGAACGGTACCGGGCTAGAGCGGCCGCCA 3') (SEQ ID NO:89) that span the junction regions. A probe against the middle of the sequence that would be integrated, 9403 (5' CAGTGCTGCAATGATACCGCGAGAC 3') (SEQ ID NO:90) is also used.

The following patterns of radioactive probe binding to DNA extracted from cells transfected with i) vector only, no radioactive signals ii) the construct containing the 3kb15a-7 4T shows radioactive signals with all three radioactive probes as is expected when the 5kb and 3kb recombination occurs with the chromosomal DNA integrating the middle sequence; the binding of the radioactive 9403 probe shows this iii) DNA from untransfected cells show no binding with the 9403 radioactive probe, iv) the lanes on the agarose gel with the plasmid 3kb15a-7-4T show radioactive signal with all probes. Obtaining this pattern i-iv) is indicative of 3kb15a-7-4T integration into the host cell genome.

### Example 3 : Design and construction of a vector for integration into the rearranged VH allele, VH₄₋₃₄ in RAMOS RA-1 and mutation of the asFP499 gene

The components of the vector for integration are as follows:
i) Construct 5 kb in PCRScript 15a-7 is modified by removing the Nae I - Xho I fragment which effectively deletes an additional Kpn I site. This new construct is herein referred to as 5kbPCRScript minus Nae I - Xho I and is 7608 bp in length.
ii) The cloned gene for asFP499, which is a fluorescent protein isolated from the sea anemone *Anemonia sulcata,* was obtained from J. Wiedenmann (University of Ulm, Germany) in the plasmid pQE32 (Qiagen, CA. USA). Four sequential PCR reactions were performed to introduce restriction sites Kpn I and Not I at the 5' and 3' ends of the asFP499 gene respectively and two C-terminal flag tags with a Sal I site at the 3' end of the second flag tag. This final product (~770 bp) is subcloned into pPCRscript (Stratagene, Texas, USA) and herein is referred to as targetPCRScriptasFP499-1.
iii) The gene encoding thymidine kinase is amplified by PCR and restriction sites Hind III and Cla I are introduced at the 5' and 3' end of the gene respectively. This product (1260 bp) is subcloned into pPCRscript (Stratagene, Texas, USA) and herein is referred to as construct TKPCRscript -64.
iv) pMClneo Poly A (3800 bp) was obtained from Stratagene (Texas, USA).

These components are assembled in the following order:

Constructs targetPCRScriptasFP499-1 and 5kbPCRScript minus Nae I - Xho I are digested with Kpn I and Sal I and run on 1.0 and 0.9 % agarose gels respectively. The desired products (~7608 bp and ~770 bp) are cut out and DNA extracted using QIAquick gel extraction kit (QiagenTM). The asFP499 gene (~770 bp) is ligated into 5kbPCRScript minus Nae I - Xho I (~7608 bp) to create 5kb-asFP499PCRscript minus NaeI-XhoI (8289 bp).

Constructs TKPCRscript -64 and 5kb-asFP499PCRscript minus Nae I - XhoI are subsequently digested with Cla I and Sal I and run on agarose gels and DNA is extracted as described above. The asFP499-5kb fragment ~5770 bp) is ligated to the TKPCRScript-64 backbone (~ 4737 bp) to generate TK-5kb-asFP499 PCRscript (10464 bp).

The TK-5kb-asFP499 cassette (~7558 bp) is digested out of TK-5kb-asFP499 PCRscript with Sal I and Hind III and ligated into pMClneo Poly A, cleaved with Sal I and Hind III (3837 bp). This new construct is designated KW 1.

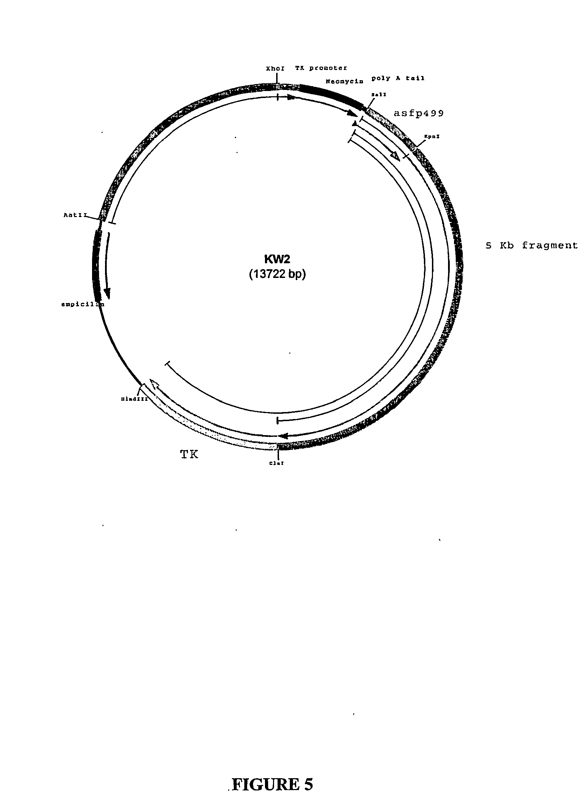

Finally, 3kbPCRscript 10-1-3 and KW1 are digested with restriction enzymes Xho I and Aat II and gel purified as described above. The 3kb fragment is ligated into the KW1 backbone ~10868 bp) yielding the final integration vector designated KW2 (13722 bp) (Figure 5). The sequence of integration vector KW2 is set out in SEQ ID NO:91.

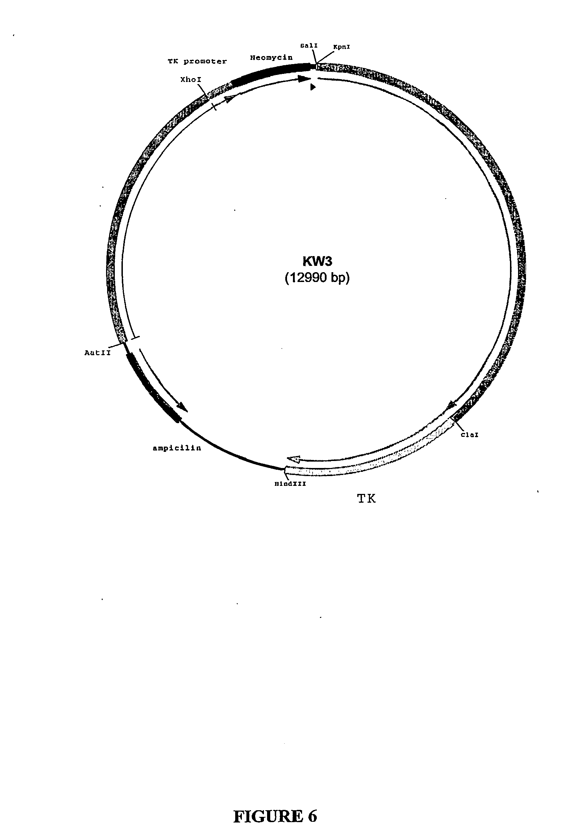

The asFP499 gene was deleted from vector KW2 to generate vector KW3 (Figure 6). The sequence of integration vector KW3 is set out in SEQ ID NO:110. It will be appreciated that any target nucleic acid molecule of interest can be inserted into this vector for use in the affinity maturation process of the present invention.

### Example 4: Optimal culturing conditions for RAMOS RA-1 cells

Optimal growth conditions for RAMOS cells were determined by performing growth curve experiments using different supplements to the medium and different seeding concentrations. RAMOS cells were seeded at 1 x 10⁴, 5 x 10⁴, 1x 10⁵, and 2 x 10⁵ (cells / ml) and cultured as 25 ml cultures in either RPMI medium with 10 % heat inactivated FBS and 1mM sodium pyruvate or RPMI medium with 15 % heat inactivated FBS, 1 mM sodium pyruvate and 50 % conditioned medium. Every 24 hours, 1 ml samples of cells were taken and the number of viable cells was determined using the Vi-Cell Viability Analyser (Beckman Coulter, CA, USA,). The results showed that RAMOS cells had a lag phase of 48 hours regardless of the seeding concentrations. Cultures reach an exponential growth phase at a density of 0.25 - 0.5 x 10⁶ cells / ml RPMI medium with 10 % heat inactivated FBS and 1 mM sodium pyruvate whereas cells growing in RPMI medium with 15 % heat inactivated FBS, 1 mM sodium pyruvate and 50 % conditioned medium did not enter an exponential phase. The optimal seeding rate for RAMOS was 1 x 10⁵ cells / ml.

Mycoplasma infection can affect transfection rates, therefore we tested RAMOS RA-1 monthly. Cells were tested using 4', 6-diamidino-2-phenylindole (DAPI) staining in which all DNA is stained specifically with this very bright dye. If mycoplasma is present small bright specks of dye are seen in the cytoplasm. Cells were fixed onto glass slides and viewed under a 100 X objective with a UV filter system. Cells were negative for mycoplasma over a period of 12 months.

### Example 5: RAMOS RA-1 cell division rate

Transfection rates in RAMOS are affected by the viability of cells in culture. To identify the optimal time during the growth cycle to transfect RAMOS cells, we first determined the cell division rate. RAMOS cells were stained with cell-permeant fluorescein-based dye carboxyfluorescein diacetate succinimidyl ester (CFSE) and analysed by flow cytometry based on established techniques in Current Protocols in Cytometry 9.11.2.

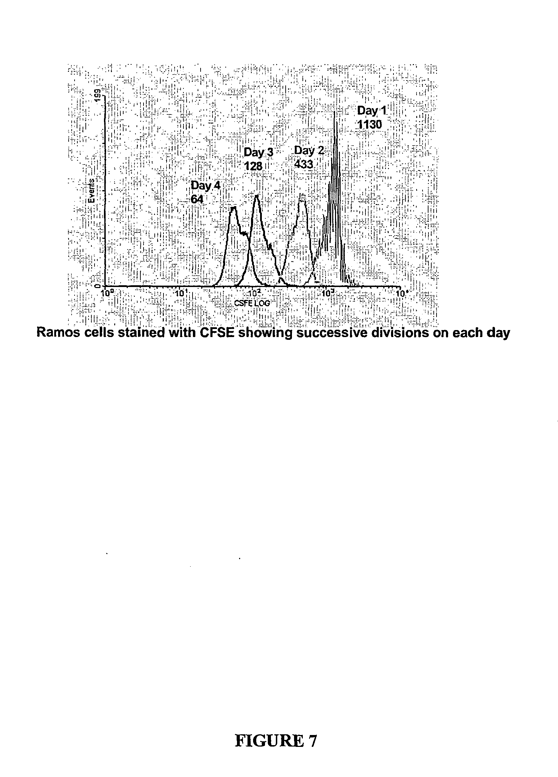

Briefly, RAMOS cells in exponential log phase growth were washed and re-suspended in phosphate buffered saline at 5x10⁶ cells / ml. A volume of 2 µl of 5 mM CFSE was added per ml of cells. Cells were incubated for 10 minutes at 37°C after which 5 volumes of ice cold RPMI + 10 % FBS was added. Cells were then incubated on ice for a further 5 minutes and washed three times in culture medium before transferring to flasks and culturing under normal conditions (see example 1). Initially cells were not analysed until 12 hours post staining to allow for the initial fluorescence decay. Thereafter cells were anlaysed by flow cytometry every 24 hours. The CFSE fluorescence was plotted against the number of cells anlaysed (Figure 7). Upon cell division, the dye is equally distributed between daughter cells, allowing the resolution of up to eight cycles of cell division by flow cytometry. In the case of using cultured cells that divide in concert, the resulting fluorescent distribution is halved per cell after each division. Therefore, these results indicate that the cell population was dividing at least once every 24 hours.

### Example 6: Optimisation of conditions for transfection of RAMOS RA-1 cells

### 1) Construction of vector for expression of asFP499 in RAMOS RA-1

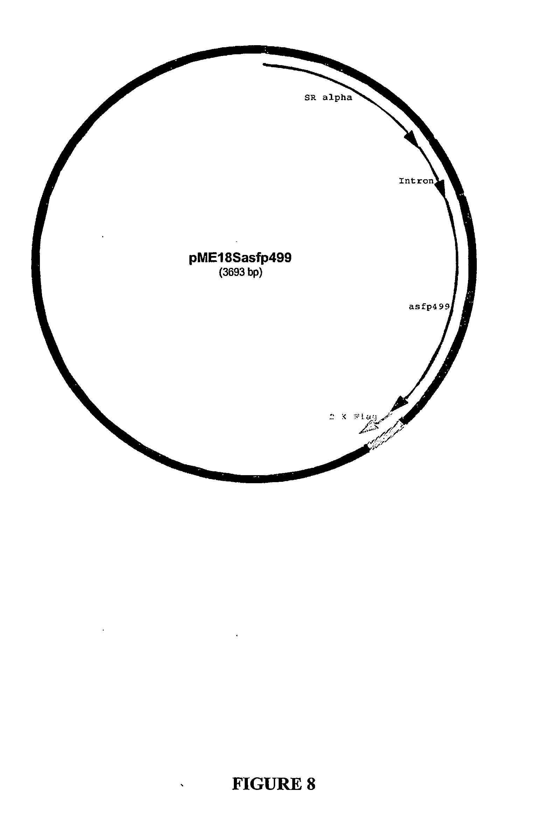

Primers were designed to add a Xho I site to the 5' end of the asFP499 gene and two flag tags, two stop codons and a Xbo I to the 3' end of the gene to allow cloning into pME18s. The primers used are 8934 (5' CCGCTCGAGATGTATCGTTCCATCAAGGAAACC 3') (SEQ ID NO:92), 8935 (5' TCTAGATTATTATTTATCATCATCATCTTTATAATCTTTATCATCATCATCTT TATAATCAGCGGCCGC 3' (SEQ ID NO:93); 8936 (5' CTAGTCTAGATTATTATTTATCATCATCATC 3') (SEQ ID NO:94), and 8398 (5' GTTATGTCCTAATTTCGAAGGCACTTGGGAGTA 3') (SEQ ID NO:95). The cloned sequence was confirmed by DNA sequence analysis. The resulting construct, referred to as pME18sasFP499 (3693 bp) (Figure 8) (SEQ ID NO:8) was used for subsequent transfection of RAMOS cells.

### ii) Transfection of pME1 8sasFP499 into RAMOS RA-1 cells

A number of different transfection reagents or methods can be used to transfect mammalian cells including Calcium Phosphate coprecipitation, Lipofectamine (Invitrogen), FuGENE6 (Roche, Germany), SuperFect (Qiagen, CA, USA), Effectene (Qiagen, CA, USA), GenePORTER (Gene Therapy Systems, CA, USA) and Metafectene (Biontex, Germany). Electroporation is another method commonly used to transfect mammalian cells. Electroporation can be carried out using different electroporators such as the Gene Pulser (BioRad, CA, USA) or the Electro Square Porator ECM 830 (BTX, Fisher Biotech, Australia) and various voltage and capacitance settings can also be used. Since RAMOS RA-1 cells are of B-lymphoid origin and it is known that lymphoid cells can be difficult to transfect, we optimised transfection of this cell line.

The efficiency of transfection of RAMOS RA-1 cells was monitored by flow cytometric analysis using an EPICS Elite (Beckman Coulter, CA, USA). Samples of transfected cells were stained with 1 µg / mL Propidium Iodode (PI). The live cell population (based on forward and side scatter characteristics and PI staining) was gated and the percentage of Fluorescent Protein (FP) positive cells was assessed. FP expression was also assessed by fluorescence microscopy using an Olympus IX70 microscope and Olympus U-RFL-T burner.

In order to optimise the transfection process, a number of different transfection parameters were tested including: set voltage, set capacitance, amount of DNA, concentration of DEAE Dextran and analysis time post transfection.

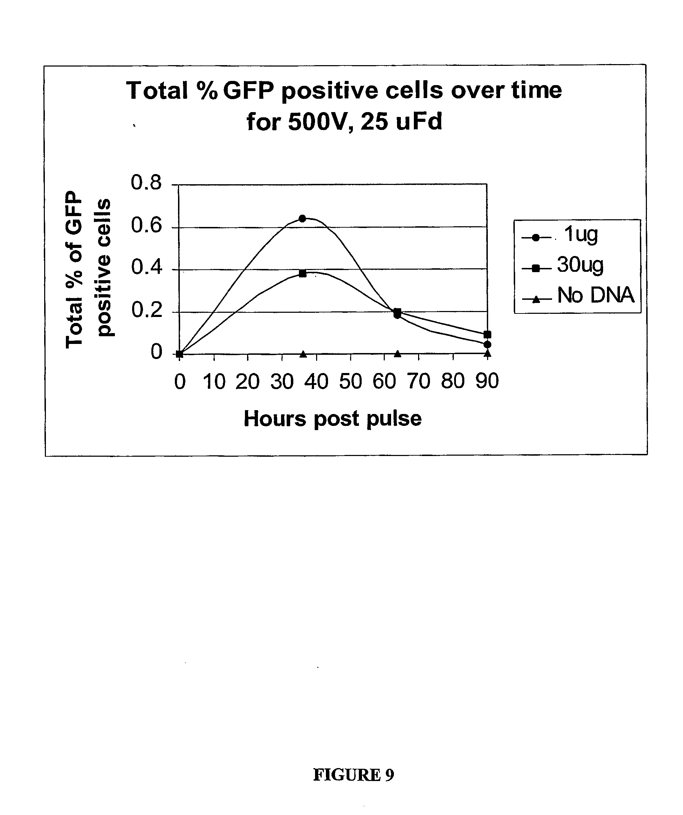

Figures 9 show the results of a representative set of optimisation experiments. The combination of 550 V and 25 µfd resulted in the highest transfection efficiency. No significant effect on the transfection efficiency was observed using 1 µg or 30 µg of pME18sEGFP. The optimum time to analyse FP expression was between 24 and 36 hours post electroporation. DEAE Dextran varying from 25 µg / ml to 1 mg / ml was tested and concentrations between 25 µg / ml and 100 µg / ml were optimal for transfection but concentrations above 500 µg / ml were found to be toxic to the cells (data not shown).

Using the optimum conditions, transfection rates of between 0.5 % and 3.5 % were achieved with the average being approximately 1.0 %.

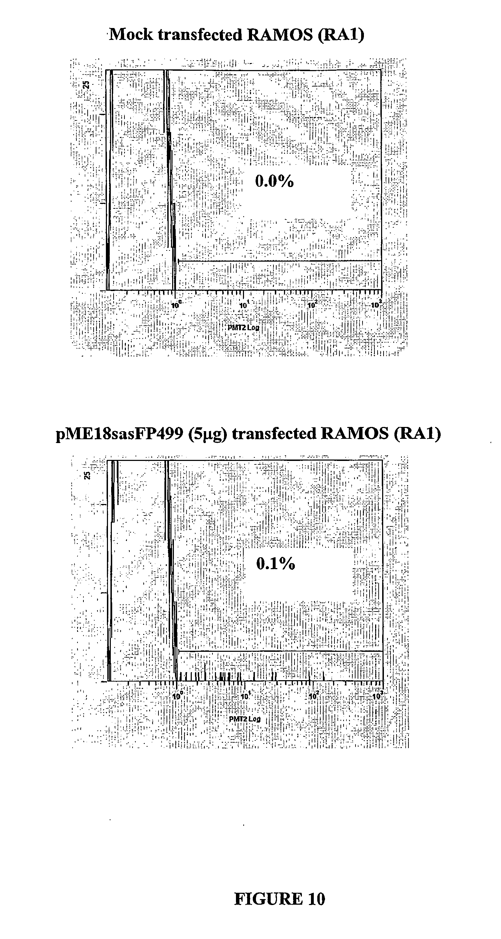

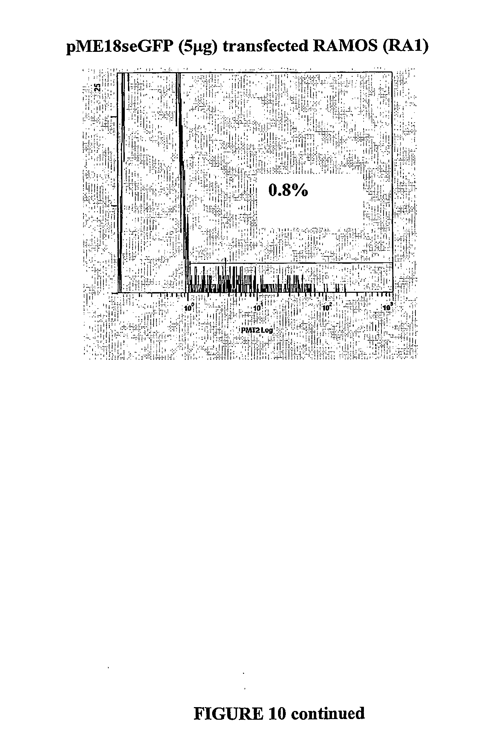

Figure 10 shows the comparison of RAMOS RA-1 cells transfected with pME18sEGFP, pME18sasFP499 or mock transfected. The level of expression of asFP499 in RAMOS RA-1 was assessed 24 hours post transfection and was found to be approximately 10 fold lower than that of EGFP.

### Example 7: Quantitation of the number of IgM molecules on the surface of RAMOS RA-1 cells

It is known in the art that the loss of surface IgM may be used to monitor integration into the rearranged VH allele.

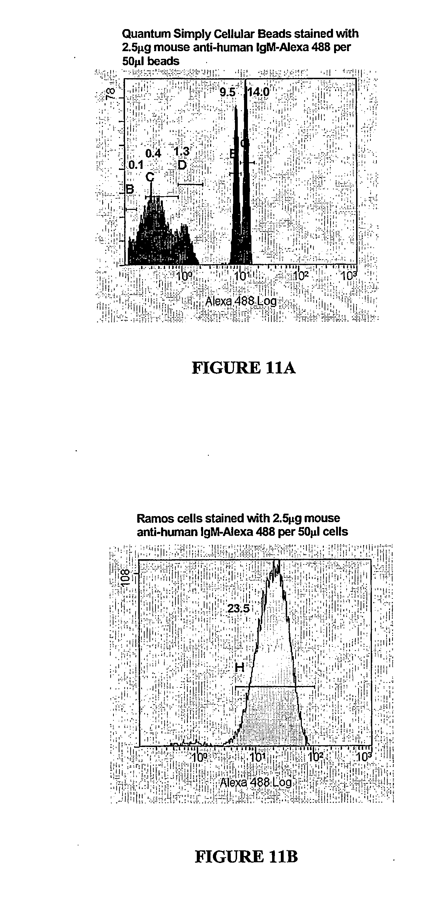

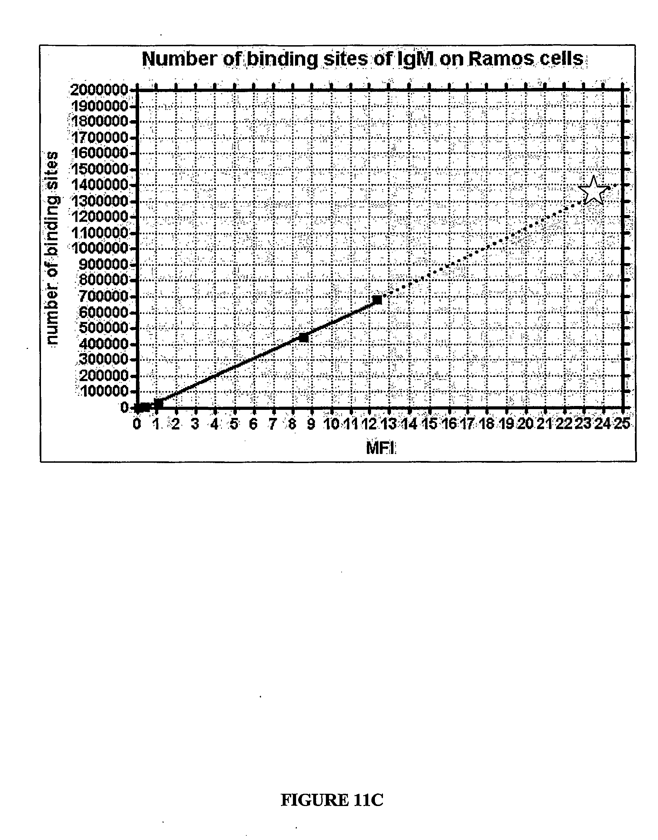

We therefore determined the number of natural IgM molecules on the surface of RAMOS cells. RAMOS cells (50 µl) and Quantum Simply Cellular™ beads (Bangs Laboratories, IN, USA) (50 µl) were separately incubated on ice for 1 hour with a saturating amount (2.5 µg) of a mouse anti-human IgM monoclonal antibody (Southern Biotech, AL, USA) conjugated to Alexa Fluor 488™ (Molecular Probes, OR, USA). Cells and beads were then analysed by flow cytometry using an EPICS Elite (Beckman-Coutler). Fluorescence intensity (at 488 nm) was plotted against the number of cells or beads analysed (Figures 11 a and 11b). Five distinct populations of beads (labeled B, C, D, E, G) which have defined numbers of anti-mouse IgG binding sites (0, 2000, 20 000, 46 000 and 68 000) were observed (Figure 11a). Figure 11b (H) shows a normal distribution of fluorescence intensity, ranging from 2 to 100, as expected for a population of cells. The mean fluorescence for each of these populations was plotted against the corresponding number of predetermined binding sites to generate a linear line graph (Figure 11c) from which we extrapolated the average number of IgM molecules on RAMOS cells. The mean fluorescence intensity was calculated as 23.5 which corresponded to 1.25 X 10⁶ molecules for RAMOS RA-1.

### Example 8 : Monitoring cell surface IgM loss on RAMOS RA-1

RAMOS RA-1 cell surface IgM decreases with time in culture. We therefore established the rate of natural loss of surface IgM in order to use this characteristic as a marker for integration. To evaluate the percentage of the population that were IgM positive, RAMOS RA-1 cells were stained for IgM and analysed by flow cytometry.

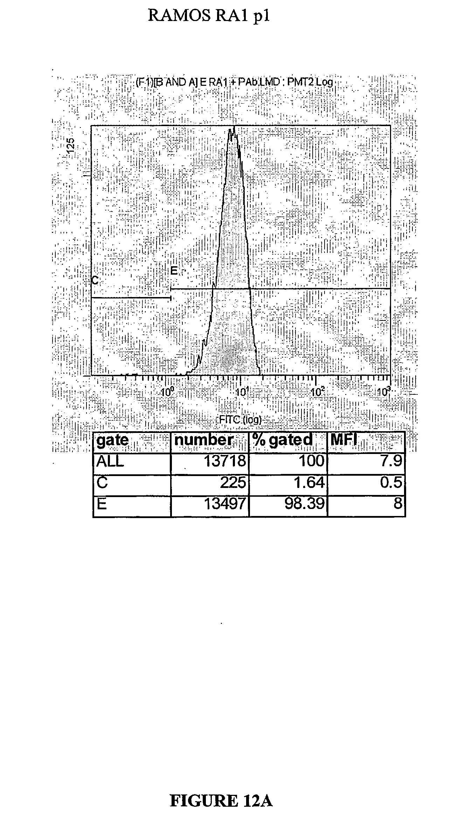

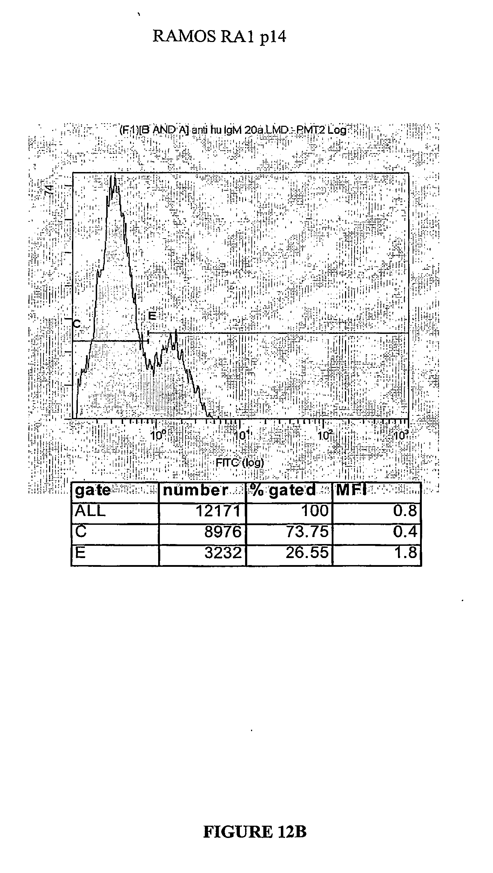

Briefly, cells were washed and resuspended at 1x10⁶ cells / 100 µl. A sheep polyclonal antibody against human IgM (µ chain specific) FITC conjugated (Chemicon, CA, USA) (10 µl) was added to cells which were incubated on ice for 1hour. Cells were then washed with 2 ml of cold wash buffer (PBS + 2 % FBS, 0.01 % sodium azide) and resuspended in 500 µl this buffer with 5 µl propidium iodide (1mg / ml) (Sigma-Aldrich, Australia). Cells were anlaysed by flow cytometry using an EPICS Elite (Beckman Coulter, CA, USA). Cells were gated on the basis of forward and side scatter and negative propidium iodide. In passage one of RAMOS RA-1 98.39 % of the cell population was positive for surface IgM (Figure 12a) by passage 14, 26.55 % of the cell population was positive (Figure 12b). This loss is attributed to either a high mutation rate or a growth advantage of IgM negative mutants. Therefore, RAMOS RA-1 cells were presorted to remove IgM negative cells and the IgM⁺ cells quantitated prior to transfection as the IgM loss is used as a measure of integration.

To further investigate IgM loss on RAMOS, we quantitated the number of cell surface IgM molecules using the methodology described above. We observed that the average number of cell surface IgM molecules significantly decreased over time. In passage 4, RAMOS RA 1 cells possessed an average 9.0 x 10⁵ molecules of IgM on their surface, however by passage 16 the number of IgM molecules had decreased to 4.1 x 10⁵ molecules (Figure 12). Together these data indicate that the decrease in cell surface IgM is dependent on passage number.

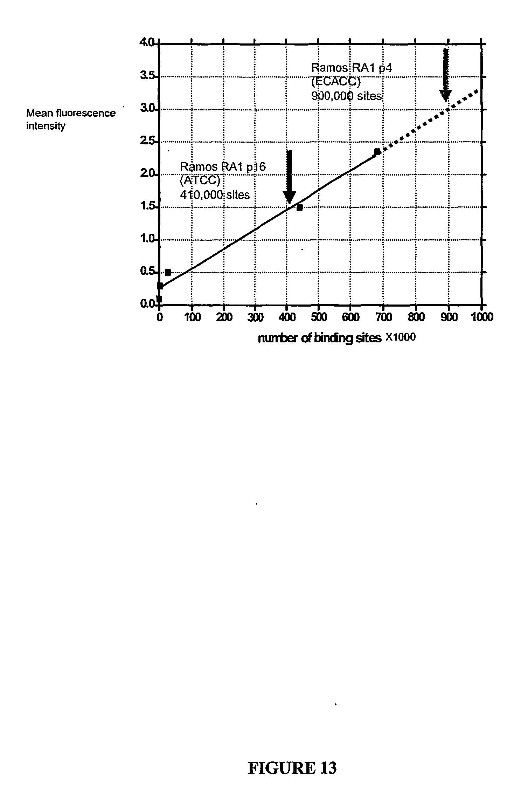

We have also observed and quantitated differences in the rate of IgM loss between RAMOS strains, 'RAMOS RA-1' supplied by American Tissue Culture Collection and 'RAMOS' supplied by European Collection of Cell Culture (ECAC) (Figure 13). Together these data suggest that although IgM loss is a marker for integration reported widely in the literature, we have opted to use an antibiotic marker, such as neomycin as a positive selection marker and thymidine kinase as a negative selection marker for integration in our system.

### Example 9: Surface display of asFP499 using the anchor domain of CD26

For cell surface display of the asFP499 gene product on RAMOS cells we used the transmembrane domain of CD26 (Tanaka,T et al., 1992, J. Immunol. 149 (2), 481-486) as an anchor.
Transmembrane domain of CD26
Accession No: M74777 (gi 180082)
Peptide sequence: MKTPWKVLLGLLGAAALVTIITVPVVLLNK (SEQ ID NO:96) Nucleotide sequence: 10-100

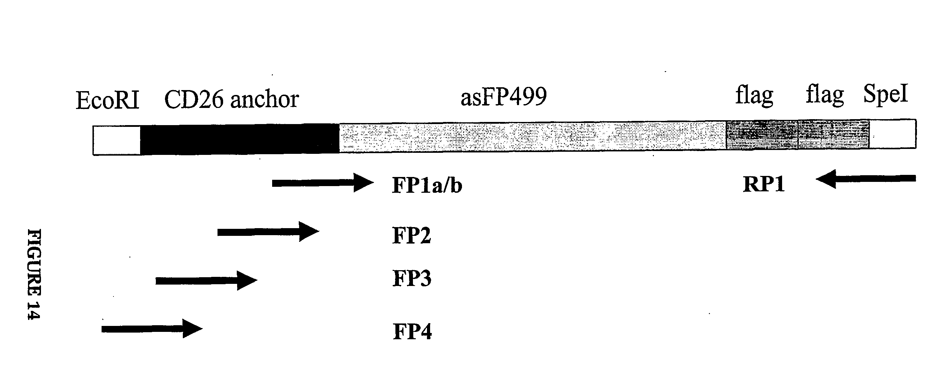

This anchor sequence was added to the 3' end of the asFP499 gene, to provide an N-terminal anchor on the protein (CD26asFP499). This was achieved by sequential overlap extension PCR using primers that partially overlapped the existing DNA end and also added new sequence. A representation of this sequential overlap PCR is shown in Figure 14. This method can be used to add sequence to either the 3' or 5' end of a sequence. Primers are listed in Table 6.

**Table 6: Primers used for addition of CD26 anchor sequence to asFP499**

| Primer | Sequence |
|---|---|
| 9712 | |
| 9713 | |
| 9726 | |
| 9727 | |
| 9728 | |
| 9963 | |

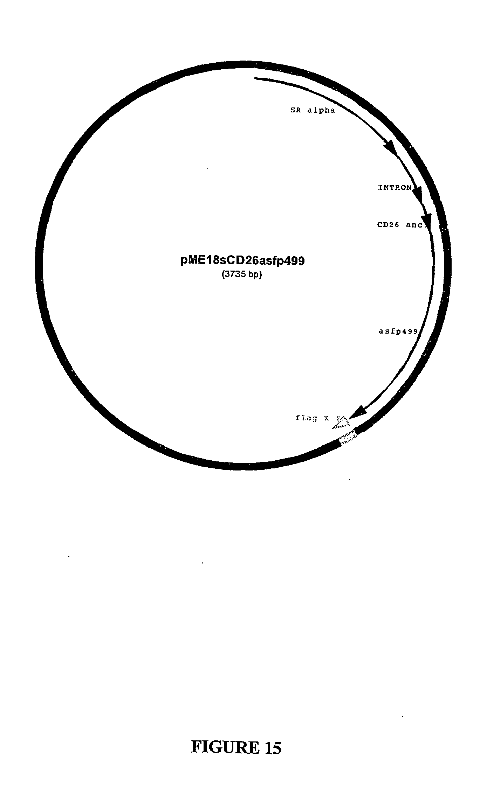

The final PCR product was cloned into pME18s as described previously except that the restriction sites SpeI and EcoRI were used. The cloned sequence was confirmed by DNA sequence analysis. The resulting vector was designated pME18sCD26asFP and is shown in Figure 15. The sequence of pME18sCD26asFP is set out in SEQ ID NO:104.

### Example 10: Transfection and analysis of CD26asFP499

To demonstrate cell surface display of CD26asFP499, the plasmid pME18sasFP499CD26 was transfected into RAMOS RA-1 and control HEK 293T cells.

RAMOS RA-1 cells were transfected as previously described (Example 6). Transfections in HEK 293T cells, which were 60 %-90 % confluent, were carried out using FuGENE6 (Roche, Germany) according to the manufacturer's instructions. 5 µg of DNA was used in each transfection and pME18sasFP499 was used as a positive control.

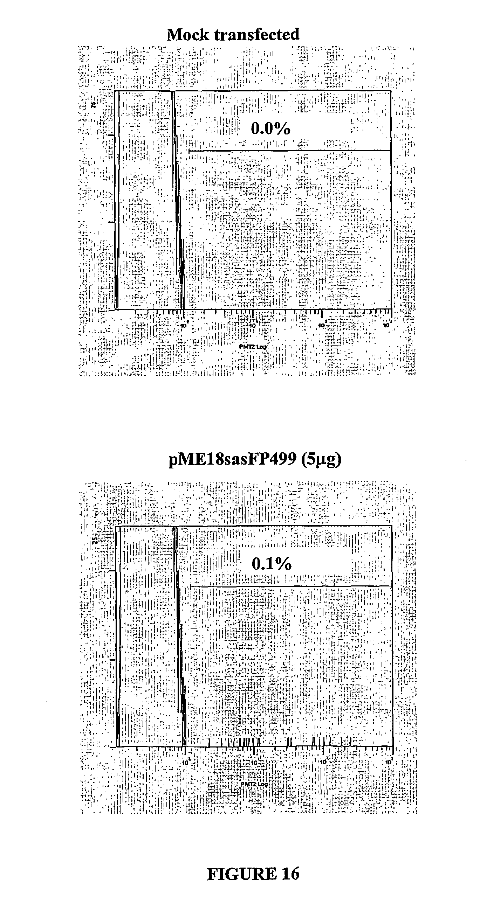

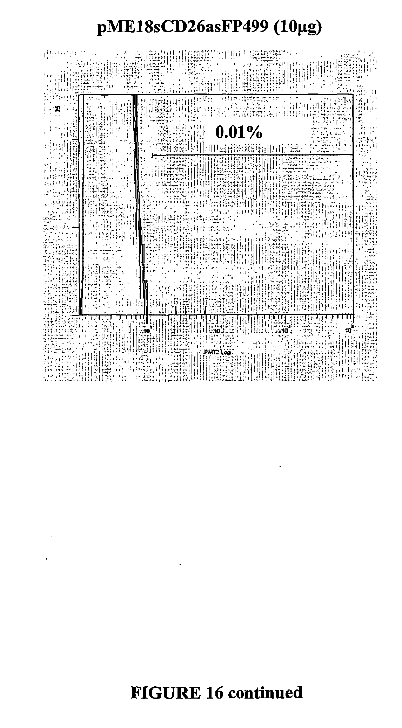

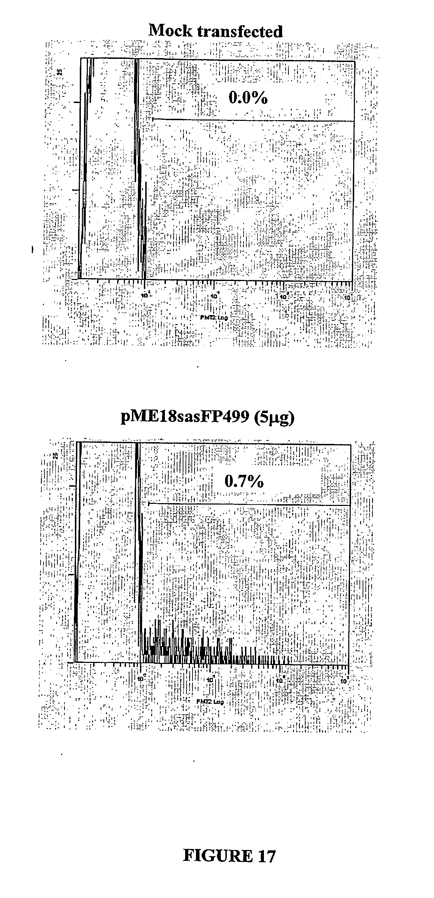

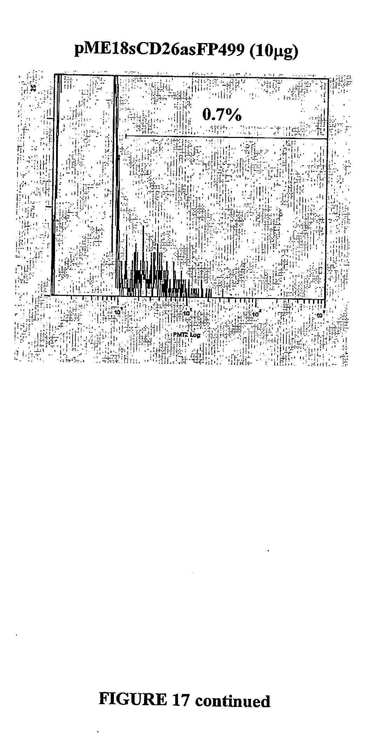

Efficiency of transfection was assessed by flow cytometry as previously described. Figure 16 shows the flow cytometry data for transfection of RAMOS RA-1 cells with pME18sCD26asFP499. It can be seen that the efficiency of transfection with this vector in this cell line is very low (0.01 % corresponds to 4 positive cells in this case). The data for transfection of HEK 293T cells is shown in Figure 17. In this cell line, the transfection efficiency of pME18sCD26asFP499 is equal to that of pME18sasFP499 and the expression of both vectors is improved in this cell line. The mean fluorescence intensity of pME18sCD26asFP499 was lower than that of pME18sasFP499.

HEK 293 T cells transfected with pME18sCD26asFP499 were analysed by confocal microscopy using a Nikon C1 (Coherent Life Sciences, Australia). The majority of cells showed a diffuse fluorescence at their periphery indicating expression of asFP499CD26 at the cell membrane, whereas bright fluorescence was observed uniformly throughout control cells transfected with pME18sasFP499 (data not shown).

### Example 11: Cloning of the coding region of AICDA (AID) gene into pME18s

Activation Induced Cytidine Deaminase (AID), the protein product of the differentiation specific AICDA gene has been shown to be a B-cell-specific factor required and essential for the processes of Class Switch Recombination (CSR) and Somatic Hypermutation (SHM) in B-cells. Its ectopic expression in a number of mammalian cell systems including non B-cell systems has shown the ability of AID protein to induce and/or enhance hypermutation (Martin *et. al* 2002, Okazaki *et. al* 2002, Yoshikawa *et. al* 2002).

### Extraction of total mRNA from RAMOS RA-1

Cells were harvested and centrifuged at 1500 rpm and resuspended in PBS. Total cellular mRNA was extracted using a GenoPrep^{™} mRNA isolation kit (Scientifix, Australia) according to the manufacturer's instructions. Briefly, after removing the supernatant, 700 µl of Lysis and Binding solution was added to cells (1x10⁶). This mixture was combined with 50 µl (250 µg) of GenoPrepTM mRNA magnetic beads and incubated at room temperature for 5 minutes. Beads were magnetically collected and washed with 500 µl of washing solution I. Beads were then washed twice with 500 µl of washing solution II. mRNA-bead complexes were resuspended in 20 µl sterile water and then incubated at 65 °C for 2 minutes. Beads were magnetically collected and the mRNA-containing supernatant was transferred to a new tube.

The human AICDA (AID) gene was amplified from RAMOS RA-1 total RNA using the Superscript One-step RT-PCR with platinum Taq kit (Invitrogen, CA. USA). The reaction included 1 X reaction mix, forward primer 9645 (5' ATG GACAGCCTCTTGATGAACCGGAGGA 3') (SEQ ID NO:105) 10pM, reverse primer 9646 (5' CAAAGTCCCAAAGTACGAAATGCGT 3') (SEQ ID NO:106) 10pM, template RNA (∼150 ng), RT / Taq mix (1.0 µl) and sterile water in a final volume of 50 µl. Cycling conditions were as follows; one cycle of 55°C for 30 minutes, 94°C for 2 minutes, 35 cycles of 94°C for 30 seconds, 55°C for 30 seconds, 68°C for one minute, and one cycle of 72°C for 7 minutes.

The RT-PCR product (596 bp) was amplified by PCR using Platinum Pfx polymerase (Invitrogen CA., USA) as previously described. This product was then cloned into pPCRScript using the PCR-Script Amp cloning kit (Stratagene, Texas, USA) at the *Srf I* site. The coding region of AID was then subcloned into the pME18s using *Xho I* and *Xba I* restriction sites with primers 9792 (5' CCCTCGAGATGGACAGCCTCTTGATGAACCGGA 3') (SEQ ID NO:108) and 9793 (5' GCTCTAGACAAAGTCCCAAAGTACGAAATGCGT 3') (SEQ ID NO:109). The PCR reaction and cycling conditions were as described above. These constructs were verified by sequencing as previously described. The DNA sequence of the coding region of the AICDA gene is set out in SEQ ID NO:107.

### Example 12: Affinity maturation of an antibody fragment

The gene targeting vector KW2 is modified such that following integration and target nucleic acid expression, a chimeric protein consisting of an antibody fragment fused to an anchor is produced. This is achieved by using standard molecular biology techniques to clone the CD26 anchor sequence into KW2 and then inserting the sequence for the antibody fragment downstream of the CD26 anchor sequence. The resulting vector is called KW3.

RAMOS RA-1 cells are transfected with the gene targeting vector KW3, using the optimised protocol previously described. The cells are allowed to recover for 48 to 72 hours before G418 at 5 mg / ml and gancyclovir or FIAU are added to the media for 7 to 9 days. This results in the selection of stable transfectants and also allows time for mutation and surface display to occur. The live cell population, which consists of the stable transfectants, is sorted using flow cytometry and allowed to react with the fluorescently labelled binding partner of the displayed antibody fragment. The cells with the highest fluorescence intensity (ie highest binding) are then single cell sorted into 96 well U bottomed plates containing 100 µl of 50 % conditioned media and 50 % fresh growth media. Single cell sorting is achieved using the Autoclone function of the EPICS Elite (Beckman Coulter, CA, USA).

It is also possible to select the cells with the highest affinity gene products on the cell surface, by capturing cells with immoblised binding partner, and selecting for the highest affinity gene product by competitive elution.

If necessary, the cells can be cycled through the *in vivo* strategy (Figure 2). The single cells can be expanded to between 1x10⁵ and 1x10⁶ to allow further mutation to occur and then reacted with the labelled binding partner and single cell sorted again. This cycle of single cell sorting/expansion and mutation/re-selection can be carried out until cells displaying the molecule with the desired characteristic, in this case increased binding affinity, have been isolated. The DNA from these cells can be extracted and the mutated gene can be amplified by PCR. Alternatively, the RNA can be extracted and the gene amplified by RT-PCR. The amplified gene can then be inserted into an appropriate expression vector for high-level production of the affinity matured antibody fragment.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

## Claims

1. A method for producing and selecting a gene product with desired characteristics, the method comprising
(i) introducing into a hypermutating cell a target nucleic acid molecule encoding a gene product such that a single copy of the target nucleic acid molecule is integrated into an immunoglobulin locus of the genome of the hypermutating cell, wherein the target nucleic acid molecule is introduced into the cell by way of an integration vector comprising a sequence homologous to a region upstream of a rearranged V allele and a sequence homologous to a region downstream of a rearranged V allele;
(ii) culturing the hypermutating cell such that the target nucleic acid molecule undergoes hypermutation during DNA and/or RNA synthesis, giving rise to a population of cells expressing mutant gene products; and
(iii) selecting a mutant gene product with desired characteristics.

2. A method as claimed in claim 1 wherein the immunoglobulin locus contains a rearranged V gene.

3. A method as claimed in claim 1 wherein the immunoglobulin locus contains a rearranged VH gene.

4. A method as claimed in claim 1 wherein the immunoglobulin locus contains the rearranged VH₄₋₃₄ allele.

5. A method as claimed in any one of claims 1 to 4 wherein following integration of the target nucleic acid molecule into the immunoglobulin locus, the target nucleic acid molecule is operatively linked to a promoter.

6. A method as claimed in claim 5 wherein following integration the initiation codon of the target nucleic acid molecule is located within 2 kb of the 3' end of the promoter.

7. A method as claimed in claim 5 wherein following integration the target nucleic acid molecule is located downstream of the promoter and upstream of an intronic enhancer with or without matrix attachment regions and/or 3' enhancer.

8. A method as claimed in any one of claims 1 to 7 wherein the target nucleic acid molecule is introduced into the cell by way of an integration vector comprising a sequence homologous to a region of at least 500 bp upstream of a rearranged V allele and a sequence homologous to a region of at least 500 bp downstream of a rearranged V gene.

9. A method as claimed in any one of claims 1 to 8 wherein the method comprises a further step to increase the rate of mutation of the target nucleic acid molecule.

10. A method as claimed in claim 9 wherein the further step is to increase the levels of expression of activation-induced cytidine deaminase (AID) within the hypermutating cell.

11. A method as claimed in any one of claim 1 to 10 wherein the hypermutating cell is selected from the group consisting of RAMOS, BL2, BL41, BL70 and Nalm.

12. A vector for targeted integration into an immunoglobulin locus of a hypermutating cell, the vector comprising a sequence homologous to a region upstream of a rearranged VH gene of the hypermutating cell, a sequence homologous to a region downstream of a rearranged VH gene of the hypermutating cell and a site for integration of a target nucleic acid molecule, wherein the region upstream of the rearranged VH gene of the hypermutating cell comprises nucleotides 191 to 5190 of SEQ ID NO:1 and the region downstream of the rearranged VH gene of the hypermutating cell is a region within nucleotides 5709 to 8699 of SEQ ID NO:1.

13. A vector for targeted integration comprising a sequence as set out in nucleotides 1 to 12990 of SEQ ID NO:110.

## Patentansprüche

1. Verfahren zur Herstellung und Selektion eines Genprodukts mit gewünschten Eigenschaften, wobei das Verfahren Folgendes umfasst:
(i) das Einführen eines Zielnucleinsäuremoleküls, das für ein Genprodukt kodiert, in eine hypermutierende Zelle, sodass eine einzelne Kopie des Zielnucleinsäuremoleküls in einen Immunglobulin-Locus des Genoms der hypermutierenden Zelle integriert wird, wobei das Zielnucleinsäuremolekül mithilfe eines Integrationsvektors, der eine zu einer Region stromauf von einem umgeordneten V-Allel homologe Sequenz und eine zu einer Region stromab von einem umgeordneten V-Allel homologe Sequenz umfasst, in die Zelle eingeführt wird;
(ii) das Kultivieren der hypermutierenden Zelle, sodass das Zielnucleinsäuremolekül eine Hypermutation während einer DNA- und/oder RNA-Synthese durchläuft, was zu einer Population von Zellen führt, die mutierte Genprodukte exprimieren; und
(iii) das Selektieren eines mutierten Genprodukts mit gewünschten Eigenschaften.

2. Verfahren nach Anspruch 1, worin der Immunglobulin-Locus ein umgeordnetes V-Gen enthält.

3. Verfahren nach Anspruch 1, worin der Immunglobulin-Locus ein umgeordnetes VH-Gen enthält.

4. Verfahren nach Anspruch 1, worin der Immunglobulin-Locus das umgeordnete VH₄₋₃₄-Allel enthält.

5. Verfahren nach einem der Ansprüche 1 bis 4, worin nach der Integration des Zielnucleinsäuremoleküls in den Immunglobulin-Locus das Zielnucleinsäuremolekül operativ an einen Promoter gebunden wird.

6. Verfahren nach Anspruch 5, worin sich das Zielnucleinsäuremolekül nach der Integration des Initiationscodons innerhalb von 2 kb vom 3'-Ende des Promotors befindet.

7. Verfahren nach Anspruch 5, worin sich das Zielnucleinsäuremolekül nach der Integration stromab vom Promotor und stromauf von einem intronischen Enhancer mit oder ohne dem Kernskelett angeheftete(n) Regionen und/oder 3'-Enhancer befindet.

8. Verfahren nach einem der Ansprüche 1 bis 7, worin das Zielnucleinsäuremolekül mithilfe eines Integrationsvektors, der eine zu einer Region aus zumindest 500 bp stromauf von einem umgeordneten V-Allel homologe Sequenz und eine zu einer Region aus zumindest 500 bp stromab von einem umgeordneten V-Allel homologe Sequenz umfasst, in die Zelle eingeführt wird.

9. Verfahren nach einem der Ansprüche 1 bis 8, worin das Verfahren einen weiteren Schritt zur Steigerung der Mutationsrate des Zielnucleinsäuremoleküls umfasst.

10. Verfahren nach Anspruch 9, worin der weitere Schritt dazu dient, das Expressionsniveau von aktivierungsinduzierter Cytidindesaminase (AID) innerhalb der hypermutierenden Zelle zu erhöhen.

11. Verfahren nach einem der Ansprüche 1 bis 10, worin die hypermutierende Zelle aus der aus RAMOS, BL2, BL41, BL70 und Nalm bestehenden Gruppe ausgewählt ist.

12. Vektor zur zielgerichteten Integration in einen Immunglobulin-Locus einer hypermutierenden Zelle, wobei der Vektor eine zu einer Region stromauf von einem umgeordneten VH-Gen der hypermutierenden Zelle homologe Sequenz, eine zu einer Region stromab von einem umgeordneten VH-Gen einer hypermutierenden Zelle homologe Sequenz und eine Stelle zur Integration eines Zielnucleinsäuremoleküls umfasst, wobei die Region stromauf vom umgeordneten VH-Gen der hypermutierenden Zelle die Nucleotide 191 bis 5190 der Seq.-ID Nr. 1 umfasst und die Region stromab vom umgeordneten VH-Gen der hypermutierenden Zelle eine Region innerhalb der Nucleotide 5709 bis 8699 der Seq.-ID Nr. 1 ist.

13. Vektor zur zielgerichteten Integration, der eine Sequenz umfasst, wie sie in den Nucleotiden 1 bis 12990 der Seq.-ID Nr. 110 dargelegt ist.

## Revendications

1. Méthode de production et de sélection d'un produit génique ayant des caractéristiques recherchées, la méthode comprenant
(i) introduire dans une cellule hypermutante une molécule d'acide nucléique cible codant pour un produit génique de sorte qu'une seule copie de la molécule d'acide nucléique cible est intégrée dans un lieu d'immunoglobuline du génome de la cellule hypermutante, où la molécule d'acide nucléique cible est introduite dans la cellule par un vecteur d'intégration comprenant une séquence homologue à une région en amont d'un allèle V réagencé et une séquence homologue à une région en aval d'un allèle V réagencé;
(ii) cultiver la cellule hypermutante de façon que la molécule d'acide nucléique cible subisse une hypermutation durant une synthèse d'ADN et/ou d'ARN, donnant lieu à une population de cellules exprimant des produits géniques mutants; et
(iii)sélectionner un produit génique mutant ayant les caractéristiques recherchés.

2. Méthode selon la revendication 1, dans laquelle le lieu d'immunoglobuline contient un gène V réagencé.

3. Méthode selon la revendication 1, dans laquelle le lieu d'immunoglobuline contient un gène VH réagencé.

4. Méthode selon la revendication 1, dans laquelle le lieu d'immunoglobuline contient l'allèle VH₄₋₃₄ réagencé.

5. Méthode selon l'une quelconque des revendications 1 à 4, dans laquelle à la suite de l'intégration de la molécule d'acide nucléique cible dans le lieu d'immunoglobuline, la molécule d'acide nucléique cible est fonctionnellement liée à un promoteur.

6. Méthode selon la revendication 5, dans laquelle à la suite de l'intégration, le codon d'initiation de la molécule d'acide nucléique cible se situe dans 2 kb de l'extrémité 3' du promoteur.

7. Méthode selon la revendication 5, dans laquelle à la suite de l'intégration, la molécule d'acide nucléique cible est située en aval du promoteur et en amont d'un activateur intronique avec ou sans régions d'attachement de matrice et/ou activateur 3'.

8. Méthode selon l'une quelconque des revendications 1 à 7, dans laquelle la molécule d'acide nucléique cible est introduite dans la cellule par l'intermédiaire d'un vecteur d'intégration comprenant une séquence homologue à une région d'au moins 500 bp en amont d'un allèle V réagencé et une séquence homologue à une région d'au moins 500 bp en aval d'un gène V réagencé.

9. Méthode selon l'une quelconque des revendications 1 à 8, dans laquelle la méthode comprend une étape supplémentaire pour augmenter le taux de mutation de la molécule d'acide nucléique cible.

10. Méthode selon la revendication 9, dans laquelle l'étape supplémentaire est destinée à augmenter les niveaux d'expression de cytidine désamynase induite par l'activation (AID) dans la cellule hypermutante.

11. Méthode selon l'une quelconque des revendications 1 à 10, dans laquelle la cellule hypermutante est sélectionnée dans le groupe consistant en RAMOS, BL2, BL41, BL70 et Nalm.

12. Vecteur pour l'intégration ciblée dans un lieu d'immunoglobuline d'une cellule hypermutante, le vecteur comprenant une séquence homologue à une région en amont d'un gène VH réagencé de la cellule hypermutante, une séquence homologue à une région en aval d'un gène VH réagencé de la cellule hypermutante et un site pour l'intégration d'une molécule d'acide nucléique cible, où la région en amont du gène VH réagencé de la cellule hypermutante comprend les nucléotides 191 à 5190 de la SEQ ID NO:1, et la région en aval du gène VH réagencé de la cellule hypermutante est une région dans les nucléotides 5709 à 8699 de la SEQ ID NO:1.

13. Vecteur pour l'intégration ciblée comprenant une séquence telle qu'exposée dans les nucléotides 1 à 12990 de la SEQ ID NO:110.
